(19) Europäisches Patentamt
European Patent Office
Office européen des brevets

(11) EP 3 981 434 A1

(12) EUROPEAN PATENT APPLICATION
published in accordance with Art. 153(4) EPC

(43) Date of publication:
**13.04.2022 Bulletin 2022/15**

(21) Application number: **20819019.9**

(22) Date of filing: **08.06.2020**

(51) International Patent Classification (IPC):
***A61K 47/68*** (2017.01) ***A61K 39/00*** (2006.01)
***C07K 16/18*** (2006.01) ***A61P 35/00*** (2006.01)

(52) Cooperative Patent Classification (CPC):
**A61K 39/00; A61K 47/68; A61P 35/00; C07K 16/18**

(86) International application number:
**PCT/CN2020/094856**

(87) International publication number:
**WO 2020/244657 (10.12.2020 Gazette 2020/50)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.06.2019 CN 201910498993**
**24.03.2020 CN 202010215322**

(71) Applicants:
- **Shanghai Hansoh Biomedical Co., Ltd.**
  **Shanghai 201203 (CN)**
- **Jiangsu Hansoh Pharmaceutical Group Co., Ltd.**
  **Lianyungang, Jiangsu 222047 (CN)**

(72) Inventors:
- **HUA, Haiqing**
  **Lianyungang, Jiangsu 222047 (CN)**
- **LIU, Suxia**
  **Lianyungang, Jiangsu 222047 (CN)**
- **BAO, Rudi**
  **Lianyungang, Jiangsu 222047 (CN)**

(74) Representative: **EP&C**
**P.O. Box 3241**
**2280 GE Rijswijk (NL)**

# (54) ANTI-B7-H4 ANTIBODY-DRUG CONJUGATE AND MEDICINAL USE THEREOF

(57) Provided are an anti-B7-H4 antibody-drug conjugate and medicinal use thereof. Specifically, provided is an anti-B7-H4 antibody or antigen-binding fragment thereof, a humanized antibody comprising the CDR region of the anti-B7-H4 antibody, and antibody-drug conjugate thereof or pharmaceutically acceptable salt or solvent compound thereof, and the aforesaid antibody-drug conjugate thereof or pharmaceutical composition of a pharmaceutically acceptable salt or solvent compound thereof, and use thereof as an anti-cancer drug, particularly the use in the preparation of a drug for treating diseases or disorders having high expression of B7-H4.

EP 3 981 434 A1

## Description

### FIELD OF THE INVENTION

The present invention belongs to the field of biomedicine. Specifically, the present invention relates to an anti-B7-H4 antibody-drug conjugate and medicinal use thereof.

### BACKGROUND OF THE INVENTION

Tumor immunotherapy is a long-term sustainable hotspot of research and development in the field of tumor therapy, and T cell tumor immunotherapy is at a core position. Tumor evasion is a huge obstacle to tumor immunotherapy. Most tumors express antigens that can be recognized by the host immune system to varying degrees. However, in many cases, the inefficient activation of effector T cells triggers an insufficient immune response, and thus tumor cells promote tumor growth by their inhibitory effects on immune system. Tumor immunotherapy is to make full use of and mobilize the killer T cells and/or other immune cells in patients with tumor to kill the tumor.

Research on the CD28 receptor and ligands thereof has led to the characterization of molecules referred to as B7 superfamily. The members of the B7 family are a class of immunoglobulins with immunoglobulin-like V domains (IgV) and immunoglobulin-like C domains (IgC). Its members include co-stimulatory factors B7.1 (CD80) and B7.2 (CD86), inducible co-stimulatory factor ligand (ICOS-L/B7-H2), programmed death-1 ligand (PD-L1/B7-H1), programmed death-2 ligand (PD- L2/B7-DC), B7-H3 and B7-H4, and the like.

Human B7-H4 is a type I transmembrane protein consisting of 282 amino acids. Its coding gene is located in the p11.1 region of chromosome 1 (Choi IH et al., J Immunol. 2003 Nov 1; 171(9): 4650-4). B7-H4 has a negative regulatory effect on the immune response of T cells. B7-H4 plays a broad inhibitory role in the differentiation and development, cell cycle progression and cytokine production of CD4+ and CD8+ T cells (Sica GL et al., Immunity. 2003 Jun; 18(6): 849-61). No immune cell disorders or autoimmune phenomena were found in B7-H4 knockout mice (Zhu G et al., Blood. 2009 Feb 19; 113(8): 1759-67; Suh WK et al., Blood. Mol Cell Biol. 2006 Sep; 26(17): 6403-11). At present, the receptor of B7-H4 and signaling transduction pathway thereof are still unclear.

Recent studies have found that B7-H4 protein is abundantly expressed in a variety of tumor tissues, allowing tumor cell evasion from the attack by immune system of the body. Taking the B7-H4 molecule as a target of tumor therapy provides a new method for tumor immunotherapy.

It is currently known that human B7-H4 is expressed on cancer cells such as breast cancer, ovarian cancer, lung cancer, cervical cancer, kidney cancer, bladder cancer, and liver cancer. The expression of B7-H4 mRNA is found in the spleen, lung, thymus, liver, skeletal muscle, kidney, pancreas, testis and ovary. At protein level, low level of B7-H4 expression is found in tissues such as the breast (duct and lobule), fallopian tube epithelium, and endometrial gland. Related studies have also shown that B7-H4 is overexpressed in tumor-associated macrophages (TAM) (Kryczek, I. et al., J. Exp. Med. 2006, 203(4): 871-881), and macrophages constitute an important component of the tumor microenvironment and can represent as much as 50% of the tumor mass.

One strategy for cancer treatment is to use antibodies as carriers to deliver cytotoxic molecules into cancer cells, and then kill the cancer cells by the dissociated small molecules. Drugs used in this strategy are called antibody-drug conjugates. Adcetris and Kadcyla are currently marketed antibody-drug conjugates. At present, many multinational pharmaceutical companies are developing monoclonal antibodies against B7-H4 and/or their drug conjugates to improve the patient's own immune system response to tumors and to achieve the goal of direct killing of tumor cells. Related patents are for example WO2013025779, US20140322129 and the like. The anti-B7-H4 monoclonal antibodies of Medimmune, FivePrime and other companies are currently still under pre-clinical development; the anti-B7-H4 antibody-drug conjugates of Genentech have also been in the pre-clinical development stage.

### SUMMARY OF THE INVENTION

The objective of the present invention is to provide an anti-B7-H4 antibody-drug conjugate, which has high affinity, high selectivity, high endocytic efficiency, high anticancer activity, high stability, high safety and low toxic and side effects, achieved through the following technical solutions:
An antibody-drug conjugate of general formula (A) or a pharmaceutically acceptable salt or solvate thereof,

$$\mathbf{Ab\text{-}(L_2\text{-}L_1\text{-}D)_y} \qquad \mathbf{(A)}$$

wherein:

D is a cytotoxic drug;

$L_1$ and $L_2$ are linker units;
y is a number of 1 to 20;

Ab is a B7-H4 antibody or antigen-binding fragment thereof, which comprises antibody light chain variable region and antibody heavy chain variable region;

the antibody heavy chain variable region comprises at least one HCDR as shown in the sequence selected from the group consisting of:

SEQ ID NO:3, SEQ ID NO:4, SEQ ID NO:5,
SEQ ID NO:9, SEQ ID NO: 10, SEQ ID NO:11;

the antibody light chain variable region comprises at least one LCDR as shown in the sequence selected from the group consisting of:

SEQ ID NO: 6, SEQ ID NO: 7, SEQ ID NO: 8,
SEQ ID NO: 12, SEQ ID NO: 13, SEQ ID NO: 14.

In one embodiment of the present invention, the antibody heavy chain variable region can also comprise at least one HCDR as shown in the sequence selected from the group consisting of:

SEQ ID NO:3, SEQ ID NO:4, SEQ ID NO:5,
SEQ ID NO:9, SEQ ID NO:10, SEQ ID NO:11,
SEQ ID NO:23, SEQ ID NO:24, SEQ ID NO:25,
SEQ ID NO:29, SEQ ID NO:30, SEQ ID NO:31, or,
the antibody heavy chain variable region can also comprise at least one HCDR as shown in the sequence selected from the group consisting of:
SEQ ID NO:43, SEQ ID NO:44, SEQ ID NO:45;
the antibody light chain variable region comprises at least one LCDR as shown in the sequence selected from the group consisting of:

SEQ ID NO: 6, SEQ ID NO: 7, SEQ ID NO: 8,
SEQ ID NO: 12, SEQ ID NO: 13, SEQ ID NO: 14,
SEQ ID NO:26, SEQ ID NO:27, SEQ ID NO:28,
SEQ ID NO:32, SEQ ID NO:33, SEQ ID NO:34.

In one embodiment of the present invention, the antibody-drug conjugate of general formula (A) or a pharmaceutically acceptable salt or solvate thereof as described above, wherein, the anti-B7-H4 antibody or antigen-binding fragment thereof comprises antibody heavy chain variable region, wherein the antibody heavy chain variable region comprises:

HCDR1 as shown in SEQ ID NO:3, HCDR2 as shown in SEQ ID NO:4 and HCDR3 as shown in SEQ ID NO:5;
or,
HCDR1 as shown in SEQ ID NO:9, HCDR2 as shown in SEQ ID NO:10 and HCDR3 as shown in SEQ ID NO: 11.

In one embodiment of the present invention, the antibody-drug conjugate of general formula (A) or a pharmaceutically acceptable salt or solvate thereof as described above, wherein, the anti-B7-H4 antibody or antigen-binding fragment thereof comprises antibody heavy chain variable region, wherein the antibody heavy chain variable region comprises:

HCDR1 as shown in SEQ ID NO:23, HCDR2 as shown in SEQ ID NO:24 and HCDR3 as shown in SEQ ID NO:25;
or,
HCDR1 as shown in SEQ ID NO:29, HCDR2 as shown in SEQ ID NO:30 and HCDR3 as shown in SEQ ID NO:31.

In one embodiment of the present invention, the antibody-drug conjugate of general formula (A) or a pharmaceutically acceptable salt or solvate thereof as described above, wherein, the anti-B7-H4 antibody or antigen-binding fragment thereof comprises antibody heavy chain variable region, wherein the antibody heavy chain variable region comprises:

HCDR1 as shown in SEQ ID NO:43, HCDR2 as shown in SEQ ID NO:44 and HCDR3 as shown in SEQ ID NO:25; or,
HCDR1 as shown in SEQ ID NO:29, HCDR2 as shown in SEQ ID NO:45 and HCDR3 as shown in SEQ ID NO:31.

In one embodiment of the present invention, the antibody-drug conjugate of general formula (A) or a pharmaceutically acceptable salt or solvate thereof as described above, wherein the antibody heavy chain variable region of the Ab comprises the CDRs of any one selected from the group consisting of the following (1) to (4):

(1) HCDR1 as shown in SEQ ID NO:3, HCDR2 as shown in SEQ ID NO:4 and HCDR3 as shown in SEQ ID NO:5;
(2) HCDR1 as shown in SEQ ID NO:9, HCDR2 as shown in SEQ ID NO:10 and HCDR3 as shown in SEQ ID NO:11;
(3) HCDR1 as shown in SEQ ID NO:23, HCDR2 as shown in SEQ ID NO:24 and HCDR3 as shown in SEQ ID NO:25; or,
(4) HCDR1 as shown in SEQ ID NO:29, HCDR2 as shown in SEQ ID NO:30 and HCDR3 as shown in SEQ ID NO:31.

The antibody heavy chain variable region of the Ab can also comprise CDRs selected from the group consisting of the following (5) to (6):

(5) HCDR1 as shown in SEQ ID NO:43, HCDR2 as shown in SEQ ID NO:44 and HCDR3 as shown in SEQ ID NO:25; or,
(6) HCDR1 as shown in SEQ ID NO:29, HCDR2 as shown in SEQ ID NO:45 and HCDR3 as shown in SEQ ID NO:31.

In one embodiment of the present invention, the antibody-drug conjugate of general formula (A) or a pharmaceutically acceptable salt or solvate thereof as described above, wherein, the anti-B7-H4 antibody or antigen-binding fragment thereof comprises antibody light chain variable region, wherein the antibody light chain variable region comprises:

LCDR1, LCDR2 and LCDR3 as shown in SEQ ID NO:6, SEQ ID NO: 7 and SEQ ID NO:8, respectively;
or LCDR1, LCDR2 and LCDR3 as shown in SEQ ID NO:12, SEQ ID NO:13 and SEQ ID NO: 14, respectively.

In some embodiment of the present invention, the antibody-drug conjugate of general formula (A) or a pharmaceutically acceptable salt or solvate thereof as described above, wherein, the anti-B7-H4 antibody or antigen-binding fragment thereof comprises antibody light chain variable region, wherein the antibody light chain variable region comprises:

LCDR1, LCDR2 and LCDR3 as shown in SEQ ID NO:26, SEQ ID NO:27 and SEQ ID NO:28, respectively;
or LCDR1, LCDR2 and LCDR3 as shown in SEQ ID NO:32, SEQ ID NO:33 and SEQ ID NO:34, respectively.

In one embodiment of the present invention, the antibody-drug conjugate of general formula (A) or a pharmaceutically acceptable salt or solvate thereof as described above, wherein, the antibody heavy chain variable region of the Ab comprises the CDRs of any one selected from the group consisting of the following (1) to (4):

(1) LCDR1 as shown in SEQ ID NO:6, LCDR2 as shown in SEQ ID NO:7 and LCDR3 as shown in SEQ ID NO:8;
(2) LCDR1 as shown in SEQ ID NO:12, LCDR2 as shown in SEQ ID NO:13 and LCDR3 as shown in SEQ ID NO:14;
(3) LCDR1 as shown in SEQ ID NO:26, LCDR2 as shown in SEQ ID NO:27 and LCDR3 as shown in SEQ ID NO:28; or,
(4) LCDR1 as shown in SEQ ID NO:32, LCDR2 as shown in SEQ ID NO:33 and LCDR3 as shown in SEQ ID NO:34.

In one embodiment of the present invention, the antibody-drug conjugate of general formula (A) or a pharmaceutically acceptable salt or solvate thereof as described above, wherein, the heavy chain and light chain variable regions of the anti-B7-H4 antibody or antigen-binding fragment thereof comprises:

(1) HCDR1 as shown in SEQ ID NO:3, HCDR2 as shown in SEQ ID NO:4 and HCDR3 as shown in SEQ ID NO:5, respectively; and
LCDR1 as shown in SEQ ID NO:6, LCDR2 as shown in SEQ ID NO:7 and LCDR3 as shown in SEQ ID NO:8; or,
(2) HCDR1 as shown in SEQ ID NO:9, HCDR2 as shown in SEQ ID NO:10 and HCDR3 as shown in SEQ ID NO: 11, respectively; and
LCDR1 as shown in SEQ ID NO:12, LCDR2 as shown in SEQ ID NO:13 and LCDR3 as shown in SEQ ID NO:14.

In one embodiment of the present invention, the antibody-drug conjugate of general formula (A) or a pharmaceutically acceptable salt or solvate thereof as described above, wherein, the heavy chain and light chain variable regions of the anti-B7-H4 antibody or antigen-binding fragment thereof comprises:

(3) HCDR1 as shown in SEQ ID NO:23, HCDR2 as shown in SEQ ID NO:24 and HCDR3 as shown in SEQ ID NO:25; and
LCDR1 as shown in SEQ ID NO:26, LCDR2 as shown in SEQ ID NO:27 and LCDR3 as shown in SEQ ID NO:28; or,
(4) HCDR1 as shown in SEQ ID NO:29, HCDR2 as shown in SEQ ID NO:30 and HCDR3 as shown in SEQ ID NO:31; and
LCDR1 as shown in SEQ ID NO:32, LCDR2 as shown in SEQ ID NO:33 and LCDR3 as shown in SEQ ID NO:34.

In one embodiment of the present invention, the antibody-drug conjugate of general formula (A) or a pharmaceutically acceptable salt or solvate thereof as described above, wherein, the heavy chain and light chain variable regions of the anti-B7-H4 antibody or antigen-binding fragment thereof comprises:

(5) HCDR1 as shown in SEQ ID NO:43, HCDR2 as shown in SEQ ID NO:44 and HCDR3 as shown in SEQ ID NO:25, respectively; and
LCDR1 as shown in SEQ ID NO:26, LCDR2 as shown in SEQ ID NO:27 and LCDR3 as shown in SEQ ID NO:28; or,
(6) HCDR1 as shown in SEQ ID NO:29, HCDR2 as shown in SEQ ID NO:45 and HCDR3 as shown in SEQ ID NO:31, respectively; and
LCDR1 as shown in SEQ ID NO:32, LCDR2 as shown in SEQ ID NO:33 and LCDR3 as shown in SEQ ID NO:34.

In one embodiment of the present invention, the antibody-drug conjugate of general formula (A) or a pharmaceutically acceptable salt or solvate thereof as described above, wherein, the antibody heavy chain variable region and the antibody light chain variable region of the Ab comprises the CDRs of any one selected from the group consisting of the following (1) to (4):

(1) HCDR1 as shown in SEQ ID NO:3, HCDR2 as shown in SEQ ID NO:4 and HCDR3 as shown in SEQ ID NO:5; and
LCDR1 as shown in SEQ ID NO:6, LCDR2 as shown in SEQ ID NO:7 and LCDR3 as shown in SEQ ID NO:8;
(2) HCDR1 as shown in SEQ ID NO:9, HCDR2 as shown in SEQ ID NO:10 and HCDR3 as shown in SEQ ID NO:11; and
LCDR1 as shown in SEQ ID NO:12, LCDR2 as shown in SEQ ID NO:13 and LCDR3 as shown in SEQ ID NO:14;
(3) HCDR1 as shown in SEQ ID NO:23, HCDR2 as shown in SEQ ID NO:24 and HCDR3 as shown in SEQ ID NO:25; and
LCDR1 as shown in SEQ ID NO:26, LCDR2 as shown in SEQ ID NO:27 and LCDR3 as shown in SEQ ID NO:28; or,
(4) HCDR1 as shown in SEQ ID NO:29, HCDR2 as shown in SEQ ID NO:30 and HCDR3 as shown in SEQ ID NO:31; and
LCDR1 as shown in SEQ ID NO:32, LCDR2 as shown in SEQ ID NO:33 and LCDR3 as shown in SEQ ID NO:34.

The antibody heavy chain variable region and antibody light chain variable region of the Ab can also comprise CDRs selected from the group consisting of the following (5) or (6):

(5) HCDR1 as shown in SEQ ID NO:43, HCDR2 as shown in SEQ ID NO:44 and HCDR3 as shown in SEQ ID NO:25; and
LCDR1 as shown in SEQ ID NO:26, LCDR2 as shown in SEQ ID NO:27 and LCDR3 as shown in SEQ ID NO:28;
(6) HCDR1 as shown in SEQ ID NO:29, HCDR2 as shown in SEQ ID NO:45 and HCDR3 as shown in SEQ ID NO:31; and
LCDR1 as shown in SEQ ID NO:32, LCDR2 as shown in SEQ ID NO:33 and LCDR3 as shown in SEQ ID NO:34.

In a preferred embodiment, the antibody-drug conjugate of general formula (A) or a pharmaceutically acceptable salt or solvate thereof as described above, wherein the Ab is a murine antibody or fragment thereof, a chimeric antibody or fragment thereof, a human antibody or fragment thereof, and a humanized antibody or fragment thereof.
In a preferred embodiment, the antibody-drug conjugate of general formula (A) or a pharmaceutically acceptable salt or solvate thereof as described above, wherein, the Ab further comprises light chain framework region and heavy chain framework region sequences, which are respectively derived from human germline light chain and heavy chain sequences or mutant sequence(s) thereof;
In a preferred embodiment, the antibody-drug conjugate of general formula (A) or a pharmaceutically acceptable salt or solvate thereof as described above, wherein, the Ab further comprises heavy chain constant region(s), wherein the heavy chain constant region(s) comprise those derived from human IgG1 or variant thereof, IgG2 or variant thereof,

IgG3 or variant thereof or IgG4 or variant thereof, preferably those derived from human IgG1, IgG2 or IgG4, more preferably IgG1 heavy chain constant region with enhanced ADCC toxicity after amino acid mutation, most preferably the heavy chain constant region as shown in SEQ ID NO: 54;

In a preferred embodiment, the antibody-drug conjugate of general formula (A) or a pharmaceutically acceptable salt or solvate thereof as described above, wherein, the Ab further comprises light chain constant region derived from human κ chain, λ chain or variant thereof, preferably that derived from human κ chain, more preferably the light chain constant region as shown in SEQ ID NO:55.

In a preferred embodiment, the antibody-drug conjugate of general formula (A) or a pharmaceutically acceptable salt or solvate thereof as described above, wherein, the Ab comprises light chain variable region with the following sequences: SEQ ID NO:16 or SEQ ID NO:18.

In a preferred embodiment, the antibody-drug conjugate of general formula (A) or a pharmaceutically acceptable salt or solvate thereof as described above, wherein, the Ab comprises light chain variable region with the following sequences: SEQ ID NO: 16, SEQ ID NO:18, SEQ ID NO:36, SEQ ID NO:38, or light chain variable regions with at least 70%, 75%, 80%, 85%, 90%, 95% or 99% homology to SEQ ID NO:16, SEQ ID NO:18, SEQ ID NO:36, SEQ ID NO:38.

In a preferred embodiment, the antibody-drug conjugate of general formula (A) or a pharmaceutically acceptable salt or solvate thereof as described above, wherein, the Ab comprises heavy chain variable region with the following sequences: SEQ ID NO:15 or SEQ ID NO: 17.

In a preferred embodiment, the antibody-drug conjugate of general formula (A) or a pharmaceutically acceptable salt or solvate thereof as described above, wherein, the Ab comprises heavy chain variable region with the following sequences: SEQ ID NO:15, SEQ ID NO:17, SEQ ID NO:35, SEQ ID NO:37, or heavy chain variable regions with at least 70%,75%, 80%, 85%, 90%, 95% or 99% homology to SEQ ID NO:15, SEQ ID NO:17, SEQ ID NO:35, SEQ ID NO:37.

In a preferred embodiment, the antibody-drug conjugate of general formula (A) or a pharmaceutically acceptable salt or solvate thereof as described above, wherein, the heavy chain variable region and the light chain variable region of the Ab is any one selected from the group consisting of the following:

(1) the heavy chain variable region as shown in SEQ ID NO: 15 and the light chain variable region as shown in SEQ ID NO: 16;
(2) the heavy chain variable region as shown in SEQ ID NO: 17 and the light chain variable region as shown in SEQ ID NO: 18.

In a preferred embodiment, the antibody-drug conjugate of general formula (A) or a pharmaceutically acceptable salt or solvate thereof as described above, wherein, the heavy chain variable region and the light chain variable region of the Ab is any one selected from the group consisting of the following:

(1) the heavy chain variable region as shown in SEQ ID NO: 15 and the light chain variable region as shown in SEQ ID NO: 16;
(2) the heavy chain variable region as shown in SEQ ID NO: 17 and the light chain variable region as shown in SEQ ID NO: 18;
(3) the heavy chain variable region as shown in SEQ ID NO: 35 and the light chain variable region as shown in SEQ ID NO: 36; or,
(4) the heavy chain variable region as shown in SEQ ID NO: 37 and the light chain variable region as shown in SEQ ID NO: 38.

In a preferred embodiment, the antibody-drug conjugate of general formula (A) or a pharmaceutically acceptable salt or solvate thereof as described above, wherein, the Ab comprises light chain with the following sequences: SEQ ID NO:20 or SEQ ID NO:22.

In a preferred embodiment, the antibody-drug conjugate of general formula (A) or a pharmaceutically acceptable salt or solvate thereof as described above, wherein, the Ab comprises light chain with the following sequences: SEQ ID NO:20, SEQ ID NO:22, SEQ ID NO:40, SEQ ID NO:42, or full-length light chain with at least 80%, 85%, 90%, 95% or 99% homology to SEQ ID NO:20, SEQ ID NO:22, SEQ ID NO:40, SEQ ID NO:42.

In a preferred embodiment, the antibody-drug conjugate of general formula (A) or a pharmaceutically acceptable salt or solvate thereof as described above, wherein, the Ab comprises heavy chain with the following sequences: SEQ ID NO: 19 or SEQ ID NO:21.

In a preferred embodiment, the antibody-drug conjugate of general formula (A) or a pharmaceutically acceptable salt or solvate thereof as described above, wherein, the Ab comprises heavy chain with the following sequences: SEQ ID NO:19, SEQ ID NO:21, SEQ ID NO:39, SEQ ID NO:41, or full-length heavy chain with at least 80%, 85%, 90%, 95% or 99% homology to SEQ ID NO:19, SEQ ID NO:21, SEQ ID NO:39, SEQ ID NO:41.

In a preferred embodiment, the antibody-drug conjugate of general formula (A) or a pharmaceutically acceptable

salt or solvate thereof as described above, wherein, the Ab comprises:

(1) the light chain of SEQ ID NO: 20 and the heavy chain of SEQ ID NO: 19; or,
(2) the light chain of SEQ ID NO: 22 and the heavy chain of SEQ ID NO: 21.

In a preferred embodiment, the antibody-drug conjugate of general formula (A) or a pharmaceutically acceptable salt or solvate thereof as described above, wherein, the Ab comprises:

(1) the light chain of SEQ ID NO: 20 and the heavy chain of SEQ ID NO: 19; or,
(2) the light chain of SEQ ID NO: 22 and the heavy chain of SEQ ID NO: 21; or,
(3) the light chain of SEQ ID NO: 40 and the heavy chain of SEQ ID NO: 39; or,
(4) the light chain of SEQ ID NO: 42 and the heavy chain of SEQ ID NO: 41.

In a preferred embodiment, the antibody-drug conjugate of general formula (A) or a pharmaceutically acceptable salt or solvate thereof as described above, wherein, the antigen-binding fragment of the anti-B7-H4 antibody is selected from the group consisting of Fab, Fab', $F(ab')_2$, single-chain antibody (scFv), dimerized V region (diabody), disulfide bond stabilized V region (dsFv) and antigen-binding fragments of a peptide comprising CDRs.

In a preferred embodiment, the antibody-drug conjugate of general formula (A) or a pharmaceutically acceptable salt or solvate thereof as described above, wherein, the cytotoxic drug is selected from the group consisting of toxin, chemotherapeutic, antibiotic, radioisotope and nucleolytic enzyme; preferably tubulin inhibitor or DNA topoisomerase inhibitor that inhibits cell division; more preferably DM1, DM3, DM4, SN-38, MMAF or MMAE; further preferably the tubulin inhibitor SN-38, MMAE or MMAF. Wherein, the structure of MMAF and SN-38 is as shown in the following formula:

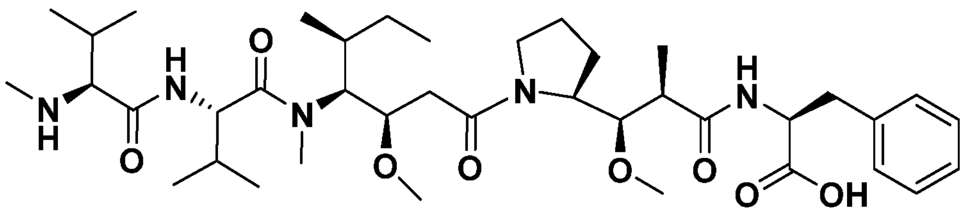

MMAF

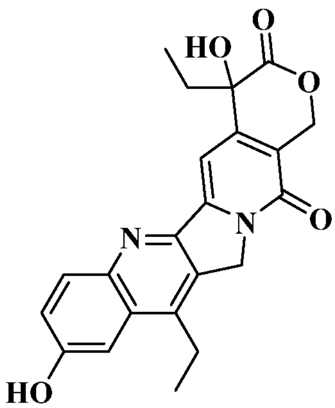

SN-38

In a preferred embodiment, the antibody-drug conjugate of general formula (A) or a pharmaceutically acceptable salt or solvate thereof as described above, wherein, the cytotoxic drug is selected from camptothecin derivatives, preferably Exatecan,

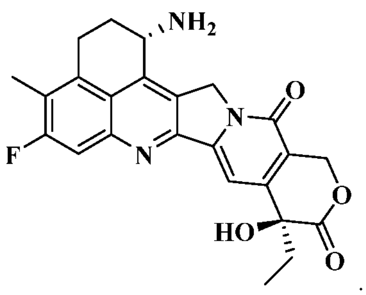

In a preferred embodiment, the antibody-drug conjugate of general formula (A) or a pharmaceutically acceptable salt or solvate thereof as described above, which is a compound of general formula (I) or a pharmaceutically acceptable salt or solvate thereof,

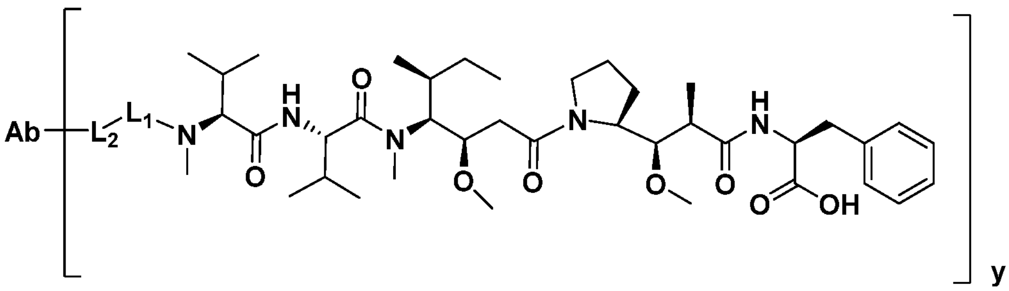

(I)

wherein:

$L_1$ and $L_2$ are linker units;
y is a number selected from 1 to 8, preferably a number selected from 2 to 4;
Ab is an anti-B7-H4 antibody or antigen-binding fragment thereof as defined above.

In a preferred embodiment, the antibody-drug conjugate of general formula (A) or a pharmaceutically acceptable salt or solvate thereof as described above, which is an antibody-drug conjugate of general formula (II) or a pharmaceutically acceptable salt or solvate thereof:

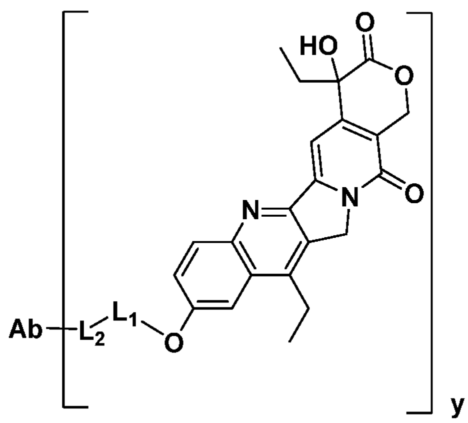

(II)

wherein:

$L_1$ and $L_2$ are linker units;
y is a number selected from 1 to 8, preferably a number selected from 2 to 4;

Ab is an anti-B7-H4 antibody or antigen-binding fragment thereof as defined above.

In a preferred embodiment, the antibody-drug conjugate of general formula (A) or a pharmaceutically acceptable salt or solvate thereof as described above, which is an antibody-drug conjugate of general formula (III) or a pharmaceutically acceptable salt or solvate thereof,

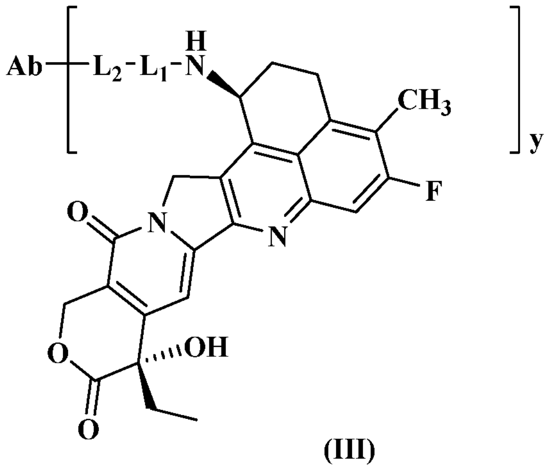

(III)

wherein:

$L_1$ and $L_2$ are linker units;
y is a number selected from 1 to 10, preferably a number selected from 2 to 8, more preferably a number selected from 4 to 8;
or, y is preferably a number selected from 2 to 10, further preferably a number selected from 6 to 10, more preferably a number of 7 to 9, and most preferably an integer of 7, 8, 9;
Ab is an anti-B7-H4 antibody or antigen-binding fragment thereof as defined above.

In a preferred embodiment, the antibody-drug conjugate or a pharmaceutically acceptable salt or solvate thereof as described above, wherein, the $L_1$ is as shown in the following general formula (B):

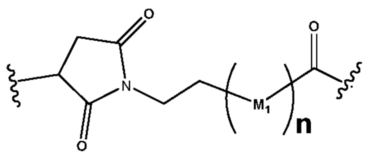

( B )

wherein:

$M_1$ is $-CR_1R_2-$;
$R_1$ and $R_2$ are the same or different, and are independently selected from the group consisting of hydrogen, alkyl, halogen, hydroxyl and amino;
N is an integer of 0 to 5, preferably 1, 2 or 3.

In a preferred embodiment, the antibody-drug conjugate or a pharmaceutically acceptable salt or solvate thereof as described above, wherein, the $L_2$ is as shown in the following general formula (C):

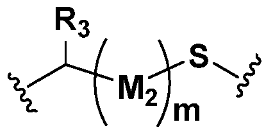

( C )

wherein:

$M_2$ is $-CR_4R_5-$;
$R_3$ is selected from the group consisting of hydrogen atom, halogen, hydroxyl, amino, alkyl, alkoxyl and cycloalkyl;
$R_4$ and $R_5$ are the same or different, and are independently selected from the group consisting of hydrogen, alkyl, halogen, hydroxyl and amino;
m is an integer of 0 to 5, preferably 1, 2 or 3.

In a preferred embodiment, the antibody-drug conjugate or a pharmaceutically acceptable salt or solvate thereof as described above, wherein, the $L_2$ is as shown in the following general formula (D):

$$-K^1-K^2-K^3-K^4- \quad (D)$$

wherein:

$K^1$ is

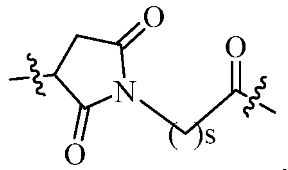

,

s is an integer of 2 to 8, further preferably an integer of 4 to 8, more preferably an integer of 4 to 6;
$K^2$ is $-NR^1(CH_2CH_2O)_pCH_2CH_2C(O)-$, $-NR^1(CH_2CH_2O)_pCH_2C(O)-$, $-S(CH_2)_pC(O)-$ or a single bond, p is an integer of 1 to 20, preferably 1 to 6;
$R_1$ is selected from the group consisting of hydrogen, deuterium, hydroxyl, amino, alkyl, halogen, haloalkyl, deuterated alkyl and hydroxyalkyl;
$K^3$ is a tetrapeptide residue, preferably, the tetrapeptide residue is a peptide residue formed by amino acids selected from the group consisting of two or more of phenylalanine, glycine, valine, lysine, citrulline, serine, glutamate and aspartate; more preferably the tetrapeptide residue GGFG;
$K^4$ is $-NR^2(CR^3R^4)t-$, $R^2$, $R^3$ or $R^4$ are each independently hydrogen, deuterium, hydroxyl, amino, alkyl, halogen, haloalkyl, deuterated alkyl and hydroxyalkyl, and t is 1 or 2,
preferably, as for the $L_2$,
$K^1$ is

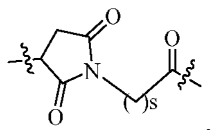

,

s is 5;
$K^2$ is a bond;
$K^3$ is the tetrapeptide residue GGFG;
$K^4$ is $-NR^2(CR^3R^4)t-$, $R^2$, $R^3$ or $R^4$ are each independently hydrogen, deuterium, hydroxyl, amino, $C_{1-6}$ alkyl, halogen, $C_{1-6}$ haloalkyl, $C_{1-6}$ deuterated alkyl and $C_{1-6}$ hydroxyalkyl, and t is 1 or 2.

In a preferred embodiment, the antibody-drug conjugate or a pharmaceutically acceptable salt or solvate thereof

as described above, wherein, the $K^1$ terminus of the linker unit $-L_2-$ is linked to the Ab, and the $K^4$ terminus is linked to $L_1$.

In a preferred embodiment, the antibody-drug conjugate or a pharmaceutically acceptable salt or solvate thereof as described above, wherein, $L_1$ is $-O-(CR^aR^b)_m-CR^5R^6-C(O)-$, $-O-CR^5R^6-(CR^aR^b)_m-$, $-O-CR^5R^6-$, $-NH-(CR^aR^b)_m-CR^5R^6-C(O)-$ or $-S-(CR^aR^b)_m-CR^5R^6-C(O)-$;

$R^a$ and $R^b$ are each independently selected from the group consisting of hydrogen, deuterium, halogen and alkyl;
$R^5$ is haloalkyl or cycloalkyl;
$R^6$ is selected from the group consisting of hydrogen, haloalkyl and cycloalkyl;
or, $R^5$ and $R^6$ and the carbon atom to which they are linked form a cycloalkyl;
m is 0, 1, 2, 3 or 4.

Preferably, the O terminus of $L_1$ is linked to the linker unit $L_2$.

In a preferred embodiment, the antibody-drug conjugate or a pharmaceutically acceptable salt or solvate thereof as described above, wherein, the $L_1$ is as shown in the following general formula (E):

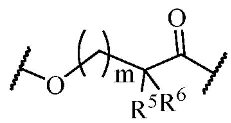

(E),

$R^5$ is haloalkyl or cycloalkyl,
$R^6$ is selected from the group consisting of hydrogen, haloalkyl and cycloalkyl, or, $R^5$ and $R^6$ and the carbon atom to which they are linked form a cycloalkyl;
preferably,
$R^5$ is selected from the group consisting of $C_{1-6}$ haloalkyl and $C_{3-6}$ cycloalkyl,
$R^6$ is selected from the group consisting of hydrogen, $C_{1-6}$ haloalkyl and cycloalkyl,
or, $R^5$ and $R^6$ and the carbon atom to which they are linked form a $C_{3-6}$ cycloalkyl;
m is an integer of 0 to 4;
more preferably, general formula (E) is selected from the group consisting of the following substituents:

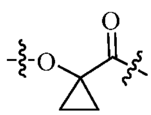 , 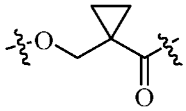 , 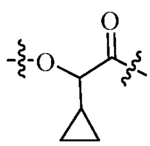 , 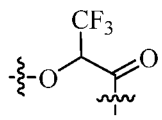 , 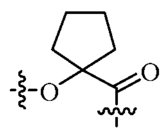 , 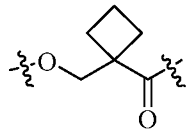

and

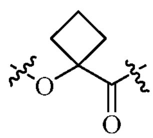 ;

In a more preferred embodiment, the antibody-drug conjugate or a pharmaceutically acceptable salt or solvate thereof as described above, wherein, the $-L_2-L_1-$ is the following structure:

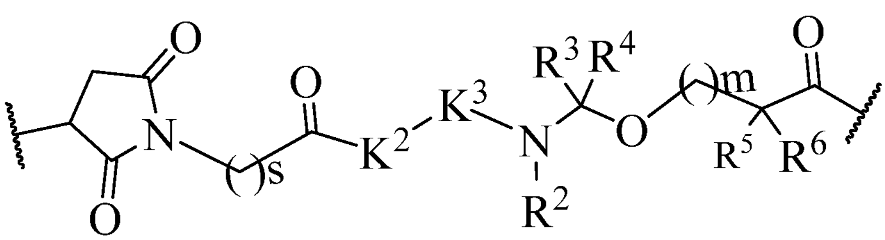

$K^2$ is a bond;
$K^3$ is the tetrapeptide residue GGFG;
$R^5$ is haloalkyl or $C_{3-6}$ cycloalkyl;
$R^6$ is selected from the group consisting of hydrogen, haloalkyl and $C_{3-6}$ cycloalkyl;
or, $R^5$ and $R^6$ and the carbon atom to which they are linked form a $C_{3-6}$ cycloalkyl;
$R^2$, $R^3$ or $R^4$ are each independently hydrogen or alkyl;
s is an integer of 2 to 8; preferably, s is 4, 5 or 6;
m is an integer of 0 to 4;
preferably, the $-L_2-L_1-$ is selected from the group consisting of the following structures:

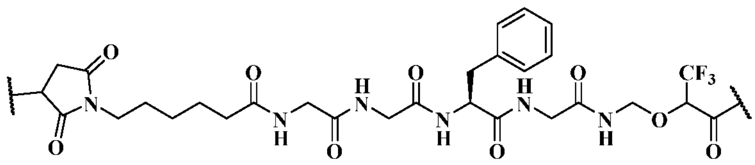

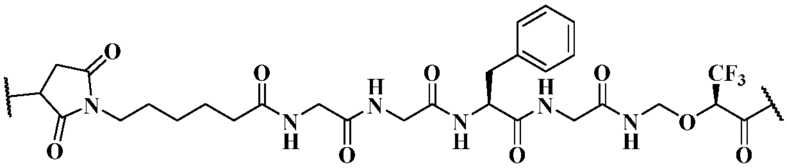

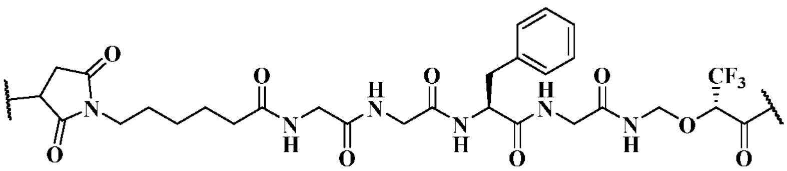

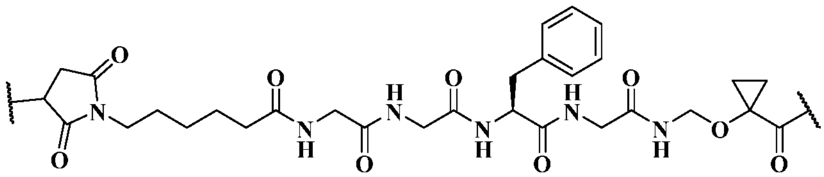

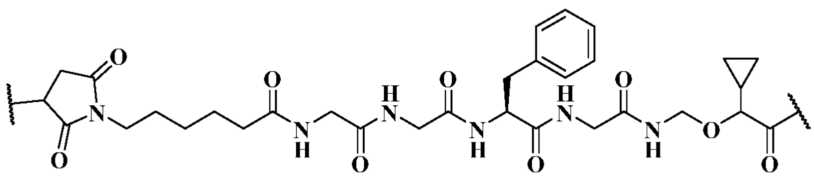

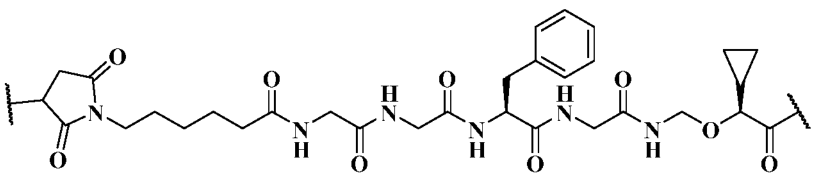

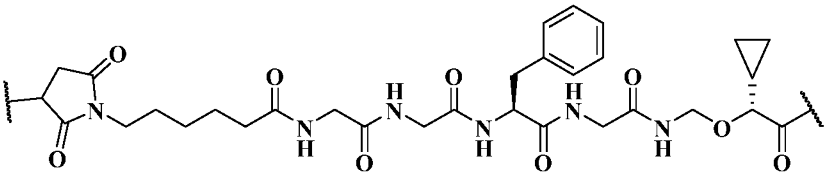

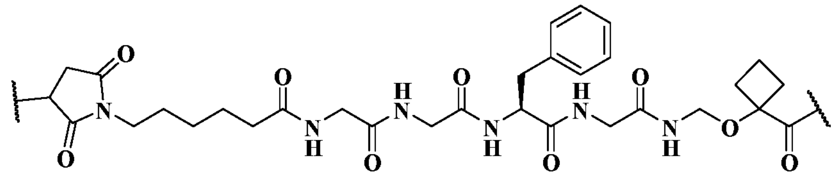

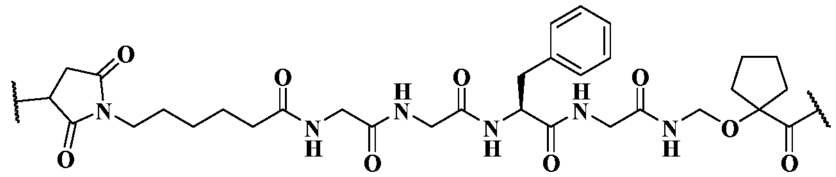

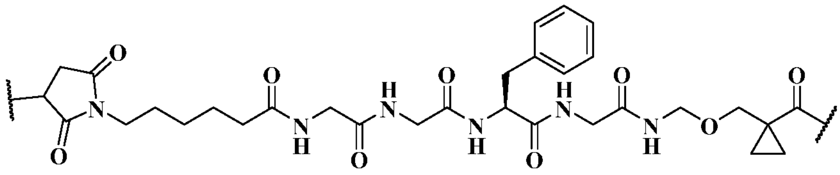

and

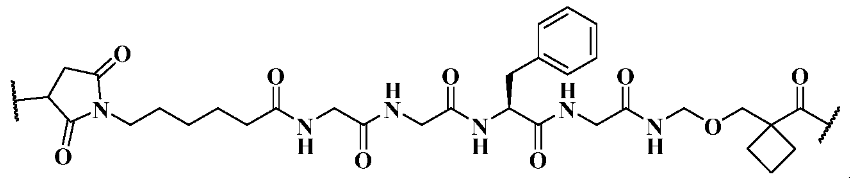

In a preferred embodiment, the antibody-drug conjugate or a pharmaceutically acceptable salt or solvate thereof as described above, which is the antibody-drug conjugate of general formula (IV) or a pharmaceutically acceptable salt or solvate thereof:

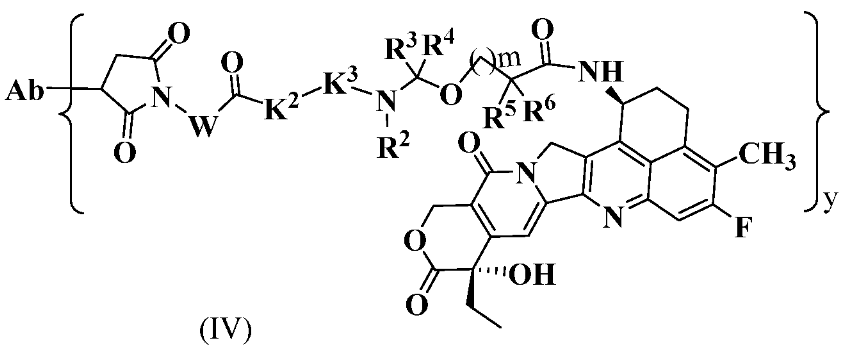

(IV)

wherein:

W is selected from the group consisting of $C_{1-8}$ alkyl, $C_{1-8}$ alkyl-cycloalkyl or linear heteroalkyl of 1 to 8 atoms, the heteroalkyl comprises 1 to 3 heteroatom(s) selected from the group consisting of N, O or S, wherein the $C_{1-8}$ alkyl, cycloalkyl or linear heteroalkyl is each independently optionally further substituted with one or more substituents selected from the group consisting of halogen, hydroxyl, cyano, amino, alkyl, chloroalkyl, deuterated alkyl, alkoxyl and cycloalkyl;

$K^2$ is selected from the group consisting of $-NR^1(CH_2CH_2O)_{p1}CH_2CH_2C(O)-$, $-NR^1(CH_2CH_2O)_{p1}CH_2C(O)-$, $-S(CH_2)_{p1}C(O)-$ or bond, $R^1$ is selected from the group consisting of hydrogen atom, alkyl, haloalkyl, deuterated alkyl and hydroxyalkyl, and $p_1$ is an integer of 1 to 20;

$K^3$ is a peptide residue consisting of 2 to 7 amino acids, the amino acids can be substituted or unsubstituted. When

substituted, the substituents can be substituted at any available attachment point, and the substituents are one or more independently selected from the group consisting of halogen, hydroxyl, cyano, amino, alkyl, chloroalkyl, deuterated alkyl, alkoxyl and cycloalkyl;

$R^2$ is independently selected from the group consisting of hydrogen atom, alkyl, haloalkyl, deuterated alkyl and hydroxyalkyl;

$R^3$ and $R^4$ are each independently selected from the group consisting of hydrogen atom, halogen, alkyl, haloalkyl, deuterated alkyl and hydroxyalkyl;

$R^5$ is selected from the group consisting of halogen, haloalkyl, deuterated alkyl, cycloalkyl, heterocyclyl, aryl and heteroaryl;

$R^6$ is selected from the group consisting of hydrogen atom, halogen, haloalkyl, deuterated alkyl, cycloalkyl, heterocyclyl, aryl and heteroaryl;

or, $R^5$ and $R^6$ and the carbon atom to which they are linked form a cycloalkyl or heterocyclyl;

m is an integer of 0 to 4;

y is 1 to 10 and y is a decimal or an integer, preferably, y is a number of 2 to 10, more preferably y is a number of 4 to 10, further preferably a number of 6 to 9, and most preferably an integer of 7, 8 or 9;

Ab is an anti-B7-H4 antibody or antigen-binding fragment thereof.

In a more preferred embodiment, the antibody-drug conjugate or a pharmaceutically acceptable salt or solvate thereof as described above, which is an antibody-drug conjugate of general formula (I-A) or a pharmaceutically acceptable salt or solvate thereof:

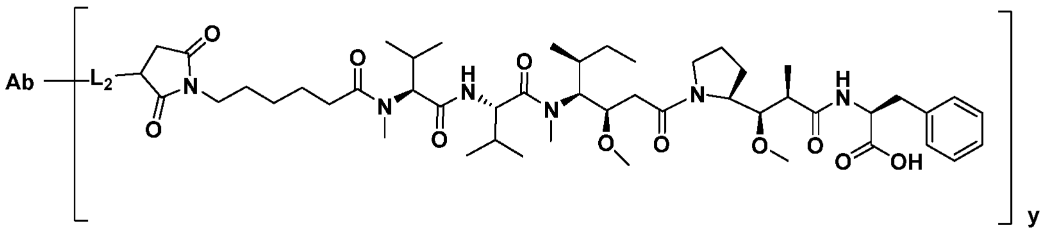

( I-A )

y is 1 to 10, y is a decimal or an integer.

In a more preferred embodiment, the antibody-drug conjugate or a pharmaceutically acceptable salt or solvate thereof as described above, which is an antibody-drug conjugate of general formula (I-B) or a pharmaceutically acceptable salt or solvate thereof:

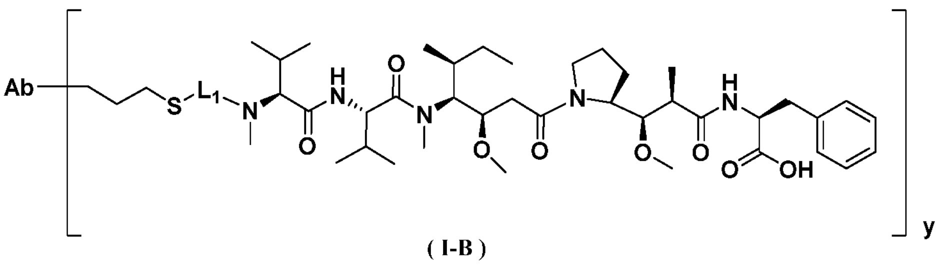

( I-B )

y is 1 to 10, y is a decimal or an integer.

In a more preferred embodiment, the antibody-drug conjugate or a pharmaceutically acceptable salt or solvate thereof as described above, which is an antibody-drug conjugate of general formula (II-A) or a pharmaceutically acceptable salt or solvate thereof:

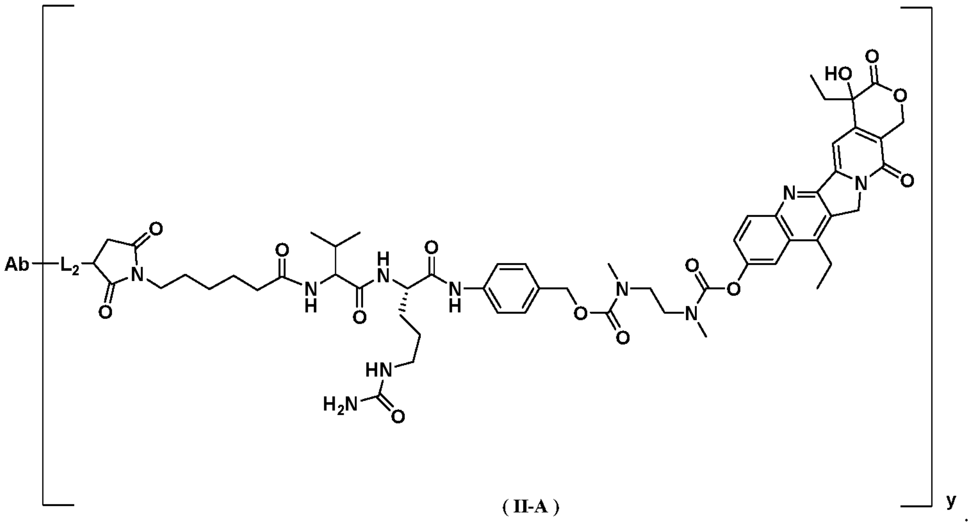

( II-A )

In a more preferred embodiment, the antibody-drug conjugate or a pharmaceutically acceptable salt or solvate thereof as described above, which is an antibody-drug conjugate of general formula (II-B) or a pharmaceutically acceptable salt or solvate thereof:

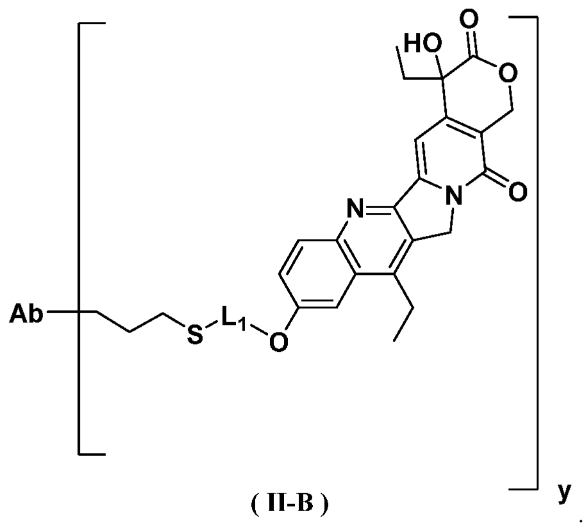

( II-B )

In a more preferred embodiment, the antibody-drug conjugate or a pharmaceutically acceptable salt or solvate thereof as described above, which is an antibody-drug conjugate of general formula (IV-A) or a pharmaceutically acceptable salt or solvate thereof:

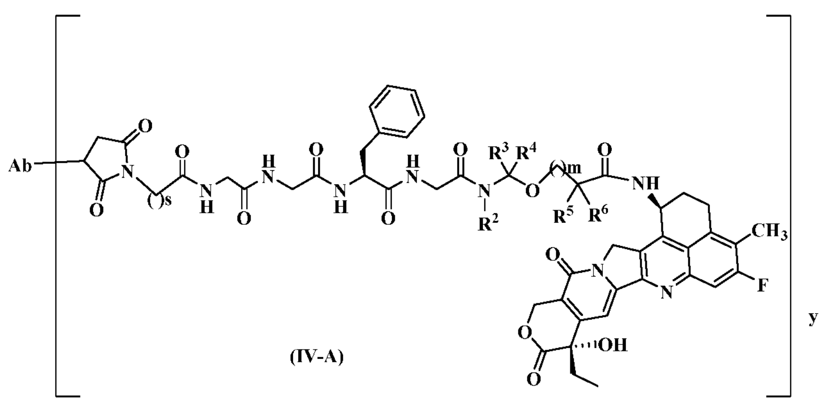

(IV-A)

wherein, s is an integer of 2 to 8, $R^2$ to $R^6$, m and y are as defined in the above general formula (IV); preferably, s is an integer of 4, 5 or 6, y is a number of 4 to 10, preferably a number of 6 to 9, and more preferably 7 or 8.

In a more preferred embodiment, the antibody-drug conjugate or a pharmaceutically acceptable salt or solvate thereof as described above, selected from the group consisting of the following compounds:

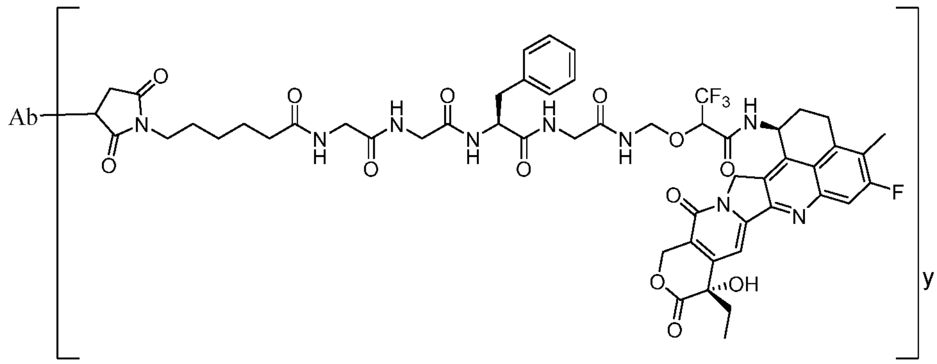

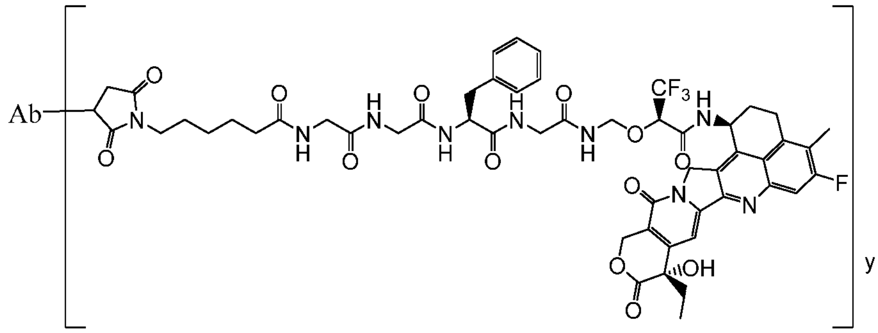

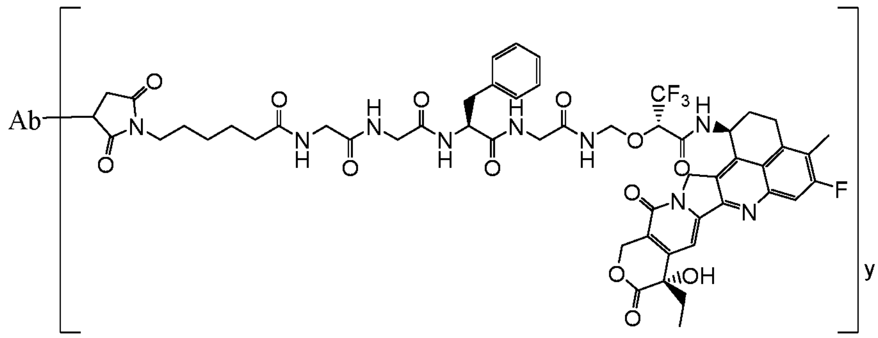

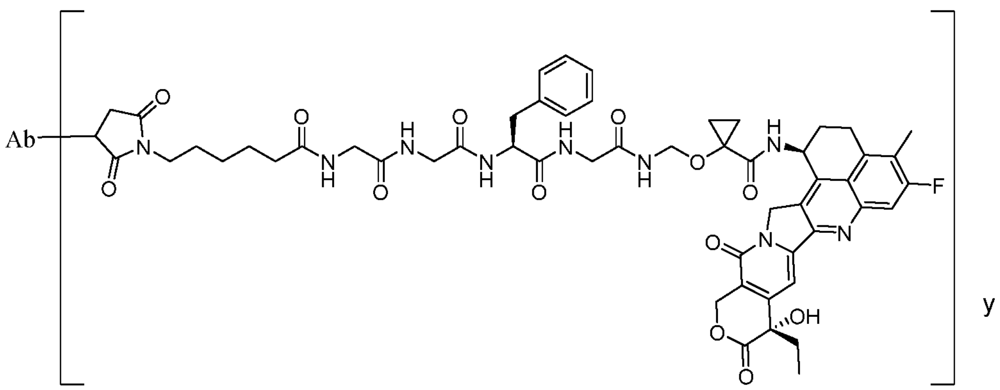

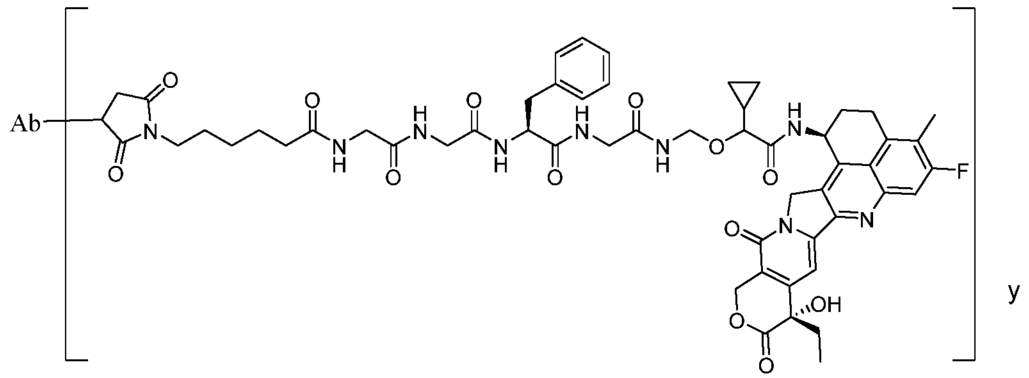

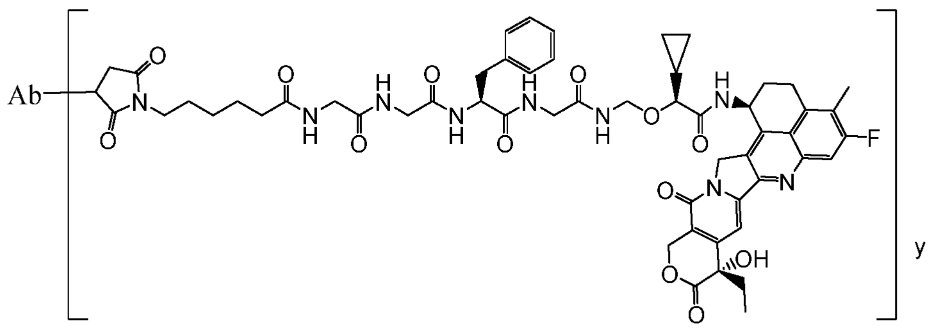

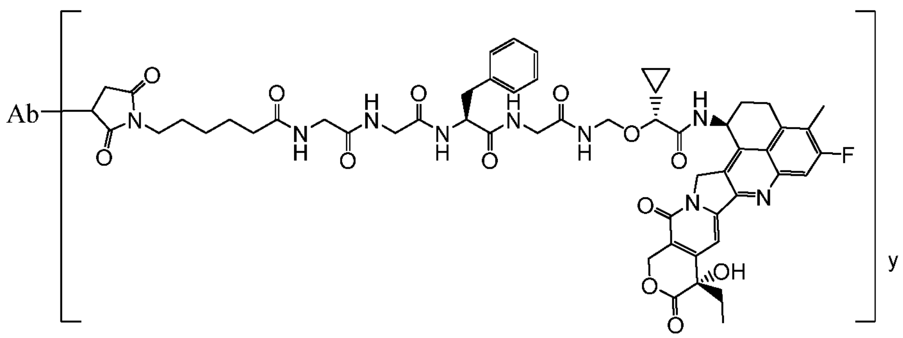

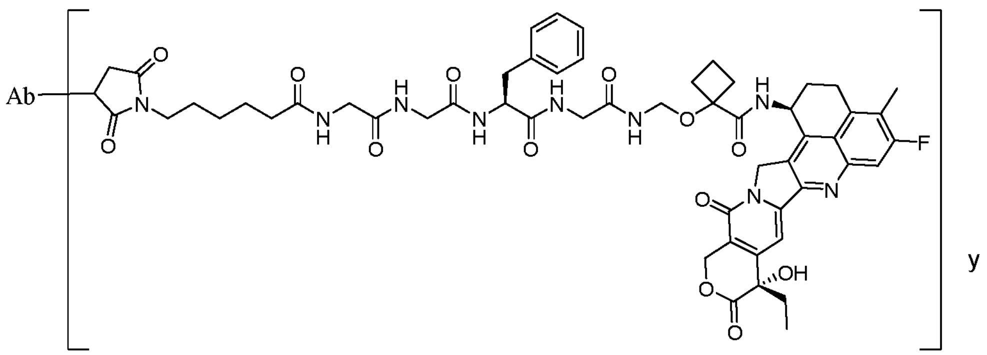

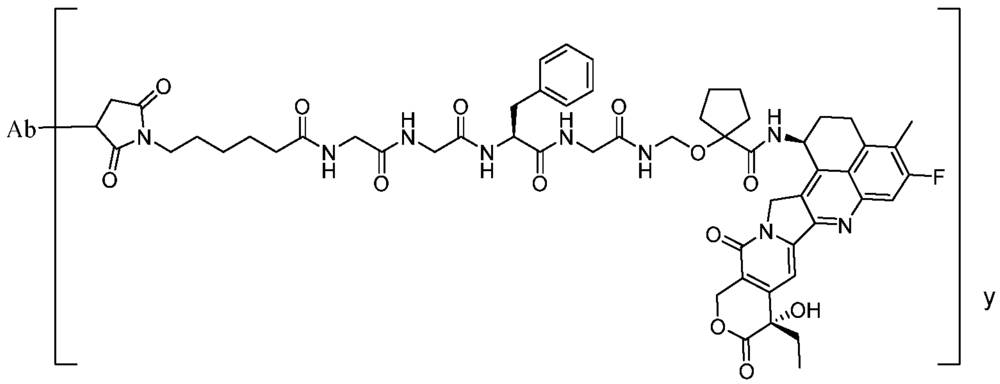

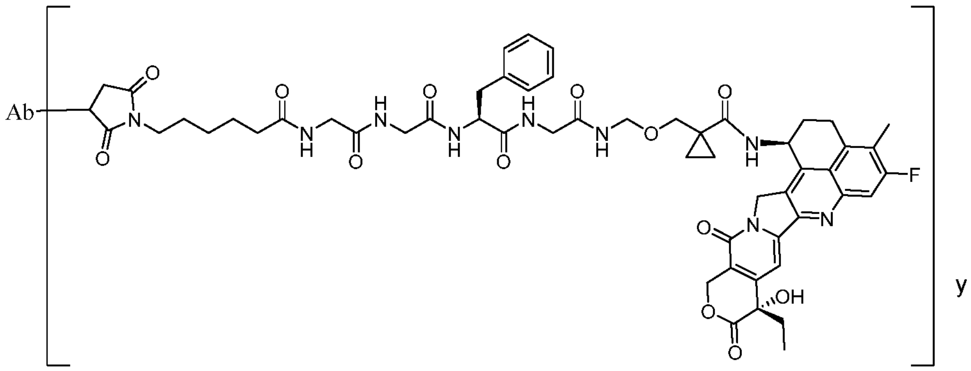

and

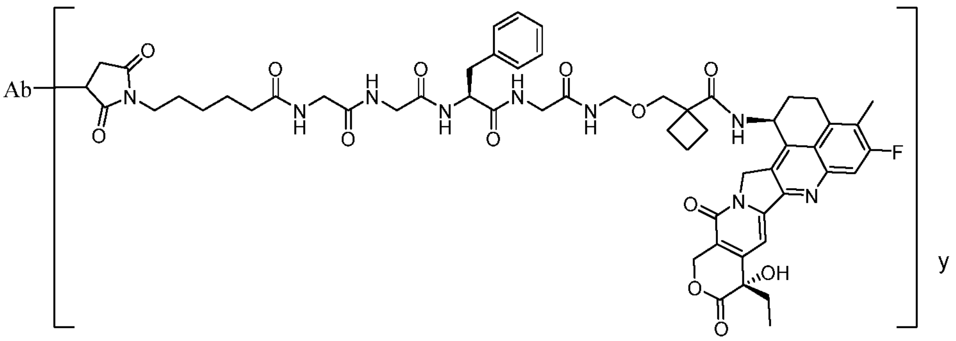

In a most preferred embodiment, the antibody-drug conjugate or a pharmaceutically acceptable salt or solvate thereof as described above, which is selected from the group consisting of the following compounds:

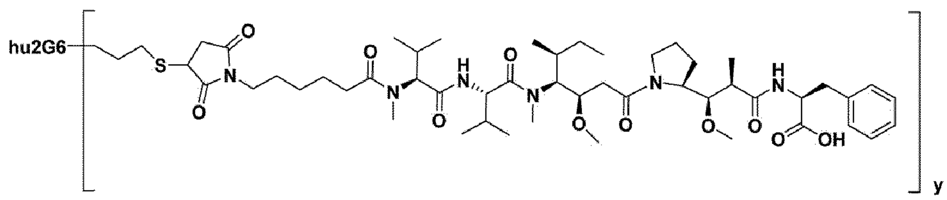

(Compound 1)

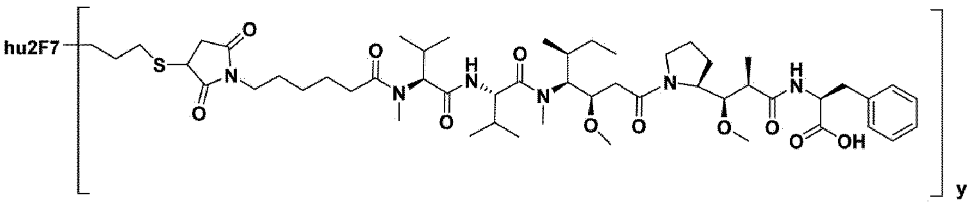

(Compound 2)

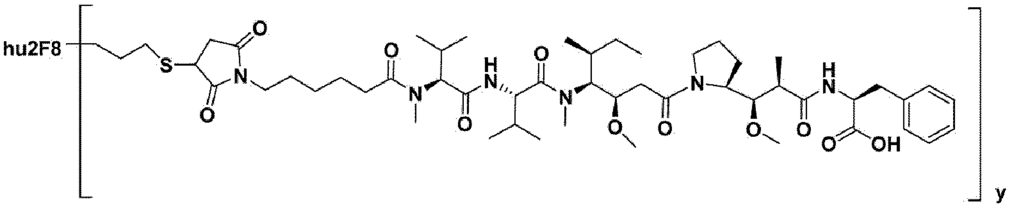

(Compound 3)

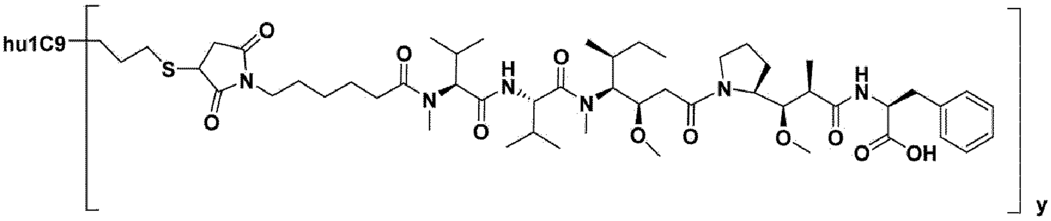

(Compound 4)

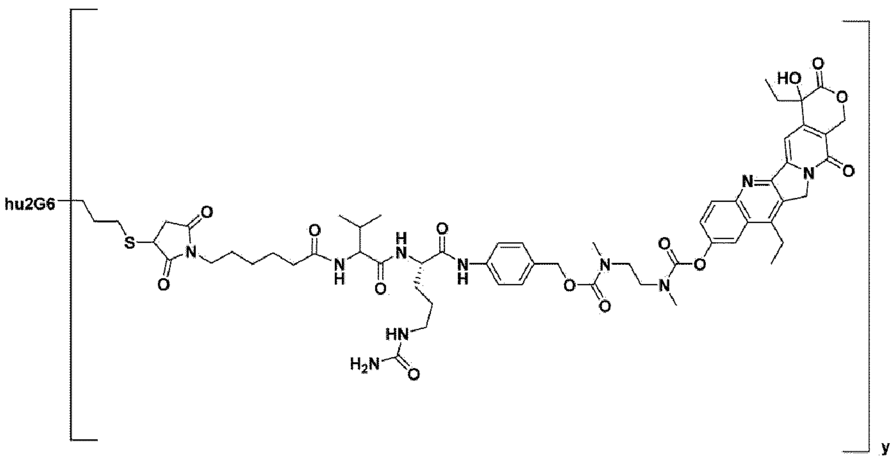

(Compound 5)

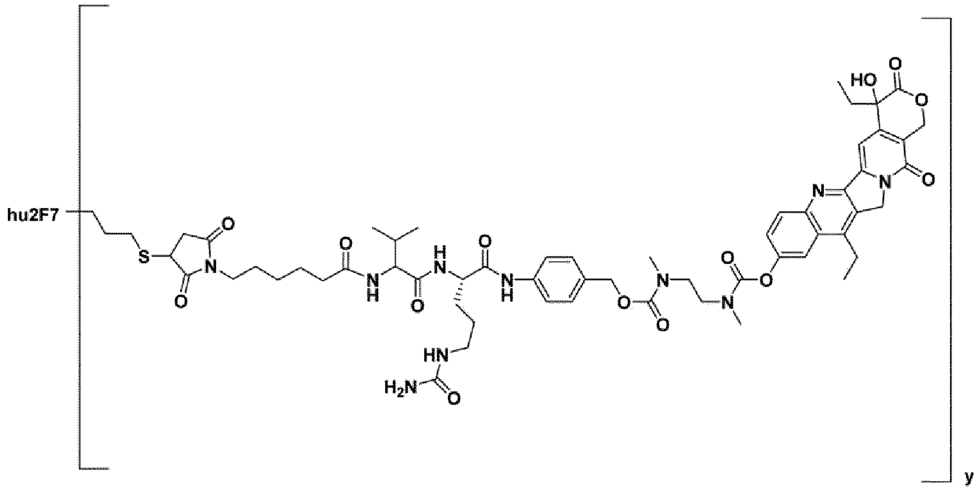

(Compound 6)

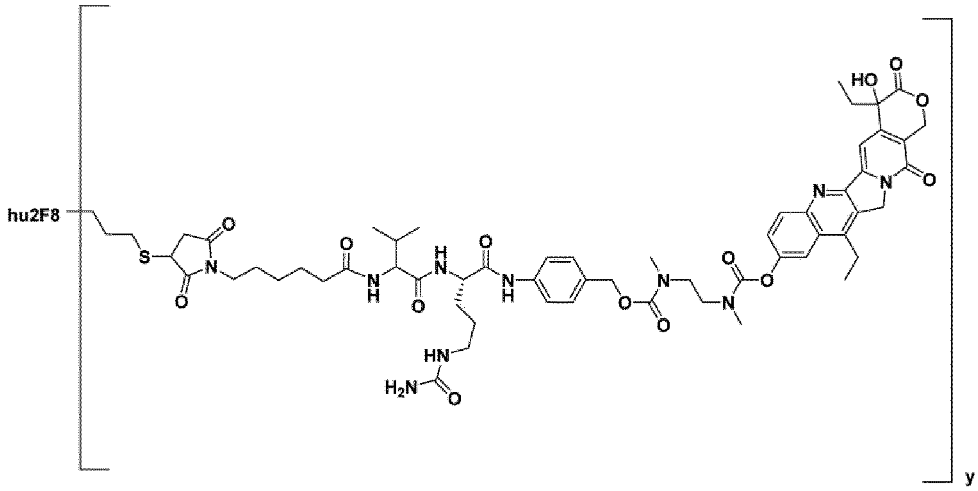

(Compound 7)

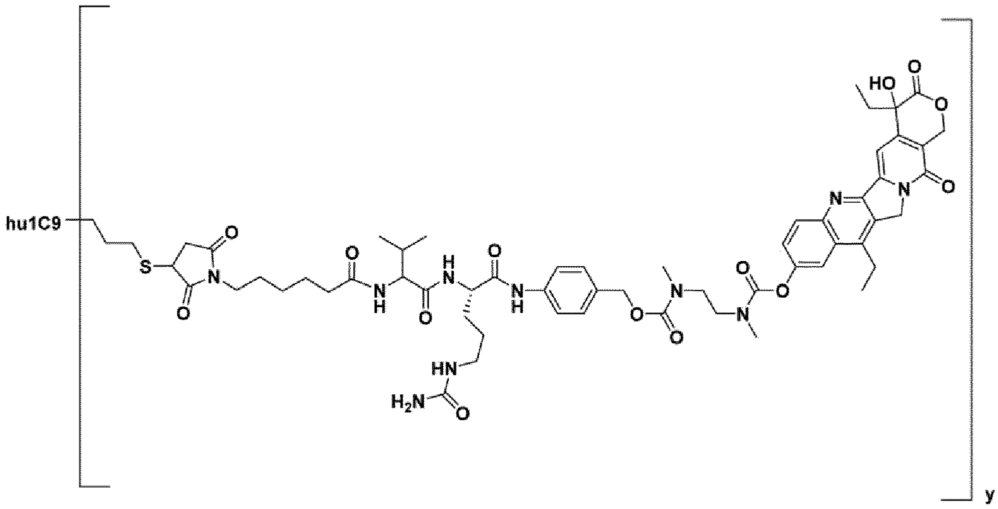

(Compound 8)

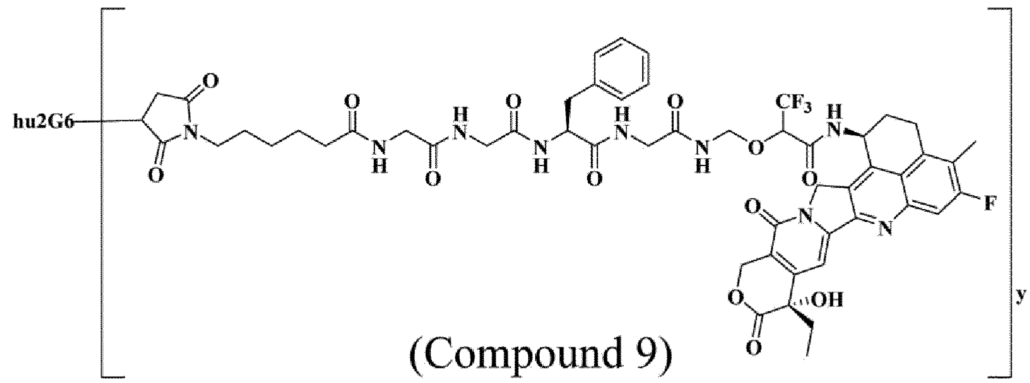

(Compound 9)

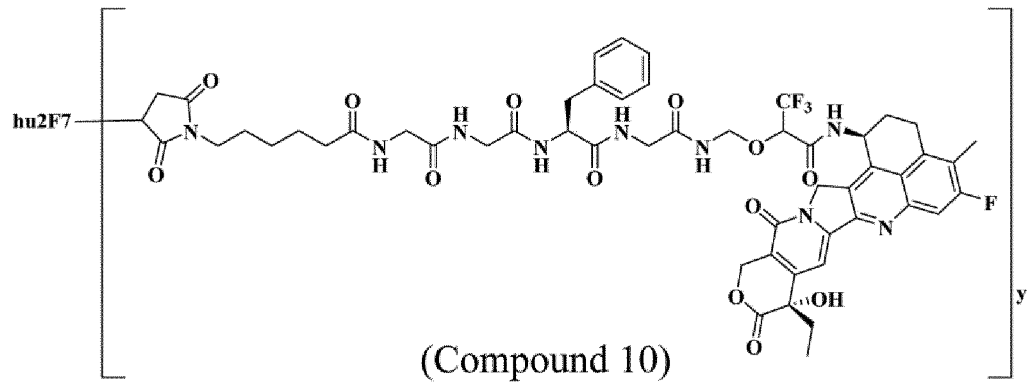

(Compound 10)

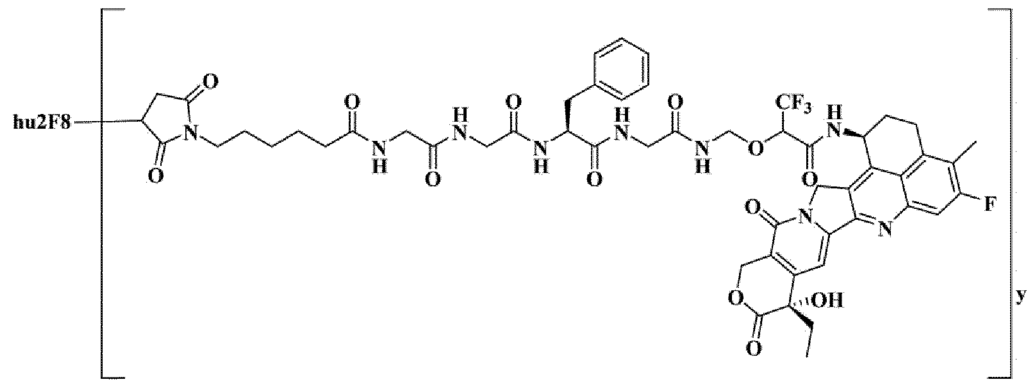

(Compound 11)

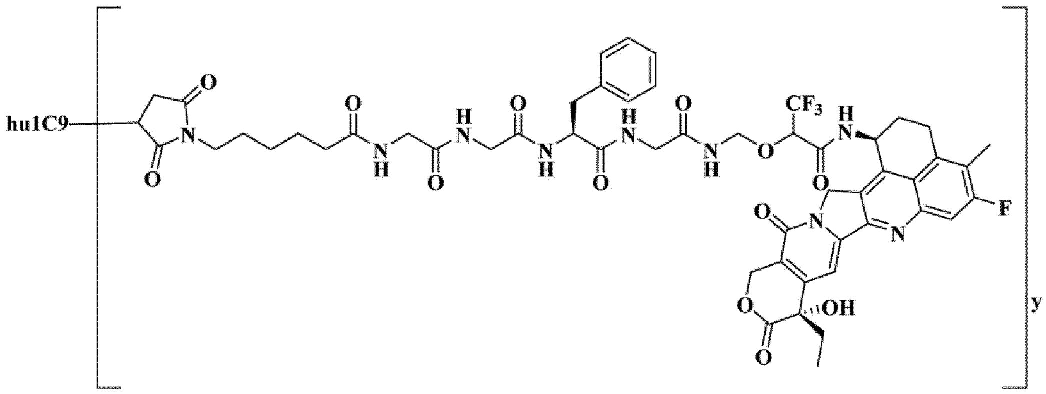

(Compound 12)

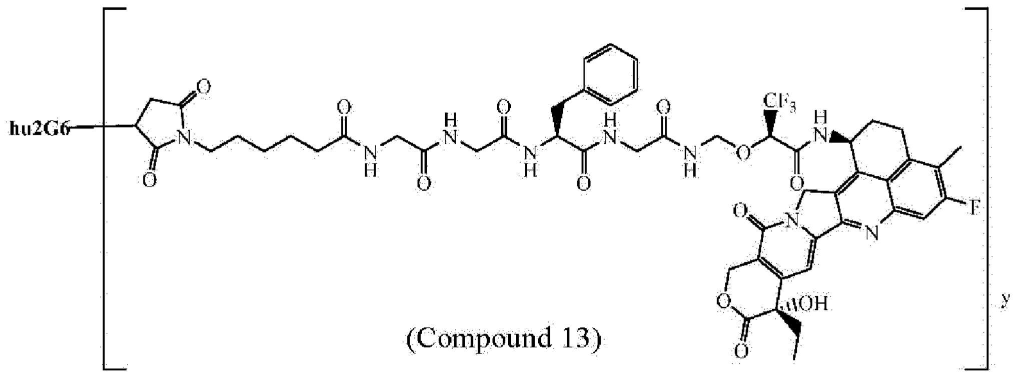

(Compound 13)

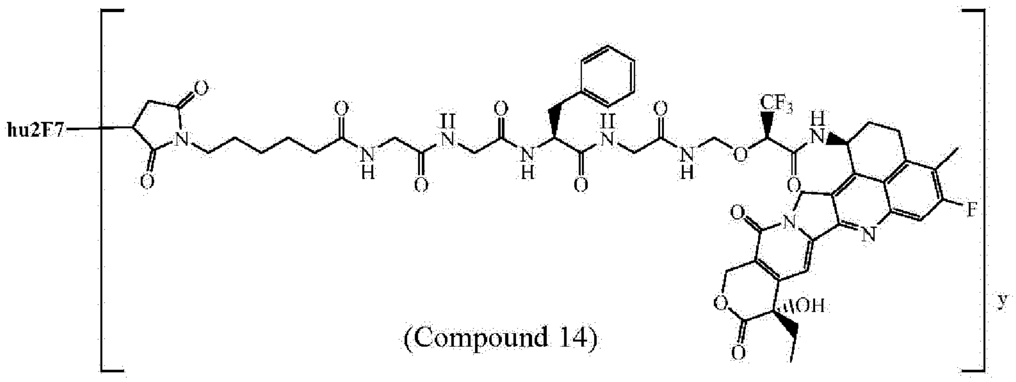

(Compound 14)

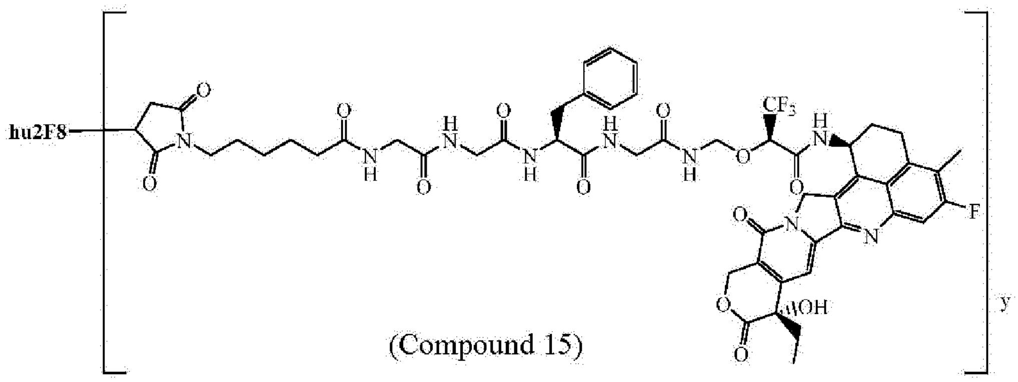

(Compound 15)

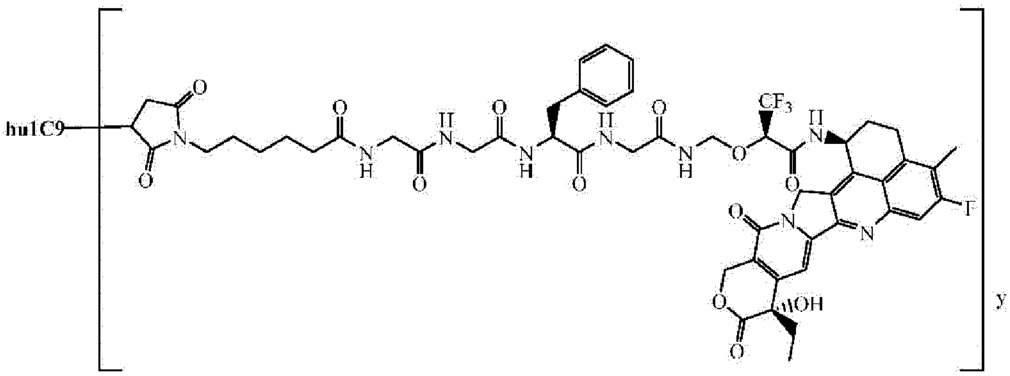

(Compound 16)

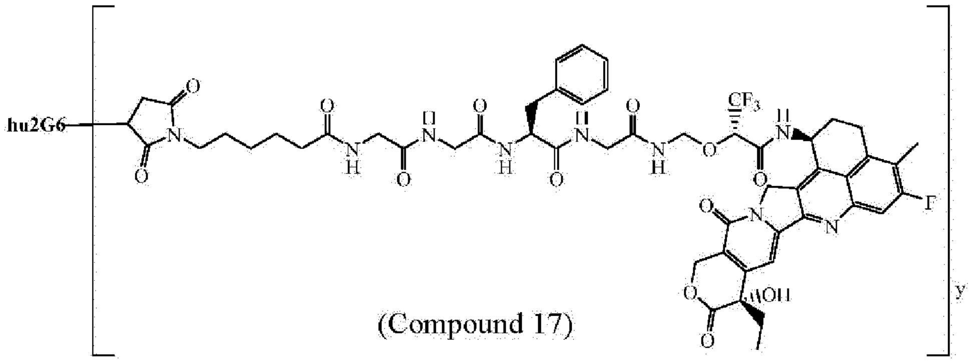

(Compound 17)

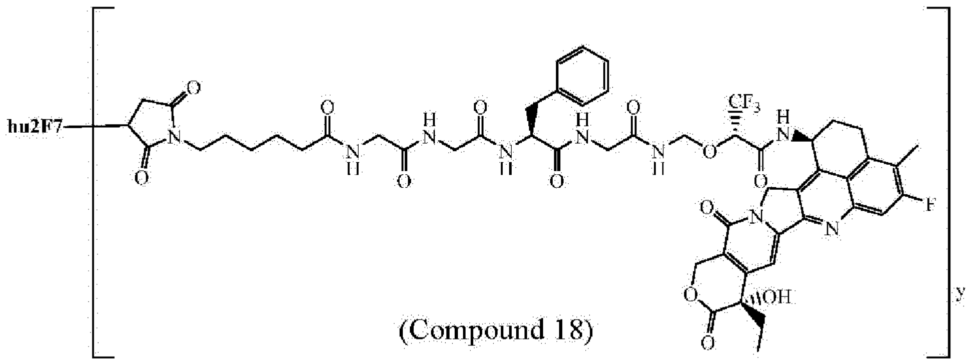

(Compound 18)

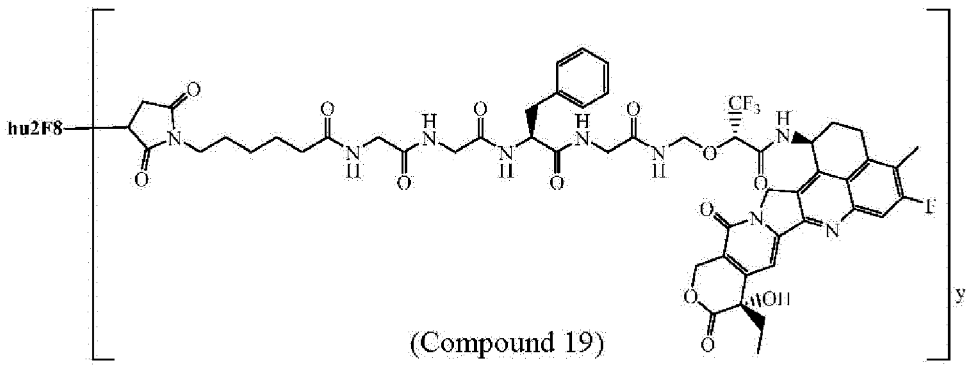

(Compound 19)

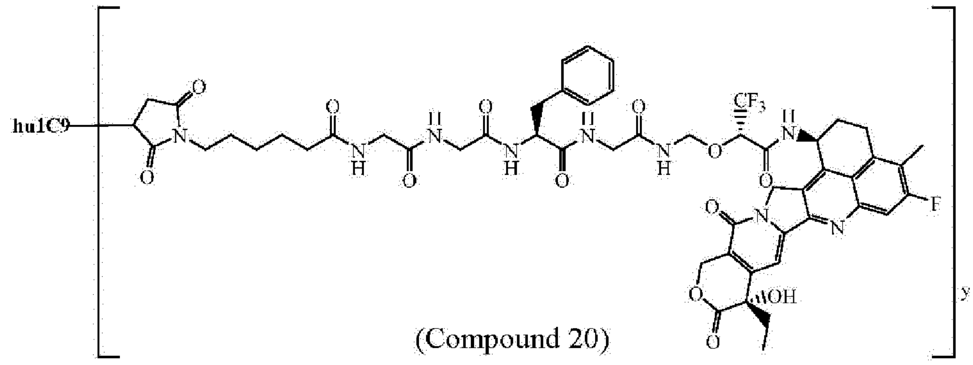

(Compound 20)

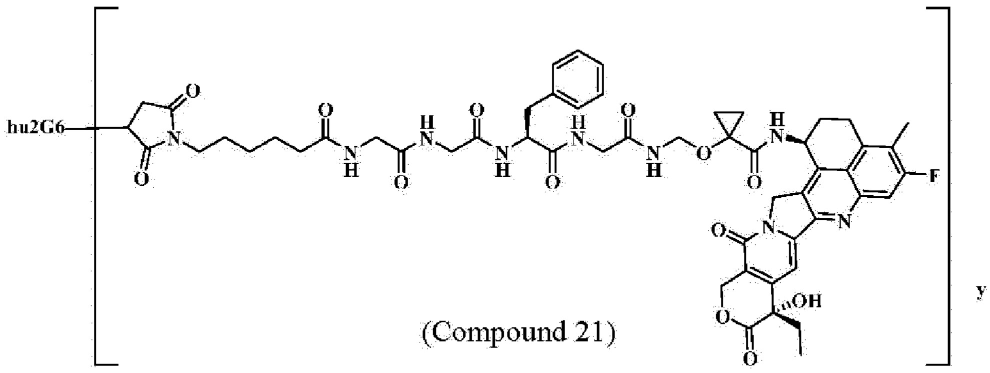

(Compound 21)

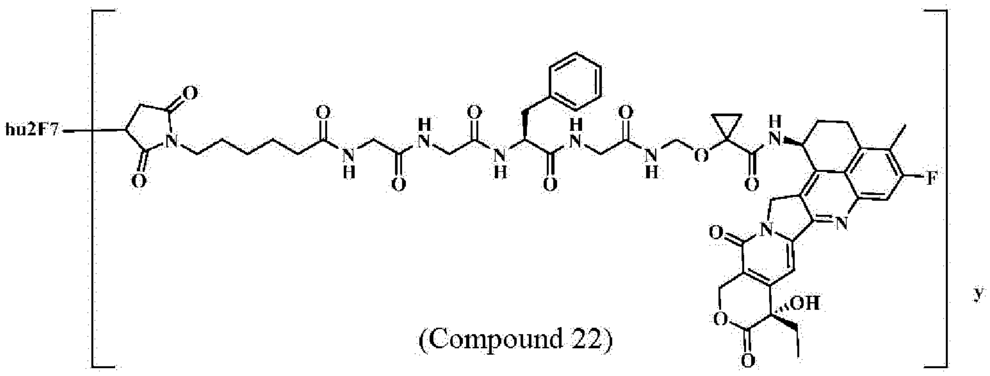

(Compound 22)

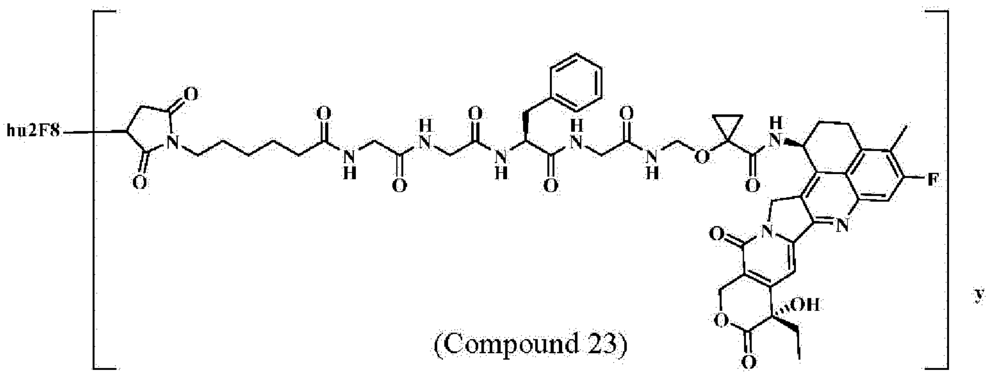

(Compound 23)

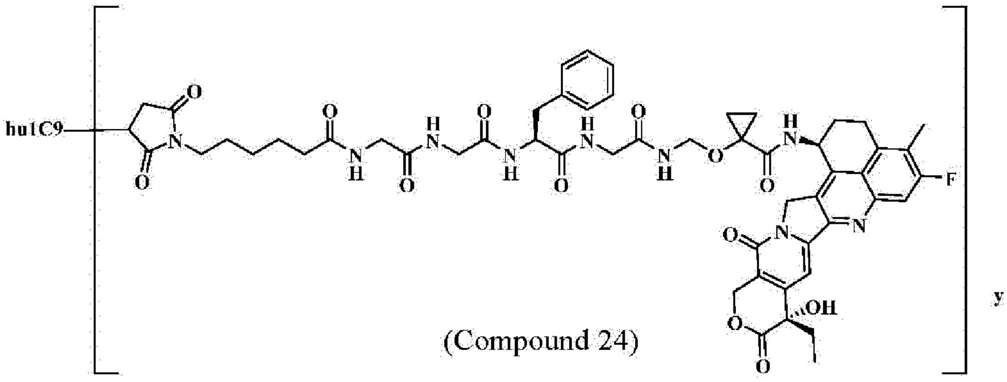

(Compound 24)

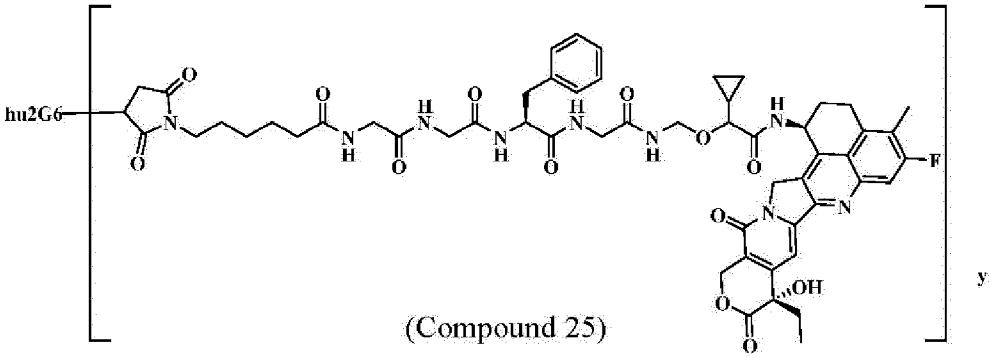

(Compound 25)

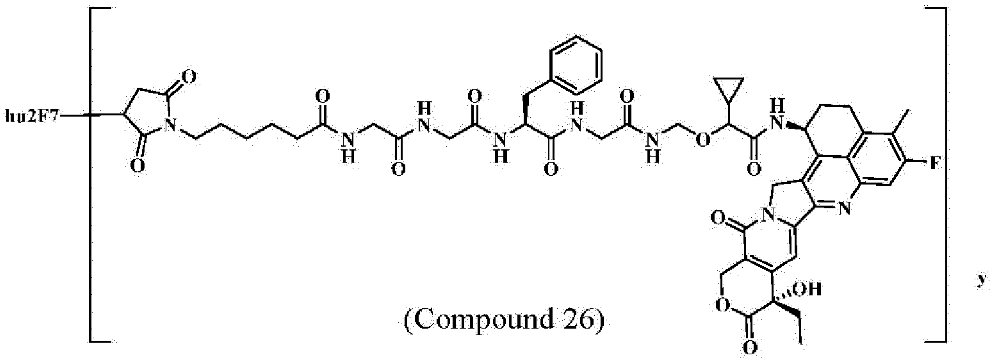

(Compound 26)

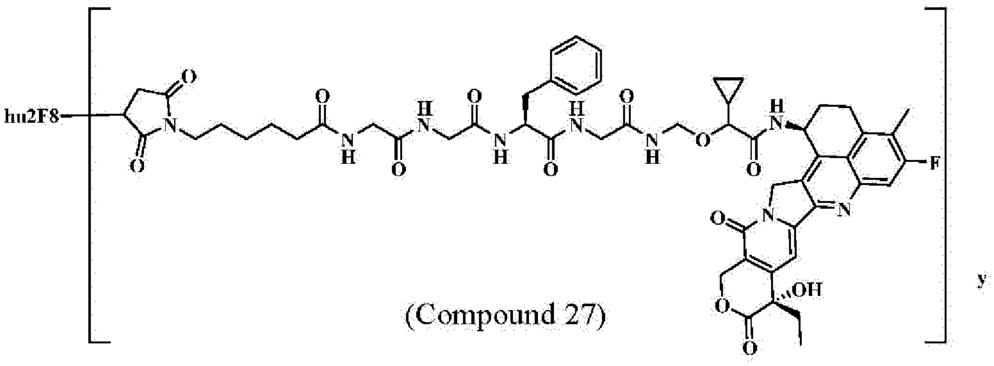

(Compound 27)

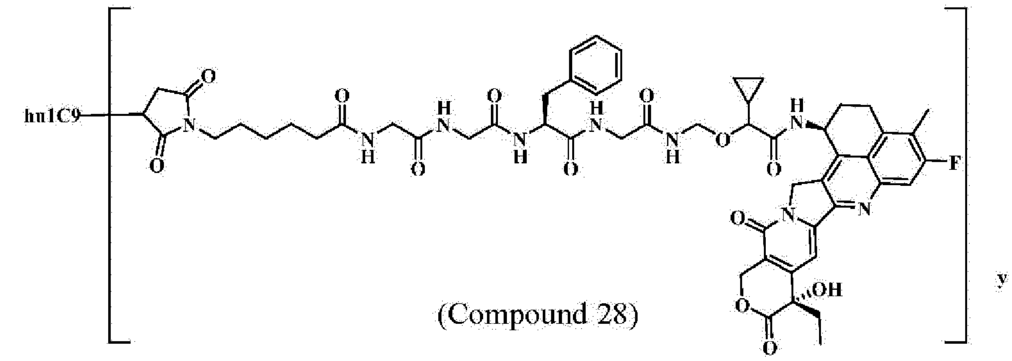

(Compound 28)

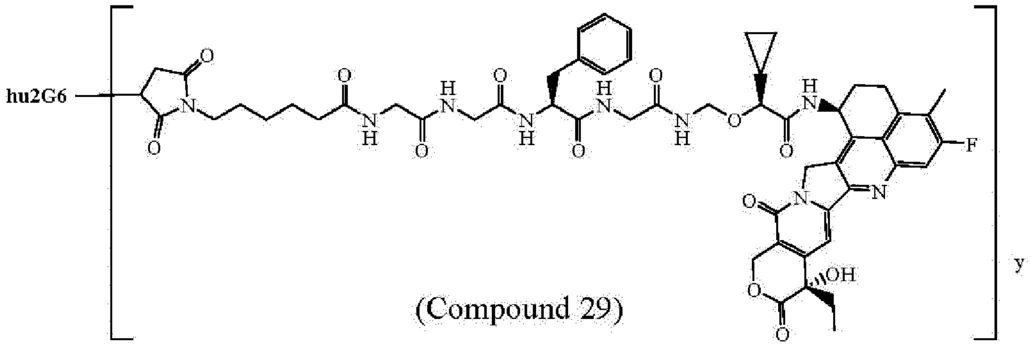

(Compound 29)

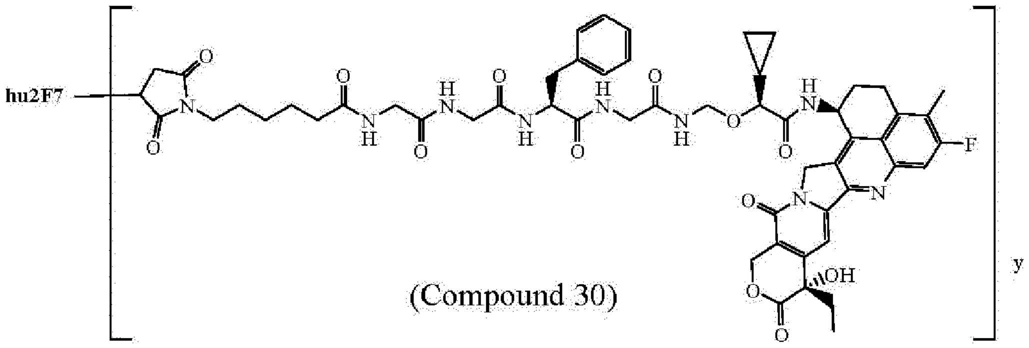

(Compound 30)

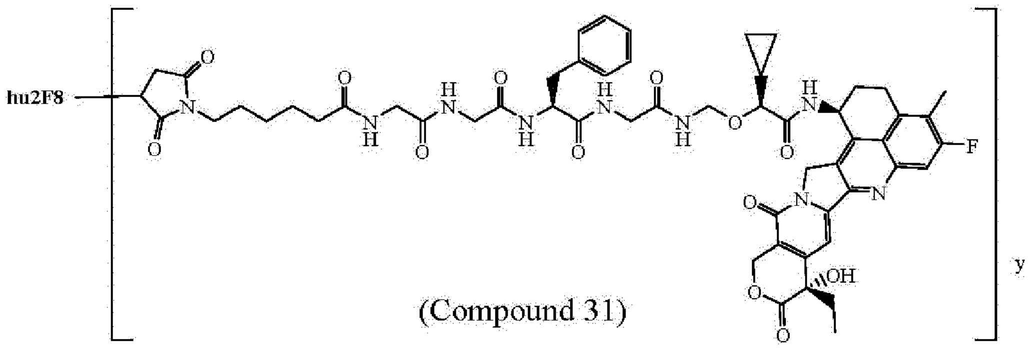

(Compound 31)

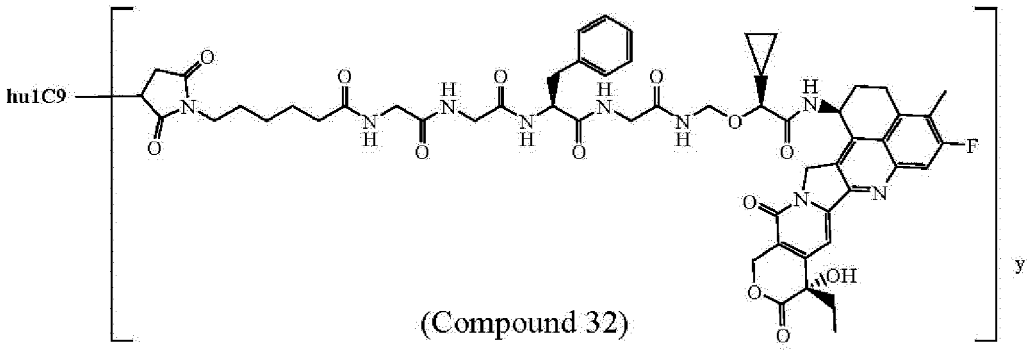

(Compound 32)

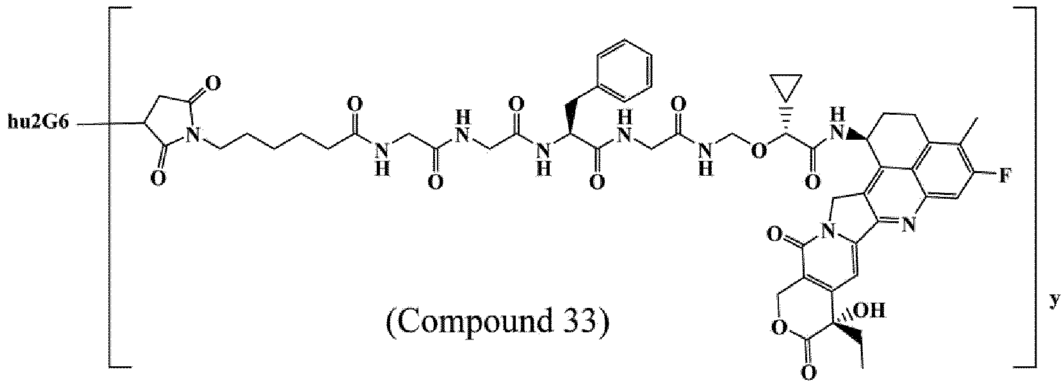

(Compound 33)

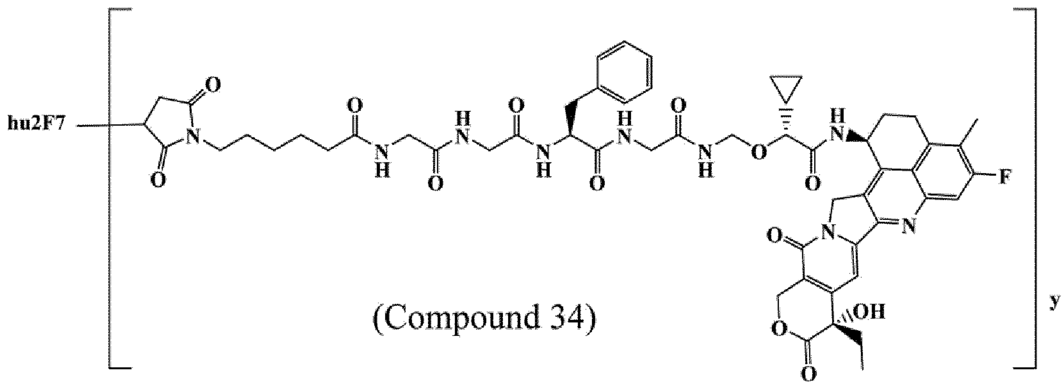

(Compound 34)

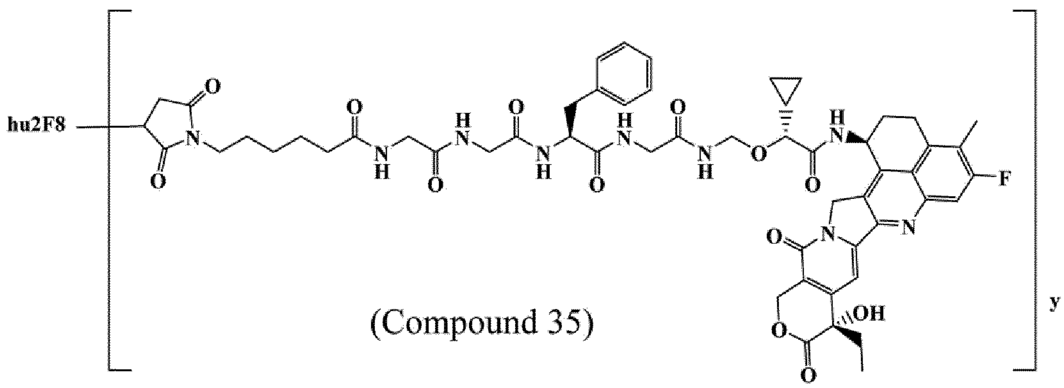

(Compound 35)

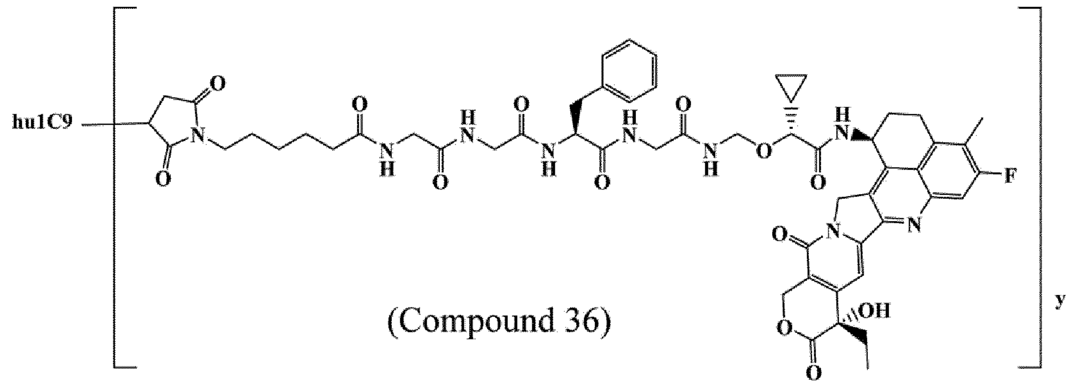

(Compound 36)

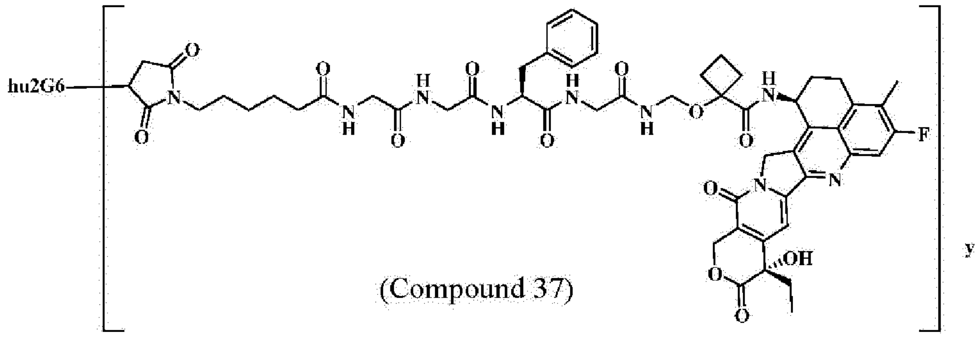

(Compound 37)

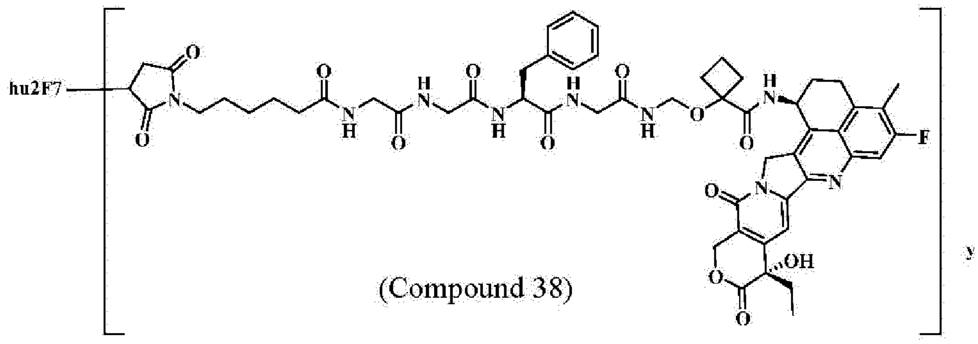

(Compound 38)

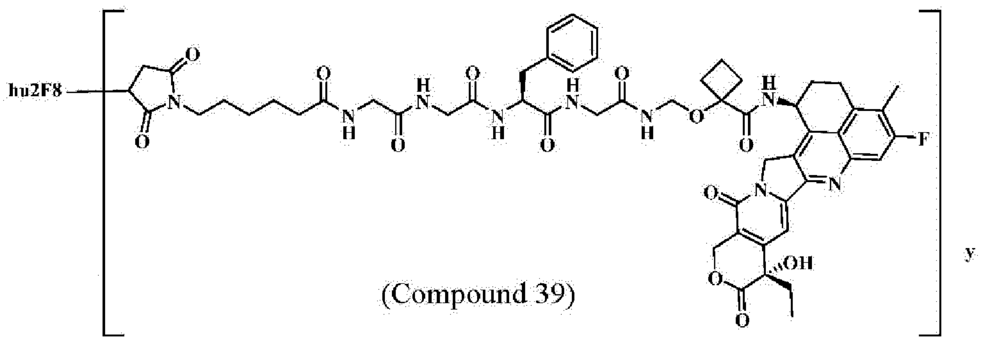

(Compound 39)

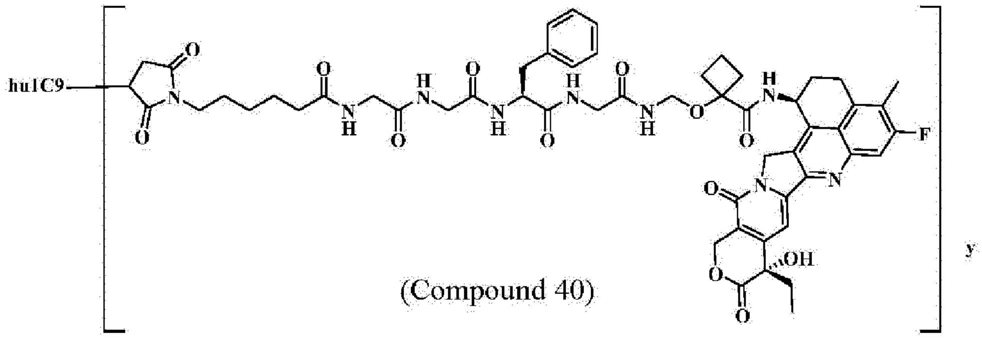

(Compound 40)

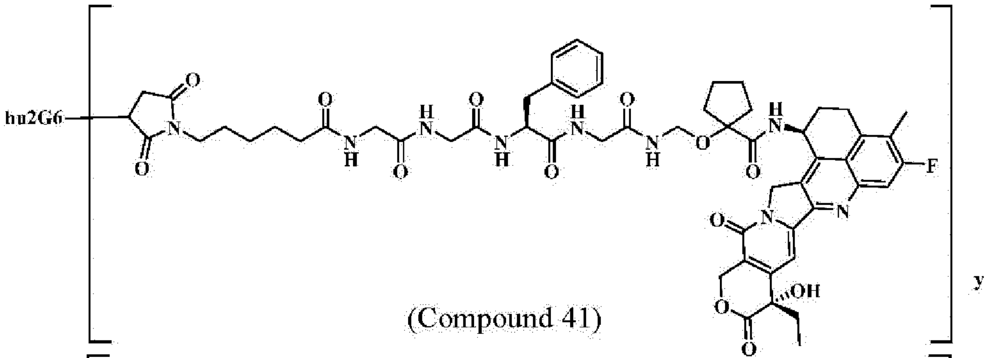

(Compound 41)

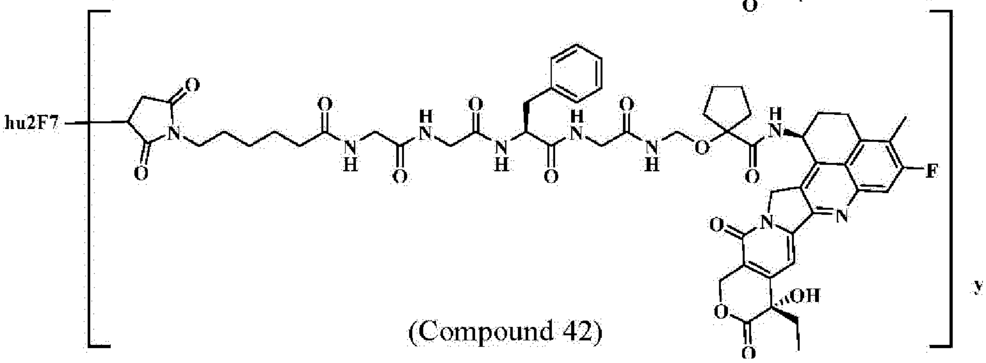

(Compound 42)

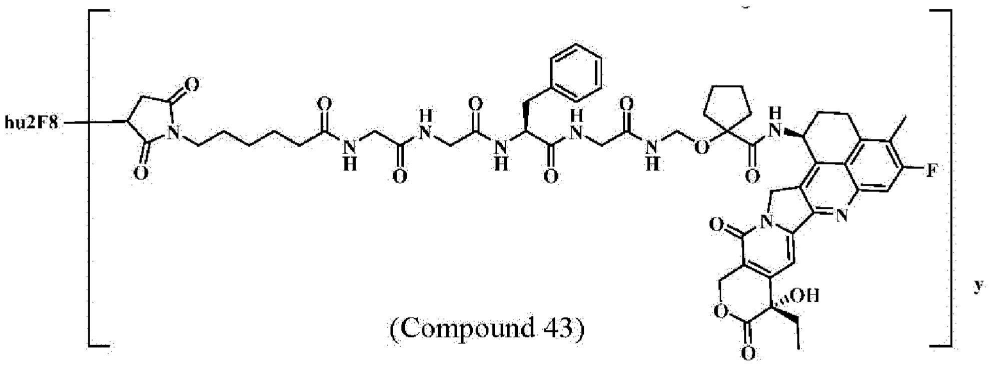

(Compound 43)

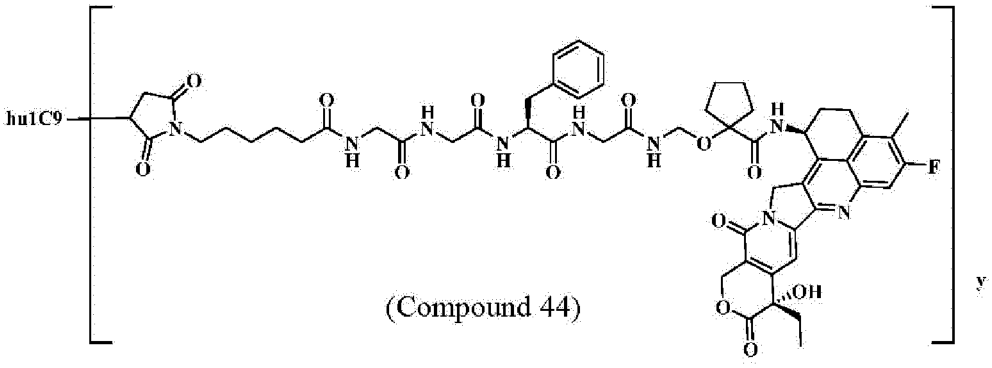

(Compound 44)

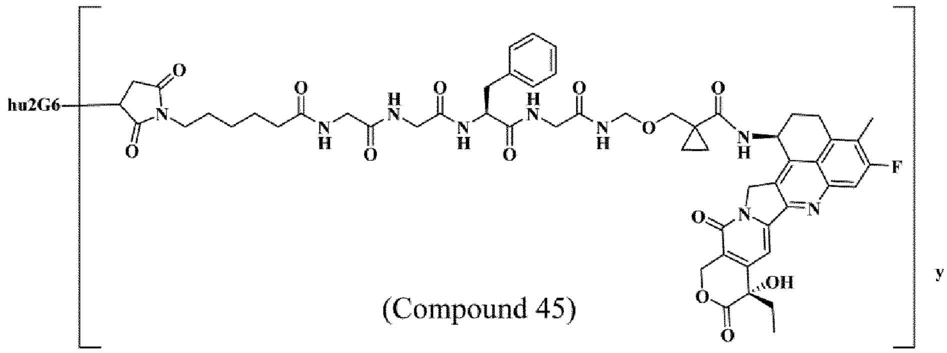

(Compound 45)

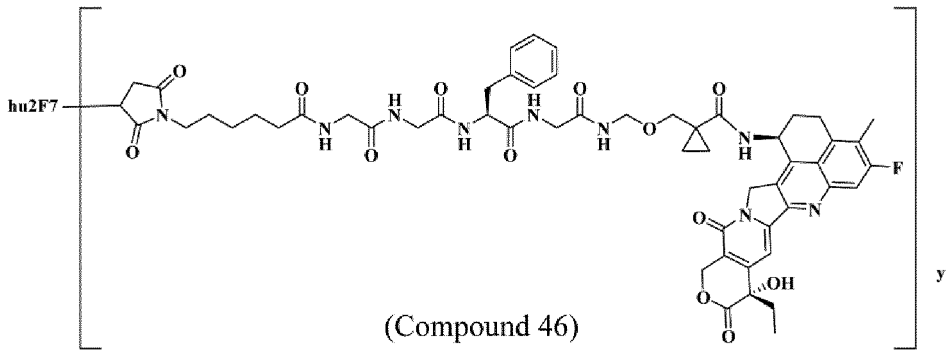

(Compound 46)

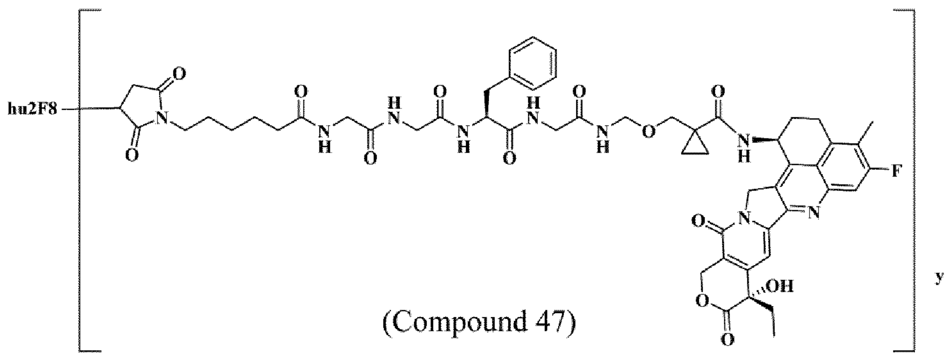

(Compound 47)

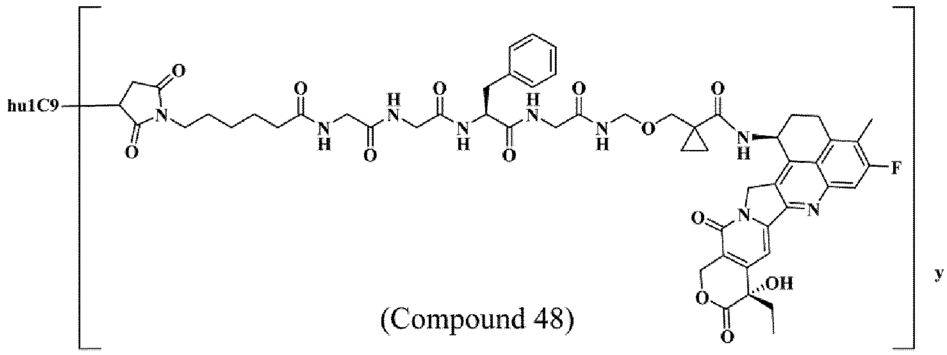

(Compound 48)

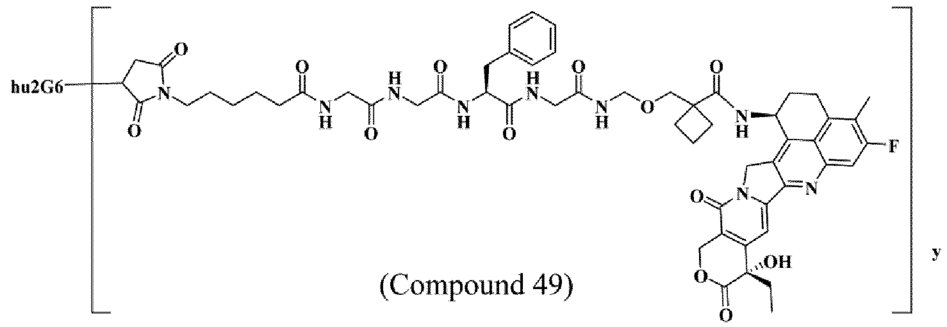

(Compound 49)

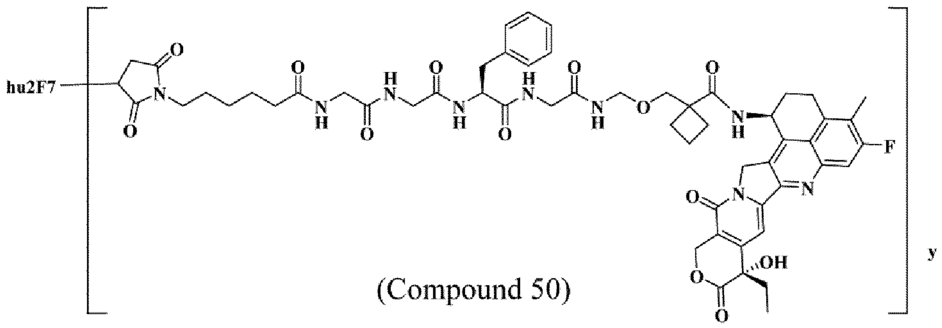

(Compound 50)

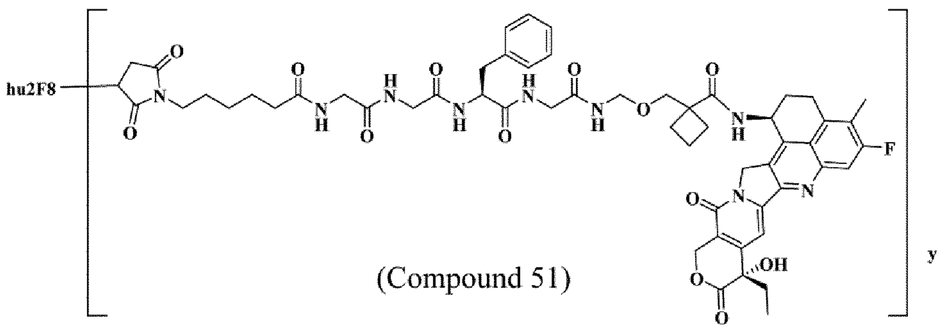

(Compound 51)

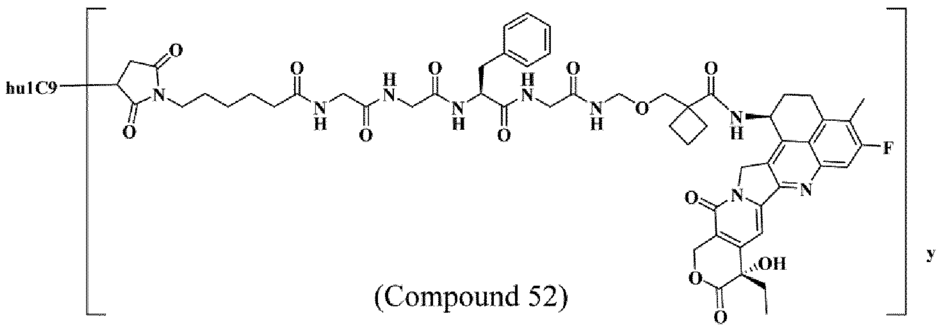

(Compound 52)

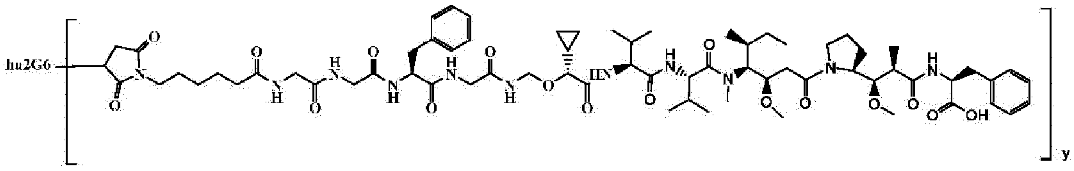

(Compound 53)

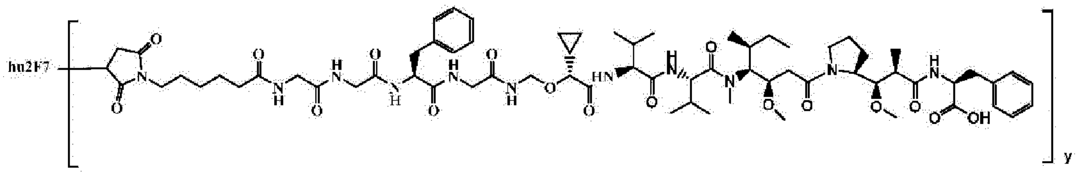

(Compound 54)

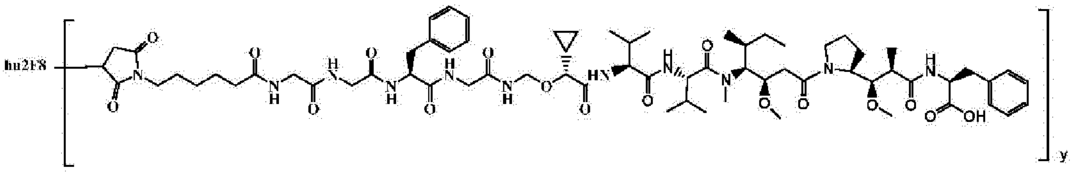

(Compound 55)

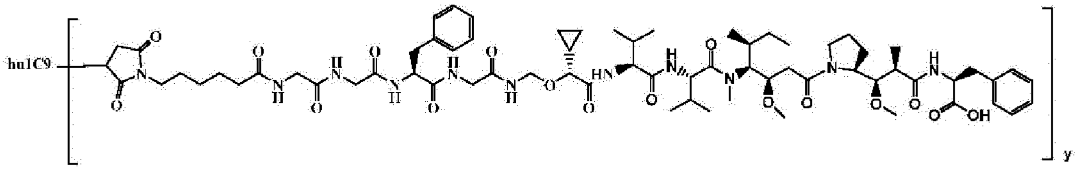

(Compound 56)

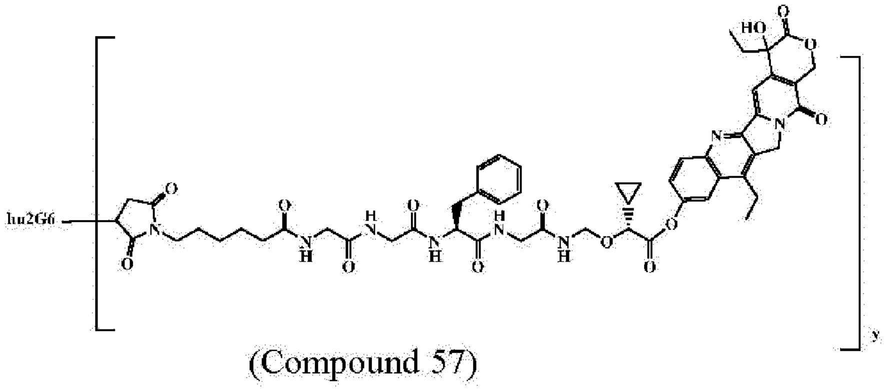

(Compound 57)

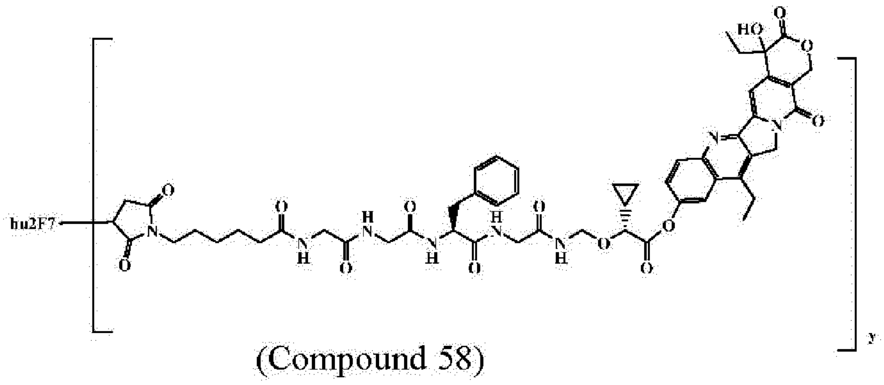

(Compound 58)

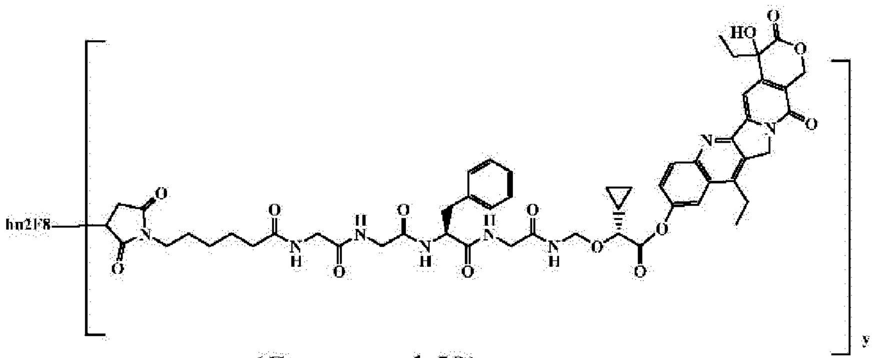

(Compound 59)

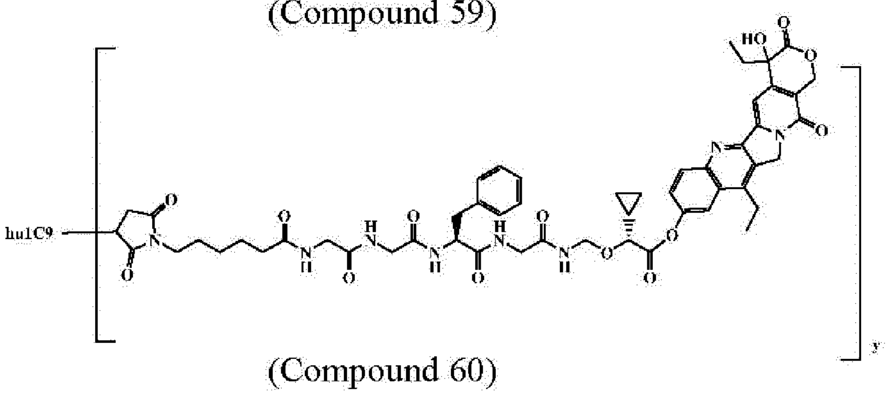

(Compound 60)

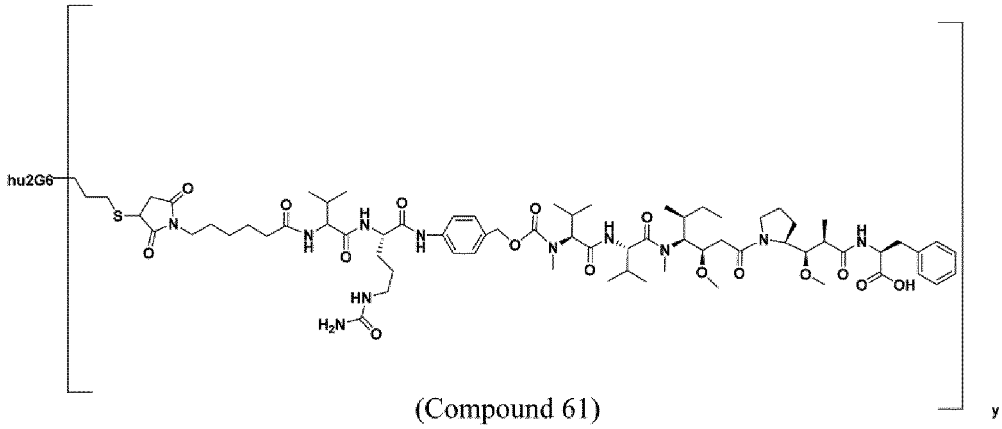

(Compound 61)

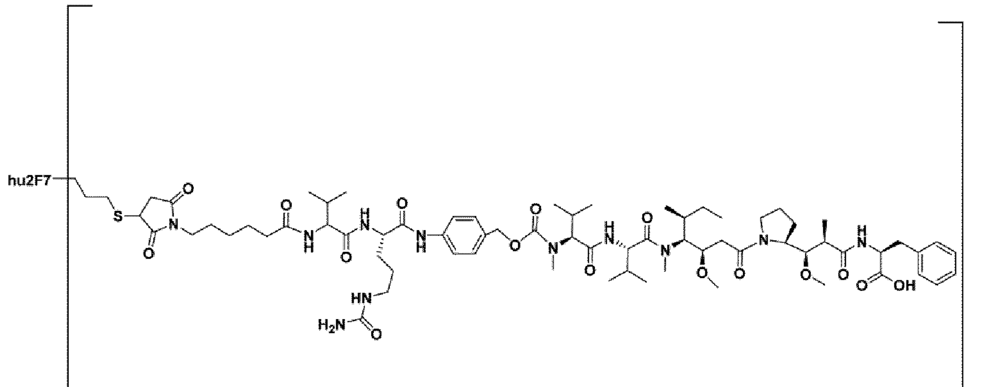

(Compound 62)

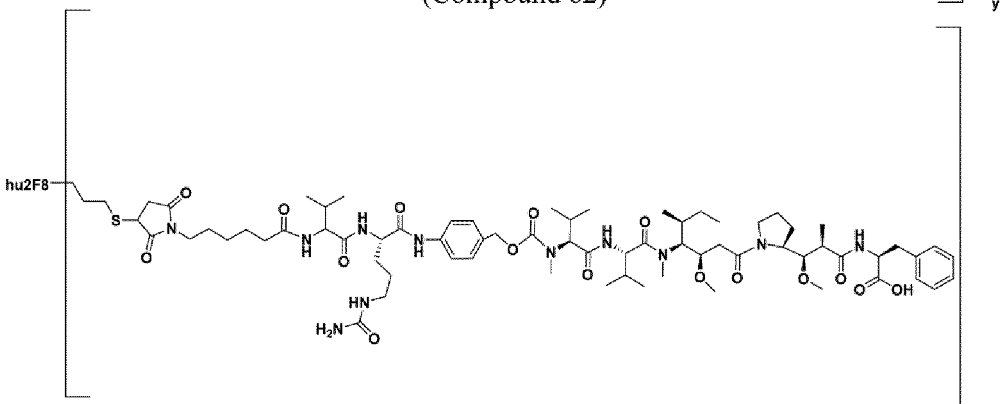

(Compound 63)

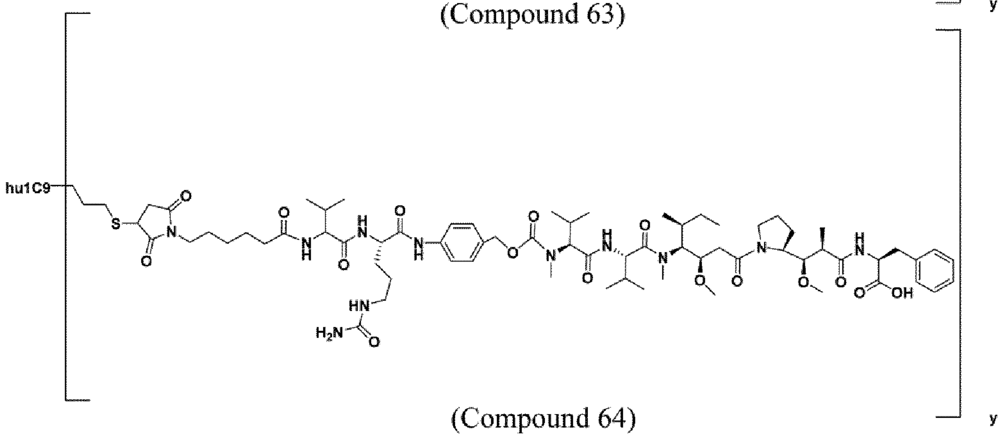

(Compound 64)

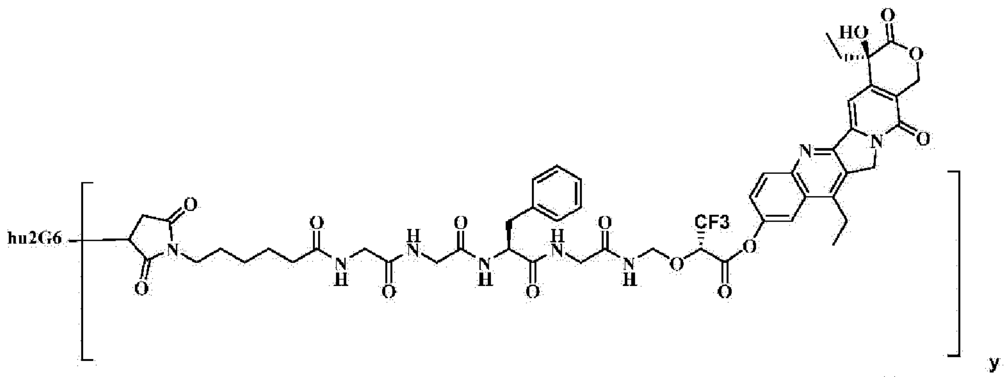

(Compound 65)

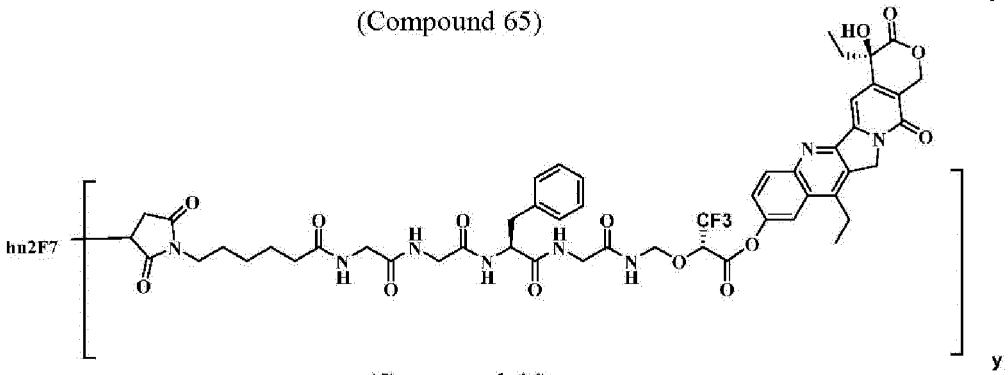

(Compound 66)

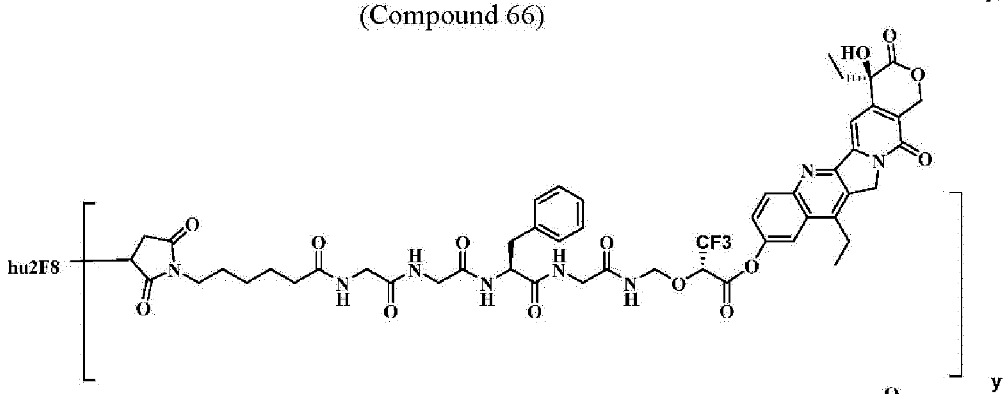

(Compound 67)

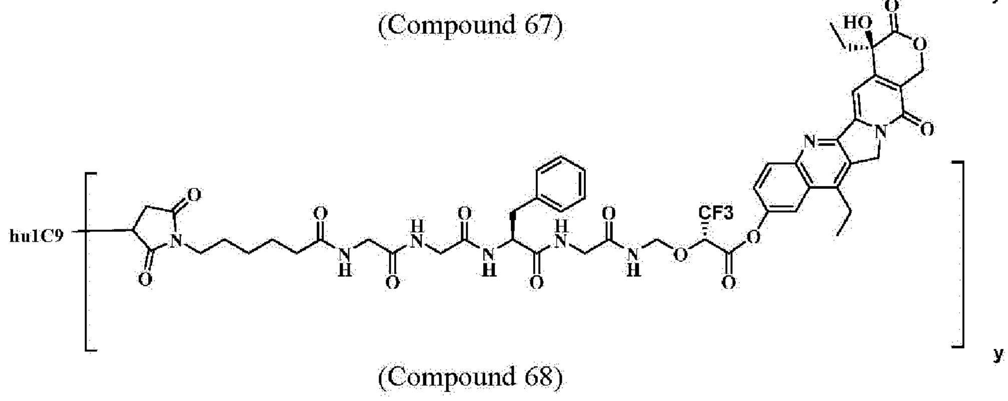

(Compound 68)

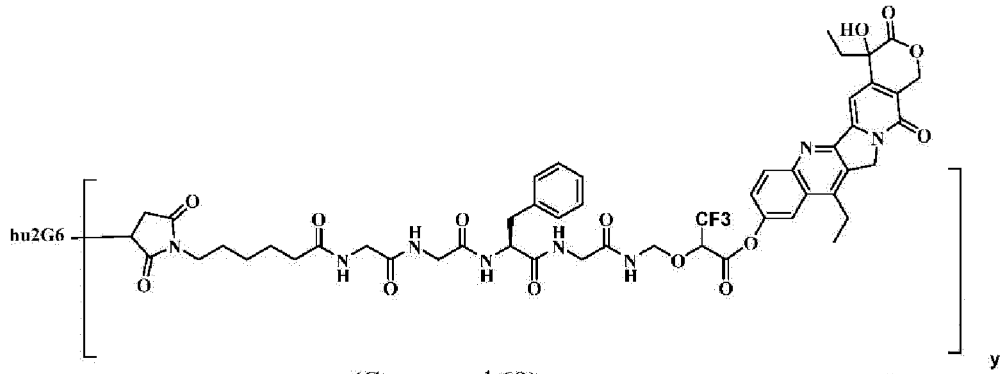

(Compound 69)

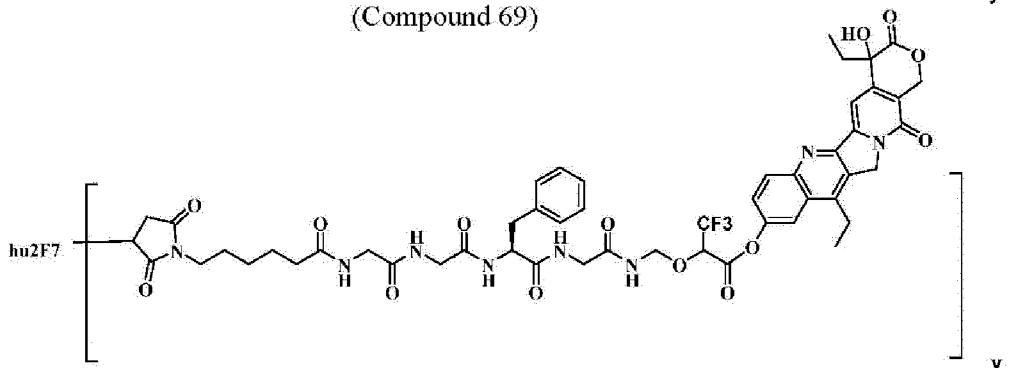

(Compound 70)

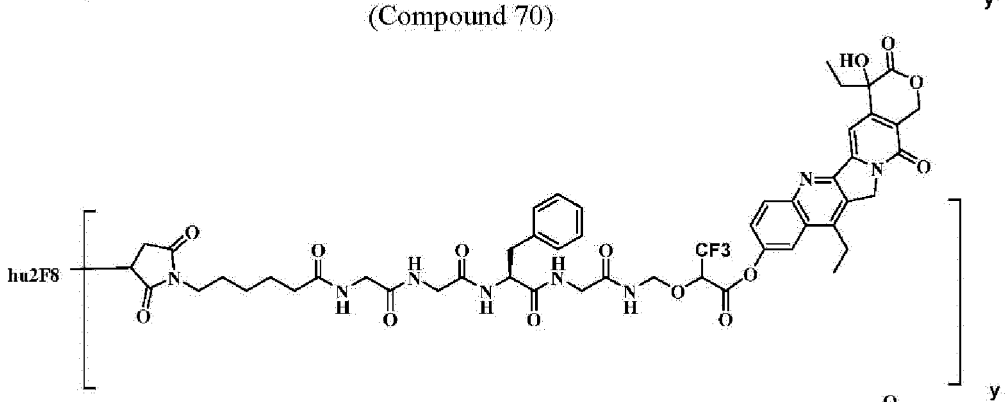

(Compound 71)

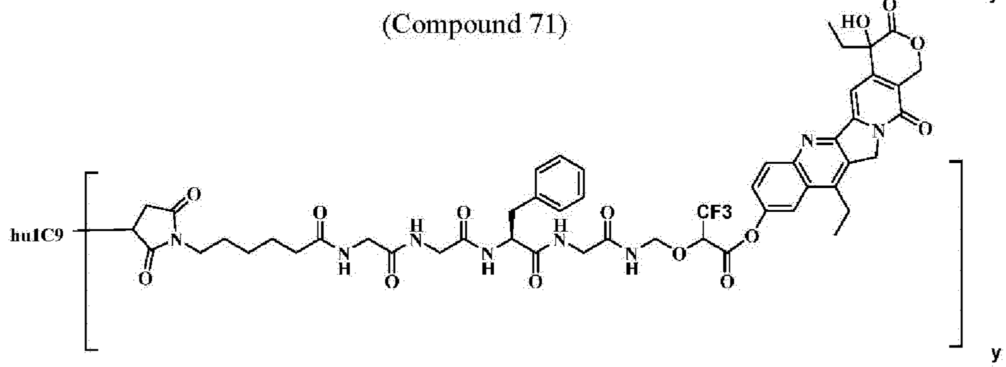

(Compound 72)

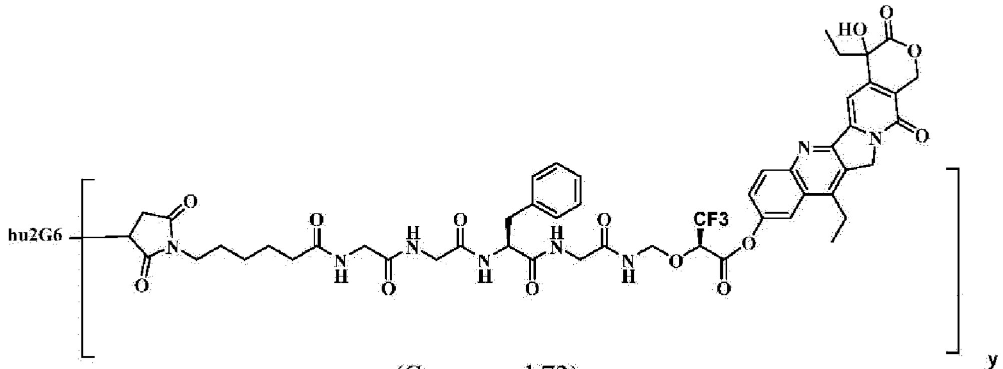

(Compound 73)

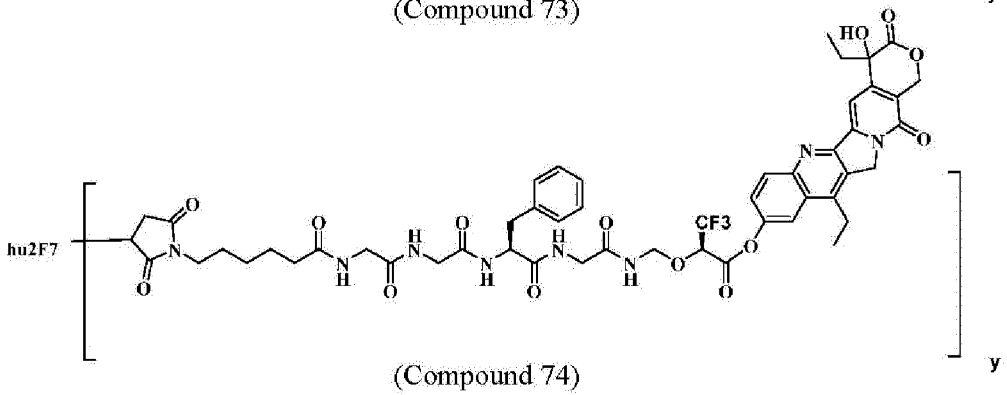

(Compound 74)

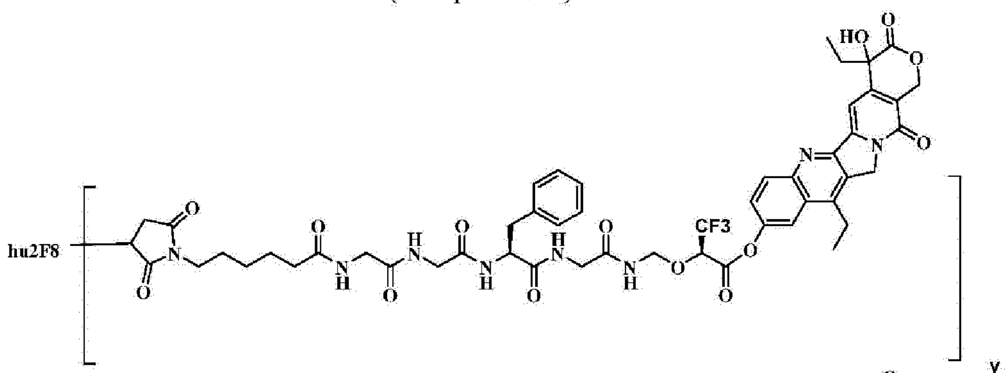

(Compound 75)

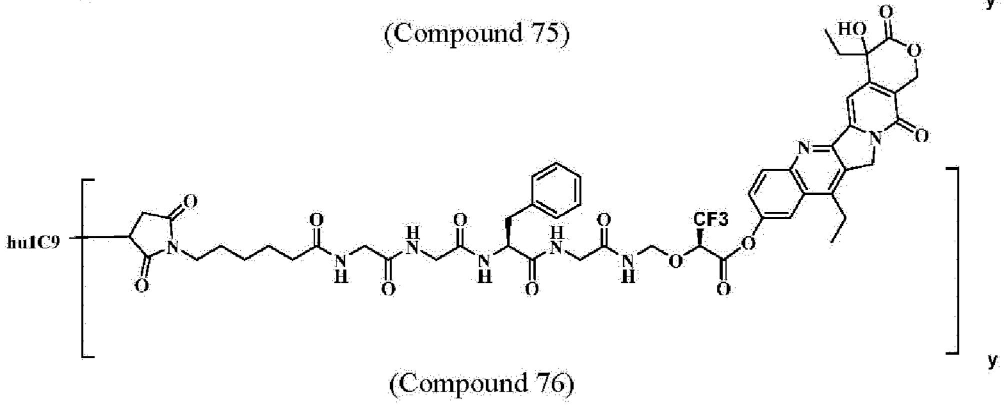

(Compound 76)

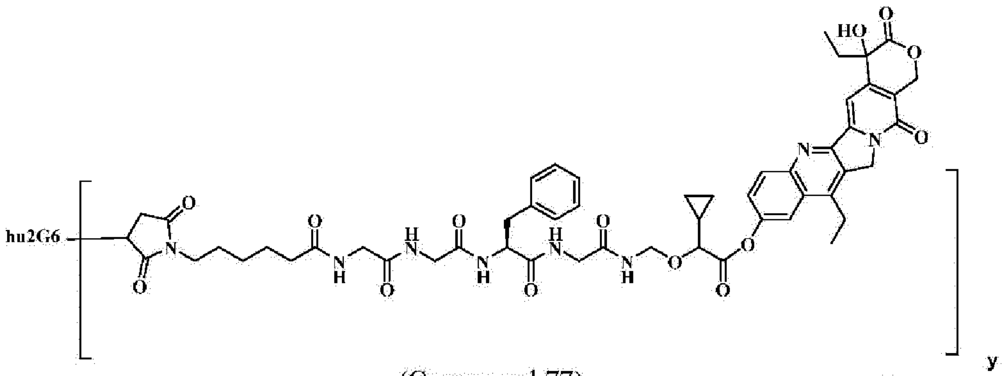

(Compound 77)

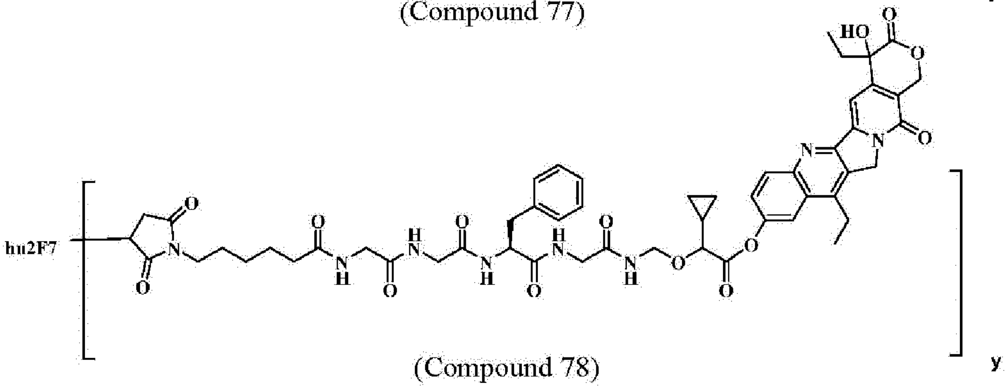

(Compound 78)

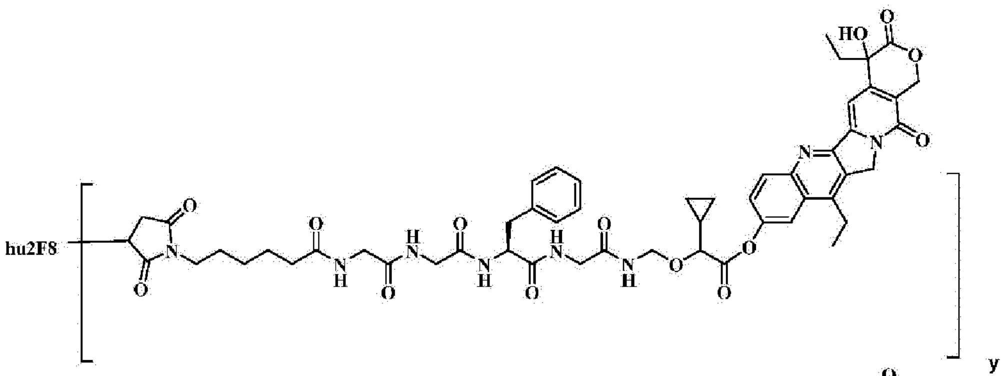

(Compound 79)

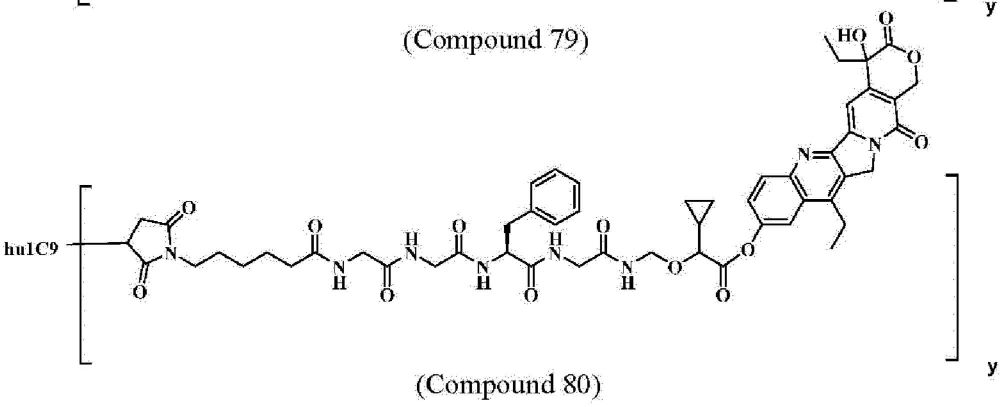

(Compound 80)

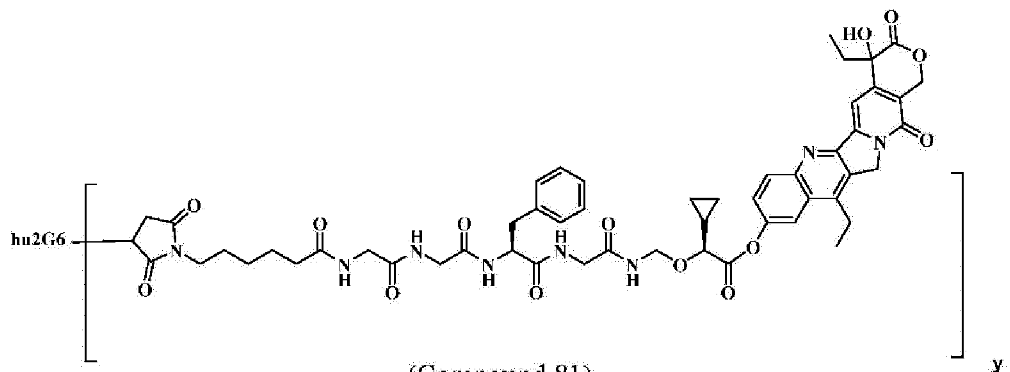

(Compound 81)

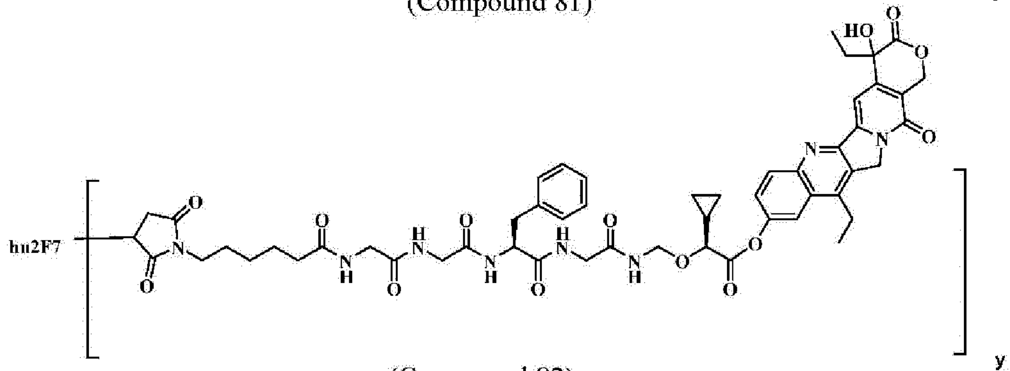

(Compound 82)

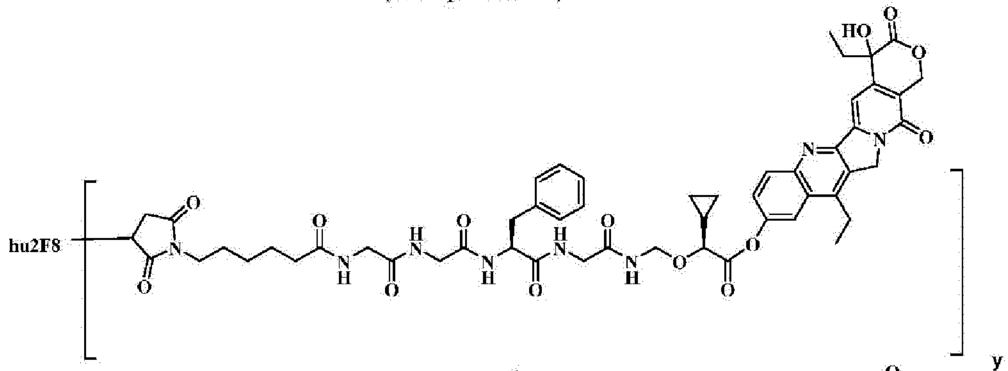

(Compound 83)

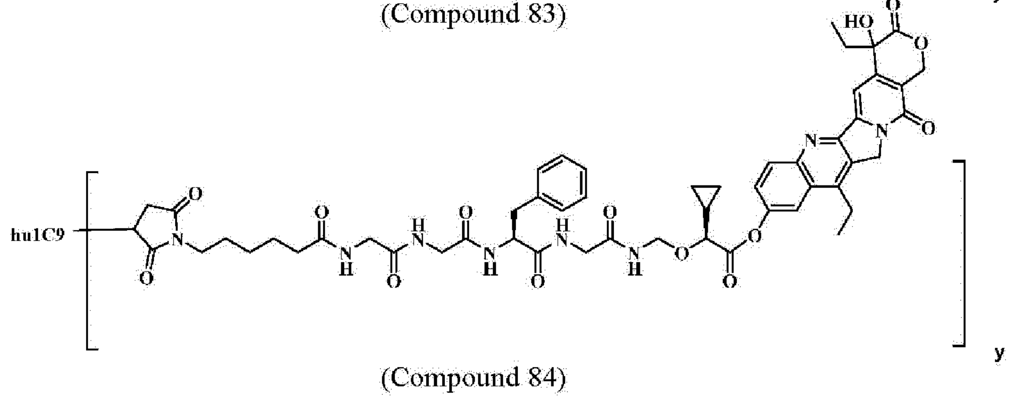

(Compound 84)

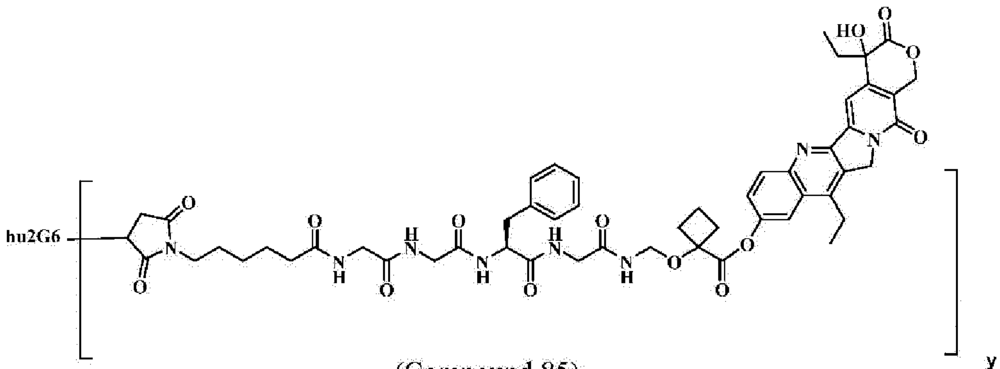

(Compound 85)

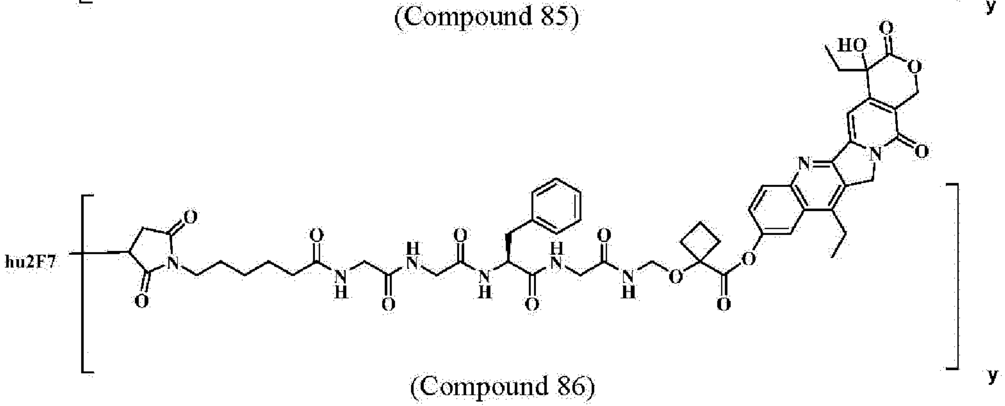

(Compound 86)

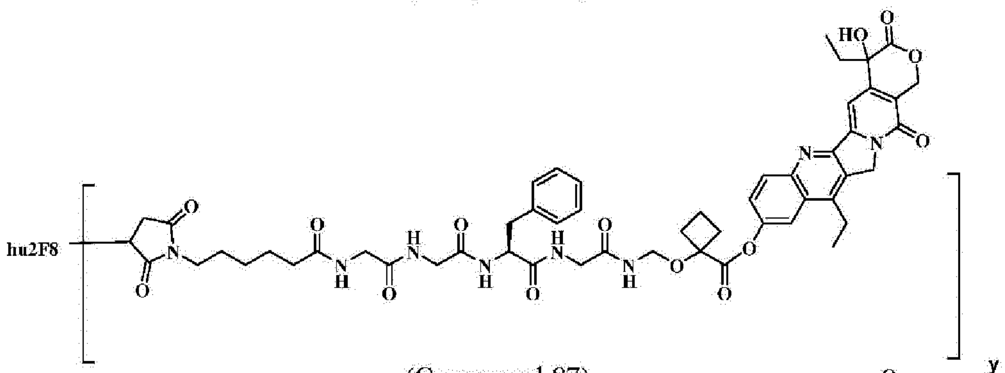

(Compound 87)

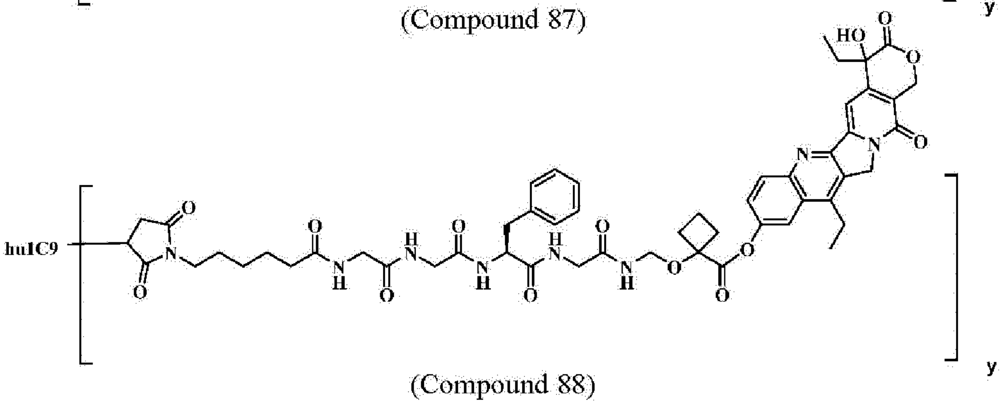

(Compound 88)

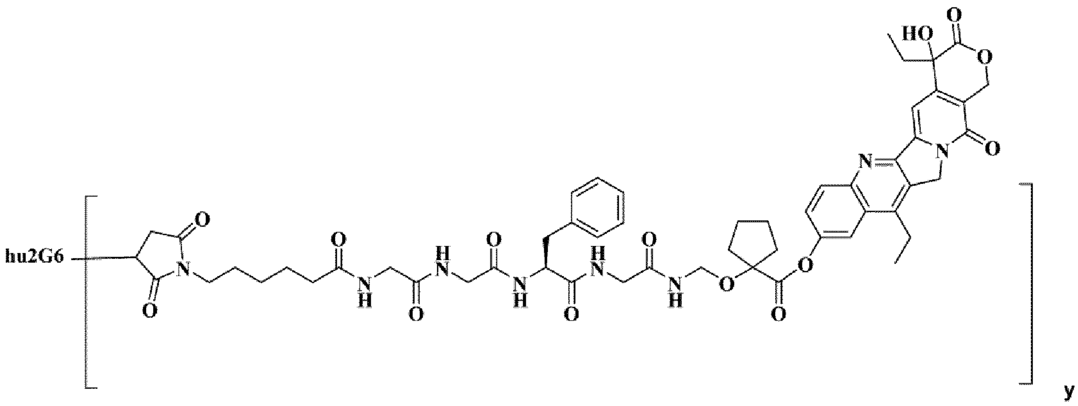

(Compound 89)

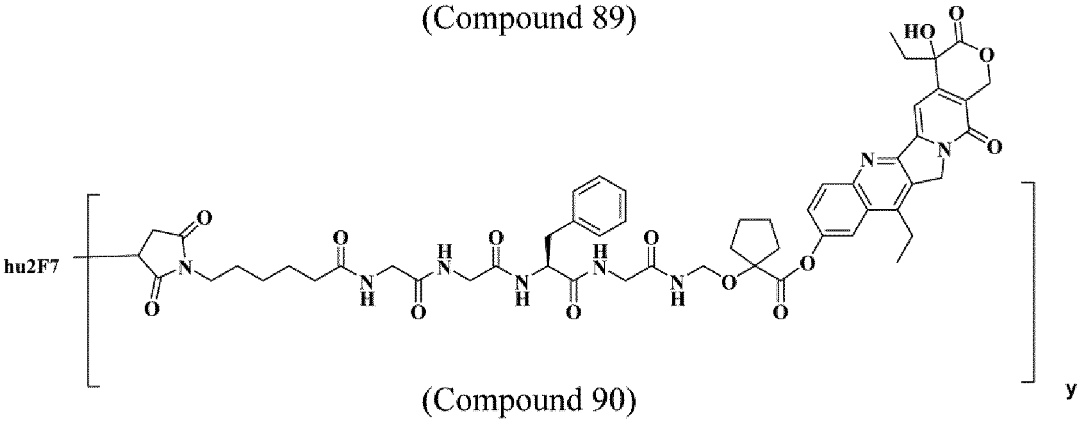

(Compound 90)

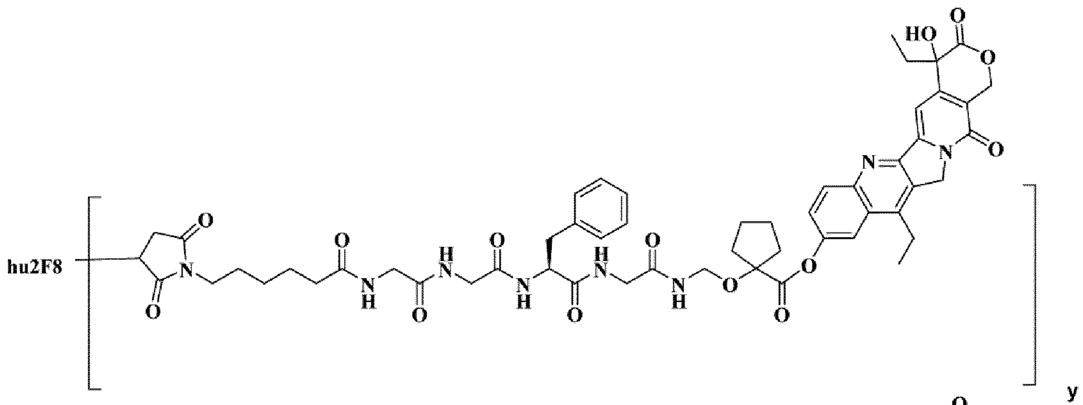

(Compound 91)

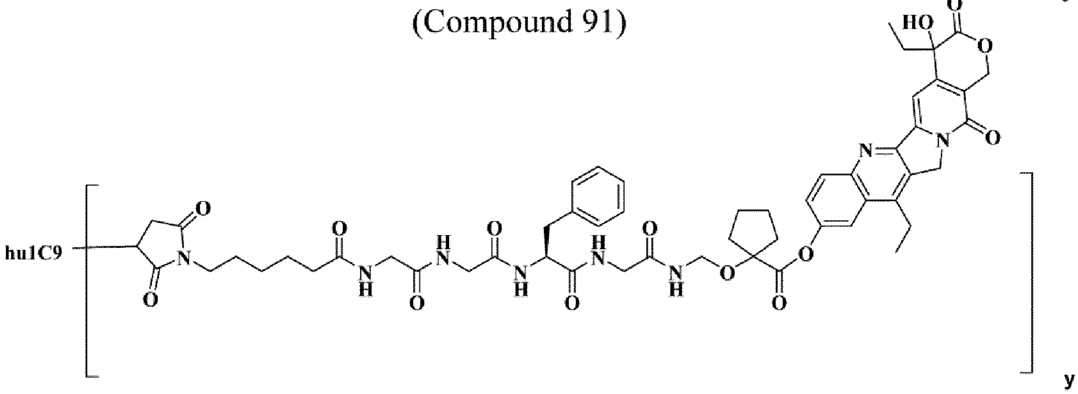

(Compound 92)

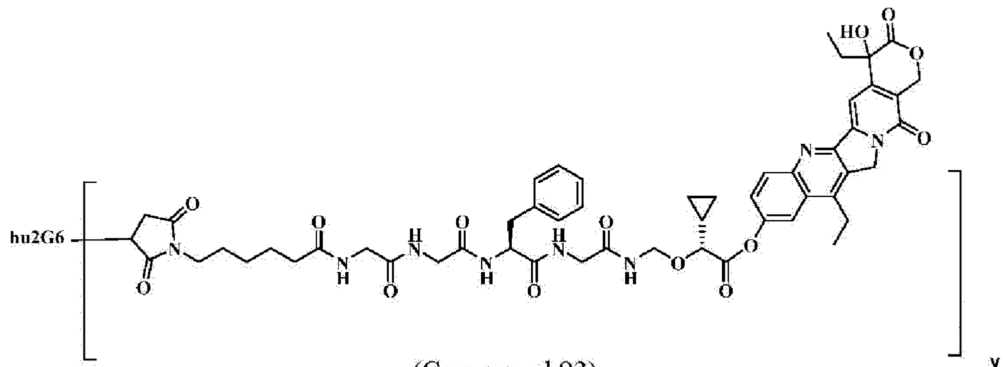

(Compound 93)

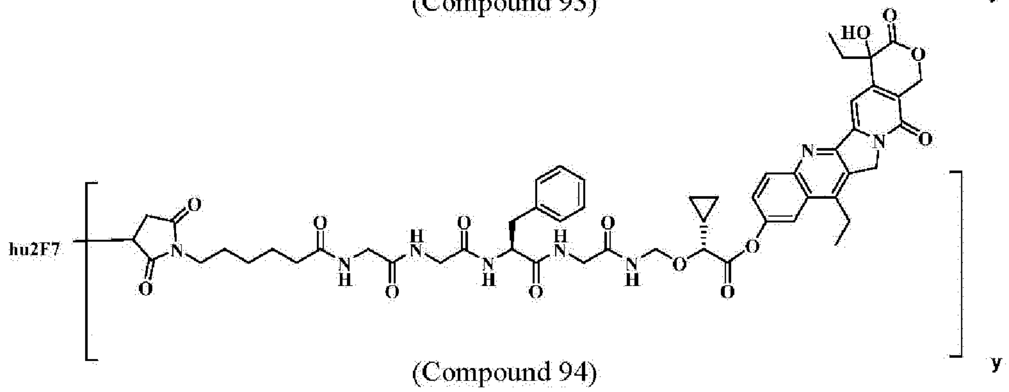

(Compound 94)

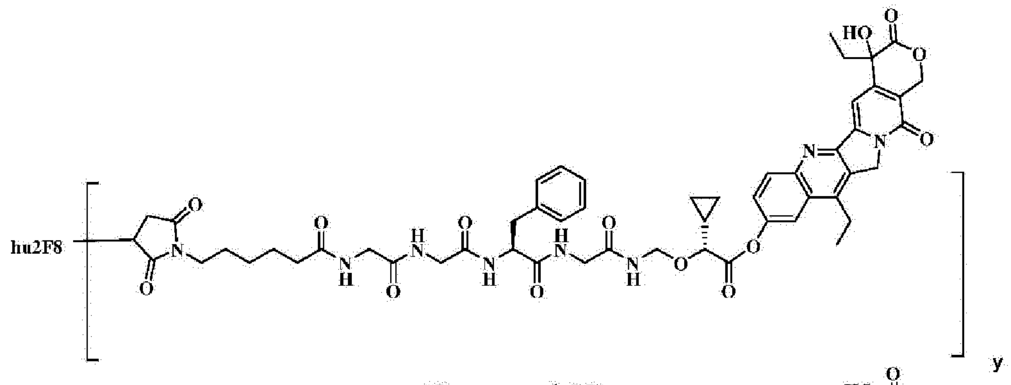

(Compound 95)

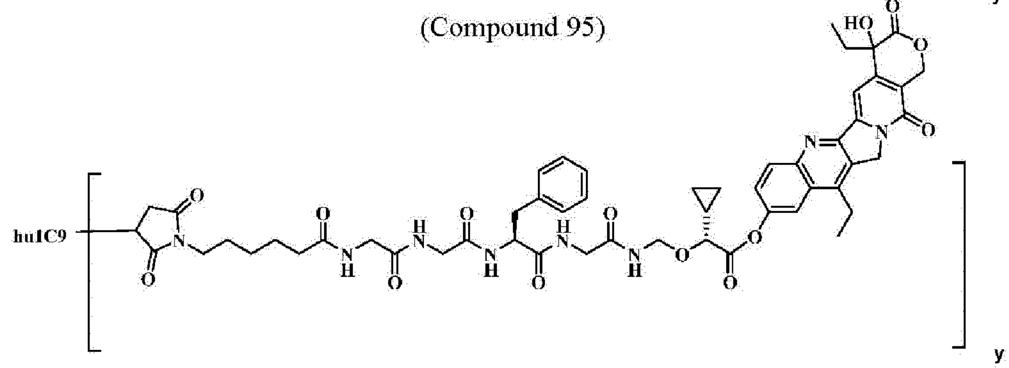

(Compound 96)

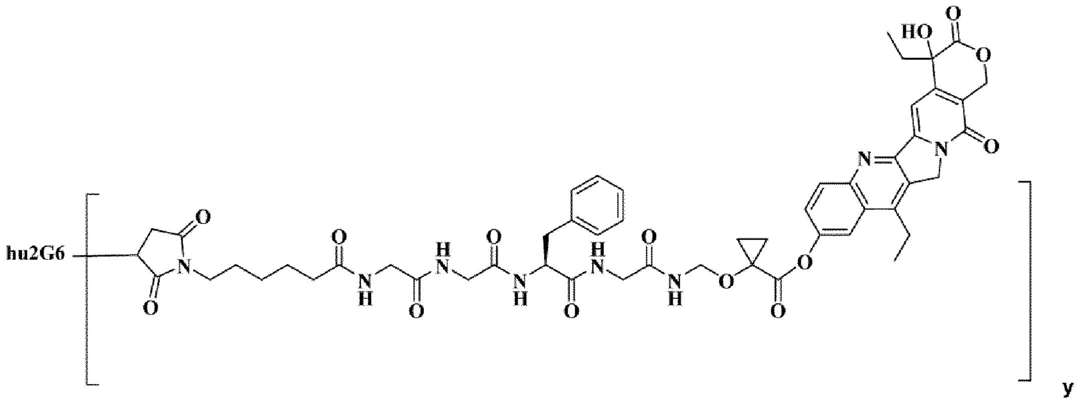

(Compound 97)

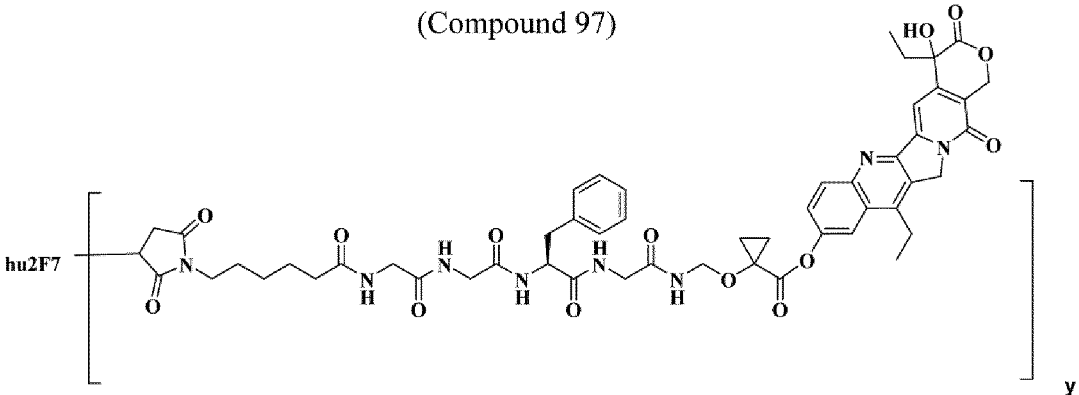

(Compound 98)

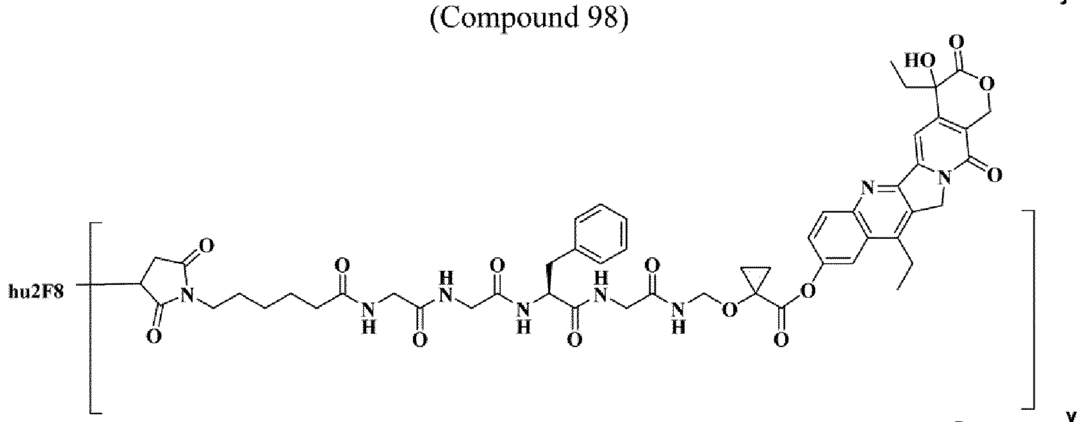

(Compound 99)

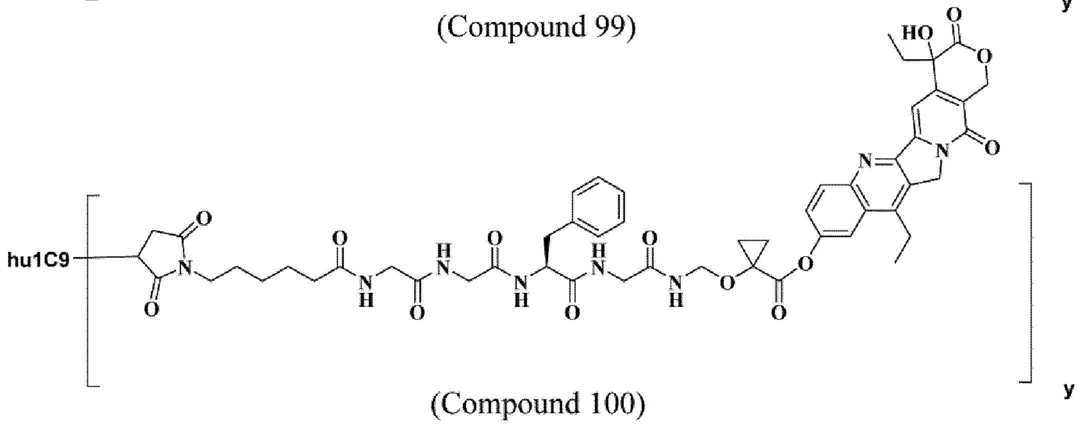

(Compound 100)

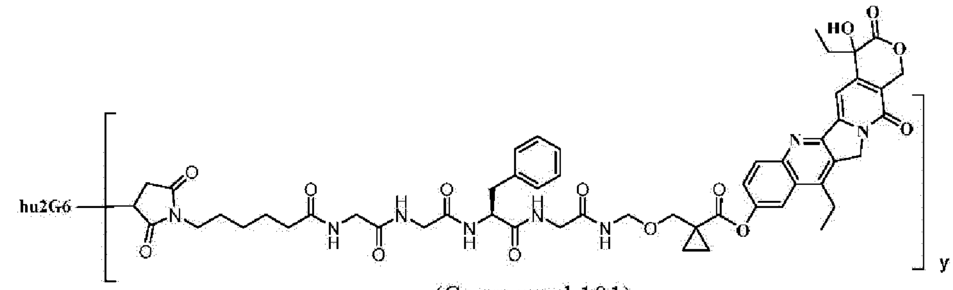

(Compound 101)

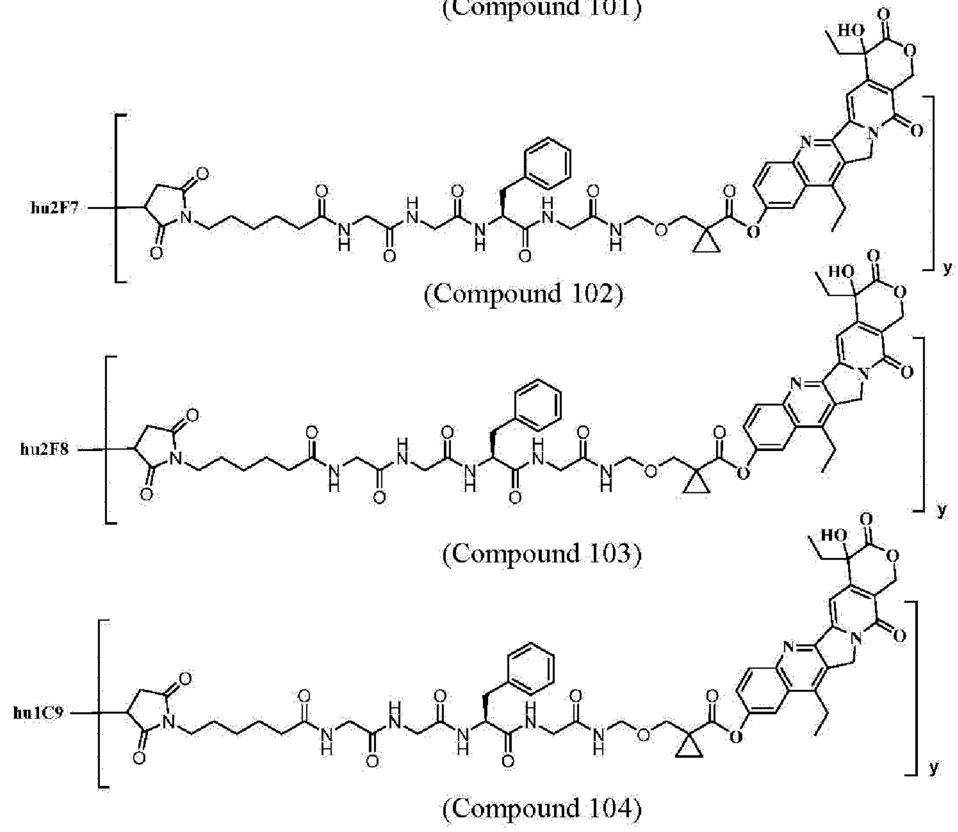

(Compound 102)

(Compound 103)

(Compound 104)

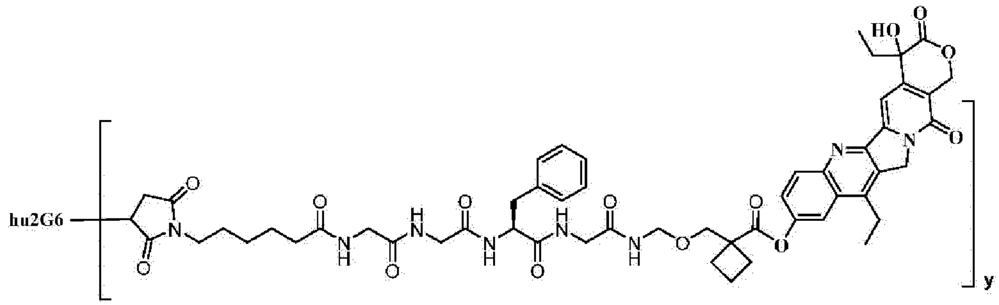

(Compound 105)

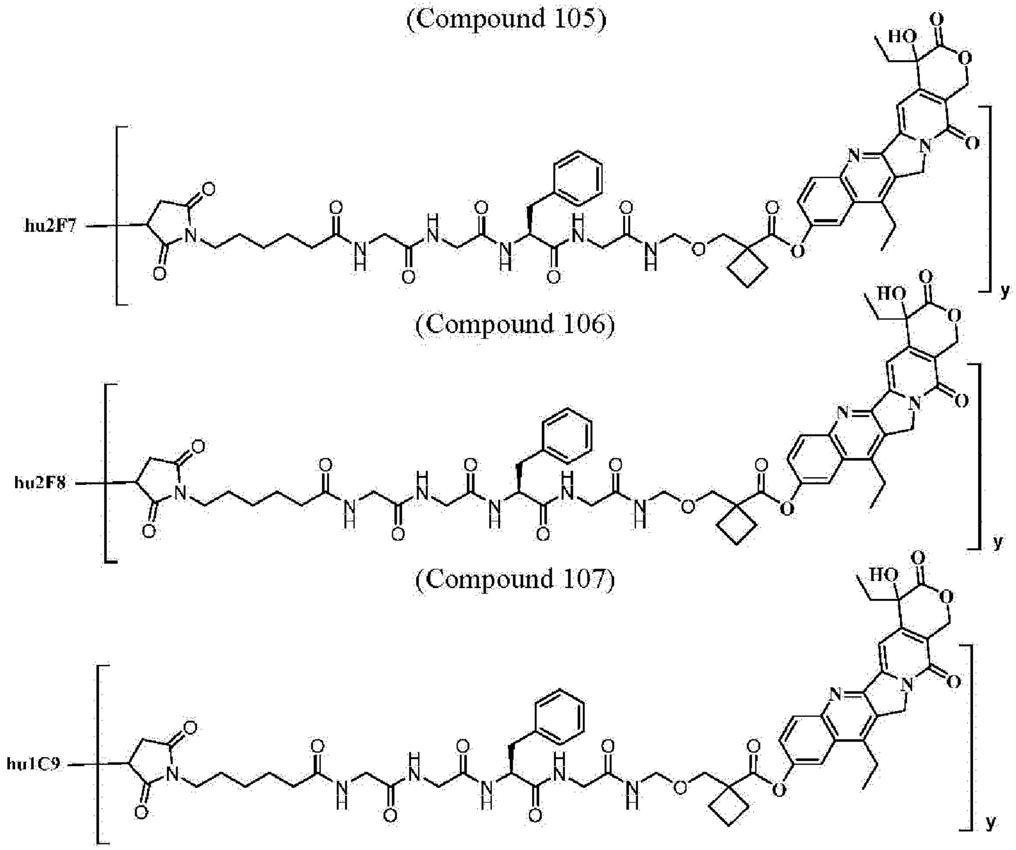

(Compound 106)

(Compound 107)

(Compound 108)

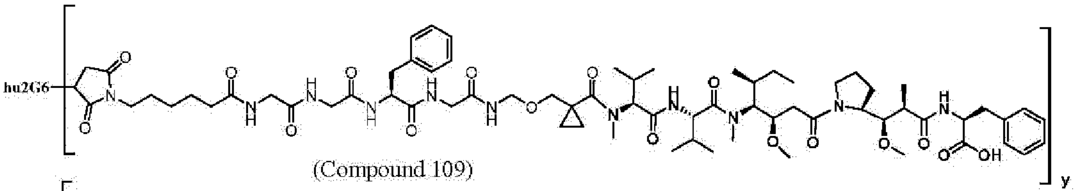

(Compound 109)

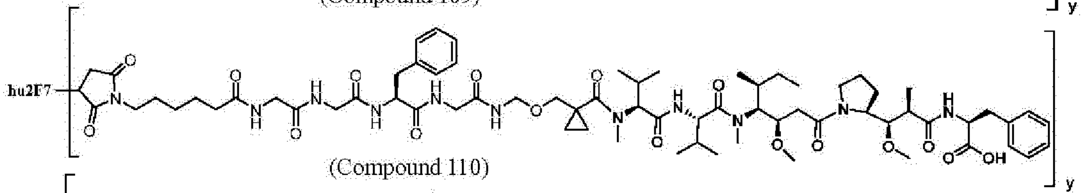

(Compound 110)

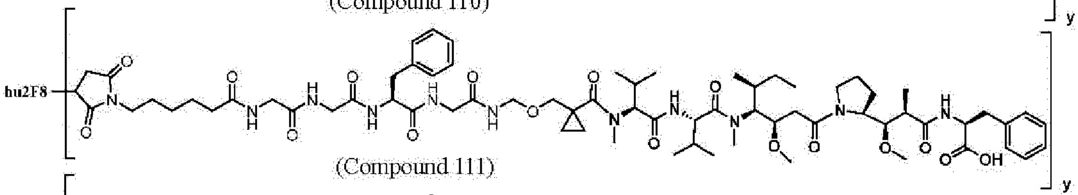

(Compound 111)

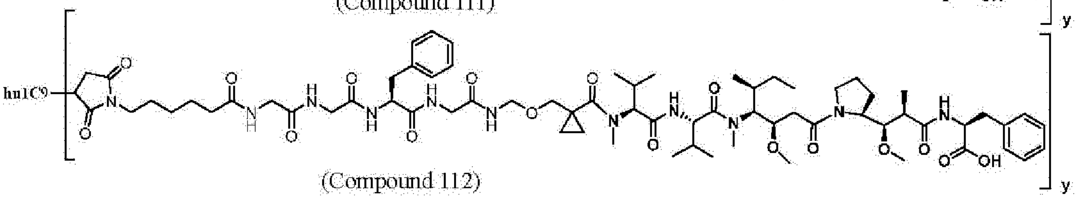

(Compound 112)

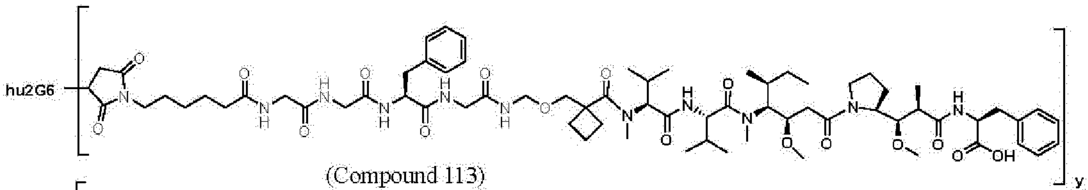

(Compound 113)

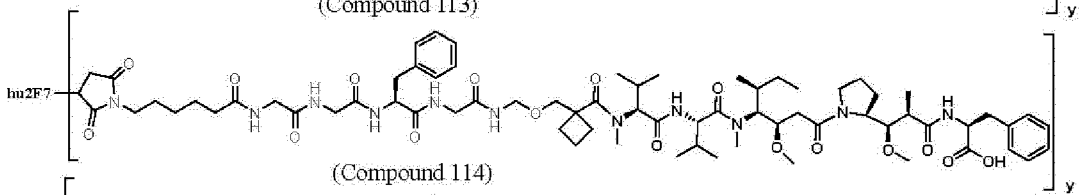

(Compound 114)

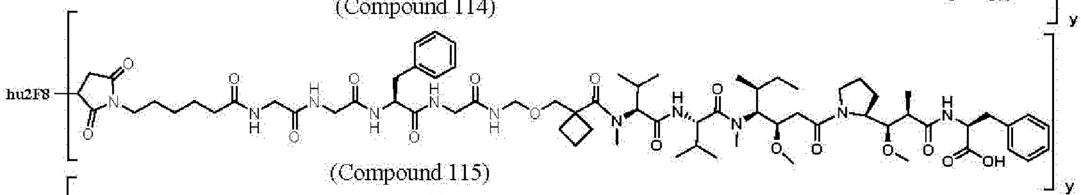

(Compound 115)

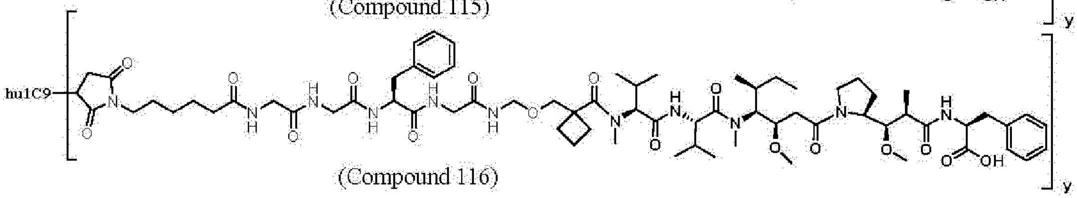

(Compound 116)

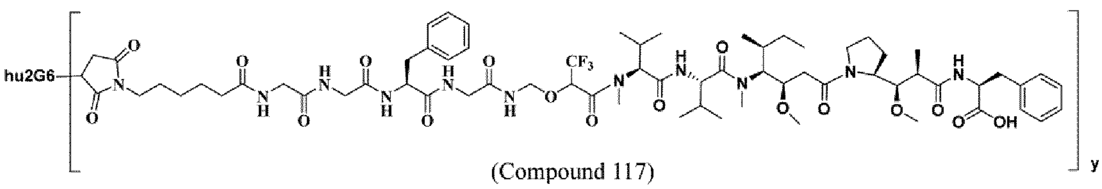

(Compound 117)

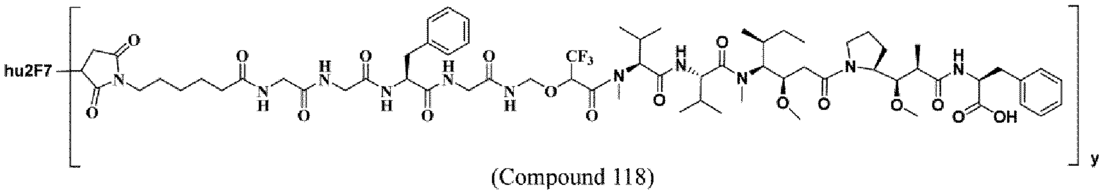

(Compound 118)

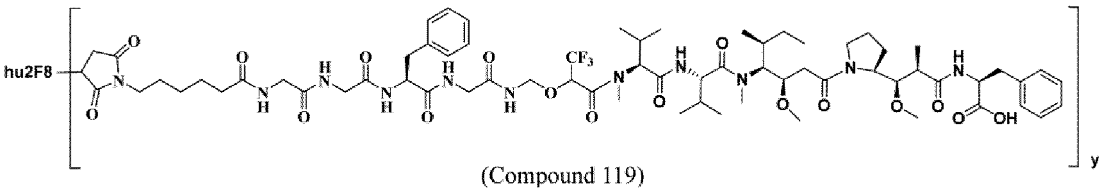

(Compound 119)

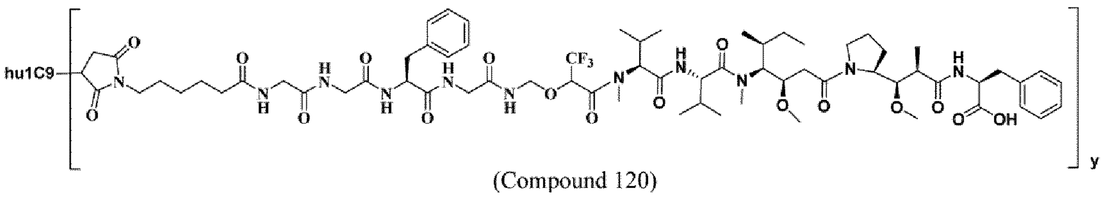

(Compound 120)

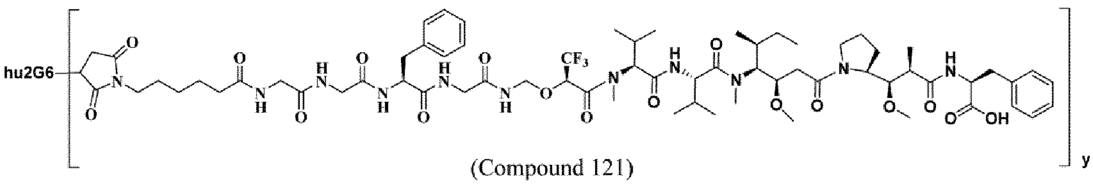

(Compound 121)

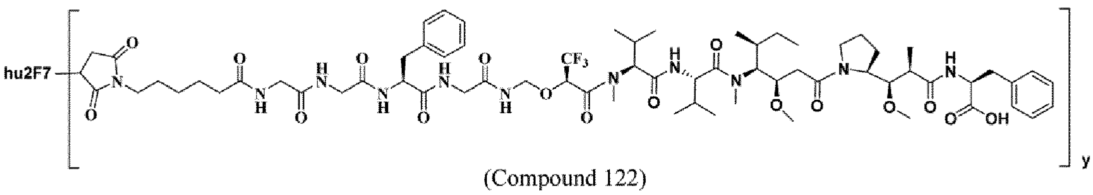

(Compound 122)

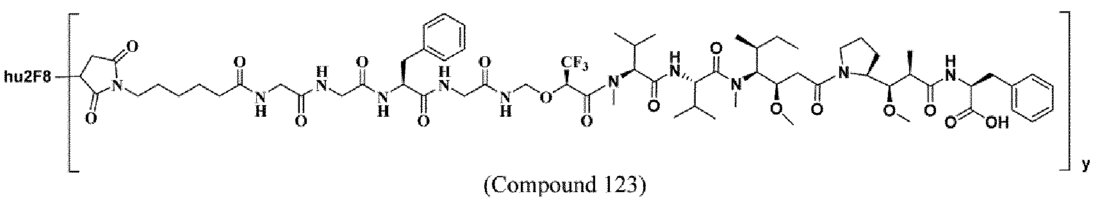

(Compound 123)

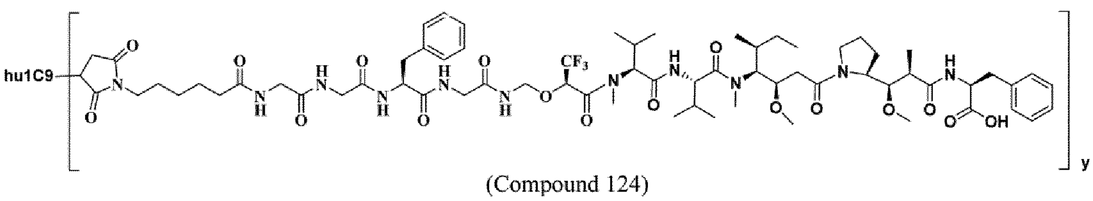

(Compound 124)

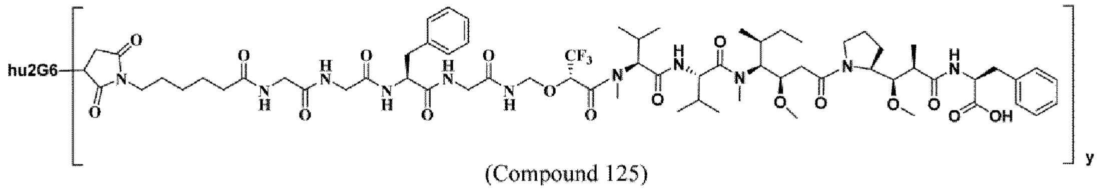

(Compound 125)

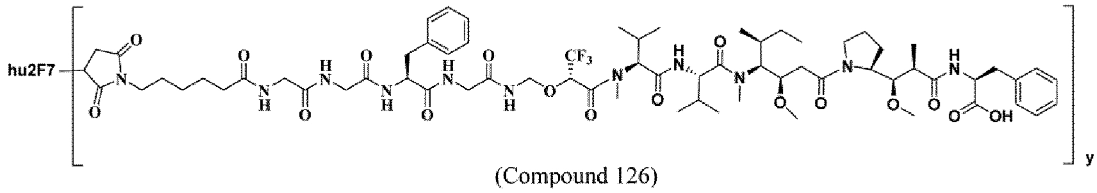

(Compound 126)

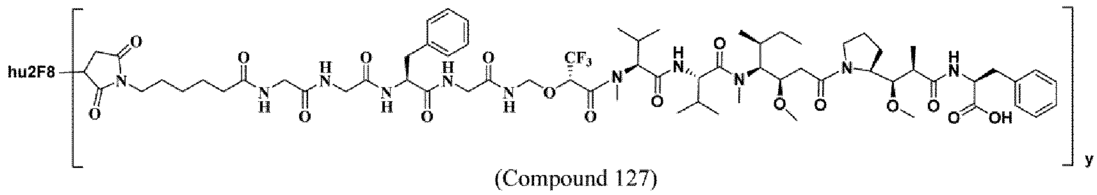

(Compound 127)

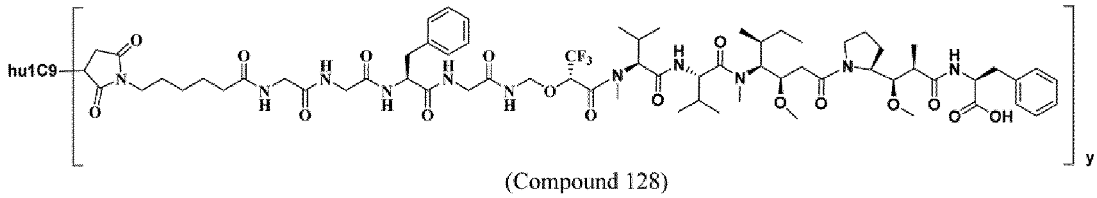

(Compound 128)

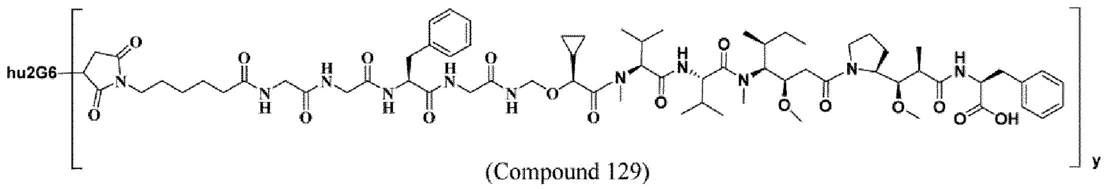

(Compound 129)

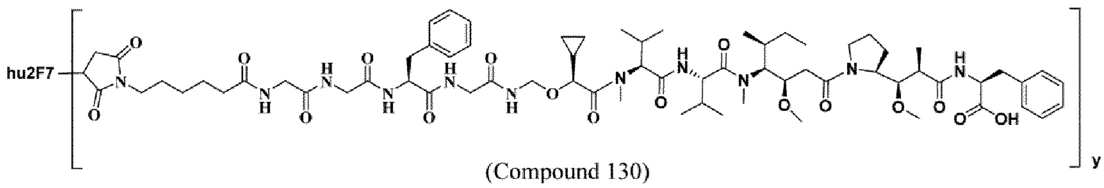

(Compound 130)

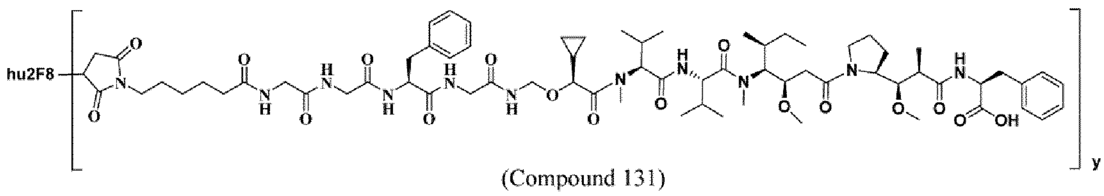

(Compound 131)

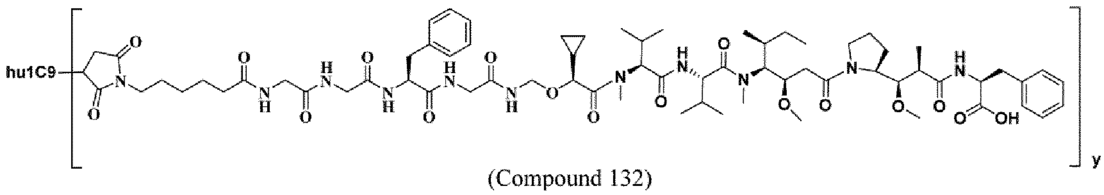

(Compound 132)

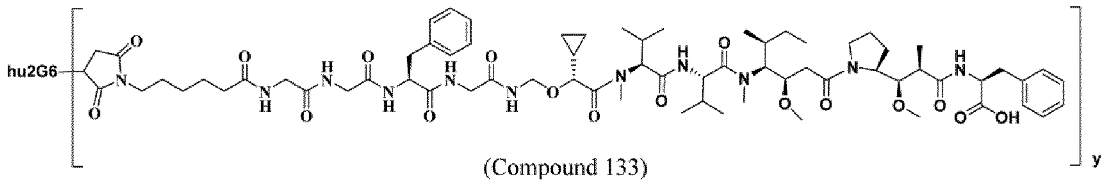

(Compound 133)

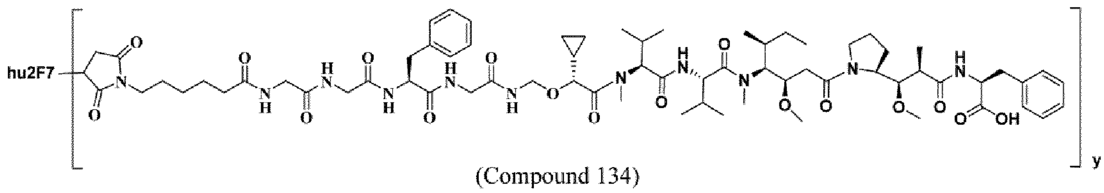

(Compound 134)

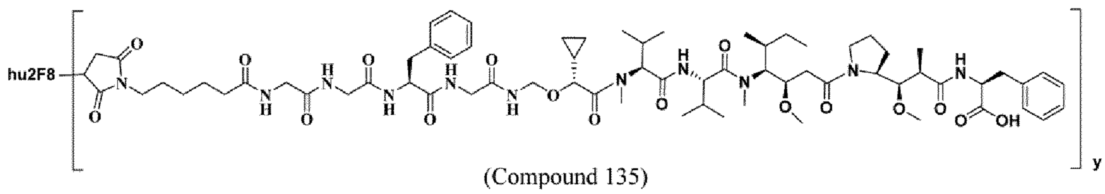

(Compound 135)

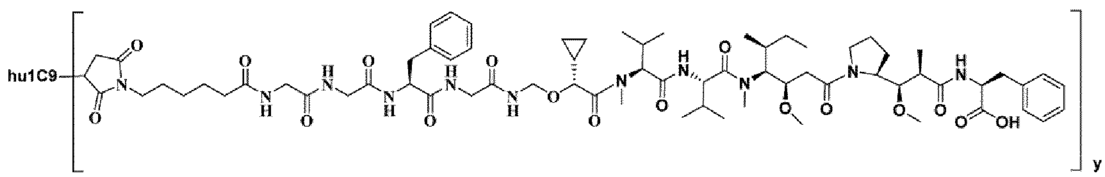

(Compound 136)

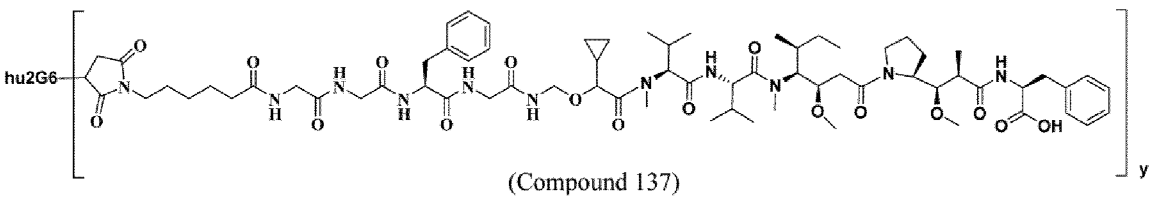

(Compound 137)

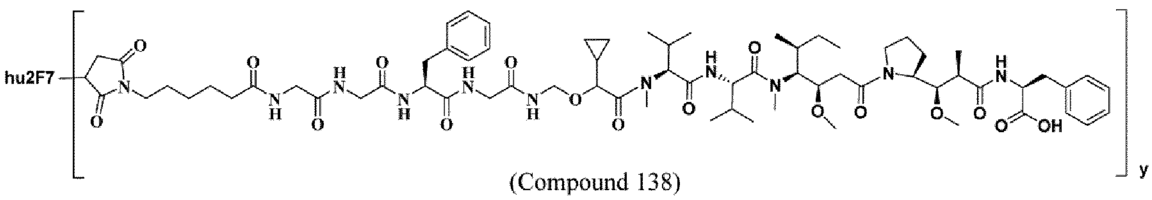

(Compound 138)

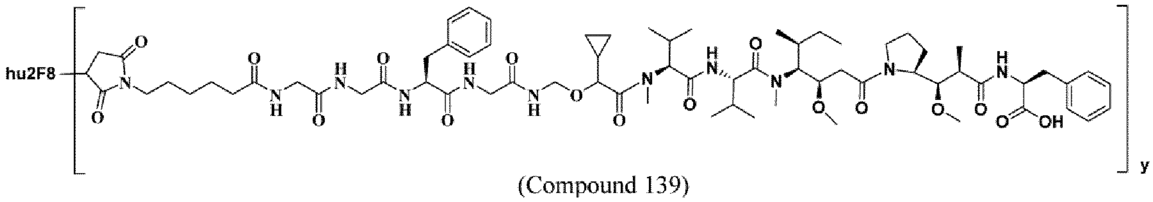

(Compound 139)

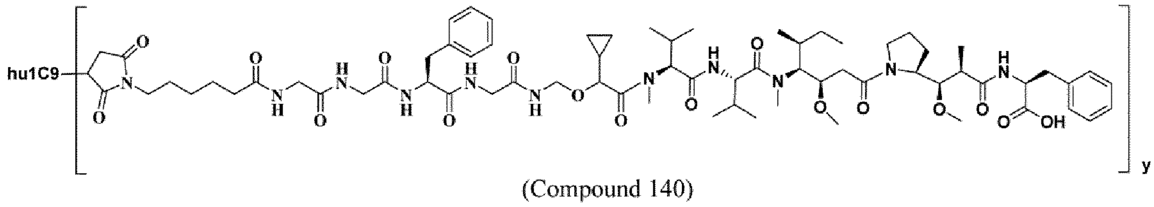

(Compound 140)

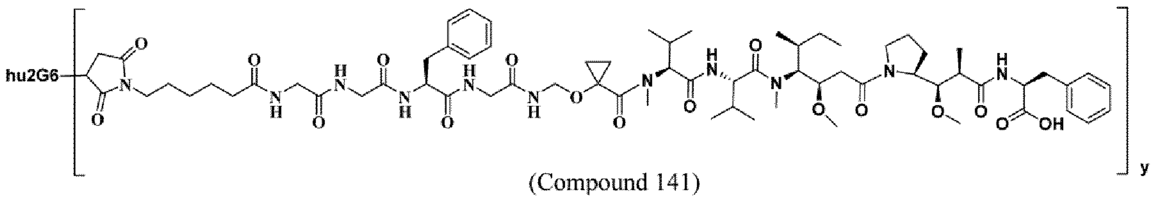

(Compound 141)

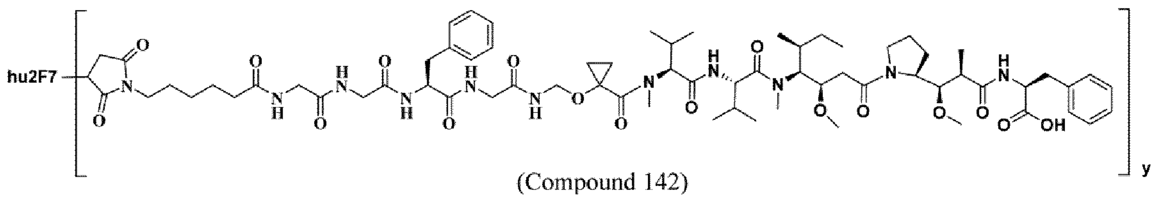

(Compound 142)

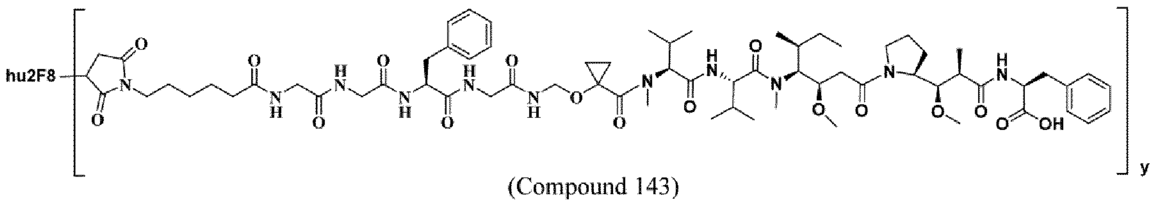

(Compound 143)

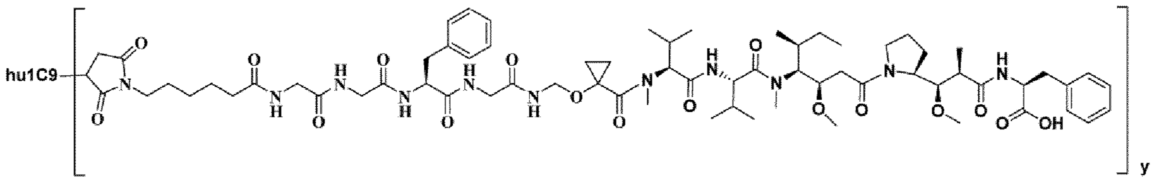

(Compound 144)

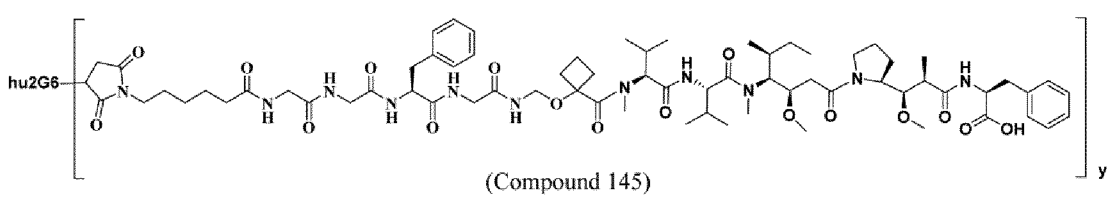

(Compound 145)

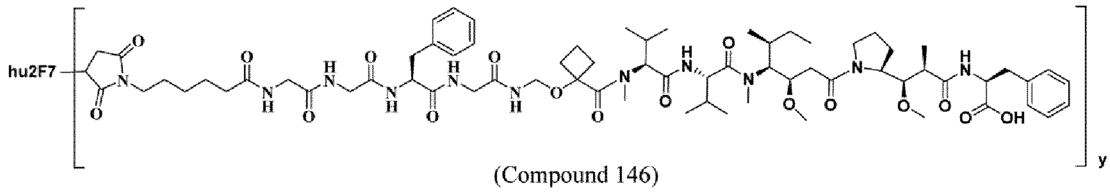

(Compound 146)

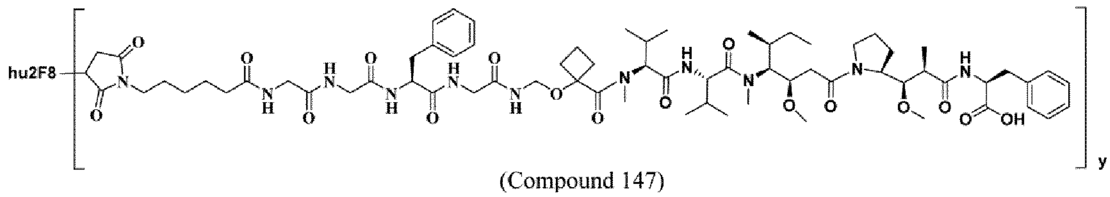

(Compound 147)

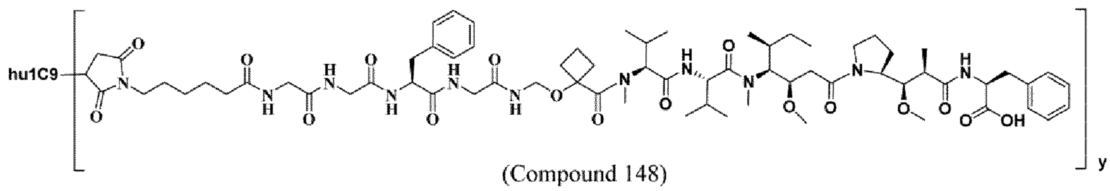

(Compound 148)

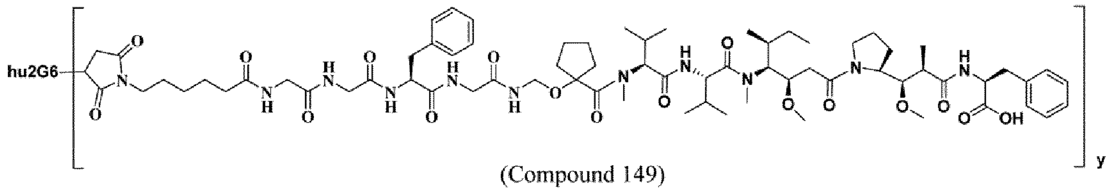

(Compound 149)

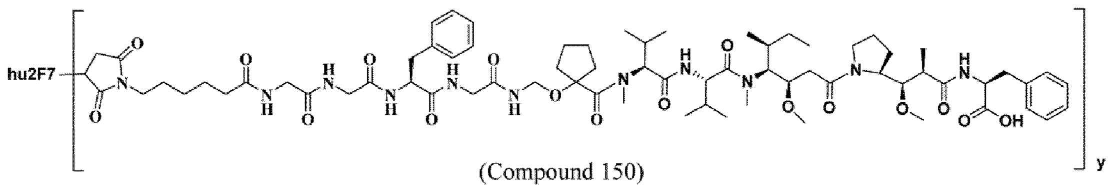

(Compound 150)

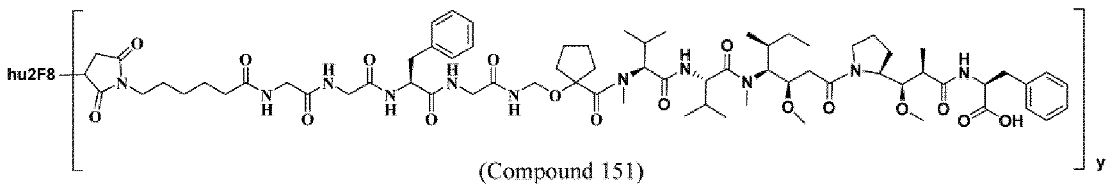

(Compound 151)

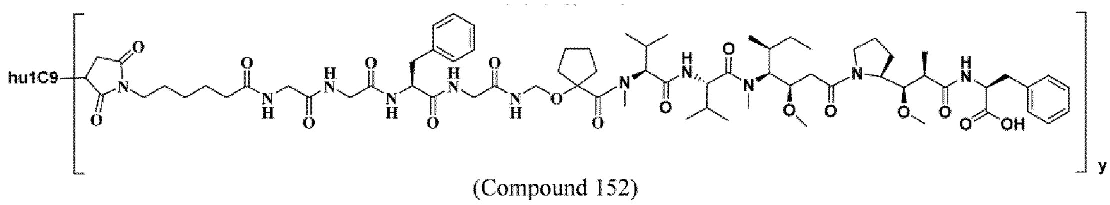

(Compound 152)

wherein, y is a number selected from 1 to 10, preferably a number selected from 2 to 10, further, a number selected from 6 to 10, more preferably a number of 7 to 9, and most preferably an integer of 7, 8, 9; or, y is a number selected from 2 to 10, preferably a number of 4 to 8, more preferably a number of 6 to 8, further preferably a number of 7 to 8,

and most preferably 8.

In a preferred embodiment, the present invention relates to method for preparing the antibody-drug conjugate of general formula (IV) or a pharmaceutically acceptable salt or solvate thereof, which comprises the following steps:

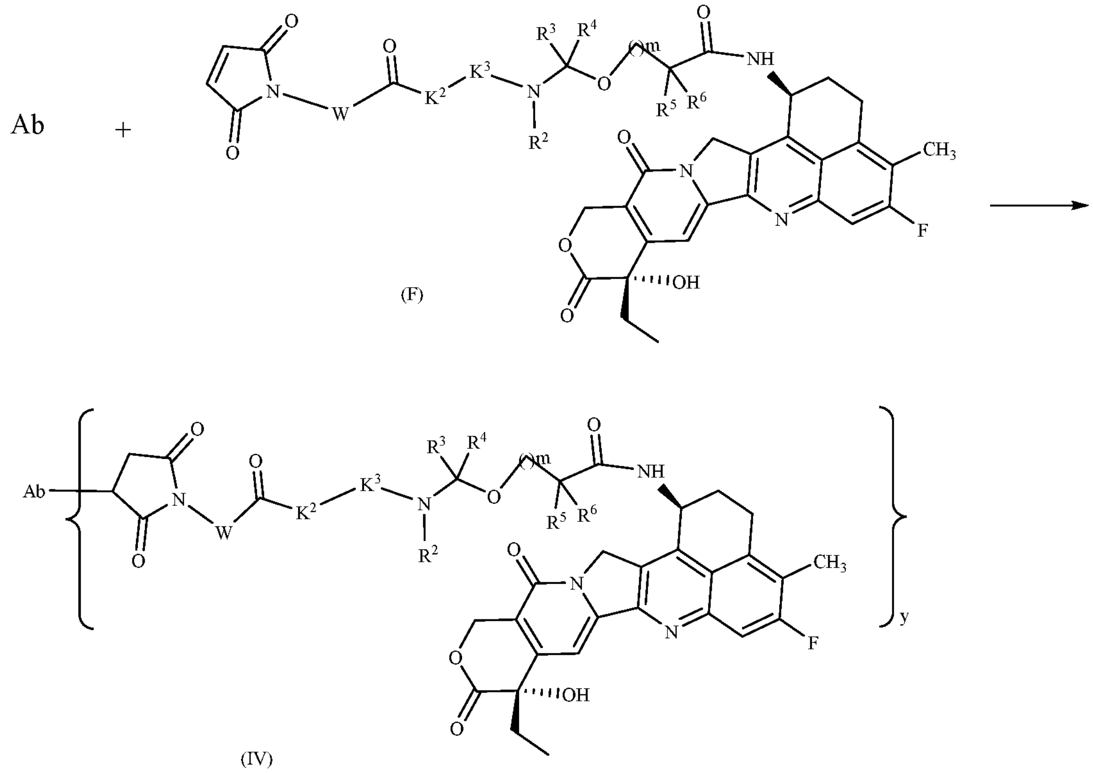

(F)

(IV)

after Ab is reduced, it is subjected to coupling reaction with general formula (F) to obtain the compound of general formula (IV);

wherein:

Ab is an anti-B7H4 antibody or antigen-binding fragment thereof;

W, $K^2$, $K^3$, $R^2$ to $R^6$, m and y are as defined in general formula (IV).

In a preferred embodiment, the general formula (F) is a compound of general formula (F-1):

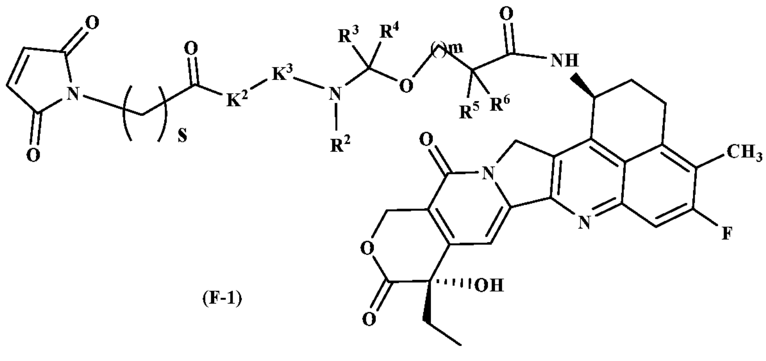

(F-1)

or a tautomer, mesomer, racemate, enantiomer, diastereomer or mixture form thereof, or a pharmaceutically acceptable salt thereof,

wherein

$K^2$, $K^3$, $R^2$ to $R^6$, s and m are as defined in the above $-L_2-L_1-$.

In a preferred embodiment, the compound of general formula (F) or general formula (F-1) is selected from the

group consisting of:

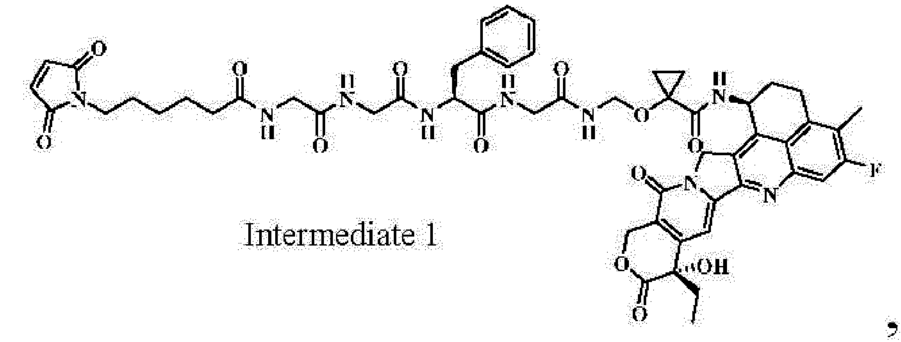

Intermediate 1

,

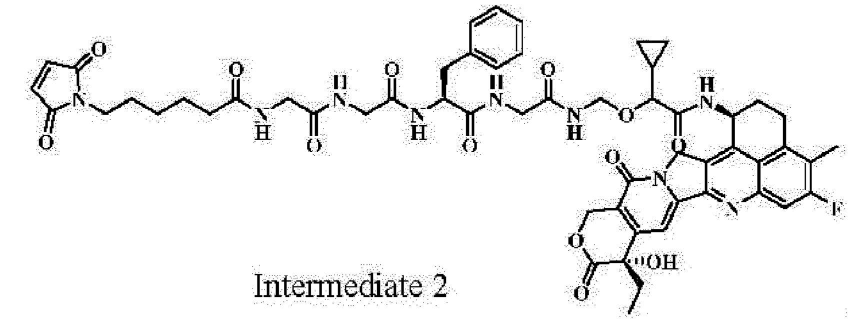

Intermediate 2

,

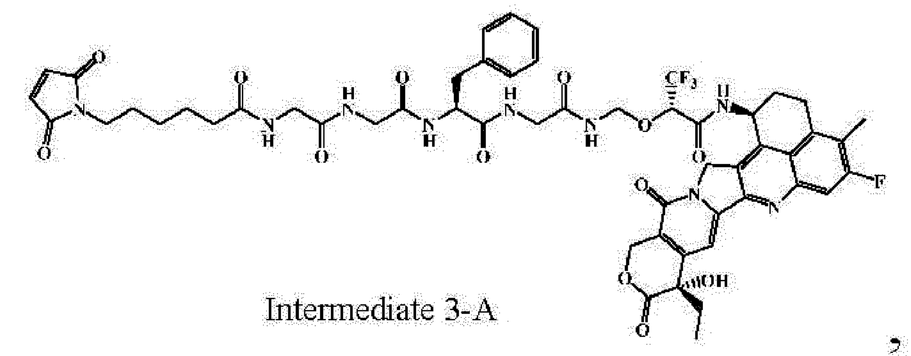

Intermediate 3-A

,

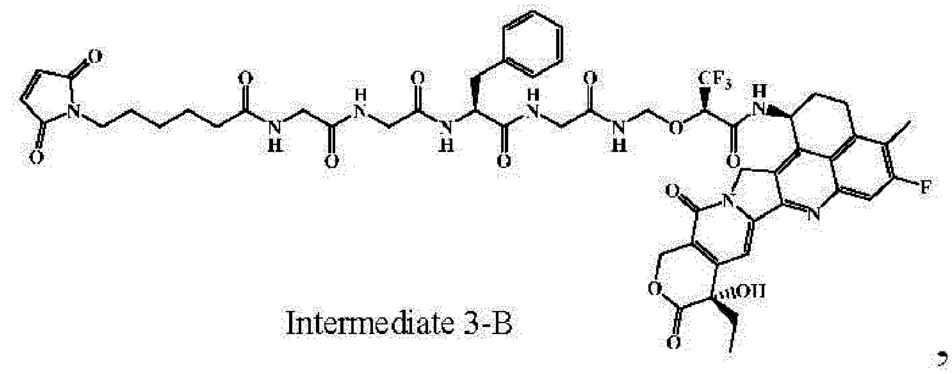

Intermediate 3-B

,

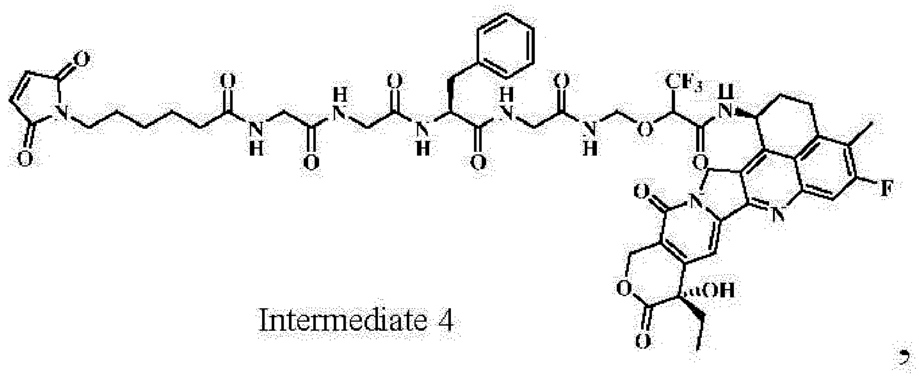

Intermediate 4

,

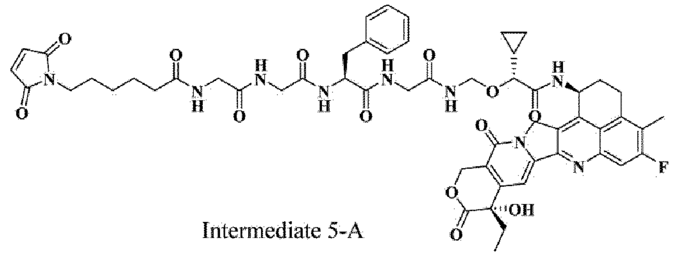

Intermediate 5-A

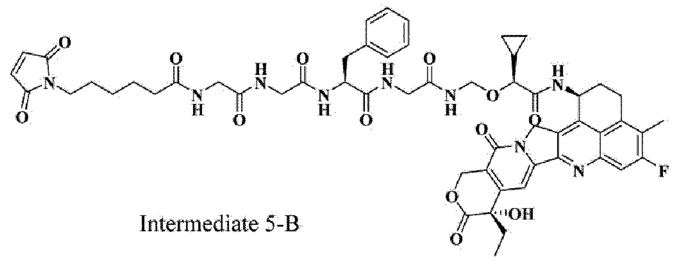

Intermediate 5-B

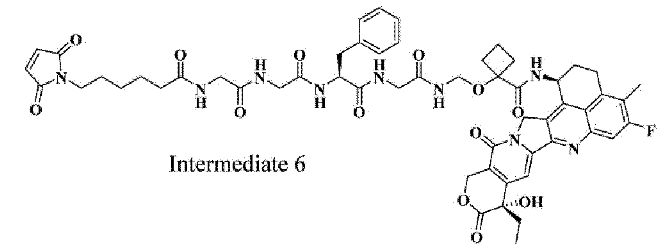

Intermediate 6

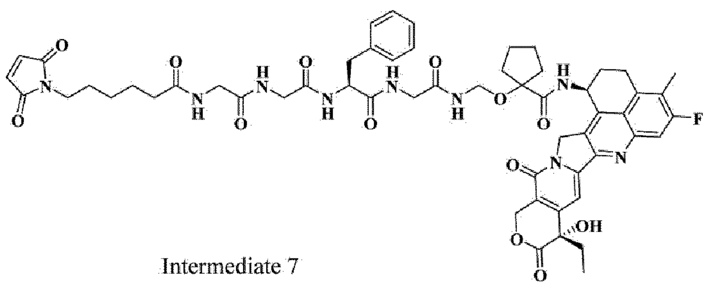

Intermediate 7

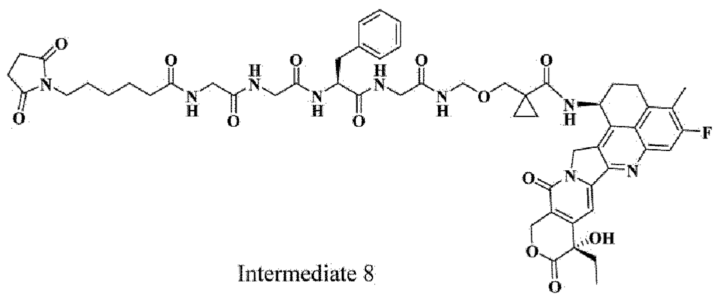

Intermediate 8

and

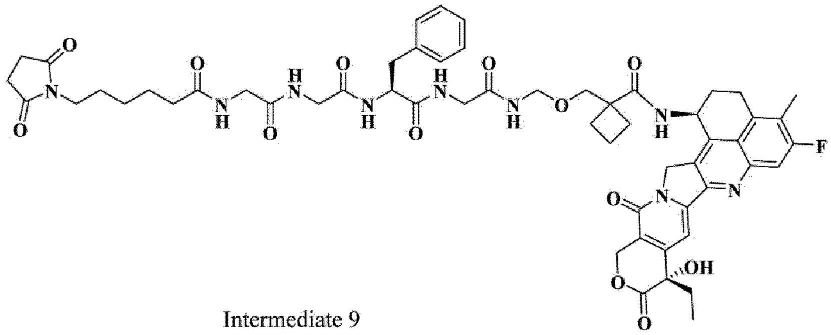

Intermediate 9

.

In a preferred embodiment, the present invention relates to a pharmaceutical composition, which comprises the antibody-drug conjugate according to any one of the present invention, or a pharmaceutically acceptable salt or solvate thereof, and one or more pharmaceutically acceptable excipients, diluents or carriers.

In a preferred embodiment, the present invention relates to use of the antibody-drug conjugate of general formula (A), or a pharmaceutically acceptable salt or solvate thereof, or a pharmaceutical composition thereof, in preparing a medicament for treating a disease related to human B7-H4, preferably a medicament for treating a cancer with high B7-H4 expression.

In a more preferred embodiment, the cancer is selected from the group consisting of astroblastoma of human brain, human pharyngeal cancer, adrenal tumor, AIDS- related cancer, alveolar soft-part sarcoma, astrocytoma, bladder cancer, bone cancer, brain and spinal cord cancer, metastatic brain tumor, breast cancer, carotid body tumor, cervical cancer, chondrosarcoma, chordoma, chromophobe cell carcinoma of kidney, clear cell carcinoma, colon cancer, colorectal cancer, connective tissue proliferative small round cell tumor, ependymoma, Ewing's sarcoma, extraosseous mucoid chondrosarcoma, fibrogenesis imperfecta ossium of bone, fibrous dysplasia of bone, gallbladder or cholangiocarcinoma, gastric cancer, gestational trophoblastic disease, germ cell tumor, head and neck cancer, hepatocellular carcinoma, islet cell tumor, Kaposi's sarcoma, kidney cancer, leukemia, liposarcoma/malignant lipomatous tumor, liver cancer, lymphoma, lung cancer, medulloblastoma, melanoma, meningioma, multiple endocrine neoplasia, multiple myeloma, myelodysplastic syndrome, neuroblastoma, neuroendocrine tumor, ovarian cancer, pancreatic cancer, papillary thyroid cancer, parathyroid adenoma, pediatric cancer, peripheral schwannoma, pheocytoma, pituitary tumor, prostate cancer, posterior uveal melanoma, renal metastatic cancer, rhabdoid tumor, rhabdomyosarcoma, sarcoma, skin cancer, soft tissue sarcoma, squamous cell carcinoma, synovial sarcoma, testicular cancer, thymic cancer, thyroid metastatic cancer and uterine cancer.

## DESCRIPTION OF THE DRAWINGS

Figure 1: *In vivo* efficacy of antibody-drug conjugates: hu2G6-MC-MMAF and hu2F7-MC-MMAF showed inhibitory and killing effects on MX-1 xenograft tumors at the doses of 1.5 mg/kg and 3 mg/kg.

Figure 2: HPLC spectra of hu2F7-MC-MMAF with drug loads of 2 and 4, respectively.

Figure 3: The *in vivo* anti-tumor efficacy of hu2F7-MC-MMAF with drug loads of 2 and 4, respectively.

Figure 4: The *in vivo* anti-tumor efficacy of hu2F7-MC-MMAF with drug loads of 2 and 4, respectively. The figure shows the tumors of mice in each group on day 21.

## DETAILED DESCRIPTION OF THE INVENTION

1. Terms

For easier understanding of the present invention, certain technical and scientific terms are specifically defined below. Unless otherwise clearly defined elsewhere in this document, all other technical and scientific terms used herein have the meanings commonly understood by those of ordinary skill in the art to which the present invention belongs.

The three-letter codes and one-letter codes of amino acids used in the present invention are as described in J. Biol. Chem, 243, p3558 (1968).

The term "antibody" described in the present invention refers to an immunoglobulin, which is a tetrapeptide chain structure consisting of two identical heavy chains and two identical light chains linked by interchain disulfide bonds. The composition and order of amino acids in the immunoglobulin heavy chain constant region(s) are different, so their antigenicity is also different. According to this, immunoglobulins can be classified into five types, also known as isotypes of immunoglobulins, namely IgM, IgD, IgG, IgA and IgE, and their corresponding heavy chains are $\mu$ chain, $\delta$ chain, $\gamma$ chain, $\alpha$ chain and $\varepsilon$ chain, respectively. The same type of Ig can be classified into different subclasses according to the difference in their amino acid composition of the hinge region and the number and position of heavy chain disulfide bonds. For example, IgG can be classified into IgG1, IgG2, IgG3 and IgG4. The light chain is classified into $\kappa$ chain or $\lambda$ chain according to the difference in the constant region. Each of the five types of Ig can have a $\kappa$ chain or a $\lambda$ chain.

In the present invention, the antibody light chain variable region of the present invention can further comprise light chain constant region, which comprises human or murine $\kappa$, $\lambda$ chains or variant thereof.

In the present invention, the antibody heavy chain variable region of the present invention can further comprise heavy chain constant region, which comprises human or murine IgG1, 2, 3, 4 or variant thereof.

The sequence of about 110 amino acids near the N-terminus of the antibody heavy and light chains varies greatly and is the variable region (V region); the remaining amino acid sequence near the C-terminus is relatively stable and is the constant region (C region). The variable region comprises 3 hypervariable regions (HVR) and 4 framework region(s) (FR) with relatively conservative sequences. The 3 hypervariable regions determine the specificity of the antibody, and are also known as complementarity determining regions (CDR). Each light chain variable region (VL) and heavy chain variable region (VH) consists of 3 CDR regions and 4 FR regions, arranged from the amino terminus to the carboxyl terminus in the order of: FR1, CDR1, FR2, CDR2, FR3, CDR3, FR4. The 3 CDR regions of the light chain refer to LCDR1, LCDR2 and LCDR3; the 3 CDR regions of the heavy chain refer to HCDR1, HCDR2 and HCDR3. The number and position of the CDR amino acid residues of the VL region and VH region of the antibody or antigen-binding fragment of the present invention comply with the known Kabat or Chothia numbering criteria and Kabat or AbM definition criteria (http://bioinf.org.uk/abs/).

The term "antigen presenting cell" or "APC" is a cell that presents foreign antigen complexed with MHC on its surface. T cells utilize T cell receptors (TCRs) to recognize such complexes. Examples of APCs include, but are not limited to, dendritic cells (DCs), peripheral blood mononuclear cells (PBMCs), monocytes, B lymphoblasts and monocyte-derived dendritic cells (DCs). The term "antigen presentation" refers to the process by which APCs capture antigens and enables them to be recognized by T cells, for example as a component of MHC-I/MHC-II conjugate.

The term "B7-H4" refers to a member of the human B7 protein family, also known as CD276, which is a type I transmembrane protein with four Ig-like extracellular domains. B7-H4 is one of the immune checkpoint proteins expressed on the surface of antigen-presenting cells or cancer cells, and has inhibitory effect on the functional activation of T cells. The term "B7-H4" includes any variant or isoform of B7-H4 that is naturally expressed by cells. The antibodies of the present invention can cross-react with B7-H4 derived from non-human species. As another option, the antibodies can also be specific for human B7-H4 and may not show cross-reactivity with other species. B7-H4 or any variant or isoform thereof can be isolated from cells or tissues naturally expressing the same, or produced by recombinant techniques using techniques commonly used in the art and those described herein. Preferably, the anti-B7-H4 antibody targets human B7-H4 with normal glycosylation pattern.

The term "recombinant human antibody" includes human antibodies prepared, expressed, created or isolated by recombinant methods, and the techniques and methods involved are well known in the art, for example (1) antibodies isolated from transgenic or transchromosomal animals (for example mice) expressing human immunoglobulin genes, or hybridomas prepared therefrom; (2) antibodies isolated from host cells transformed to express the antibodies, such as transfectionomas; (3) antibodies isolated from recombinant combinatorial human antibody libraries; as well as (4) antibodies prepared, expressed, created or isolated by splicing human immunoglobulin gene sequences to other DNA sequences and by other methods. Such recombinant human antibodies comprise variable regions and constant region(s), which utilize specific human germline immunoglobulin sequences encoded by germline genes, but also comprise subsequent rearrangements and mutations such as those occur during antibody maturation.

The term "murine antibody" in the present invention is a monoclonal antibody against human B7-H4 prepared according to the knowledge and skills in the art. During preparation, the test subject is injected with B7-H4 antigen, and

then hybridomas expressing antibodies with the desired sequences or functional properties are isolated. In a preferred embodiment of the present invention, the murine B7-H4 antibody or antigen-binding fragment thereof can further comprise the light chain constant region of murine κ, λ chain or variant thereof, or further comprise the heavy chain constant region of murine IgG1, IgG2, IgG3 or IgG4 or variant thereof.

The term "human antibody" includes antibodies with variable and constant region(s) of human germline immunoglobulin sequences. The human antibodies of the present invention can comprise amino acid residues that are not encoded by human germline immunoglobulin sequences (such as mutations introduced by random or site-specific mutagenesis *in vitro* or by somatic mutations *in vivo*). However, the term "human antibody" does not include antibodies in which CDR sequences derived from the germline of another mammalian species (such as mouse) have been grafted onto human framework sequences (namely "humanized antibodies").

The term "humanized antibody", also known as CDR-grafted antibody, refers to the antibody produced by grafting murine CDR sequences into the framework of human antibody variable regions. It can overcome the strong immune response reactions induced by chimeric antibodies carrying a large amount of murine protein components. In order to avoid the decrease in activity caused by the decrease in immunogenicity, the human antibody variable regions can be subjected to minimal reverse mutation to maintain the activity.

The term "chimeric antibody" is an antibody formed by fusing the variable region of a murine antibody with the constant region of a human antibody, which can alleviate the immune response induced by murine antibody. Establishing a chimeric antibody requires first establishing a hybridoma that secrets murine specific monoclonal antibody, then cloning the variable region gene from the murine hybridoma cells, and then cloning the constant region gene of the human antibody as necessary, linking the murine variable region gene with the human constant region gene to form a chimeric gene, which is inserted into a human expression vector, and finally expressing the chimeric antibody molecule in a eukaryotic industrial system or a prokaryotic industrial system. The human antibody constant region(s) can be selected from the heavy chain constant region(s) of human IgG1, IgG2, IgG3 or IgG4 or variant thereof, preferably comprising human IgG2 or IgG4 heavy chain constant region(s), or using IgG1 with enhanced ADCC (antibody-dependent cell-mediated cytotoxicity) toxicity after amino acid mutations.

The term "antigen-binding fragment" refers to antigen-binding fragments and antibody analogs of an antibody, which usually comprise at least part of the antigen-binding region or variable regions (for example, one or more CDRs) of the parental antibody. The antibody fragment retains at least some of the binding specificity of the parental antibody. Generally, when the activity is represented on a mole basis, the antibody fragment retains at least 10% of the parental binding activity. Preferably, the antibody fragment retains at least 20%, 50%, 70%, 80%, 90%, 95% or 100% or more of the binding affinity of the parental antibody to the target. Examples of antigen-binding fragments include, but are not limited to: Fab, Fab', F(ab')2, Fv fragment, linear antibody, single-chain antibody, nanobody, domain antibody and multispecific antibody. Engineered antibody variants are reviewed in Holliger and Hudson (2005) Nat. Biotechnol. 23: 1126-1136.

The "Fab fragment" consists of the CHI and variable regions of one light chain and one heavy chain. The heavy chain of a Fab molecule cannot form disulfide bonds with another heavy chain molecule.

The "Fc" region comprises two heavy chain fragments comprising the CHI and CH2 domains of the antibody. The two heavy chain fragments are held together by two or more disulfide bonds and through the hydrophobic interaction of the CH3 domain.

The "Fab' fragment" comprises a light chain and a part of a heavy chain that comprises the VH domain, the CHI domain and the region between the CHI and CH2 domains, so that interchain disulfide bonds can be formed between the two heavy chains of two Fab' fragments, so as to form the F(ab')2 molecule.

The "F(ab')2 fragment" comprises two light chains and two heavy chains comprising a part of the constant region between the CHI and CH2 domains, thereby forming interchain disulfide bonds between the two heavy chains. Therefore, the F(ab')2 fragment consists of two Fab' fragments held together by disulfide bonds between the two heavy chains.

The "Fv region" comprises variable regions from both the heavy chain and the light chain, but lacks the constant region(s).

The term "multispecific antibody" is used in its broadest sense, which encompasses antibodies with polyepitope specificity. These multispecific antibodies include, but are not limited to: antibodies comprising heavy chain variable region (VH) and light chain variable region (VL), wherein the VH-VL unit has multi-epitope specificity; antibodies with two or more VL and VH regions, each VH-VL unit binds to different targets or different epitopes of the same target; antibodies with two or more single variable regions, each single variable region binds to different targets or different epitopes of the same target; full-length antibodies, antibody fragments, diabodies, bispecific diabodies and triabodies, antibody fragments that are covalently or non-covalently linked together, and the like.

The term "single-chain antibody" is a single-chain recombinant protein formed by linking the heavy chain variable region (VH) and light chain variable region (VL) of an antibody through a linker peptide. It is the smallest antibody fragment with complete antigen binding site.

The term "domain antibody fragment" is an immunoglobulin fragment with immunological functions that comprises only the heavy chain variable region or the light chain variable region. In some cases, two or more VH regions are covalently linked to a peptide linker to form a bivalent domain antibody fragment. The two VH regions of the bivalent domain antibody fragment can target the same or different antigens.

The term "binding to B7-H4" in the present invention refers to the ability to interact with human B7-H4. The term "antigen-binding site" of the present invention refers to a three-dimensional site that is scattered on the antigen and is recognized by the antibody or antigen-binding fragment of the present invention.

The terms "specifically binds" and "selectively binds" used in the present invention refer to the binding of an antibody to an epitope on a predetermined antigen. Generally, when recombinant human B7-H4 is used as the analyte and an antibody is used as the ligand, when measured by surface plasmon resonance (SPR) technology in an instrument, the antibody binds to the predetermined antigen at an equilibrium dissociation constant (KD) of about less than $10^{-7}$ M or even less, and its binding affinity to the predetermined antigen is at least twice of its binding affinity to non-specific antigens (other than the predetermined antigen or closely related antigens, such as BSA and the like). The term "antibody that recognizes...antigen" can be used interchangeably with the term "antibody that specifically binds to..." herein.

The term "cross-reactivity" refers to the ability of the antibodies of the present invention to bind to B7-H4 from different species. For example, the antibody of the present invention that binds to human B7-H4 can also bind to B7-H4 of another species. Cross-reactivity is measured by detecting specific reactivity with purified antigens in binding assays (for example SPR and ELISA), or by binding or functional interaction with cells that physiologically express B7-H4. Methods of determining cross-reactivity include standard binding assays as described herein, for example surface plasmon resonance (SPR) analysis or flow cytometry.

The terms "inhibition" or "blocking" can be used interchangeably and encompass both partial and complete inhibition/blocking. The inhibition/blocking of a ligand preferably reduces or alters the normal level or type of activity that present when ligand binding occurs without inhibition or blocking. Inhibition and blocking are also intended to include any measurable reduction in binding affinity of the ligand when being in contact with anti-B7-H4 antibody, compared to the binding affinity of the ligand not being in contact with anti-B7-H4 antibody.

The term "inhibition of growth" (for example when referring to cells) is intended to include any measurable reduction in cell growth.

The terms "induced immune response" and "enhanced immune response" can be used interchangeably and refer to immune response to the stimulation by a specific antigen (i.e. passive or adaptive). The term "induce" used in expression "inducing CDC or ADCC" refers to stimulating a specific direct cell killing mechanism.

The "ADCC" in the present invention, i.e. antibody-dependent cell-mediated cytotoxicity, means that cells expressing Fc receptors directly kill the target cells coated with antibodies by recognizing the Fc segment of the antibodies. The ADCC effect function of antibodies can be enhanced, reduced or eliminated by modifying the Fc segment of IgG. The modification refers to mutations in the heavy chain constant region of an antibody.

The methods for producing and purifying antibodies and antigen-binding fragments are well-known and can be found in the prior art, such as Antibodies: A Laboratory Manual, Cold Spring Harbor, chapters 5-8 and 15. For example, mice can be immunized with human B7-H4 or fragment thereof, and the obtained antibodies can be renatured and purified, and amino acid sequencing can be performed by using conventional methods. Antigen-binding fragments can also be prepared by using conventional methods. The antibody or antigen-binding fragment of the present invention is genetically engineered to introduce one or more human FR regions onto the non-human CDR regions. The human FR germline sequences can be obtained from the ImmunoGeneTics (IMGT) website http://imgt.cines.fr, or from The Immunoglobulin FactsBook, 2001ISBN012441351.

The engineered antibodies or antigen-binding fragments of the present invention can be prepared and purified by conventional methods. The cDNA sequences of the corresponding antibodies can be cloned and recombined into GS expression vectors. The recombinant immunoglobulin expression vectors can stably transfect CHO cells. As a more recommended prior art, mammalian expression systems can lead to glycosylation of antibodies, especially at the highly conserved N-terminus of the Fc region. Stable clones are obtained by expressing antibodies that specifically bind to human antigens. Positive clones are expanded in serum-free medium of bioreactors to produce antibodies. The culture medium into which the antibodies are secreted can be purified and collected by conventional techniques. The antibodies can be filtered and concentrated by conventional methods. Soluble mixtures and multimers can also be removed by conventional methods, for example molecular sieves and ion exchange. The resulting product needs to be frozen immediately, such as at -70°C, or lyophilized.

The antibody of the present invention refers to monoclonal antibody. The monoclonal antibody (mAb) described in the present invention refers to an antibody obtained from a single cloned cell strain, the cell strain is not limited to a eukaryotic, prokaryotic or phage cloned cell strain. Monoclonal antibodies or antigen-binding fragments can be obtained by recombination using, for example, hybridoma technology, recombination technology, phage display technology, synthesis technology (such as CDR-grafting) or other existing technologies.

"administering" and "treating", when applied to animals, humans, experimental subjects, cells, tissues, organs or biological fluids, refer to contacting the exogenous medicament, therapeutic agent, diagnostic agent or composition with the animals, humans, subjects, cells, tissues, organs or biological fluids. "administering" and "treating" can refer to for example treatment, pharmacokinetics, diagnosis, research and experimental methods. Treating cells includes contacting reagents with the cells, and contacting reagents with fluids, wherein the fluids are in contact with the cells. "administering" and "treating" also mean treating for example cells with reagents, diagnostics, binding compositions or with another type of cells *in vitro* and *ex vivo.* "Treating", when applied to human, veterinary or research subjects, refers to therapeutic treatment, preventive or prophylactic measures, research and diagnostic applications.

"Treatment" refers to administering an internal or external therapeutic agent, for example a composition comprising any one of the binding compounds of the present invention, to a patient with one or more disease symptoms on which the therapeutic agent is known to have therapeutic effect. Generally, the therapeutic agent is given in an amount effective to alleviate one or more disease symptoms in the treated subject or population, either to induce the regression of such symptoms or to inhibit the development of such symptoms to any clinically measurable extent. The amount of therapeutic agent that is effective to alleviate any specific disease symptom (also referred to as a "therapeutically effective amount") can vary according to a variety of factors, for example the patient's disease state, age and body weight, and the ability of the drug to produce the desired therapeutic effect in the patient. Whether the disease symptoms have been alleviated can be evaluated by any clinical testing methods commonly used by doctors or other health care professionals for evaluating the severity or progression of the symptoms. Although the embodiments of the present invention (for example treatment methods or products) may be ineffective in alleviating each disease symptom of interest, but they should reduce the disease symptom of interest in a statistically significant number of patients, as determined by any statistical testing methods known in the art, such as Student t-test, chi-square test, Mann and Whitney's U test, Kruskal-Wallis test (H test), Jonckheere-Terpstra test and Wilcoxon test.

The term "essentially consisting of......" or variant thereof used throughout the specification and claims means to comprise all the elements or element groups described, and optionally comprise other elements similar or different in nature to the elements described, which does not significantly change the basic or new properties of the given dosing regimen, method, or composition. As a non-limiting example, the binding compound essentially consisting of the mentioned amino acid sequence can also comprise one or more amino acids, which do not significantly affect the properties of the binding compound.

The term "naturally occurring" applied to a certain object in the present invention refers to the fact that the object can be found in nature. For example, a polypeptide sequence or polynucleotide sequence is deemed as naturally occurring, if it exists in organisms (including virus) and can be isolated from natural sources, and has not been intentionally modified artificially in the laboratory.

The "effective amount" includes an amount sufficient to ameliorate or prevent a symptom or condition of a medical condition. The effective amount also refers to an amount sufficient to allow or facilitate diagnosis. The effective amount for a particular patient or veterinary subject can vary depending on the following factors: such as the condition to be treated, the general health condition of the patient, the method, route and dose of drug administration, and the severity of side effects. The effective amount can be the maximum dose or dosing regimen that avoids significant side effects or toxic effects.

"Exogenous" refers to substances produced outside organisms, cells or human bodies according to backgrounds. "Endogenous" refers to substances produced inside cells, organisms or human bodies according to backgrounds.

"Homology" refers to the sequence similarity between two polynucleotide sequences or between two polypeptides. When the positions in the two sequences aligned are occupied by the same base or amino acid monomer subunit, for example if each position of two DNA molecules is occupied by adenine, then the molecules are deemed as homologous at that position. The homology percentage between two sequences is a function of the number of matched or homologous positions shared by the two sequences divided by the number of positions aligned $\times$ 100%. For example, in the optimal sequence alignment, if 6 out of 10 positions in the two sequences are matched or homologous, then the two sequences are 60% homologous. Generally, the alignment is made when two sequences are aligned to obtain the maximum homology percentage.

The expressions "cell", "cell line" and "cell culture" used herein can be used interchangeably, and all such names include progeny thereof. Therefore, the words "transformant" and "transformed cell" include primary test cells and cultures derived therefrom, regardless of the number of passages. It should also be understood that due to intentional or unintentional mutations, all offspring cannot be exactly the same in terms of DNA content. Mutant progeny with the same function or biological activity as those screened in the original transformed cells is included. It can be clearly understood from the context, when different names are referred to.

"Optional" or "optionally" means that: an event or circumstance that follows the wording "pptional" or "optionally" could occur, but does not have to occur, and the description includes occasions when the event or circumstance occurs or does not occur. For example, "optionally comprising 1 to 3 antibody heavy chain variable regions" means that the antibody heavy chain variable regions of particular sequences can but does not have to be present.

"Pharmaceutical composition" means a mixture containing one or more of the compounds described herein, or a physiologically/pharmaceutically acceptable salt or a prodrug thereof, and other chemical components, as well as other components such as physiological/pharmaceutically acceptable carriers and excipients. The object of the pharmaceutical composition is to promote drug administration to organisms, to facilitate the absorption of the active ingredient and thereby exerting the biological activity. The preparation of conventional pharmaceutical compositions is described in the Chinese Pharmacopoeia.

"Pharmaceutically acceptable salt" refers to a salt of the antibody-drug conjugate of the present invention, which is safe and effective for use in mammals and has the desired biological activity. The antibody-drug conjugate of the present invention comprises at least one amino group, thus it can form a salt with an acid. Non-limiting examples of pharmaceutically acceptable salts include: hydrochloride, hydrobromide, hydroiodide, sulfate, hydrogen sulfate, citrate, acetate, succinate, ascorbate, oxalate, nitrate, sorbate, hydrogen phosphate, dihydrogen phosphate, salicylate, hydrogen citrate, tartrate, maleate, fumarate, formate, benzoate, methanesulfonate, ethanesulfonate, benzenesulfonate and p-toluenesulfonate.

The "solvate" refers to a pharmaceutically acceptable solvate formed by the antibody-drug conjugate compound of the present invention and one or more solvent molecules. Non-limiting examples of solvent molecules include: water, ethanol, acetonitrile, isopropanol and ethyl acetate.

The "cytotoxic drug", when used in the present invention, refers to a substance that inhibits the function of cells and/or causes cell death or destruction.

The "tubulin inhibitor" refers to a class of compounds that interfere with the process of cell mitosis by inhibiting the polymerization of tubulin or promoting the aggregation of tubulin, thereby exerting an anti-tumor effect. Non-limiting examples thereof include: maytansinoids, calicheamicin, taxanes, vincristine, colchicine, dolastatin/auristatin/monomethyl auristatin E (MMAE)/ monomethyl auristatin F (MMAF).

"Linker" refers to a chemical moiety by which an antibody is covalently attached to a covalent bond or atomic chain of a drug. Non-limiting examples of linkers include: arylene, heteroarylene, PEG, polymethyleneoxy, succinate, succinamide, diglycolate, malonate and caproamide.

"Drug load" (DAR) is represented by y, which is the average number of cytotoxic drugs per antibody in formula (A). The range of drug load in the present invention can be 1-20 cytotoxic drugs (D) per antibody. The antibody-drug conjugate of general formula (A) is a collection of antibodies conjugated to a certain range (1-20) of cytotoxic drugs. The drug load (DAR) in an antibody-drug conjugate from coupling reaction can be characterized by conventional means, for example mass spectrometry, HPLC and ELISA. By these means, the quantitative distribution of the antibody-drug conjugate on the y value can be determined.

2. Abbreviations
MC = 6-maleimidohexanoyl
VC = valine-citrulline
PAB = p-aminobenzyloxycarbonyl
MMAE = monomethyl auristatin E (MW 718)
MMAF = a variant of monomethyl auristatin E, which has phenylalanine at the C-terminus of the molecule (MW 731.5)

The examples are incorporated below for further description of the present invention, but these examples do not limit the scope of the present invention. The experimental methods with unspecified conditions in the examples of the present invention generally follow conventional conditions, such as Antibodies: A Laboratory Manual and Molecular Cloning: A Laboratory Manual, Cold Spring Harbor; or according to the conditions recommended by the raw material or commodity manufacturer. The reagents with unspecified sources are conventional reagents purchased on the market.

**Example 1: Antigen preparation and stable cell line construction**

The sequence encoding his-tagged human B7-H4 (huB7-H4-His) and the sequence encoding Fc-tagged human B7-H4 (huB7-H4-Fc) were synthesized by Integrated DNA Technology (IDT) of CRO (the template sequences of the above B7-H4 recombinant proteins were all designed by the present invention) and respectively cloned into pTT5 vectors (Biovector). After the recombinant B7-H4 proteins were expressed in 293T cells, they were purified by the following experimental methods, and the purified proteins could be used in the following experiments of the examples.

Sequence of huB7-H4-His:

MASLGQILFWSIISIIIILAGAIALIIGFGISGRHSITVTTVASAGNIGEDGILSCTFEP
DIKLSDIVIQWLKEGVLGLVHEFKEGKDELSEQDEMFRGRTAVFADQVIVGNAS
LRLKNVQLTDAGTYKCYIITSKGKGNANLEYKTGAFSMPEVNVDYNASSETLR
CEAPRWFPQPTVVWASQVDQGANFSEVSNTSFELNSENVTMKVVSVLYNVTIN
NTYSCMIENDIAKATGDIKVTESEIKRRSHLQLLNSKADYKDDDDKGSHHHHH
HHH

SEQ ID NO: 1

Sequence of huB7-H4-Fc:

FGISGRHSITVTTVASAGNIGEDGILSCTFEPDIKLSDIVIQWLKEGVLGLVHEFKE
GKDELSEQDEMFRGRTAVFADQVIVGNASLRLKNVQLTDAGTYKCYIITSKGKG
NANLEYKTGAFSMPEVNVDYNASSETLRCEAPRWFPQPTVVWASQVDQGANF
SEVSNTSFELNSENVTMKVVSVLYNVTINNTYSCMIENDIAKATGDIKVTESEIK
RRSHLQLLNSKAGSGGGGDKTHTCPPCPAPELLGGPSVFLFPPKPKDTLMISRTP
EVTCVVVDVSHEDPEVKFNWYVDGVEVHNAKTKPREEQYNSTYRVVSVLTVL
HQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRW
QQGNVFSCSVMHEALHNHYTQKSLSLSPGK

SEQ ID NO: 2

Purification steps of huB7-H4-His:

The HEK293 cell expression supernatant sample was centrifuged at high speed to remove impurities. The buffer was replaced against PBS and added with imidazole to a final concentration of 5 mM. The nickel column was equilibrated with PBS solution containing 5 mM imidazole and washed with 2-5 times the column volume. The supernatant sample obtained after replacement was loaded onto the column. The column was rinsed with PBS solution containing 5 mM imidazole until the A280 reading dropped to baseline. Then the chromatography column was rinsed with PBS+10 mM imidazole to remove non-specifically bound protein impurities, and the effluent was collected. The target protein was eluted with PBS solution containing 300 mM imidazole, and the elution peak was collected. The collected eluate was further purified by ion exchange (SP column). Preparing A solution: 0.01 M PB, pH 8.0. Preparing B solution: A solution + 1 M NaCl. First, the PBS solution of imidazole with eluted target protein was replaced against solution A, the SP column was equilibrated by using solution A, and the sample was loaded. The concentration gradient of solution B was 0-100%. The sample was eluted with 10 times the column volume, and each elution peak was collected. The obtained protein was identified as correct by electrophoresis, peptide map and liquid chromatography-mass spectrometry (LC-MS), and then aliquoted for use.

Purification steps of huB7-H4-Fc:

The HEK293 cell expression supernatant sample was centrifuged at high speed to remove impurities. The buffer was replaced against PBS. The Protein A affinity column was equilibrated with 10 mM PBS buffer and was washed 2-5 times the column volume. The supernatant sample obtained after replacement was loaded onto the column. The column was washed with buffer 25 times the column volume until the A280 reading dropped to baseline. The target protein was eluted with 0.8% acetate buffer of pH 3.5, and the elution peaks were collected. After aliquoting, 1 M Tris-Cl pH 8.0 buffer was immediately added for neutralization. Then the solution was replaced against PBS pH 6.9 by using Millipore's Amico-15 filter column. The obtained protein was identified by electrophoresis, peptide map and liquid chromatography-mass spectrometry (LC-MS), and then aliquoted for use.

Construction of stable CHO-S cell pool:
The full-length sequences encoding human or cynomolgus monkey B7-H4 protein (huB7-H4 or cyB7-H4) were synthesized by Integrated DNA Technology (IDT) (the template sequences of the above B7-H4 recombinant proteins were all designed by the present invention), and respectively cloned into the modified pcDNA3.1 vector, pcDNA3.1/puro (Invitrogen #V79020). CHO-S (ATCC) cells were cultured in CD-CHO medium (Life Technologies, #10743029) to $0.5 \times 10^6$ cells/ml. 10 μg of vector encoding huB7-H4 or cyB7-H4 gene was mixed with 50 μl LF-LTX (Life Technologies, #A12621) in 1 ml Opti-MEM medium (Life Technologies, #31985088). The mixture was incubated at room temperature for 20 minutes and added into the culture medium of CHO cells. The cells were placed in a carbon dioxide incubator for culture. After 24 hours, the culture medium was replaced with new medium and added with 10 μg/ml puromycin. After that, the culture medium was replaced with new medium every 2-3 days, and a stable CHO-S cell pool was obtained after 10-12 days of selection.

**Example 2: Obtaining mouse hybridoma and antibody sequences**

A total of 5 Balb/c and 5 A/J female 10-weeks-old mice were immunized with the human antigen huB7-H4-Fc. Sigma Complete Freund's Adjuvant (CFA) and Sigma Incomplete Freund's Adjuvant (IFA) were used. The immunogen and the immune adjuvant were fully mixed and emulsified at a ratio of 1:1 to make a stable "water-in-oil" liquid. The injection dose was 25 μg/200 μL/mouse.

Day 01: First immunization, complete Freund's adjuvant.
Day 21: Second immunization, incomplete Freund's adjuvant.
Day 35: Third immunization, incomplete Freund's adjuvant.
Day 42: Blood sampling and serum titer test (blood after 3 immunizations).
Day 49: Fourth immunization, incomplete Freund's adjuvant.
Day 56: Blood sampling and serum titer test (blood after 4 immunizations).

Indirect ELISA method was used to detect the affinity of antibody or serum: huB7-H4-His protein was diluted to a concentration of 1μg/ml with PBS pH7.4, and added at 100μl/well into a 96-well high-affinity ELISA plate, and refrigerated at 4°C for incubation overnight (16-20 hours). The plate was washed 4 times with PBST (pH 7.4 PBS containing 0.05% Tween-20). 3% bovine serum albumin (BSA) blocking solution diluted with PBST was added at 150 μl/well and incubated at room temperature for 1 hour for blocking. After completion of the blocking, the blocking solution was discarded, and the plate was washed 4 times with PBST buffer. The antibody or serum to be tested was diluted with PBST containing 3% BSA, to obtain a gradient of 10 doses (10-fold dilution) starting from 1 μM, and added into the microtiter plate at 100 μl/well and incubated at room temperature for 1 hour. After completion of the incubation, the plate was washed 4 times with PBST, HRP-labeled goat anti-human secondary antibody (Abcam, cat#ab97225) diluted with PBST containing 3% BSA was added at 100 μl/well, and incubated for 1 hour at room temperature. The plate was washed 4 times with PBST, then TMB chromogenic substrate (Cell Signaling Technology, cat#7004S) was added at 100 μl/well and incubated at room temperature in dark for 1 minute. The stop solution (Cell Signaling Technology, cat#7002S) was added at 100 μl/well to terminate the reaction. The absorbance value was read at 450 nm with a microplate reader (BioTek, model Synergy HI). The data were analyzed to obtain the binding affinity of the antibody or serum to the human B7-H4 antigen.

The serum titer and the ability of the immunized mouse serum to bind to cell surface antigens were evaluated by using indirect ELISA method. The cell fusion was performed according to the detection results of titer (greater than 100,000 times of dilution) . The immunized mice with high serum titer, affinity and FACS binding were selected for one final immunization and then sacrificed. The spleen cells and SP2/0 myeloma cells were fused and plated to obtain hybridomas. The target hybridomas were screened by indirect ELISA and monoclonal cell strains were established by limiting-dilution method. Among the obtained positive antibody strains, the hybridoma strains expressing non-specific binding antibodies were further excluded by comparing CHO-S cells stably expressing huB7-H4 and cyB7-H4, with blank CHO-S cells. The target hybridomas were screened by using methods similar to that of the *in vitro* cell binding experiment in Example 5, and established as monoclonal cell strains by limiting-dilution method. Hybridoma cells at logarithmic growth phase were collected. RNA was extracted with Trizol (Invitrogen, 15596-018) and subjected to reverse transcription (PrimeScript™ Reverse Transcriptase, Takara #2680A). The cDNA obtained by reverse transcription was amplified by PCR with a mouse Ig-primer set (Novagen, TB326 Rev.B 0503) and sequenced. Finally, the sequences of 4 strains of murine antibodies were obtained for humanization and construction of antibody-drug conjugates.

The heavy chain and light chain variable region sequences of murine monoclonal antibody 2G6 are as follows:

2G6 HCVR

EVQLVESGGGLVKPGGSLKLSCAASGFTFSRYGMSWVRQTPEKRLEWVAGING
GGSYTYYLDTVKGRFTISRDNSRNTLYLQMSSLRSEDTAMYYCVSQGSNYYFD
YWGQGTTLTVSS SEQ ID NO: 46

2G6 LCVR

DIRMTQSPSSMSVSLGDTVSITCHASQGISSNIGWLQQKPGKSFKALIYHGTNLE
DGVPSRFSGSGSGADYSLTISSLESEDFADYYCVQYAQFPYTFGGGTKLEIK

SEQ ID NO: 47

The heavy chain and light chain variable regions of murine monoclonal antibody 2G6 comprise the following CDR sequences:

| Name | Sequence | No. |
|---|---|---|
| HCDR1 | GFTFSRYGMS | SEQ ID NO: 3 |
| HCDR2 | GINGGGSYTYYLDTVKG | SEQ ID NO: 4 |
| HCDR3 | QGSNYYFDY | SEQ ID NO: 5 |
| LCDR1 | HASQGISSNIG | SEQ ID NO: 6 |
| LCDR2 | HGTNLED | SEQ ID NO: 7 |
| LCDR3 | VQYAQFPYT | SEQ ID NO: 8 |

The sequences of the heavy chain and light chain variable regions of murine monoclonal antibody 2F7 are as follows:

2F7 HCVR

EVQLVESGGGLVQPGGSLKLSCAASGFTFSNYYMSWVRQTPEKRLEWVAYVSS
GGGSTYYSDSVKGRFTISRDNAKNTLYLQMSSLKPEDTAMYYCTRESYSQGNY
FDYWGQGTTLTVSS

SEQ ID NO: 48

2F7 LCVR

DIVMTQSPATLSVTPGDRVSLSCRASQSISDYLHWYQQKSHESPRLLIKFASQSIS
GIPSRFSGSGSGSDFTLSINSVEPEDVGVYYCQNGHSFSLTFGAGTKLELK

SEQ ID NO: 49

The heavy chain and light chain variable regions of murine monoclonal antibody 2F7 comprise the following CDR sequences:

| Name | Sequence | No. |
|---|---|---|
| HCDR1 | GFTFSNYYMS | SEQ ID NO: 9 |

(continued)

| Name | Sequence | No. |
|---|---|---|
| HCDR2 | YVSSGGGSTYYSDSVKG | SEQ ID NO: 10 |
| HCDR3 | ESYSQGNYFDY | SEQ ID NO: 11 |
| LCDR1 | RASQSISDYLH | SEQ ID NO: 12 |
| LCDR2 | FASQSIS | SEQ ID NO: 13 |
| LCDR3 | QNGHSFSLT | SEQ ID NO: 14 |

The sequences of the heavy chain and light chain variable regions of murine monoclonal antibody 2F8 are as follows:

2F8 HCVR

QVQLQQPGSVLVRPGASVKLSCKASGYTFTNSWMNWAKLRPGQGLEWIGGIYP
NSGNIEYNEKFKGKATLTVDTSSSTAYMDLTSLTSEDSAVYYCARDSRFSYWGQ
GTLVTVSA

SEQ ID NO: 50

2F8 LCVR

DIVMTQSHKFMSTSVGDRVSITCKASQDVRTAVAWYQQKPGQSPKLLISSTSYR
YTGVPDRFTGSGSGTDFTFIISSVQAEDLAVYYCQQHYSTPLTFGAGTKLELK

SEQ ID NO: 51

The heavy chain and light chain variable regions of murine monoclonal antibody 2F8 comprise the following CDR sequences:

| Name | Sequence | No. |
|---|---|---|
| HCDR1 | GYTFTNSWMN | SEQ ID NO: 23 |
| HCDR2 | GIYPNSGNIEYNEKFKG | SEQ ID NO: 24 |
| HCDR3 | DSRFSY | SEQ ID NO: 25 |
| LCDR1 | KASQDVRTAVA | SEQ ID NO: 26 |
| LCDR2 | STSYRYT | SEQ ID NO: 27 |
| LCDR3 | QQHYSTPLT | SEQ ID NO: 28 |

The sequences of the heavy chain and light chain variable regions of murine monoclonal antibody 1C9 are as follows:

1C9 HCVR

QVQLQQPGSVLVRPGASVKLSCKASGDTFTTYWMNWVKQRPGQGLEWIGGIY LNSGSSEYNEKFKGKATLSVDTSSSTAYMDLSSLTSEDSAVYYCARDSRFSYWG QGTLVTVSA

SEQ ID NO: 52

1C9 LCVR

DIVMTQSHKFLSTSVGDRVSITCKASQDVSTAVAWYQQKPGQSPELLISSASYRY TGVPDRFTGSGSGTDFTFTISSVQAEDLAVYYCQQHYNTPLTFGAGTQLELK

SEQ ID NO: 53

The heavy chain and light chain variable regions of murine monoclonal antibody 1C9 comprise the following CDR sequences:

| Name | Sequence | No. |
|---|---|---|
| HCDR1 | GDTFTTY | SEQ ID NO: 29 |
| HCDR2 | YLNSGS | SEQ ID NO: 30 |
| HCDR3 | DSRFSY | SEQ ID NO: 31 |
| LCDR1 | KASQDVSTAVA | SEQ ID NO: 32 |
| LCDR2 | SASYRYT | SEQ ID NO: 33 |
| LCDR3 | QQHYNTPLT | SEQ ID NO: 34 |

**Example 3: Humanization experiment of murine antibodies**

Humanization of the murine anti-human B7-H4 monoclonal antibodies was performed by using methods as published in many documents in the art. Briefly, parental (murine antibody) constant domains were replaced with human constant domains. In the present invention, human germline antibody sequences were selected according to the homology between the murine antibodies and human antibodies, and the murine antibodies 2G6, 2F7, 2F8 and 1C9 were humanized. The CDR regions of the murine antibodies 2G6 and 2F7 were grafted onto the selected corresponding humanized templates to replace the humanized variable regions, and then recombined with the IgG constant region(s) (preferably, IgG1 for the heavy chain, andκ for the light chain). Then, based on the three-dimensional structure of the murine antibodies, the embedded residues, the residues directly interacting with the CDR regions and the residues with significant influence on the conformation of VL and VH were subjected to back mutation, and chemically unstable amino acid residues of the CDR regions were optimized, wherein the HCDR1 of murine monoclonal antibody 2F8 was optimized to GYTFTSSWMN (SEQ ID NO: 43), HCDR2 was optimized to GIYPNRGNIEY NEKFKG (SEQ ID NO: 44), the HCDR2 of murine monoclonal antibody 1C9 was optimized to YLNRGS (SEQ ID NO: 45), and the designed humanized antibodies comprising the following combination of light and heavy chain variable region sequences were obtained.

The heavy and light chain variable regions of humanized antibody hu2G6 are as follows:

hu2G6 HCVR

EVQLLESGGGLVQPGGSLRLSCAASGFTFSRYGMSWVRQAPGKGLEWVSGING
GGSYTYYLDTVKGRFTISRDNARNTLYLQMSSLRAEDTAVYYCVSQGSNYYFD
YWGQGTLVTVSS

SEQ ID NO: 15

hu2G6 LCVR

DIRMTQSPSSLSASVGDRVTITCHASQGISSNIGWLQQKPGKAPKALIYHGTNLE
DGVPSRFSGSGSGADYTLTISSLQPEDFATYYCVQYAQFPYTFGGGTKVEIK

SEQ ID NO: 16

The heavy and light chain variable regions of humanized antibody hu2F7 are as follows:

hu2F7 HCVR

EVQLVESGGGLVQPGGSLRLSCAASGFTFSNYYMSWVRQAPGKGLEWVAYVSS
GGGSTYYSDSVKGRFTISRDNAKNTLYLQMSSLRAEDTAVYYCTRESYSQGNYF
DYWGQGTTVTVSS

SEQ ID NO: 17

hu2F7 LCVR

EIVMTQSPATLSLSPGERATLSCRASQSISDYLHWYQQKPGQSPRLLIKFASQSIS
GIPARFSGSGSGTDFTLTISSLEPEDFAVYYCQNGHSFSLTFGQGTKLEIK

SEQ ID NO: 18

The heavy and light chain variable regions of humanized antibody hu2F8 are as follows:

hu2F8 HCVR

EVQLVQSGAEVKKPGASVKVSCKASGYTFTSSWMNWVRQAPGQRLEWMGGI
YPNRGNIEYNEKFKGRVTLTVDTSASTAYMELSSLRSEDTAVYYCARDSRFSYW
GQGTLVTVSS

SEQ ID NO: 35

hu2F8 LCVR

DIQMTQSPSSLSASVGDRVTITCKASQDVRTAVAWYQQKPGKAPKLLISSTSYRY
TGVPSRFSGSGSGTDFTFTISSLQPEDIATYYCQQHYSTPLTFGGGTKVEIK

SEQ ID NO:36

The heavy and light chain variable regions of humanized antibody hu1C9 are as follows:

hu1C9 HCVR

EVQLVQSGAEVKKPGASVKVSCKASGDTFTTYWMNWVRQAPGQRLEWMGGI
YLNRGSSEYNEKFKGRVTLTVDTSASTAYMELSSLRSEDTAVYYCARDSRFSYW
GQGTLVTVSS

SEQ ID NO:37

hu1C9 LCVR

DIQMTQSPSSLSASVGDRVTITCKASQDVSTAVAWYQQKPGKAPKLLISSASYRY
TGVPSRFSGSGSGTDFTFTISSLQPEDIATYYCQQHYNTPLTFGGGTKVEIK

SEQ ID NO:38;

;

The variable regions were recombined with the IgG constant region(s) (preferably, IgG1 for the heavy chain, andκ for the light chain). Exemplary heavy and light chain constant region sequences are shown as follows,

IgG1 C:

ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTC
PPCPAPELLGGPSVFLFPPKPKDTLMISRTPEVTCVVVDVSHEDPEVKFNWYVD

GVEVHNAKTKPREEQYNSTYRVVSVLTVLHQDWLNGKEYKCKVSNKALPAPIE
KTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSL
SLSPGK

SEQ ID NO: 54

Ig kappa C:

RTVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGEC

SEQ ID NO: 55;

;

Humanized antibodies consisting of the following exemplary light and heavy chain sequences were obtained after linking:

hu2G6 HC

EVQLLESGGGLVQPGGSLRLSCAASGFTFSRYGMSWVRQAPGKGLEWVSGING
GGSYTYYLDTVKGRFTISRDNARNTLYLQMSSLRAEDTAVYYCVSQGSNYYFD
YWGQGTLVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKK
VEPKSCDKTHTCPPCPAPELLGGPSVFLFPPKPKDTLMISRTPEVTCVVVDVSHE
DPEVKFNWYVDGVEVHNAKTKPREEQYNSTYRVVSVLTVLHQDWLNGKEYK
CKVSNKALPAPIEKTISKAKGQPREPQVYTLPPSREEMTKNQVSLTCLVKGFYPS
DIAVEWESNGQPENNYKTTPPVLDSDGSFFLYSKLTVDKSRWQQGNVFSCSVM
HEALHNHYTQKSLSLSPGK

SEQ ID NO: 19

hu2G6 LC

DIRMTQSPSSLSASVGDRVTITCHASQGISSNIGWLQQKPGKAPKALIYHGTNLE
DGVPSRFSGSGSGADYTLTISSLQPEDFATYYCVQYAQFPYTFGGGTKVEIKRTV
AAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC

SEQ ID NO: 20

hu2F7 HC

EVQLVESGGGLVQPGGSLRLSCAASGFTFSNYYMSWVRQAPGKGLEWVAYVSS
GGGSTYYSDSVKGRFTISRDNAKNTLYLQMSSLRAEDTAVYYCTRESYSQGNYF
DYWGQGTTVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDK

KVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEY
KCKVSNKALPAPIEKTISKAKGQPREPQVYTLPPSREEMTKNQVSLTCLVKGFYP
SDIAVEWESNGQPENNYKTTPPVLDSDGSFFLYSKLTVDKSRWQQGNVFSCSVM
HEALHNHYTQKSLSLSPGK

SEQ ID NO: 21

hu2F7 LC

EIVMTQSPATLSLSPGERATLSCRASQSISDYLHWYQQKPGQSPRLLIKFASQSIS
GIPARFSGSGSGTDFTLTISSLEPEDFAVYYCQNGHSFSLTFGQGTKLEIKRTVAAP
SVFIFPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEC

SEQ ID NO: 22

hu2F8 HC

EVQLVQSGAEVKKPGASVKVSCKASGYTFTSSWMNWVRQAPGQRLEWMGGI
YPNRGNIEYNEKFKGRVTLTVDTSASTAYMELSSLRSEDTAVYYCARDSRFSYW
GQGTLVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVE
PKSCDKTHTCPPCPAPELLGGPSVFLFPPKPKDTLMISRTPEVTCVVVDVSHEDP
EVKFNWYVDGVEVHNAKTKPREEQYNSTYRVVSVLTVLHQDWLNGKEYKCK
VSNKALPAPIEKTISKAKGQPREPQVYTLPPSREEMTKNQVSLTCLVKGFYPSDI
AVEWESNGQPENNYKTTPPVLDSDGSFFLYSKLTVDKSRWQQGNVFSCSVMHE
ALHNHYTQKSLSLSPGK

SEQ ID NO:39

hu2F8 LC

DIQMTQSPSSLSASVGDRVTITCKASQDVRTAVAWYQQKPGKAPKLLISSTSYRY
TGVPSRFSGSGSGTDFTFTISSLQPEDIATYYCQQHYSTPLTFGGGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC

SEQ ID NO:40

hu1C9HC

EVQLVQSGAEVKKPGASVKVSCKASGDTFTTYWMNWVRQAPGQRLEWMGGI
YLNRGSSEYNEKFKGRVTLTVDTSASTAYMELSSLRSEDTAVYYCARDSRFSYW
GQGTLVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVE

PKSCDKTHTCPPCPAPELLGGPSVFLFPPKPKDTLMISRTPEVTCVVVDVSHEDP
EVKFNWYVDGVEVHNAKTKPREEQYNSTYRVVSVLTVLHQDWLNGKEYKCK
VSNKALPAPIEKTISKAKGQPREPQVYTLPPSREEMTKNQVSLTCLVKGFYPSDI
AVEWESNGQPENNYKTTPPVLDSDGSFFLYSKLTVDKSRWQQGNVFSCSVMHE
ALHNHYTQKSLSLSPGK

SEQ ID NO:41

hu1C9 LC

DIQMTQSPSSLSASVGDRVTITCKASQDVSTAVAWYQQKPGKAPKLLISSASYRY
TGVPSRFSGSGSGTDFTFTISSLQPEDIATYYCQQHYNTPLTFGGGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC

SEQ ID NO:42.

**Example 4: Expression of anti-B7-H4 humanized antibodies**

The recombinant vector was designed according to the sequences of the humanized antibodies. The heavy chain vector was designed as follows: signal peptide + humanized heavy chain. The light chain vector was designed as follows: signal peptide + humanized light chain.

The above sequences were inserted into the pCEP4 vector (ThermoFisher #V04450). The expression vector was synthesized according to the above design. The vector plasmid was obtained and extracted in large scale and sent for sequencing for verification. The qualified plasmid was transfected into human 293F cells (ThermoFisher # R79007) with PEI (ThermoFisher # BMS1003-A). The 293F cells were continuously cultured with serum-free medium (Shanghai OPM Biosciences, OPM-293 CD03) to the logarithmic growth phase and used for cell transfection. 21.4μg of humanized antibody light chain plasmid and 23.6μg of humanized antibody heavy chain plasmid were dissolved in 10ml Opti-MEM® I Reduced Serum Medium (GIBCO, 31985-070) and mixed well, then 200 μg PEI was added and mixed well, incubated at RT for 15 min. 50 mL of cells were then added. Cell culture conditions: 5% $CO_2$, 37°C, 125 rpm/min. During the culture, supplements were added on the day 1 and day 3 until the cell viability was less than 70%. The cell supernatant was collected, centrifuged and filtered. The centrifuged and filtered cell culture medium was loaded onto the antibody purification affinity column. The column was washed with phosphate buffer. The sample was eluted with glycine hydrochloride buffer (pH 2.7 0.1 M Gly-HCl), neutralized with 1 M Tris hydrochloride pH 9.0, and dialyzed against phosphate buffer to finally obtain the purified humanized antibody, which could be used in experiments of each Example.

**Examples 5: Detection *of in vitro* binding ability of humanized antibodies**

The designed humanized antibodies were tested in experiments *in vitro* as follows:
1. In vitro cell binding experiment:
The cultured MX-1 cells expressing human B7-H4 (CLS Cell Lines Service GmbH #300296) were collected, the cell density was adjusted with PBS at pH 7.4, and the cells were plated on a 96-well plate with V-shaped bottom at $1\times10^5$

cells per well. The plate was centrifuged at 2000 rpm for 5 minutes and the supernatant was removed. 100 μl of the gradient diluted humanized antibody solution (diluted with PBS containing 0.5% BSA, starting from 1 μM, a 3-fold gradient of 10 doses) was added to each well, mixed well and incubated at 4°C on a shaker for 1 hour. The plate was centrifuged at 2000 rpm for 5 minutes and the supernatant was removed. The cells were washed twice with PBS. 100 μl of FITC-labeled goat anti-human secondary antibody (Abcam, cat#ab97224) diluted with 0.5% BSA in PBS was added to each well, mixed well and incubated for 30 minutes at 4°C on a shaker. The plate was centrifuged at 2000 rpm for 5 minutes and the supernatant was removed. The cells were washed twice with PBS and then resuspended in PBS. The signal was detected by using a flow cytometer (BECKMAN COULTER, model DxFLEX), and a concentration curve was plotted for result analysis. The results are as shown in Table 1. The humanized antibodies hu2G6 and hu2F7 both bind positively to MX-1 cells with high expression of B7-H4.

Table 1. Affinity ($EC_{50}$) of each humanized antibody to MX-1 cells

| Antibody name | FACS Binding $EC_{50}$ (nM) with MX-1 cells |
|---|---|
| hu2G6 | 14.3 |
| hu2F8 | 7.26 |
| hu1C9 | 7.25 |
| hu2F7 | 7.32 |

2. Affinity kinetics experiment:

The Biacore method is a generally acknowledged method for objectively detecting the affinity and kinetics between proteins. The affinity and binding kinetics of the antibodies to be tested of the present invention were analyzed by Biacore T200 (GE). The anti-B7-H4 antibodies to be tested of the present invention were covalently linked to CM5 (GE) chips by using the kit provided by Biacore and the NHS standard amino couplingmethod. Then 50 nM of human huB7-H4-His protein diluted in the same buffer was injected at a flow rate of 10 uL/min. After the injection, the samples were all regenerated with the regeneration reagent in the kit. The antigen-antibody binding kinetics was tracked for 3 minutes and the dissociation kinetics was tracked for 10 minutes. The obtained data were analyzed with a 1:1 (Langmuir) binding model by using BIAevaluation software of GE. The affinity kinetics data of the humanized antibodies calculated by this method are as shown in Table 2. The humanized antibodies hu2G6, hu2F7, hu2F8 and hu1C9 all show strong affinity to the human B7-H4 antigen protein.

Table 2. Affinity and kinetics characterization of each humanized antibody

| Antibody | Binding rate $k_a$(1/M*s) | Dissociation rate $k_d$ (1/s) | Affinity $K_D$ |
|---|---|---|---|
| hu2G6 | 7.41e+05 | 1.00e-05 | 1.35e-11 |
| hu2F8 | 4.47e+05 | 1.12e-04 | 2.50e-10 |
| hu1C9 | 3.35e+05 | 1.00e-05 | 2.98e-11 |
| hu2F7 | 3.29e+05 | 2.49e-04 | 7.57e-10 |

**Example 6: Endocytosis of anti-B7-H4 antibodies**

In order to test whether the antibodies of the present invention could be endocytosed along with human B7-H4 after binding to B7-H4, MX-1 cells were used for evaluation. MX-1 cells were trypsinized (first washed with PBS once at 37°C for about 2 min), collected and resuspended in pre-chilled FACS buffer. The cell concentration was adjusted to $1\times10^6$ cells/mL. 1 mL of the cell suspension was added to an EP tube, centrifuged at 1500 rpm for 5 minutes, and the supernatant was removed. 1 mL of the prepared antibody to be tested was added to resuspend the cells, and the final concentration of the antibody was 20 μg/ml. The cells were incubated on a 4°C shaker for 1 hour, centrifuged, and the supernatant was discarded (4°C, 1500 rpm×5 min). The cells were washed twice with FACS buffer and the supernatant was removed. 100 μL of fluorescent secondary antibody working solution was added to each tube to resuspend the cells. The cells were incubated on a 4°C shaker for 30 min, centrifuged, and the supernatant was discarded (4°C, 1500 rpm×5 min). The cells were washed twice with FACS buffer and the supernatant was removed. 1.0 mL of pre-warmed MX-1 cell complete medium was added to each tube to resuspend the cells and mixed. The cell suspension was aliquoted into 4 tubes at 200 μL per tube, which were respectively the 0 min group, blank group, 30 min group and 2 h group. The 0 min and blank groups were placed on ice, while the other groups were placed in a 37°C incubator for endocytosis for

30 min and 2 h respectively. At the corresponding time point, the EP tube was taken out and placed on ice to pre-chill for 5 min. All treatment groups were centrifuged and the supernatant was discarded (4°C, 1500 rpm×5 min). The cells were washed once with FACS buffer and the supernatant was removed. 250 μL strip buffer was added to EP tubes of all treatment groups except the 0 min group and incubated for 8 min at room temperature. The cells were centrifuged and the supernatant was discraded (4°C, 1500 rpm×5 min). The cells were washed twice with FACS buffer and the supernatant was removed. All treatment groups were added with 100 μL immunostaining fixative, placed at 4°C for more than 30 min, and detected by the flow cytometer DxFlex. Endocytosis percentage of B7-H4 antibody (%) = (average fluorescence intensity value at each time point - average fluorescence intensity value of the blank group) / (average fluorescence intensity value at zero point - average fluorescence intensity value of the blank group) × 100%. The data are shown in Table 3:

Table 3. Humanized antibody-mediated endocytosis efficiency of B7-H4 protein

| Antibody | 0 hour (%) | 0.5 hour (%) | 2 hours (%) |
|---|---|---|---|
| IgG control | 0 | 0.1 | 0.9 |
| hu2G6 | 0 | 18.6 | 28.7 |
| hu2F7 | 0 | 13.9 | 25.9 |

**Example 7: Conjugation of antibodies to MC-MMAF**

The antibodies of the present invention have cell affinity activity and endocytosis activity, making them suitable for coupling with drugs to form antibody-drug conjugates for treating B7-H4-mediated diseases. The coupling process is shown in the following equation, wherein Ab represents hu2G6 or hu2F7:

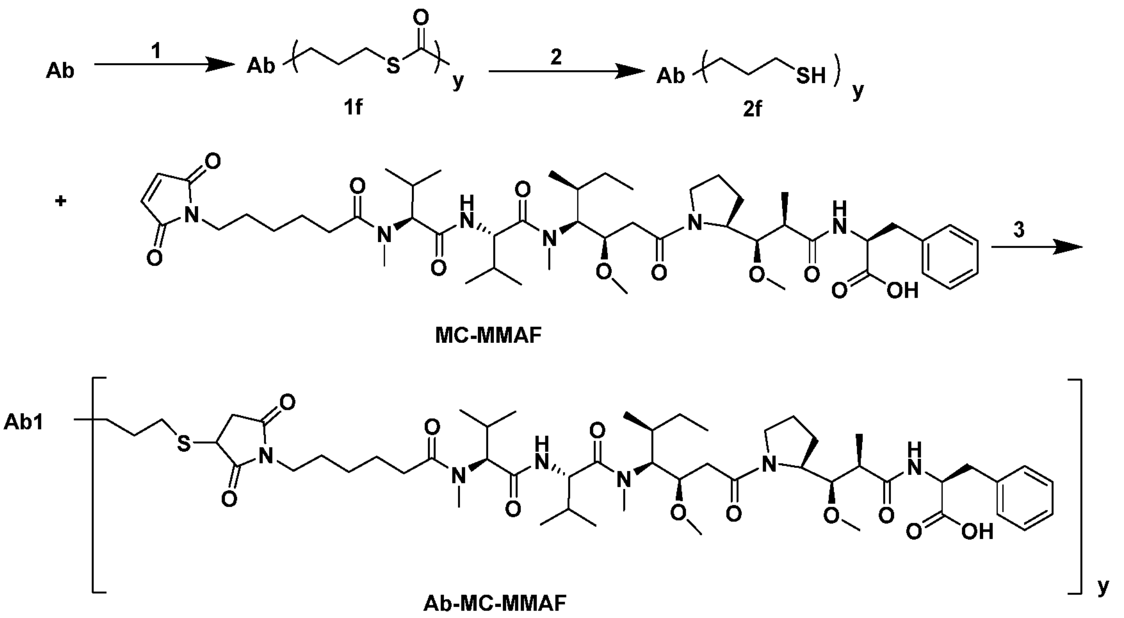

1f
2f
MC-MMAF
Ab-MC-MMAF

In the first step, S-(3-hydroxypropyl) thioacetate (0.7 mg, 5.3 mol) was dissolved in 0.9 mL acetonitrile to form a solution for later use. The above pre-prepared acetonitrile solution of S-(3-hydroxypropyl) thioacetate was added to the antibody in pH=4.3 acetate/sodium acetate buffer (10.35 mg/mL, 9.0 mL, 0.97 mol). Then 1.0 mL aqueous solution of sodium cyanoborohydride (14.1 mg, 224 mol) was added dropwise into the reaction mixture and reacted under shaking at 25°C for 2 hours. After completion of the reaction, the reaction mixture was desalted and purified by using Sephadex G25 gel column (elution phase: pH 6.5 0.05 M PBS solution) to obtain a solution of product 1f. The solution was concentrated to 10 mg/mL and directly used in the next reaction.

In the second step, lf solution (11.0 mL) was added with 0.35 mL of 2.0 M carboxyamine hydrochloride solution and reacted under shaking at 25°C for 30 minutes. Then the reaction solution was desalted and purified by using Sephadex G25 gel column (elution phase: pH 6.5 0.05 M PBS solution) to obtain a solution of product 2f (concentration 6.17 mg/mL, 14.7 mL).

In the third step, the compound MC-MMAF (1.1 mg, 1.2 mol, prepared by the method disclosed in PCT patent WO2005081711) was dissolved in 0.3 mL acetonitrile, added into 2f solution (concentration 6.17 mg/mL, 3.0 mL) and reacted under shaking at 25°C for 4 hours. Then the reaction solution was desalted and purified by Sephadex G25 gel column (elution phase: pH 6.5 0.05 M PBS solution), and filtered under sterile conditions with a filter to obtain the product Ab-MC -MMAF antibody-drug conjugate in PBS buffer (3.7 mg/mL, 4.7 mL), which was refrigerated at 4°C. The average value y of the product hu2G6-MC-MMAF determined by HIC-HPLC was 3.8, and samples of hu2G6-MC-MMAF (y=4) were obtained by HIC-HPLC purification. The average value y of the product hu2F7-MC-MMAF determined by HIC-HPLC was 3.2, and samples of hu2F7-MC-MMAF (y=2) and hu2F7-MC-MMAF (y=4) were obtained by HIC-HPLC purification.

Example 8: Conjugation of antibodies to SN-38

Antibody-conjugated drugs were prepared through the following coupling process, wherein Ab represents hu2F7:

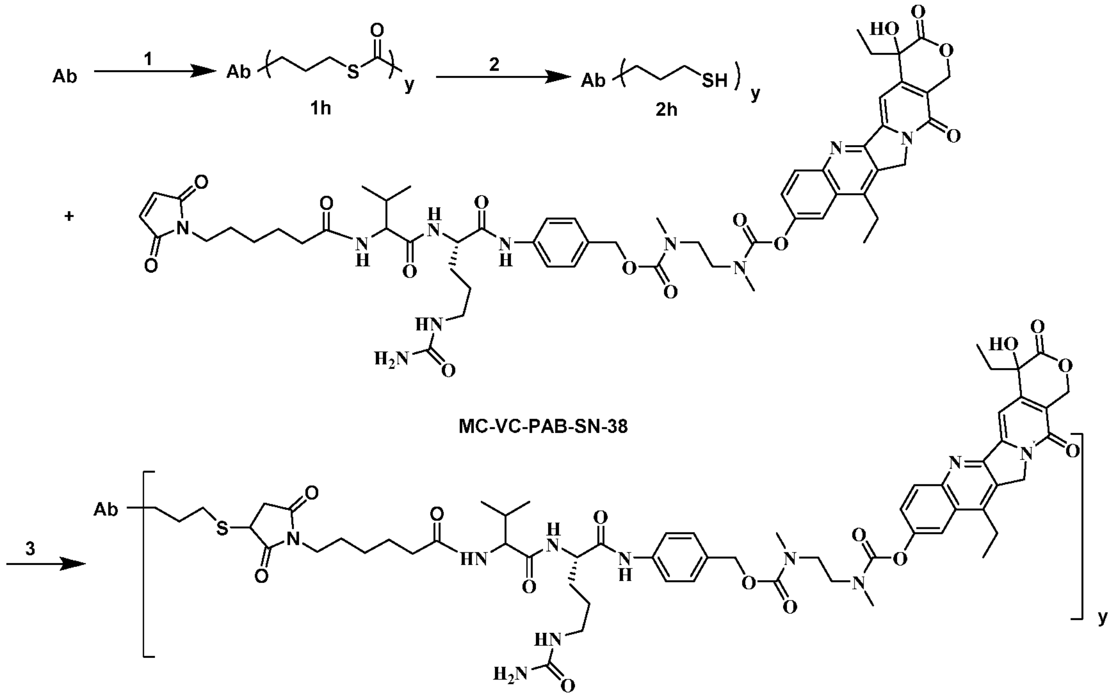


Ab-SN-38

In the first step, S-(3-hydroxypropyl) thioacetate (0.7 mg, 5.3 mol) was dissolved in 0.9 mL acetonitrile to form a solution for later use. The above pre-prepared acetonitrile solution of S-(3-hydroxypropyl) thioacetate was added to the antibody in pH=4.3 acetate/sodium acetate buffer (10.35 mg/mL, 9.0 mL, 0.97 mol). Then 1.0 mL aqueous solution of sodium cyanoborohydride (14.1 mg, 224 mol) was added dropwise into the reaction mixture and reacted under shaking at 25°C for 2 hours. After completion of the reaction, the reaction mixture was desalted and purified by using Sephadex G25 gel column (elution phase: pH 6.5 0.05 M PBS solution) to obtain a solution of product 1h. The solution was concentrated to 10 mg/mL and directly used in the next reaction.

In the second step, 1h solution (11.0 mL) was added with 0.35 mL of 2.0 M carboxyamine hydrochloride solution and reacted under shaking at 25°C for 30 minutes. Then the reaction solution was desalted and purified by using Sephadex G25 gel column (elution phase: pH 6.5 0.05 M PBS solution) to obtain a solution of product 2h (concentration 6.2 mg/mL, 15.0 mL). The 2h solution was concentrated to about 10 mg/ml and used in the next reaction.

In the third step, the compound MC-VC-PAB-SN-38 (1.3 mg, 1.2 mol) was dissolved in 0.3 mL acetonitrile, added into 2h solution (concentration 6.2 mg/mL, 3.0 mL) and reacted under shaking at 25°C for 4 hours. Then the reaction solution was desalted and purified by Sephadex G25 gel column (elution phase: pH 6.5 0.05 M PBS solution), and filtered under sterile conditions with a filter to obtain the product hu2F7-SN-38 antibody-drug conjugate in PBS buffer (3.7 mg/mL, 4.7 mL), which was refrigerated at 4°C. The average value y was determined by the ultraviolet method. Cuvettes filled with sodium succinate buffer were placed in the reference absorption cell and in the sample determination absorption cell respectively, and after deducting the solvent blank, the cuvettes filled with the test solution were placed

in the sample determination absorption cell. The absorbance at 280 nm and 370 nm was measured.

Data processing:

The antibody content $C_{mab}$ was determined by establishing a standard curve and measuring the absorption at the wavelength of 280 nm. The small molecule content $C_{Drug}$ was determined by measuring the absorption at the wavelength of 370 nm.

$$\text{Average drug load } y=C_{Drug}/C_{mab}$$

The average value y=3.7 of the product hu2F7-SN-38 was determined by the above method. Samples of hu2F7-SN-38 (y=4) were obtained by UV-HPLC purification.

**Example 9: Conjugation of antibodies to Exatecan**

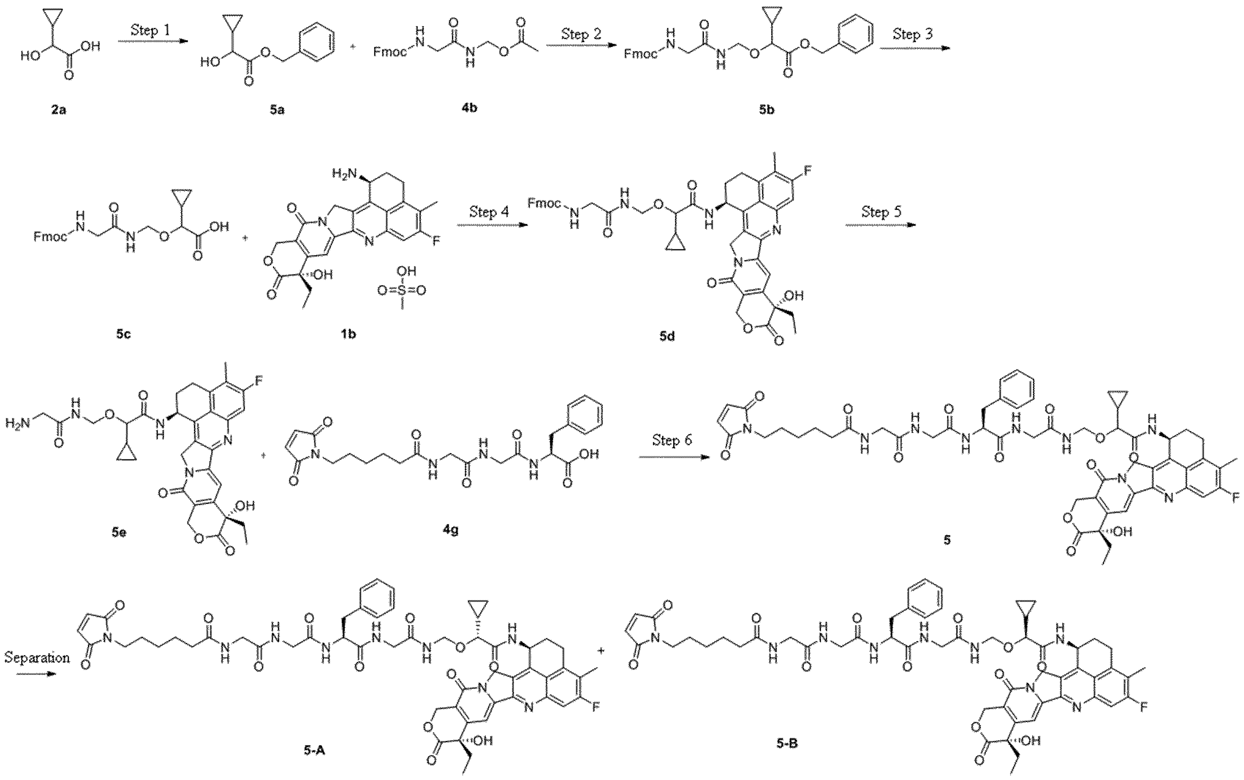


In the first step, **2a** (2 g, 17.2 mmol) was dissolved in 75 mL acetonitrile and added successively with potassium carbonate (9.27 g, 67.2 mmol), benzyl bromide (20 mL, 167.2 mmol) and tetrabutylammonium iodide (620 mg, 1.68 mmol). The reaction solution was stirred at room temperature for 48 hours and filtered through diatomaceous earth. The filter cake was rinsed with ethyl acetate (20 ml). The filtrate was pooled and concentrated under reduced pressure. The obtained residues were purified by silica gel column chromatography with developing solvent system C to obtain product **5a** (3.2 g, yield: 90.1%).

In the second step, **5a** (181.3 mg, 0.879 mmol) and **4b** (270 mg, 0.733 mmol) were added into a reaction flask, added with 6 mL tetrahydrofuran and replaced with argon three times. The reaction mixture was cooled to 0-5°C in an ice-water bath and added with potassium tert-butoxide (164 mg, 1.46 mmol), then warmed to room temperature by removing the ice bath and stirred for 40 minutes. The reaction mixture was added with 15 mL ice water and extracted with ethyl acetate (40 mL×2) and chloroform (20 mL×5). The organic phases were pooled and concentrated. The obtained residues were dissolved in 6 mL dioxane, added with 3 mL water, sodium bicarbonate (73.8 mg, 0.879 mmol) and 9-fluorene methyl chloroformate (190 mg, 0.734 mmol) and stirred at room temperature for 2 hours. The reaction solution was added with 30 mL water and extracted with ethyl acetate (20 mL×3). The organic phases were washed with saturated sodium chloride solution (30 mL), dried by using anhydrous sodium sulfate and filtered. The filtrate was concentrated under reduced pressure. The obtained residues were purified by silica gel column chromatography with

the developing solvent system C to obtain product **5b** Benzyl 10-cyclopropyl-1-(9H-fluoren-9-yl)-3,6-dioxo-2,9-dioxa-4,7-diazaundec-11-ate (73 mg, yield: 19.4%).

MS m/z (ESI): 515.0 [M+1].

In the third step, **5b** (30 mg, 0.058 mmol) was dissolved in 6.75 mL of a mixed solvent of tetrahydrofuran and ethyl acetate (V:V=2:1), added with palladium on carbon (18 mg, content 10%, dry basis), replaced with hydrogen three times, and reacted under stirring at room temperature for 1 hour. The reaction solution was filtered with diatomaceous earth. The filter cake was rinsed with ethyl acetate. The filtrate was concentrated to obtain the crude product **5c** 10-cyclopropyl-1-(9*H*-fluoren-9-yl)-3,6-dioxo-2,9-dioxa-4,7-diazaundec-11-acid (20 mg), which was directly used in the next reactopm without purification.

MS m/z (ESI): 424.9 [M+1].

In the fourth step, **1b** (15 mg, 28.2 μmol) was added into a reaction flask, added with 1.5 mL *N, N*-dimethylformamide and replaced with argon three times. The reaction mixture was cooled to 0-5°C in an ice-water bath, added with a drop of triethylamine, added with the crude product **5c** (20 mg, 47.1 μmol), added with 4-(4,6-dimethoxy-1,3,5-triazin-2-yl)-4-methylchloromorpholine (25.4 mg, 86.2 μmol) and reacted under stirring in an ice bath for 40 minutes. The reaction mixture was added with 15 mL water and extracted with ethyl acetate (20 mL×3). The organic phases were pooled. The organic phases were washed with saturated sodium chloride solution (20 mL×2), dried by using anhydrous sodium sulfate and filtered. The filtrate was concentrated under reduced pressure. The obtained residues were purified by thin layer chromatography with the developing solvent system B to obtain the title product **5d** (9H-fluoren-9-yl)methyl(2-(((1-cyclopropyl-2-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolozino[1,2-*b*]quinolin-1-yl)amino)-2-oxoethoxy)methyl)amino)-2-oxoethyl)carbamate (23.7 mg, yield: 78.9%).

MS m/z (ESI): 842.1[M+1].

In the fifth step, **5d** (30 mg, 35.7 μmol) was dissolved in 3 mL dichloromethane, added with 1.5 mL diethylamine and stirred at room temperature for 2 hours. The reaction solution was concentrated under reduced pressure, added with 1.5 mL toluene and concentrated under reduced pressure, repeating twice. The residues were added with 4.5 mL n-hexane and pulped. The supernatant was poured out after letting standing and the solid was kept. The solid residues were concentrated under reduced pressure and pumped dry to obtain the crude product **5e** 2-((2-aminoacetamido)methoxy)-2- cyclopropyl-*N*-((1*S*,9*S*)-9-ethyl-5-fluoro-9-hydroxy-4-methyl-10,13-dioxo-2,3,9,10,13, 15-hexahydro-1*H*,12*H*-benzo[*de*]pyrano[3',4':6,7]indolozino[1,2-*b*]quinolin-1-yl) acetamide (23 mg), which was directly used in the next reaction without purification.

MS m/z (ESI): 638.0[M+18].

In the sixth step, the crude product **5e** (20 mg, 32.3 μmol) was dissolved in 1 mL *N*, *N*-dimethylformamide and replaced with argon three times. The reaction mixture was cooled to 0-5°C in an ice-water bath, added with 0.5 mL of *N, N*-dimethylformamide solution of **4g** (31.8 mg, 67.3 μmol), added with 4-(4,6-dimethoxy-1,3,5-triazin-2-yl) -4-methylchloromorpholine (27.8 mg, 94.3 μmol) and reacted under stirring in an ice bath for 10 minutes. The reaction mixture was warmed to room temperature by removing the ice bath and reacted under stirring for 1 hour to produce compound **5**. The reaction solution was purified by high performance liquid chromatography (separation conditions: column: XBridge Prep C18 OBD 5 μm 19*250 mm; mobile phase: A-water (10 mmol $NH_4OAc$): B-acetonitrile, gradient elution, flow rate: 18 mL/min). The corresponding components were collected and concentrated under reduced pressure to obtain products 5-A and 5-B (3.6 mg, 2.6 mg).

MS m/z (ESI): 1074.4 [M+1].

Single configuration compound 5-A (shorter retention time)

UPLC analysis: retention time 1.14 minutes, purity: 85% (column: ACQUITY UPLC BEHC18 1.7 μm 2.1*50 mm, mobile phase: A-water (5 mmol $NH_4OAc$), B-acetonitrile).

$^1H$ NMR (400 MHz, DMSO-$d_6$): δ 8.60 (t, 1H), 8.51-8.49 (d, 1H), 8.32-8.24 (m, 1H), 8.13-8.02 (m, 2H), 8.02-7.96 (m, 1H), 7.82-7.75 (m, 1H), 7.31 (s, 1H), 7.26-7.15 (m, 4H), 6.99 (s, 1H), 6.55-6.48 (m, 1H), 5.65-5.54 (m, 1H), 5.41 (s, 2H), 5.35-5.15 (m, 3H), 4.74-4.62 (m, 2H), 4.54-4.40 (m, 2H), 3.76-3.64 (m, 4H), 3.62-3.48 (m, 2H), 3.20-3.07 (m, 2H), 3.04-2.94 (m, 2H), 2.80-2.62 (m, 2H), 2.45-2.30 (m, 3H), 2.25-2.15 (m, 2H), 2.15-2.04 (m, 2H), 1.93-1.78 (m, 2H), 1.52-1.39 (m, 3H), 1.34-1.12 (m, 5H), 0.87 (t, 3H), 0.64-0.38 (m, 4H).

Single configuration compound 5-B (longer retention time):
UPLC analysis: retention time 1.16 minutes, purity: 89% (column: ACQUITY UPLC BEHC18 1.7 μm 2.1*50 mm, mobile phase: A-water (5 mmol $NH_4OAc$), B-acetonitrile).

$^1H$ NMR (400 MHz, DMSO-$d_6$): δ 8.68-8.60 (m, 1H), 8.58-8.50 (m, 1H), 8.32-8.24 (m, 1H), 8.13-8.02 (m, 2H), 8.02-7.94 (m, 1H), 7.82-7.75 (m, 1H), 7.31 (s, 1H), 7.26-7.13 (m, 4H), 6.99 (s, 1H), 6.55-6.48 (m, 1H), 5.60-5.50 (m, 1H), 5.41 (s, 2H), 5.35-5.15 (m, 3H), 4.78-4.68 (m, 1H), 4.60-4.40 (m, 2H), 3.76-3.58 (m, 4H), 3.58-3.48 (m, 1H), 3.20-3.10 (m, 2H), 3.08-2.97 (m, 2H), 2.80-2.72 (m, 2H), 2.45-2.30 (m, 3H), 2.25-2.13 (m, 2H), 2.13-2.04 (m, 2H), 2.03-1.94 (m,

2H), 1.91-1.78 (m, 2H), 1.52-1.39 (m, 3H), 1.34-1.12 (m, 5H), 0.91-0.79 (m, 3H), 0.53-0.34 (m, 4H).

The preparation methods of other intermediates were with reference to that of intermediate 5.

The PBS buffered aqueous solution of antibody hu2F7 (pH=6.5 0.05 M PBS buffered aqueous solution; 7.3 ml, 13.8 mg/ml, 0.681 μmol) was added with a prepared aqueous solution of tris(2-carboxyethyl) phosphine (10 mM, 0.347 mL, 3.47 μmol) at 37 °C. The reaction mixture was placed in a water bath shaker and reacted under shaking at 37°C for 3 hours. The reaction was terminated, and the reaction solution was cooled to 25°C in a water bath, diluted to 14.0 ml, and 3.3 ml of the solution was taken out for the next reaction.

Compound **5-A** (3.0 mg, 3.72 μmol) was dissolved in 0.15 mL DMSO and added into the above 3.3 ml solution. The reaction mixture was placed in a water bath shaker and reacted under shaking at 25°C for 3 hours. The reaction was terminated. The reaction solution was desalted and purified by using a Sephadex G25 gel column (elution phase: pH 6.5 0.05 M PBS buffered aqueous solution, containing 0.001 M EDTA) to obtain an exemplary product of Ab-Exatecan, hu2F7-Exatecan (Compound 34) in PBS buffer (1.35 mg/mL, 13 mL), which was stored at 4°C. The average value y was determined by the ultraviolet method. Cuvettes filled with sodium succinate buffer were respectively placed in the reference absorption cell and the sample determination absorption cell, and after deducting the solvent blank, the cuvettes filled with the test solution were placed in the sample determination absorption cell. The absorbance at 280 nm and 370 nm was measured.

Data processing:

The antibody content $C_{mab}$ was determined by establishing a standard curve and measuring the absorption at the wavelength of 280 nm. The small molecule content $C_{Drug}$ was determined by measuring the absorption at the wavelength of 370 nm.

$$\text{Average drug load } y = C_{Drug}/C_{mab}$$

As for the exemplary product hu2F7-Exatecan (compound 34), y was determined to be 7.6 by the above method. Samples of hu2F7-Exatecan (y=8) were obtained by UV-HPLC purification.

The preparation methods of other antibody conjugates were with reference to that of compound 34.

**Example 10: The killing activity of antibody-drug conjugates on tumor cells**

In order to test the killing effect of the antibody-drug conjugates of the present invention on tumor cells, MX-1 breast cancer cells were used for evaluation. MX-1 cells were collected, centrifuged and counted. The cell density was adjusted to $0.44\times10^6$ cells/mL with complete medium, and the cells were plated in 60 wells of a white 96-well plate at 90 μL per well with a cell number of 40,000. The rest peripheral wells were added with 100 μL PBS. The cell plate was placed in a 37°C, 5% $CO_2$ incubator and cultured overnight. On the second day of the experiment, the antibody-drug conjugate solution was prepared with PBS in a 96-well V-bottom plate, starting from a concentration of 1000 nM (3-fold dilution and 9 concentrations). After completion of the preparation, the solution was added to a white 96-well plate at 10 μL per well in duplicates. The cell plate was placed in a 37°C, 5% $CO_2$ incubator and the culture lasted for 72 hours. On the fifth day of the experiment, the plate was detected and read. The cell culture plate was taken out. Two control wells were set up. 100 μL medium containing 40,000 cells was added to each well, and after equilibrating to room temperature, 50 μL CTG solution (Promega G7573) was added to each well. The mixture was shaken and mixed, placed in dark and let stand for 10 minutes, and then detected by using the luminescence program of the microplate reader. Maximum killing rate = (1-fluorescence value of 1000 nM well / fluorescence value of the control well)%. The experimental results are as shown in Table 4:

Table 4: Evaluation of the killing activity of antibody-drug conjugates on tumor cells

| Antibody-drug conjugate | $IC_{50}$ (nM) | Killing effect at maximum dose |
|---|---|---|
| IgG-MC-MMAF (control) | ND | ND |
| hu2G6-MC-MMAF (compound 1, y=4) | 1.1 | 48.2% |
| hu2F7-MC-MMAF (compound 2, y=4) | 3.4 | 53.9% |
| hu2F7-SN-38 (compound 6, y=4) | 78.85 | 56.4% |
| hu2F7-Exatecan (compound 34, y=8) | 80.28 | 60.39% |
| ND: No activity detectable. | | |

**Example 11: The inhibiton effect of antibody-drug conjugates on growth of tumor cells**

In order to test the killing effect of the antibody-drug conjugates of the present invention on tumor cells, SK-BR-3 (ATCC #HTB30) breast cancer cells were used for evaluation. SK-BR-3 cells were collected, centrifuged and counted. The cell density was adjusted to $0.44\times10^6$ cells/mL with complete medium, and the cells were plated in 60 wells of a white 96-well plate at 90 $\mu$L per well with a cell number of 40,000. The rest peripheral wells were added with 100 $\mu$L PBS. The cell plate was placed in a 37°C, 5% $CO_2$ incubator and cultured overnight. On the second day of the experiment, the antibody-drug conjugate solution was prepared with PBS in a 96-well V-bottom plate, starting from a concentration of 1000 nM (3-fold dilution and 9 concentrations). After completion of the preparation, the solution was added to a white 96-well plate at 10 $\mu$L per well in duplicates. Two other control wells were set up and 10 $\mu$L PBS was added to each well. The cell plate was placed in a 37°C, 5% $CO_2$ incubator and the culture lasted for 72 hours. On the fifth day of the experiment, the plate was detected and read. The cell culture plate was taken out. After equilibrating to room temperature, 50 $\mu$L CTG solution (Promega G7573) was added to each well. The mixture was shaken and mixed, placed in dark and let stand for 10 minutes, and then detected by using the luminescence program of the microplate reader. Maximum inhibition rate = (1-fluorescence value of 1000 nM well / fluorescence value of the control well)%. The experimental results are as shown in Table 5:

Table 5: Evaluation of the inhibition activity of antibody-drug conjugates on tumor cells

| Antibody-drug conjugate | $IC_{50}$ (nM) | Maximum inhibition rate |
|---|---|---|
| IgG-MC-MMAF (control) | ND | ND |
| hu2G6-MC-MMAF (compound 1, y=4) | 1.0 | 90.3% |
| hu2F7-MC-MMAF (compound 2, y=4) | 9.5 | 91.8% |
| ND: No activity detectable. | | |

**Example 12: Evaluation of the *in vivo* efficacy of antibody-drug conjugates conjugated to MMAF**

After the formation of transplanted tumors with MX-1 cells in mice, the anti-tumor effect of the antibody-drug conjugates of the present invention was evaluated. $5\times10^6$ MX-1 cells were injected subcutaneously into immunodeficient nude mice (BALB/c Nude). After 2 weeks, intravenous injection of the antibody-drug conjugates hu2G6-MC- MMAF and hu2F7-MC-MMAF was performed, at a frequency of once/week and a dose of 1.5 mg/kg or 3 mg/kg. Human IgG1 protein was used as the control at a dose of 3 mg/kg. There were 5 mice in each group of the control group or the administration group. The tumor inhibition rate was calculated by measuring the tumor volume. Tumor inhibition rate = 100% - (tumor volume of the administration group on day 21 - tumor volume of the administration group on day 0) / (tumor volume of the control group on day 21 - tumor volume of the control group on day 0). The experimental results are as shown in Figure 1 and Table 6. Both hu2G6-MC-MMAF and hu2F7-MC-MMAF show dose-dependent anti-tumor effect. Both hu2G6-MC-MMAF and hu2F7-MC-MMAF show tumor inhibition rate of more than 100% at the dose of 3 mg/kg, which means that the antibody-drug conjugates of the present invention can not only inhibit tumor growth, but also exert killing effect on the tumor already formed.

Table 6. Efficacy of administered compounds on MX-1 transplanted tumor in tumor-bearing nude mice

| Administration group | Tumor inhibition rate |
|---|---|
| hu2G6-MC-MMAF (compound 1, y=4) 1.5 mg/kg | 38.0% |
| hu2G6-MC-MMAF (compound 1, y=4) 3 mg/kg | 115.6% |
| hu2F7-MC-MMAF (compound 2, y=4) 1.5 mg/kg | 90.3% |
| hu2F7-MC-MMAF (compound 2, y=4) 3 mg/kg | 163.8% |

**Example 13: Evaluation of the *in vivo* efficacy of antibody-drug conjugates with different drug loads**

In order to further study the antibody-drug conjugates with different drug loads, the antibody-drug conjugates were prepared by the method of Example 7 and purified by HPLC to obtain hu2F7-MC-MMAF (y=2) and hu2F7-MC-MMAF (y=4) (Figure 2). After formation of transplanted tumors with MX-1 in mice, the anti-tumor effect of the antibody-drug conjugates of the present invention was evaluated. $5\times10^6$ MX-1 cells were injected subcutaneously into immunodeficient nude mice. After 2 weeks, intravenous injection of the antibody-drug conjugates hu2F7-MC-MMAF (y=2) and hu2F7-MC-MMAF (y=4) was performed, at a frequency of once/week and a dose of 3 mg/kg. Human IgG1 protein was used as the control at a dose of 3 mg/kg. There were 5 mice in each group of the control group or the administration group. The tumor inhibition rate was calculated by measuring the tumor volume. Tumor inhibition rate = 100% - (tumor volume of the administration group on day 21 - tumor volume of the administration group on day 0) / (tumor volume of the control group on day 21 - tumor volume of the control group on day 0). The experimental results are as shown in Figure 3 and Table 7. Both hu2F7-MC-MMAF (y=2) and hu2F7-MC-MMAF (y=4) show anti-tumor effect. hu2F7-MC-MMAF (y=4) has stronger anti-tumor effect than hu2F7-MC-MMAF (y=2) at the dose of 3 mg/kg, showing a tumor inhibition rate of more than 100%, which means that hu2F7-MC-MMAF (y=4) can not only inhibit tumor growth, but also exert killing effect on the tumor already formed (Figure 4).

Table 7. Efficacy of antibody-drug conjugates with different drug loads on MX-1 transplanted tumor in tumor-bearing nude mice

| Administration group | Tumor inhibition rate |
|---|---|
| hu2F7-MC-MMAF (y=2) 3 mg/kg | 87.99% |
| hu2F7-MC-MMAF (y=4) 3 mg/kg | 197.87% |

**Example 14: Evaluation of the *in vivo* efficacy of antibody-drug conjugates conjugated to Exatecan**

In order to study the antibody-drug conjugates conjugated to Exatecan, the antibody-drug conjugates were prepared by the method of Example 9 and purified by HPLC to obtain hu2F7-Exatecan (compound 34, y=8). After formation of transplanted tumors with MX-1 in mice, the anti-tumor effect of the antibody-drug conjugates of the present invention was evaluated. $5\times10^6$ MX-1 cells were injected subcutaneously into immunodeficient nude mice. After 2 weeks, intravenous injection of the antibody-drug conjugate hu2F7-Exatecan (y=8) was performed, at a frequency of once/week and a dose of 5 mg/kg and 10 mg/kg. Human IgG1 protein was used as the control at a dose of 5mg/kg. There were 5 mice in each group of the control group or the administration group. The tumor inhibition rate was calculated by measuring the tumor volume. Tumor inhibition rate = 100% - (tumor volume of the administration group on day 18 - tumor volume of the administration group on day 0) / (tumor volume of the control group on day 18 - tumor volume of the control group on day 0). The experimental results are as shown in Table 8. hu2F7-Exatecan shows a tumor inhibition rate of more than 100% at the doses of both 5 mg/kg and 10 mg/kg, which means that hu2F7-Exatecan (y=8) can not only inhibit tumor growth, but also exert killing effect on the tumor already formed.

Table 8. Efficacy of antibody-drug conjugates conjugated to Exatecan on MX-1 transplanted tumor in tumor-bearing nude mice

| Administration group | Tumor inhibition rate |
|---|---|
| hu2F7-Exatecan (compound 34, y=8) 5 mg/kg | 173.55% |
| hu2F7-Exatecan (compound 34, y=8) 10 mg/kg | 183.48% |

**Example 15: Experiment of the bystander killing activity of antibody-drug conjugates conjugated to Exatecan**

In order to study the effect of the antibody-drug conjugates conjugated to Exatecan of the present invention on killing B7-H4 negative cells through the bystander effect, the killing activity of the antibody-drug conjugates on a myeloma cell line with negative B7-H4 expression was detected by using ONE-Glo™ Luciferase Assay detection kit. MX-1 cells were collected, centrifuged, counted, adjusted to a cell suspension of $7.5\times10^6$ cells/mL with complete medium and plated onto a white 96-well plate. 33 μL of MX-1 cell suspension was added to each well in the "mixed cell group", and 33 μL PBS was added to each well in the "single cell group". NCI-H929-LUC tumor cells with negative B7-H4 expression (Cobioer catalog number CBP30061L) were collected. The cell density was adjusted to $1.5\times10^6$ cells/mL. 33 μL of NCI-H929-LUC cell suspension was added to each well in the "mixed cell group" and "single cell group". The cell plate was placed in a 37°C, 5% $CO_2$ incubator for culture overnight. On the second day of the experiment, the highest concentration of the antibody-drug conjugate hu2F7-Exatecan (compound 34, y=8) to be tested was uniformly adjusted to 3000 nM, and diluted to obtaine a 5-fold gradient of 9 doses. 33.3 μL of the diluted antibody-drug conjugate working solution was added to each well of the experimental plate and mixed gently. The cell plate was placed in a 37°C, 5% incubator and incubated for 48 hours. The cell culture plate was taken out and equilibrated to room temperature. Then 100 μL ONE-Glo™ Luciferase reagent (Promega E6120) was added to each well. The cells were shaken and lysed at room temperature

in dark for 10 minutes. The cell lysate was centrifuged at 1000 rpm/min for 1 minute, and then transferred to a 96-well plate with transparent bottom at 180 μL per well. The plate was read at a wavelength of 490 nm on a microplate reader. The data were analyzed using the Graphpad Prism software, and the experimental results are as shown in Table 9. ONE-Glo™ Luciferase reagent can specifically detect the viability of NCI-H929-LUC cells. hu2F7-Exatecan effectively kills NCI-H929-LUC cells in the "mixed cell group", indicating that in the presence of B7-H4 positive cells, hu2F7-Exatecan has a bystander killing effect, and it can not only kill B7-H4 positive cells, but can also kill the insensitive B7-H4 negative cells (in "single Cell Group").

Table 9. Evalutaion of bystander killing activity

| Experimental group | $IC_{50}$ (nM) | Maximum inhibition rate |
|---|---|---|
| Mixed cell group (MX-1:NCI-LUC=5:1) | 70.34 | 76.8% |
| Single cell group (NCI-H929-LUC) | ND | ND |
| ND: No activity detectable. | | |

## Claims

1. An antibody-drug conjugate represented by general formula (A) or a pharmaceutically acceptable salt or solvate thereof,

$$\mathbf{Ab\text{-}(L_2\text{-}L_1\text{-}D)_y} \qquad \mathbf{(A)}$$

wherein:

D is a cytotoxic drug;
$L_1$ and $L_2$ are linker units;
y is a number of 1 to 20;
Ab is a B7-H4 antibody or antigen-binding fragment thereof, which comprises antibody light chain variable region and antibody heavy chain variable region,
the antibody heavy chain variable region comprises at least one HCDR as shown in the sequence selected from the group consisting of:

SEQ ID NO:3, SEQ ID NO:4, SEQ ID NO:5,
SEQ ID NO:9, SEQ ID NO: 10, SEQ ID NO:11,
SEQ ID NO:23, SEQ ID NO:24, SEQ ID NO:25,
SEQ ID NO:29, SEQ ID NO:30, SEQ ID NO:31,

the antibody light chain variable region comprises at least one LCDR as shown in the sequence selected from the group consisting of:

SEQ ID NO: 6, SEQ ID NO: 7, SEQ ID NO: 8,
SEQ ID NO: 12, SEQ ID NO: 13, SEQ ID NO: 14,

SEQ ID NO:26, SEQ ID NO:27, SEQ ID NO:28,
SEQ ID NO:32, SEQ ID NO:33, SEQ ID NO:34.

**2.** The antibody-drug conjugate or the pharmaceutically acceptable salt or solvate thereof according to claim 1, wherein the antibody heavy chain variable region of the Ab comprises the CDRs of any one selected from the group consisting of the following (1) to (4):

(1) HCDR1 as shown in SEQ ID NO:3, HCDR2 as shown in SEQ ID NO:4 and HCDR3 as shown in SEQ ID NO:5;
(2) HCDR1 as shown in SEQ ID NO:9, HCDR2 as shown in SEQ ID NO:10 and HCDR3 as shown in SEQ ID NO:11;
(3) HCDR1 as shown in SEQ ID NO:23, HCDR2 as shown in SEQ ID NO:24 and HCDR3 as shown in SEQ ID NO:25; or,
(4) HCDR1 as shown in SEQ ID NO:29, HCDR2 as shown in SEQ ID NO:30 and HCDR3 as shown in SEQ ID NO:31.

**3.** The antibody-drug conjugate or the pharmaceutically acceptable salt or solvate thereof according to claim 1, wherein the antibody light chain variable region of the Ab comprises the CDRs of any one selected from the group consisting of the following (1) to (4):

(1) LCDR1 as shown in SEQ ID NO:6, LCDR2 as shown in SEQ ID NO:7 and LCDR3 as shown in SEQ ID NO:8;
(2) LCDR1 as shown in SEQ ID NO:12, LCDR2 as shown in SEQ ID NO:13 and LCDR3 as shown in SEQ ID NO:14;
(3) LCDR1 as shown in SEQ ID NO:26, LCDR2 as shown in SEQ ID NO:27 and LCDR3 as shown in SEQ ID NO:28; or,
(4) LCDR1 as shown in SEQ ID NO:32, LCDR2 as shown in SEQ ID NO:33 and LCDR3 as shown in SEQ ID NO:34.

**4.** The antibody-drug conjugate or the pharmaceutically acceptable salt or solvate thereof according to claim 1, wherein the antibody heavy chain variable region and antibody light chain variable region of the Ab comprises the CDRs of any one selected from the group consisting of the following (1) to (4):

(1) HCDR1 as shown in SEQ ID NO:3, HCDR2 as shown in SEQ ID NO:4 and HCDR3 as shown in SEQ ID NO:5; and
LCDR1 as shown in SEQ ID NO:6, LCDR2 as shown in SEQ ID NO:7 and LCDR3 as shown in SEQ ID NO:8;
(2) HCDR1 as shown in SEQ ID NO:9, HCDR2 as shown in SEQ ID NO:10 and HCDR3 as shown in SEQ ID NO:11; and
LCDR1 as shown in SEQ ID NO:12, LCDR2 as shown in SEQ ID NO:13 and LCDR3 as shown in SEQ ID NO:14;
(3) HCDR1 as shown in SEQ ID NO:23, HCDR2 as shown in SEQ ID NO:24 and HCDR3 as shown in SEQ ID NO:25; and
LCDR1 as shown in SEQ ID NO:26, LCDR2 as shown in SEQ ID NO:27 and LCDR3 as shown in SEQ ID NO:28; or,
(4) HCDR1 as shown in SEQ ID NO:29, HCDR2 as shown in SEQ ID NO:30 and HCDR3 as shown in SEQ ID NO:31; and
LCDR1 as shown in SEQ ID NO:32, LCDR2 as shown in SEQ ID NO:33 and LCDR3 as shown in SEQ ID NO:34.

**5.** The antibody-drug conjugate or the pharmaceutically acceptable salt or solvate thereof according to any one of claims 1 to 4, wherein the Ab is a murine antibody or fragment thereof, a chimeric antibody or fragment thereof, a human antibody or fragment thereof, and a humanized antibody or fragment thereof.

**6.** The antibody-drug conjugate or the pharmaceutically acceptable salt or solvate thereof according to any one of claims 1 to 5, wherein the Ab further comprises any one of (a), (b) and (c) or combination thereof:

(a) light chain framework region(s) and heavy chain framework region(s) derived from human germline light chain and heavy chain sequences or mutant sequence(s) thereof;
(b) heavy chain constant region(s) derived from human IgG1 or variant thereof, IgG2 or variant thereof, IgG3 or variant thereof or IgG4 or variant thereof, preferably heavy chain constant region(s) derived from human IgG1, IgG2 or IgG4, more preferably heavy chain constant region(s) of IgG1 with enhanced ADCC toxicity after amino acid mutation, most preferably the heavy chain constant region as shown in SEQ ID NO: 54;

(c) light chain constant region(s) derived from human κ chain, λ chain or variant thereof, preferably light chain constant region(s) derived from human κ chain, more preferably the light chain constant region as shown in SEQ ID NO:55.

**7.** The antibody-drug conjugate or the pharmaceutically acceptable salt or solvate thereof according to any one of claims 1 to 6, wherein the Ab comprises light chain variable region(s) of the following sequences: SEQ ID NO: 16, SEQ ID NO: 18, SEQ ID NO: 36, SEQ ID NO: 38, or light chain variable region(s) with at least 70%, 75%, 80%, 85%, 90%, 95% or 99% homology to SEQ ID NO: 16, SEQ ID NO: 18, SEQ ID NO: 36, SEQ ID NO: 38.

**8.** The antibody-drug conjugate or the pharmaceutically acceptable salt or solvate thereof according to any one of claims 1 to 7, wherein the Ab comprises heavy chain variable region(s) of the following sequences: SEQ ID NO: 15, SEQ ID NO: 17, SEQ ID NO: 35, SEQ ID NO: 37, or heavy chain variable region(s) with at least 70%, 75%, 80%, 85%, 90%, 95% or 99% homology to SEQ ID NO: 15, SEQ ID NO: 17, SEQ ID NO: 35, SEQ ID NO: 37.

**9.** The antibody-drug conjugate or the pharmaceutically acceptable salt or solvate thereof according to any one of claims 1 to 8, wherein the light chain of the Ab is selected from the group consisting of the following sequences: SEQ ID NO: 20, SEQ ID NO: 22, SEQ ID NO: 40, SEQ ID NO: 42, or full-length light chain with at least 80%, 85%, 90%, 95% or 99% homology to SEQ ID NO: 20, SEQ ID NO: 22, SEQ ID NO: 40, SEQ ID NO: 42.

**10.** The antibody-drug conjugate or the pharmaceutically acceptable salt or solvate thereof according to any one of claims 1 to 9, wherein the heavy chain of the Ab is selected from the group consisting of the following sequences: SEQ ID NO: 19, SEQ ID NO: 21, SEQ ID NO: 39, SEQ ID NO: 41, or full-length heavy chain with at least 80%, 85%, 90%, 95% or 99% homology to SEQ ID NO: 19, SEQ ID NO: 21, SEQ ID NO: 39, SEQ ID NO: 41.

**11.** The antibody-drug conjugate or the pharmaceutically acceptable salt or solvate thereof according to any one of claims 1 to 10, wherein the heavy chain variable region and light chain variable region of the Ab is any one selected from the group consisting of the following:

(1) the heavy chain variable region as shown in SEQ ID NO: 15 and the light chain variable region as shown in SEQ ID NO: 16;
(2) the heavy chain variable region as shown in SEQ ID NO: 17 and the light chain variable region as shown in SEQ ID NO: 18;
(3) the heavy chain variable region as shown in SEQ ID NO: 35 and the light chain variable region as shown in SEQ ID NO: 36; or,
(4) the heavy chain variable region as shown in SEQ ID NO: 37 and the light chain variable region as shown in SEQ ID NO: 38.

**12.** The antibody-drug conjugate or the pharmaceutically acceptable salt or solvate thereof according to claim 11, wherein the Ab is any one selected from the group consisting of the following:

(1) the light chain as shown in SEQ ID NO: 20 and the heavy chain as shown in SEQ ID NO: 19;
(2) the light chain as shown in SEQ ID NO: 22 and the heavy chain as shown in SEQ ID NO: 21;
(3) the light chain as shown in SEQ ID NO: 40 and the heavy chain as shown in SEQ ID NO: 39; or,
(4) the light chain as shown in SEQ ID NO: 42 and the heavy chain as shown in SEQ ID NO: 41.

**13.** The antibody-drug conjugate or the pharmaceutically acceptable salt or solvate thereof according to any one of claims 1 to 12, wherein the antigen-binding fragment is selected from the group consisting of Fab, Fab', $F(ab')_2$, single-chain antibody, dimerized V region, disulfide bond stabilized V region and antigen-binding fragments of a peptide comprising CDRs.

**14.** The antibody-drug conjugate or the pharmaceutically acceptable salt or solvate thereof according to any one of claims 1 to 13, wherein the cytotoxic drug is selected from the group consisting of toxin, chemotherapeutic, antibiotic, radioisotope and nucleolytic enzyme.

**15.** The antibody-drug conjugate or the pharmaceutically acceptable salt or solvate thereof according to any one of claims 1 to 14, wherein the cytotoxic drug is selected from the group consisting of tubulin inhibitor or DNA topoisomerase inhibitor that inhibits cell division; preferably DM1, DM3, DM4, SN-38, MMAF or MMAE; more preferably the tubulin inhibitor SN-38, MMAE or MMAF.

**16.** The antibody-drug conjugate or the pharmaceutically acceptable salt or solvate thereof according to any one of claims 1 to 14, wherein the cytotoxic drug is selected from camptothecin derivatives, preferably Exatecan:

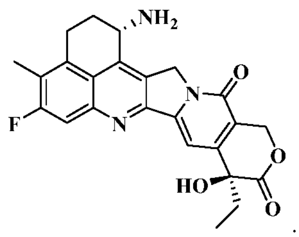

.

**17.** The antibody-drug conjugate or the pharmaceutically acceptable salt or solvate thereof according to any one of claims 1 to 15, wherein the cytotoxic drug is selected from the group consisting of:

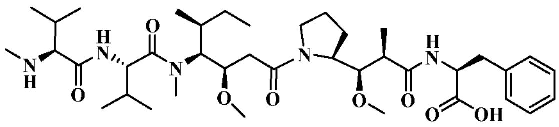

MMAF

or

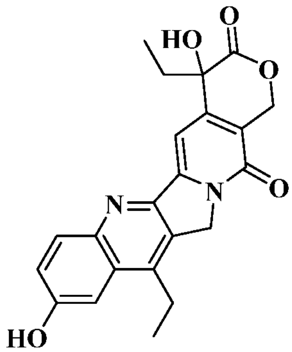

SN-38

.

**18.** The antibody-drug conjugate or the pharmaceutically acceptable salt or solvate thereof according to claim 1, the antibody-drug conjugate is as shown in general formula (I),

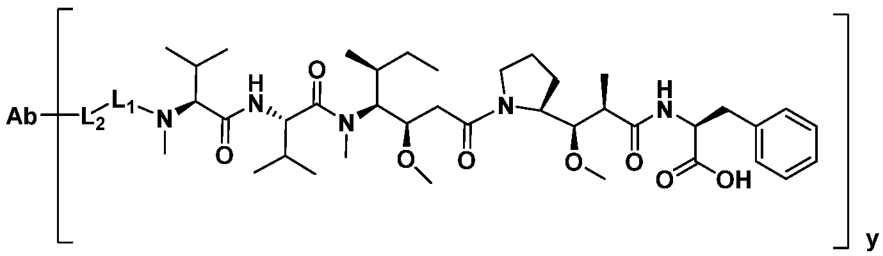

(I)

wherein:

$L_1$ and $L_2$ are linker units;
y is a number selected from 1 to 8, preferably a number selected from 2 to 4;
Ab is the B7-H4 antibody or antigen-binding fragment thereof according to any one of claims 1 to 12.

**19.** The antibody-drug conjugate or the pharmaceutically acceptable salt or solvate thereof according to claim 1, the antibody-drug conjugate is as shown in general formula (II):

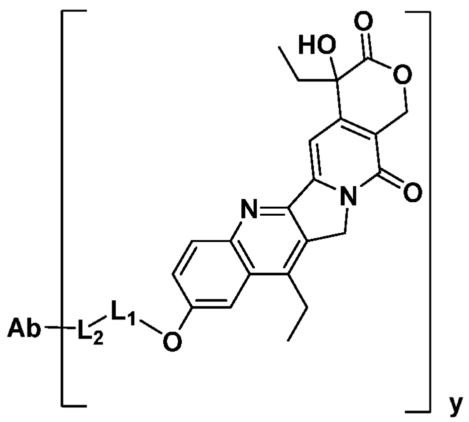

( II )

wherein:

$L_1$ and $L_2$ are linker units;
y is a number selected from 1 to 8, preferably a number selected from 2 to 4;
Ab is the B7-H4 antibody or antigen-binding fragment thereof according to any one of claims 1 to 12.

**20.** The antibody-drug conjugate or the pharmaceutically acceptable salt or solvate thereof according to claim 1, the antibody-drug conjugate is as shown in general formula (III):

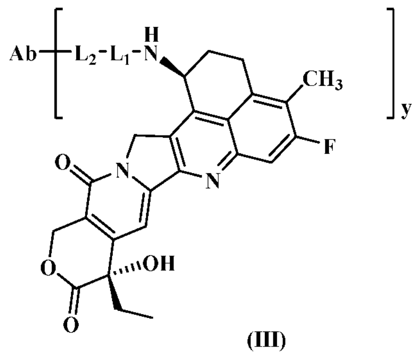

(III)

wherein:

$L_1$ and $L_2$ are linker units;
y is a number selected from 1 to 10, preferably a number selected from 2 to 8, more preferably a number of 4 to 8, further preferably a number of 6 to 8, and most preferably 8;
Ab is the B7-H4 antibody or antigen-binding fragment thereof according to any one of claims 1 to 12.

**21.** The antibody-drug conjugate or the pharmaceutically acceptable salt or solvate thereof according to any one of claims 18 to 20, wherein $L_1$ is as shown in general formula (B):

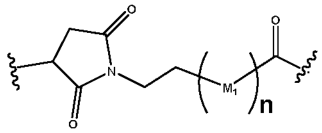

( B )

wherein:

$M_1$ is $-CR_1R_2-$;
$R_1$ and $R_2$ are the same or different, and are independently selected from the group consisting of hydrogen, alkyl, halogen, hydroxyl and amino;
n is an integer of 0 to 5, preferably 1, 2 or 3.

**22.** The antibody-drug conjugate or the pharmaceutically acceptable salt or solvate thereof according to any one of claims 18 to 20, wherein $L_2$ is as shown in general formula (C):

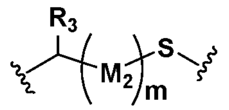

**( C )**

wherein:

$M_2$ is $-CR_4R_5-$;
$R_3$ is selected from the group consisting of hydrogen, halogen, hydroxyl, amino, alkyl, alkoxyl and cycloalkyl:

$R_4$ and $R_5$ are the same or different, and are independently selected from the group consisting of hydrogen, alkyl, halogen, hydroxyl and amino;
m is an integer of 0 to 5, preferably 1, 2 or 3.

**23.** The antibody-drug conjugate or the pharmaceutically acceptable salt or solvate thereof according to any one of claims 18 to 20, the $L_2$ is as shown in general formula (D):

$-K^1-K^2-K^3-K^4-$ (D)

wherein:

$K^1$ is

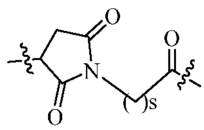

,

s is an integer of 2 to 8;
$K^2$ is selected from the group consisting of $-NR^1(CH_2CH_2O)_pCH_2CH_2C(O)-$, - $NR^1(CH_2CH_2O)_pCH_2C(O)-$, $-S(CH_2)_pC(O)-$ and single bond, p is an integer of 1 to 20, preferably 1 to 6;
$R_1$ is selected from the group consisting of hydrogen, deuterium, hydroxyl, amino, alkyl, halogen, haloalkyl, deuterated alkyl and hydroxyalkyl;
$K^3$ is a tetrapeptide residue, preferably, the tetrapeptide residue is a peptide residue formed by amino acids selected from the group consisting of two or more of phenylalanine, glycine, valine, lysine, citrulline, serine, glutamate and aspartate; more preferably the tetrapeptide residue GGFG;
$K^4$ is $-NR^2(CR^3R^4)_t-$, $R^2$, $R^3$ or $R^4$ are each independently hydrogen, deuterium, hydroxyl, amino, alkyl, halogen, haloalkyl, deuterated alkyl and hydroxyalkyl, and t is 1 or 2.

**24.** The antibody-drug conjugate or the pharmaceutically acceptable salt or solvate thereof according to claim 23, wherein the $K^1$ terminus of the linker unit $-L_2-$ is linked to the Ab, and the $K^4$ terminus is linked to $L_1$.

**25.** The antibody-drug conjugate or the pharmaceutically acceptable salt or solvate thereof according to any one of claims 18 to 20, wherein:

$L_1$ is selected from the group consisting of $-O-(CR^aR^b)_m-CR^5R^6-C(O)-$, $-O-CR^5R^6-(CR^aR^b)_m-$, $-O-CR^5R^6-$, $-NH-(CR^aR^b)_m-CR^SR^6-C(O)-$ and $-S-(CR^aR^b)_m-CR^5 R^6-C(O)-$;

$R^a$ and $R^b$ are each independently selected from the group consisting of hydrogen, deuterium, halogen and alkyl;
$R^5$ is haloalkyl or cycloalkyl;
$R^6$ is selected from the group consisting of hydrogen, haloalkyl and cycloalkyl;
or, $R^5$ and $R^6$ and the carbon atom to which they are linked form a cycloalkyl;
m is 0, 1, 2, 3 or 4.

**26.** The antibody-drug conjugate or the pharmaceutically acceptable salt or solvate thereof according to claim 25, wherein the O terminus of $L_1$ is linked to the linker unit $L_2$.

**27.** The antibody-drug conjugate or the pharmaceutically acceptable salt or solvate thereof according to claim 25, the $L_1$ is as shown in general formula (E):

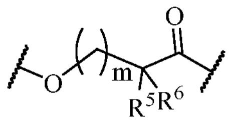

(E),

$R^5$ is selected from the group consisting of haloalkyl and cycloalkyl,
$R^6$ is selected from the group consisting of hydrogen, haloalkyl and cycloalkyl,
or, $R^5$ and $R^6$ and the carbon atom to which they are linked form a cycloalkyl; preferably,
$R^5$ is selected from the group consisting of $C_{1-6}$ haloalkyl and $C_{3-6}$ cycloalkyl,
$R^6$ is selected from the group consisting of hydrogen, $C_{1-6}$ haloalkyl and $C_{3-6}$ cycloalkyl,
or, $R^5$ and $R^6$ and the carbon atom to which they are linked form a $C_{3-6}$ cycloalkyl;
m is an integer of 0 to 4;
more preferably, general formula (E) is selected from the group consisting of the following substituents:

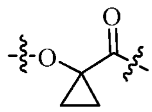 , 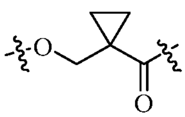 , 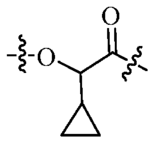 , 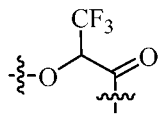 , 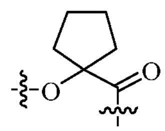 ,

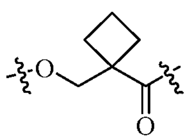 and 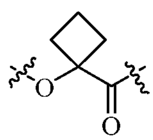 .

**28.** The antibody-drug conjugate or the pharmaceutically acceptable salt or solvate thereof according to any one of claims 1 to 27, wherein the $-L_2-L_1-$ is as shown in the following structure:

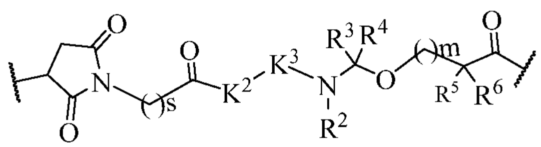

$K^2$ is a bond;
$K^3$ is the tetrapeptide residue GGFG;
$R^5$ is selected from the group consisting of haloalkyl and $C_{3-6}$ cycloalkyl;
$R^6$ is selected from the group consisting of hydrogen, haloalkyl and $C_{3-6}$ cycloalkyl;
or, $R^5$ and $R^6$ and the carbon atom) to which they are linked form a $C_{3-6}$ cycloalkyl;
$R^2$, $R^3$ or $R^4$ are each independently hydrogen or alkyl;
s is an integer of 2 to 8;
m is an integer of 0 to 4;
preferably, the $-L_2-L_1-$ is selected from the group consisting of the following structures:

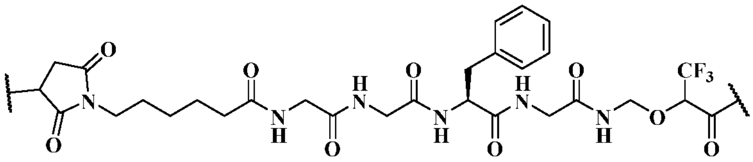

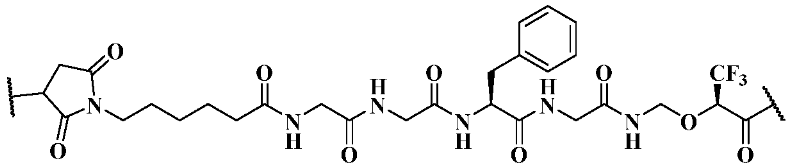

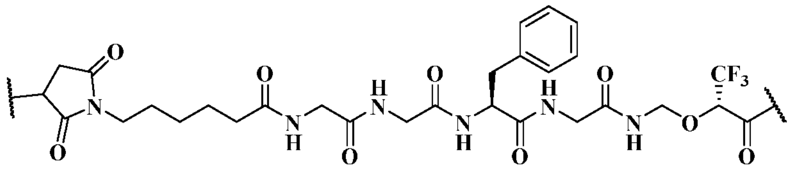

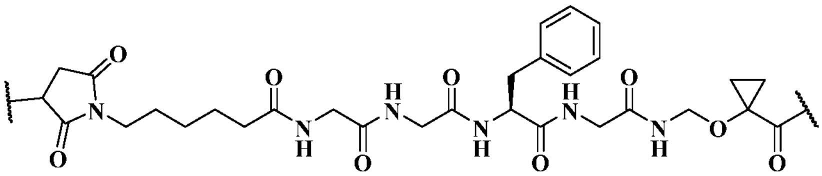

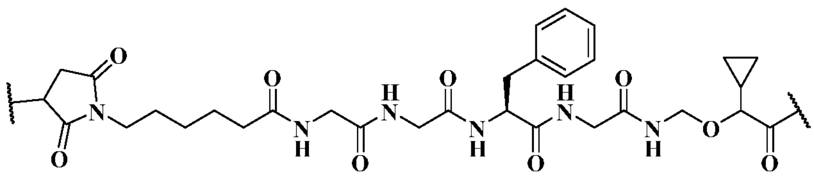

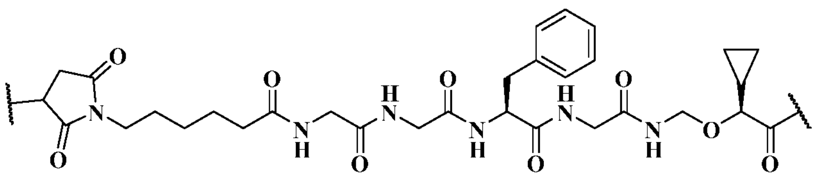

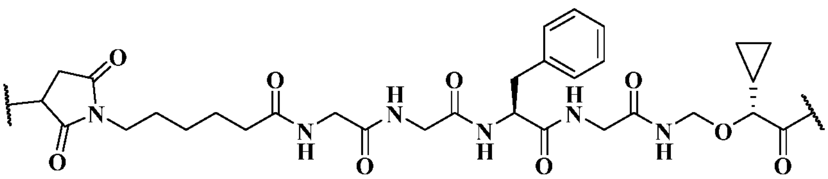

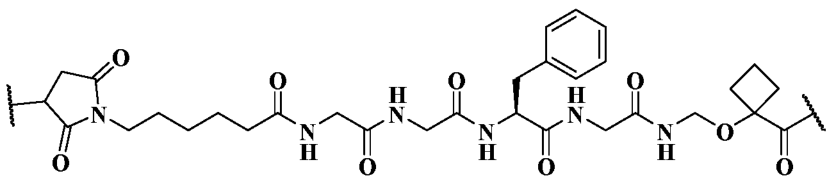

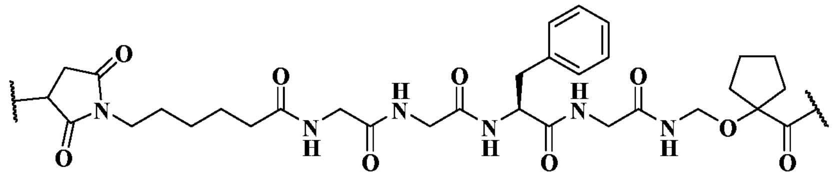

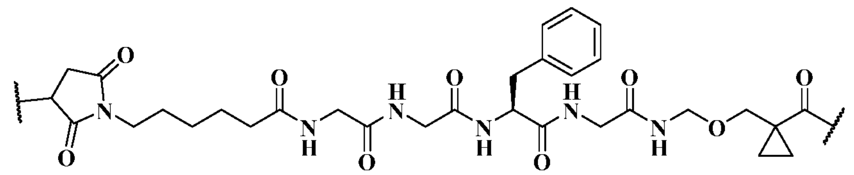

and

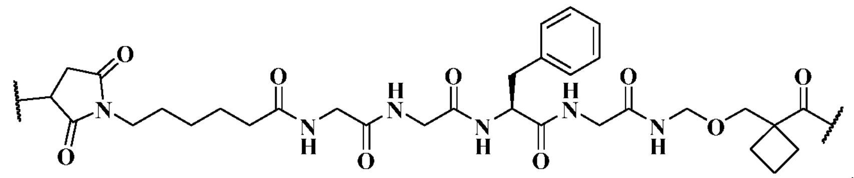

**29.** The antibody-drug conjugate or the pharmaceutically acceptable salt or solvate thereof according to any one of claims 1 to 12, wherein the antibody-drug conjugate is as shown in general formula (IV):

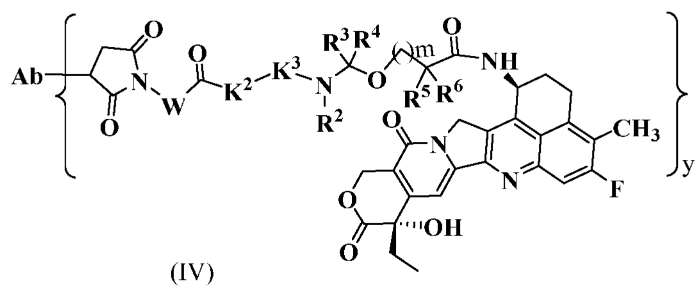

(IV)

wherein:

W is selected from the group consisting of $C_{1-8}$ alkyl, $C_{1-8}$ alkyl-cycloalkyl and linear heteroalkyl of 1 to 8 atoms, the heteroalkyl comprises 1 to 3 heteroatom(s) selected from the group consisting of N, O and S, wherein the $C_{1-8}$ alkyl, cycloalkyl and linear heteroalkyl are optionally further substituted with one or more substituents selected from the group consisting of halogen, hydroxyl, cyano, amino, alkyl, chloroalkyl, deuterated alkyl, alkoxyl and cycloalkyl;

$K^2$ is selected from the group consisting of $-NR^1(CH_2CH_2O)_{p1}CH_2CH_2C(O)-$, $-NR^1(CH_2CH_2O)_{p1}CH_2C(O)-$, $-S(CH_2)_{p1}C(O)-$ or bond, $R^1$ is selected from the group consisting of hydrogen atom, alkyl, haloalkyl, deuterated alkyl and hydroxyalkyl, and $p_1$ is an integer of 1 to 20;

$K^3$ is a peptide residue consisting of 2 to 7 amino acids, the amino acid(s) can be substituted or unsubstituted; if being substituted, the substituent(s) can be substituted at any available attachment point, and the substituent(s) is/are one or more independently selected from the group consisting of halogen, hydroxyl, cyano, amino, alkyl, chloroalkyl, deuterated alkyl, alkoxyl and cycloalkyl;

$R^2$ is independently selected from the group consisting of hydrogen atom, alkyl, haloalkyl, deuterated alkyl and hydroxyalkyl;

$R^3$ and $R^4$ are each independently selected from the group consisting of hydrogen atom, halogen, alkyl, haloalkyl, deuterated alkyl and hydroxyalkyl;

$R^5$ is selected from the group consisting of halogen, haloalkyl, deuterated alkyl, cycloalkyl, heterocyclyl, aryl and heteroaryl;

$R^6$ is selected from the group consisting of hydrogen atom, halogen, haloalkyl, deuterated alkyl, cycloalkyl,

heterocyclyl, aryl and heteroaryl;
or, $R^5$ and $R^6$ and the carbon atom to which they are linked form a cycloalkyl or heterocyclyl;
m is an integer of 0 to 4;
y is 1 to 10, y is a decimal or an integer;
Ab is an anti-B7-H4 antibody or antigen-binding fragment thereof.

**30.** The antibody-drug conjugate of general formula (I) or the pharmaceutically acceptable salt or solvate thereof according to claim 18, the antibody-drug conjugate is as shown in general formula (I-A):

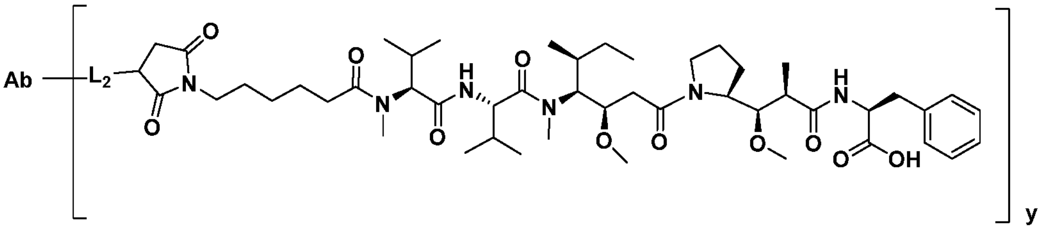

( I-A )

**31.** The antibody-drug conjugate of general formula (I) or the pharmaceutically acceptable salt or solvate thereof according to claim 18, the antibody-drug conjugate is as shown in general formula (I-B):

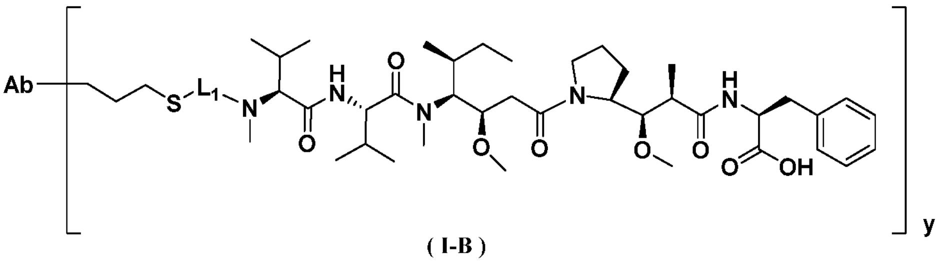

( I-B )

**32.** The antibody-drug conjugate of general formula (II) or the pharmaceutically acceptable salt or solvate thereof according to claim 19, the antibody-drug conjugate is as shown in general formula (II-A):

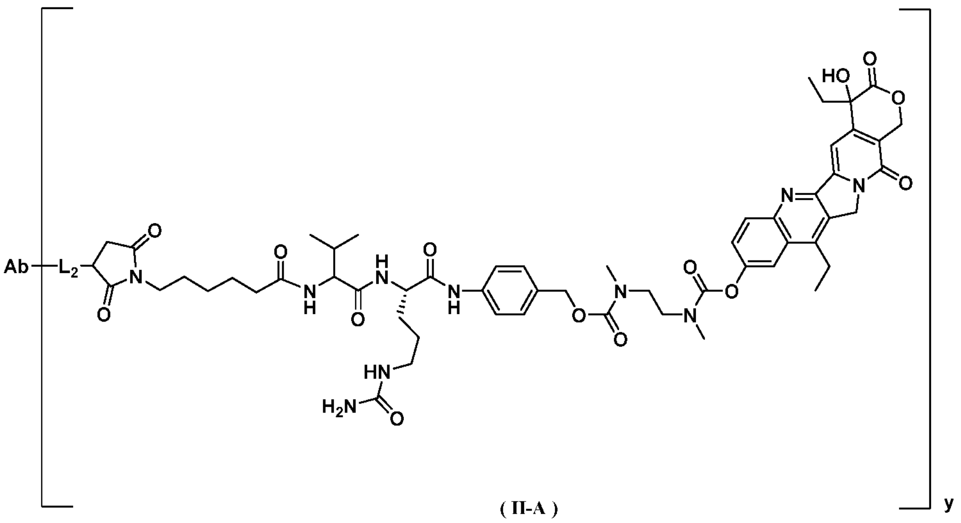

( II-A )

**33.** The antibody-drug conjugate of general formula (II) or the pharmaceutically acceptable salt or solvate thereof according to claim 19, the antibody-drug conjugate is as shown in general formula (II-B):

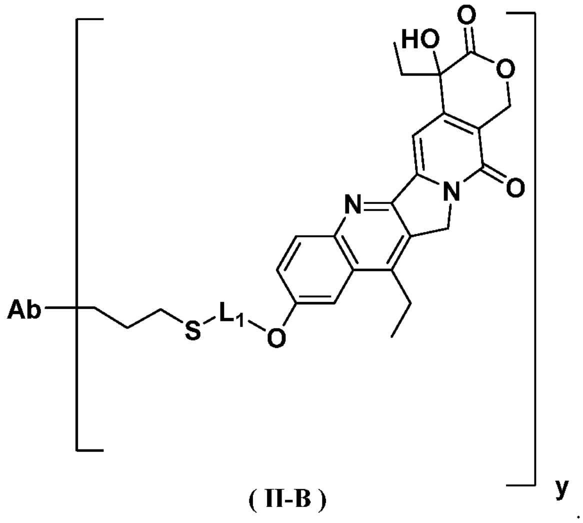

( II-B )

34. The antibody-drug conjugate or the pharmaceutically acceptable salt or solvate thereof according to claim 29, the antibody-drug conjugate is as shown in general formula (IV-A):

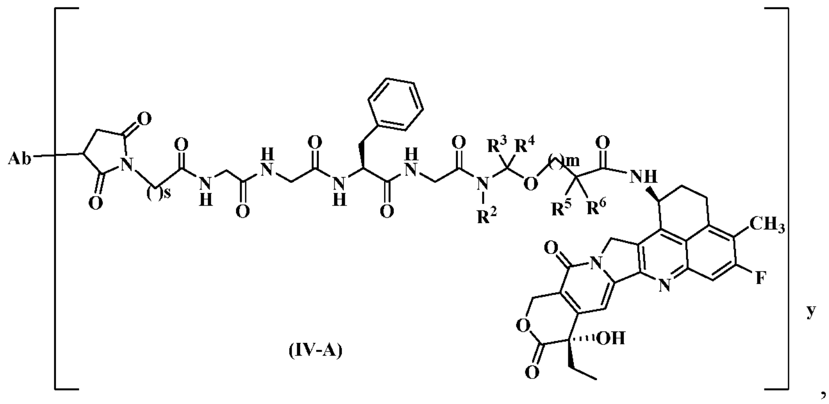

(IV-A)

preferably, the antibody-drug conjugate is shown as follows:

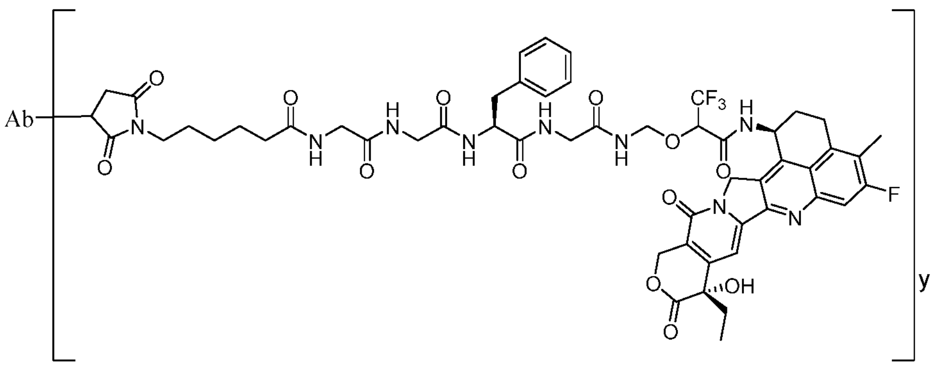

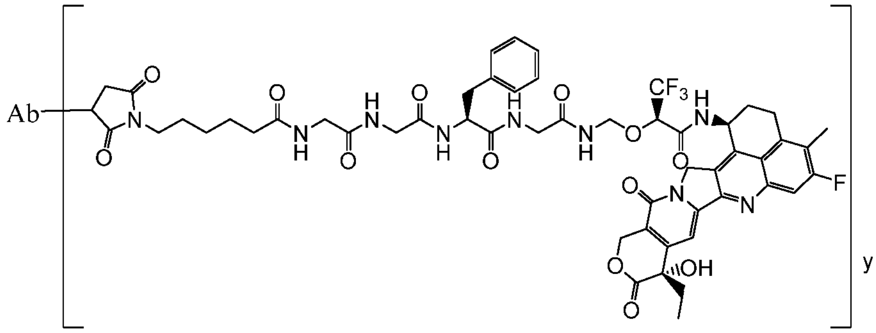

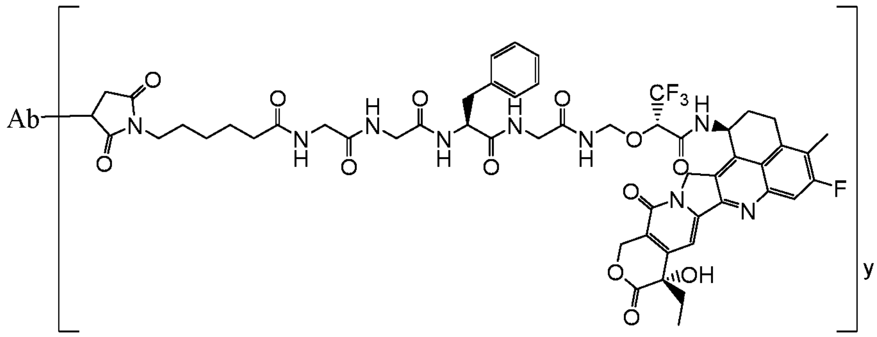

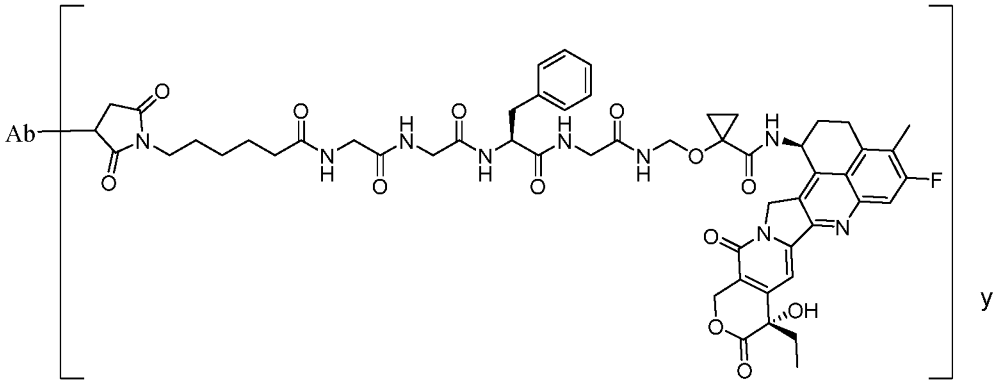

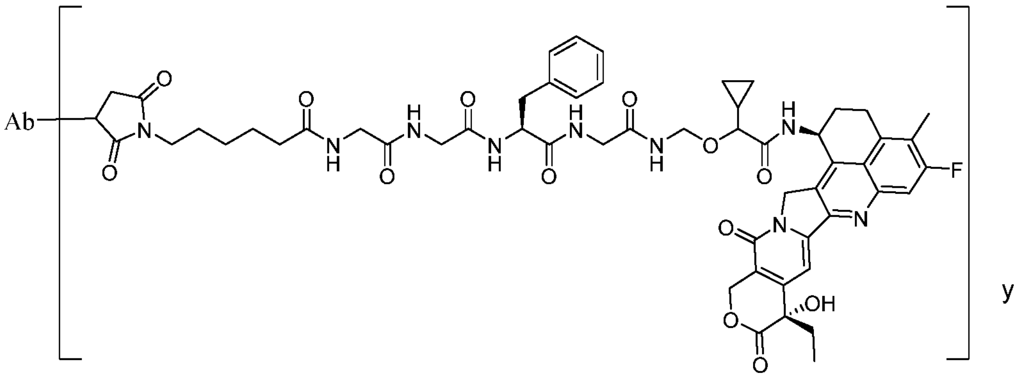

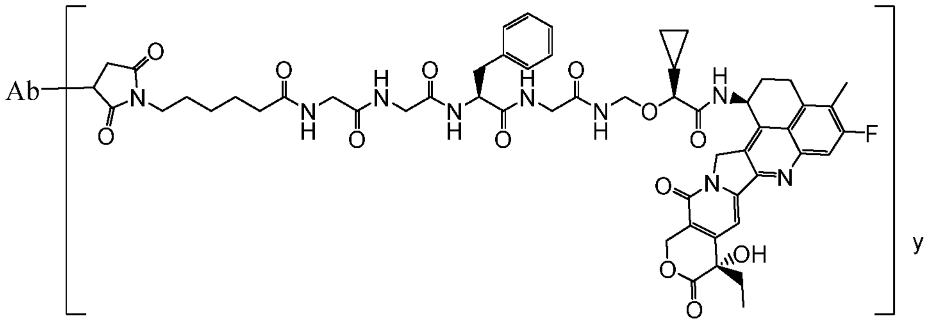

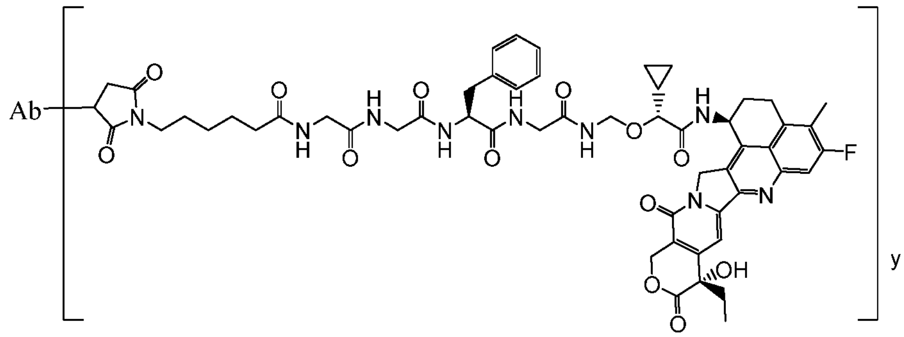

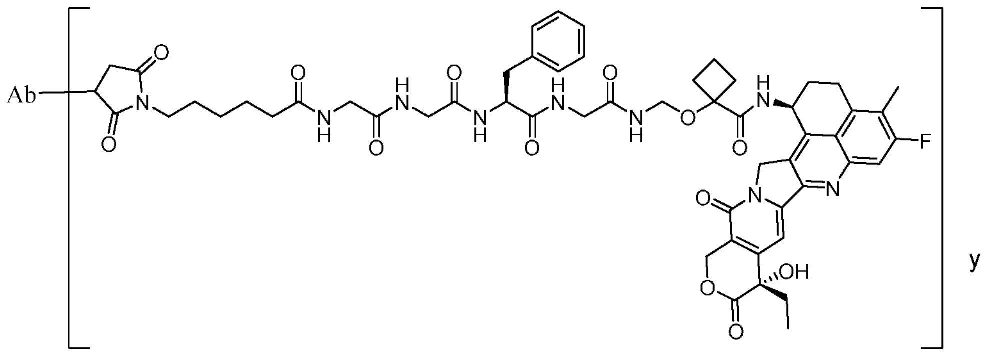

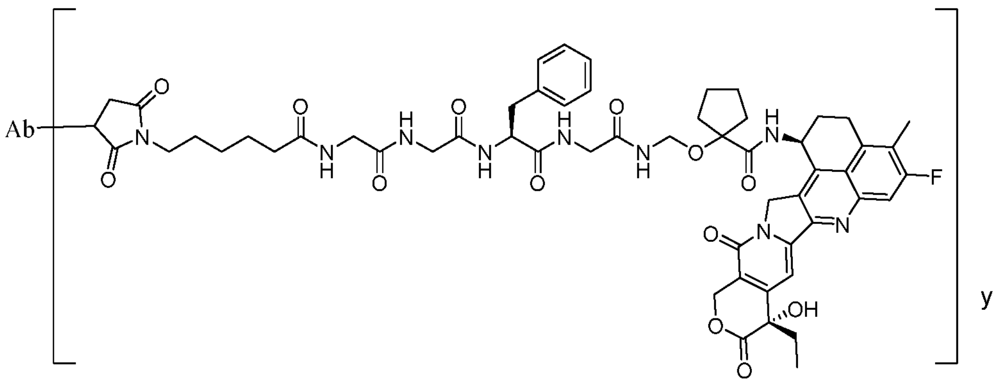

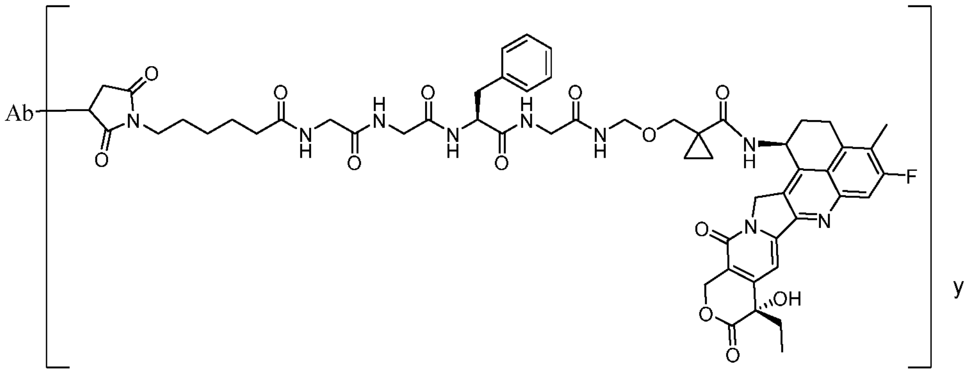

or

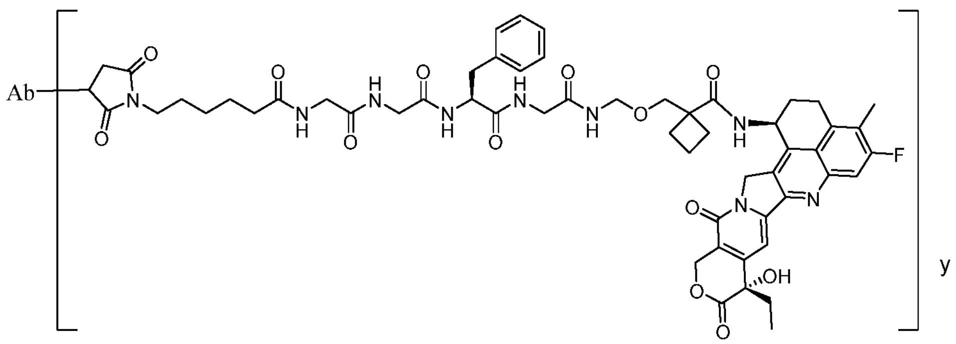

.

**35.** The antibody-drug conjugate of general formula (A) or the pharmaceutically acceptable salt or solvate thereof according to any one of claims 1 to 34, the antibody-drug conjugate is selected from the group consisting of the following compounds:

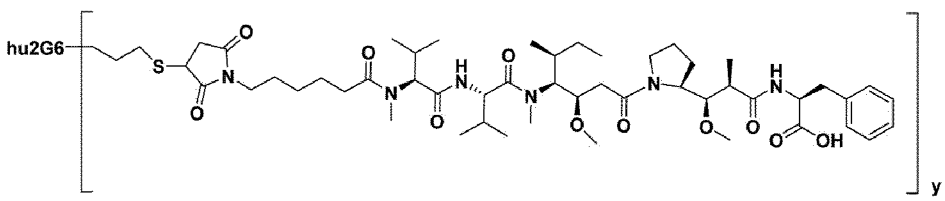

(Compound 1)

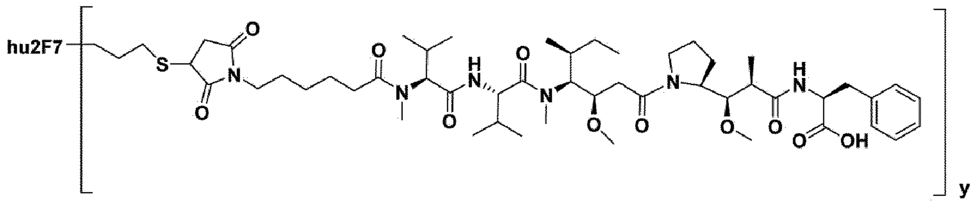

(Compound 2)

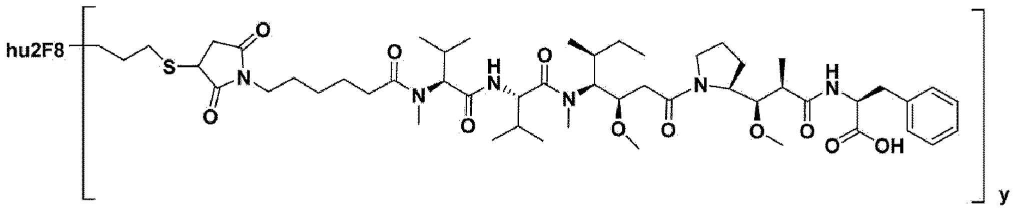

(Compound 3)

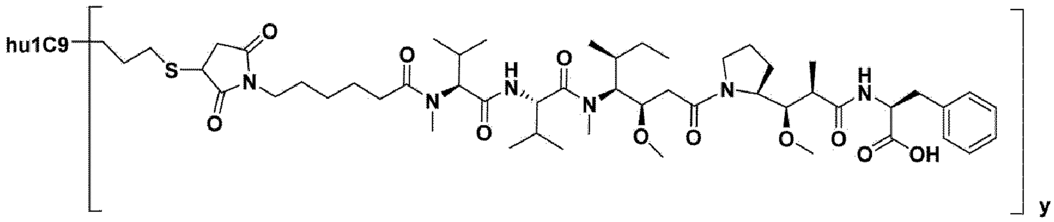

(Compound 4)

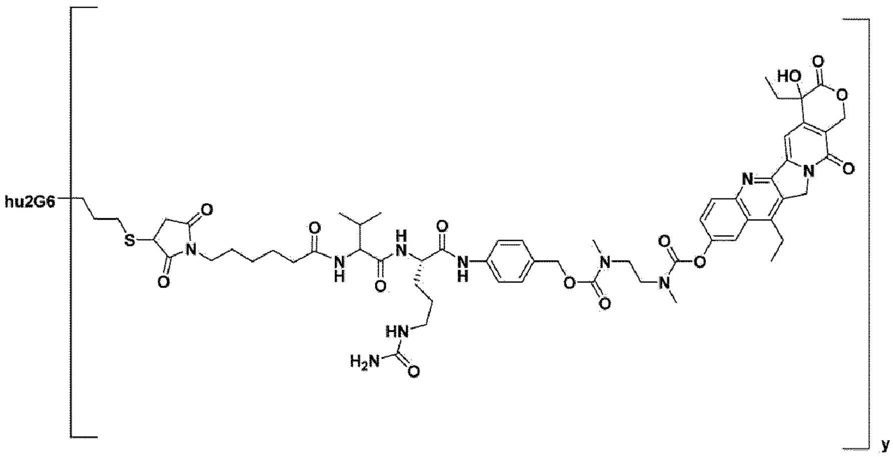

(Compound 5)

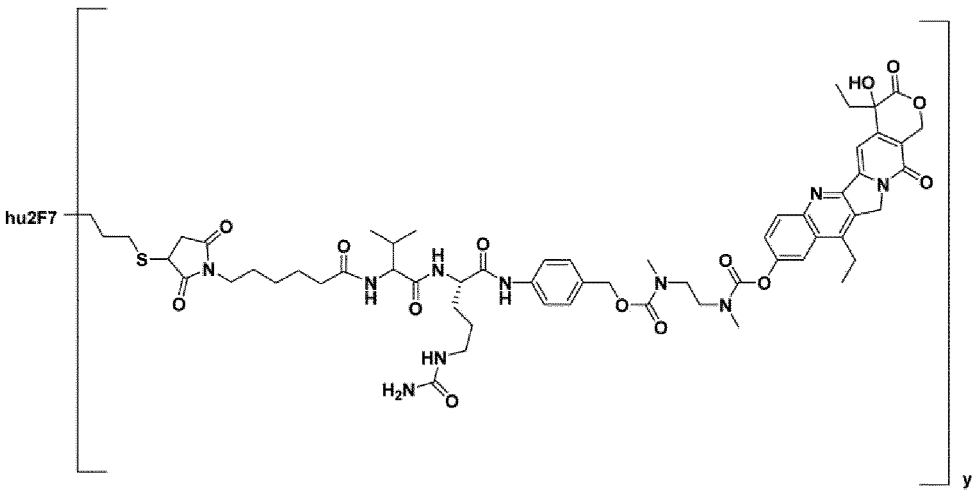

(Compound 6)

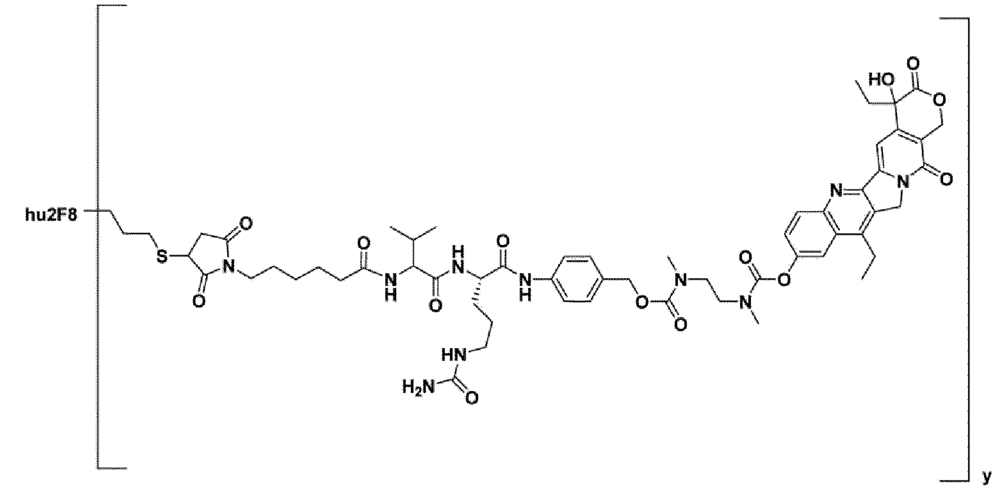

(Compound 7)

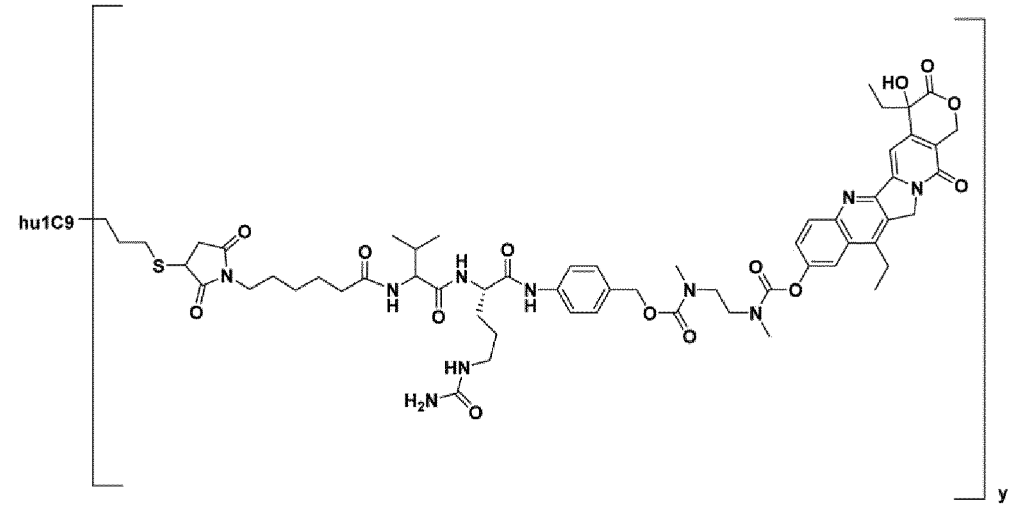

(Compound 8)

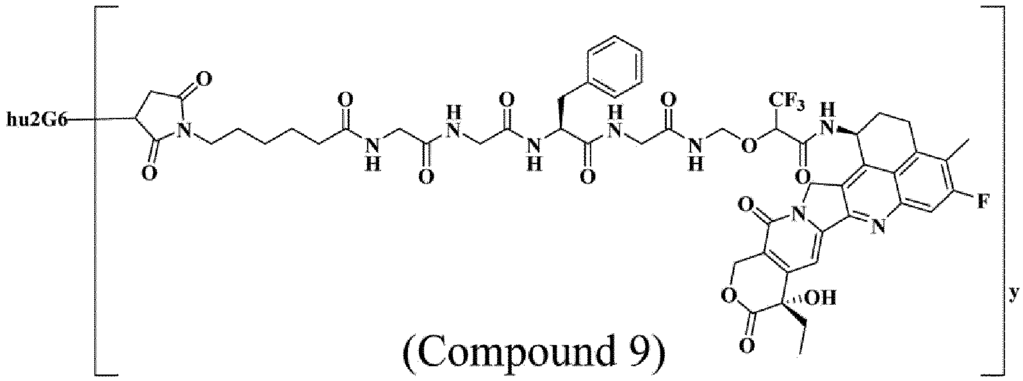

(Compound 9)

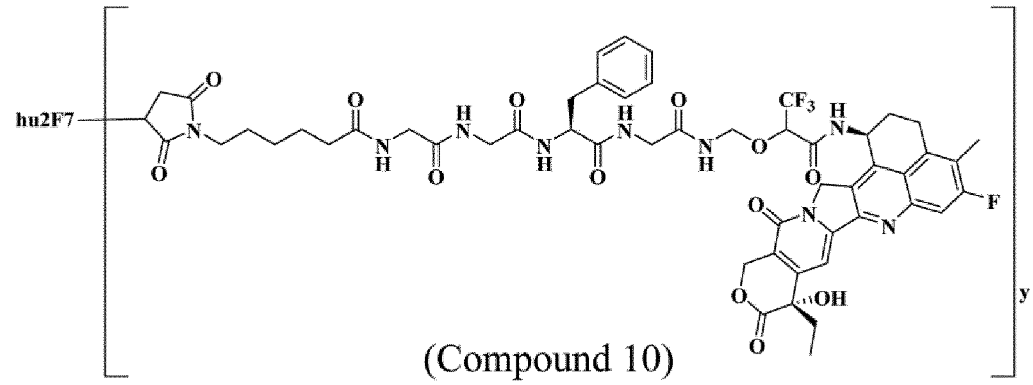

(Compound 10)

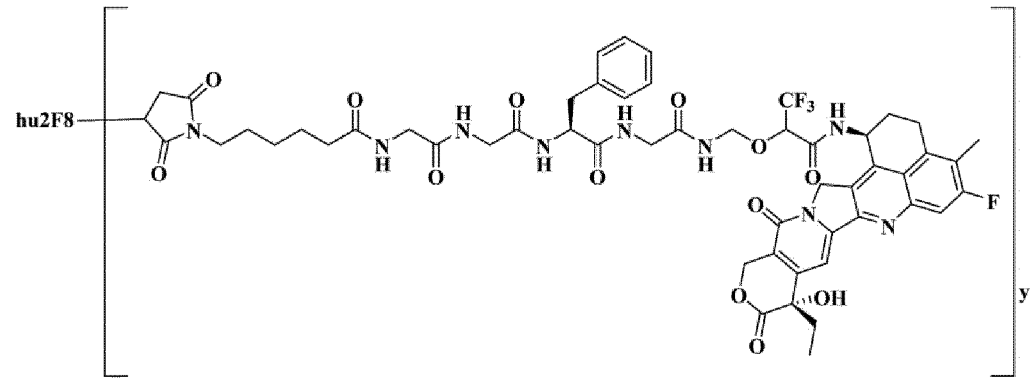

(Compound 11)

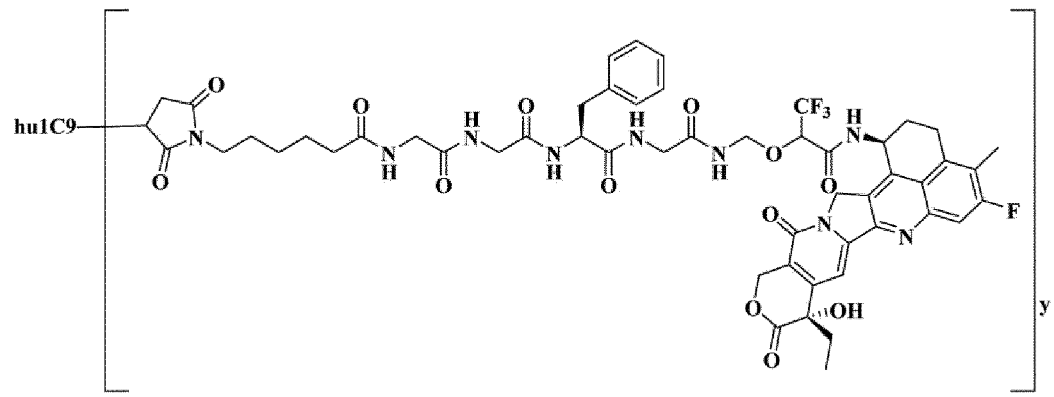

(Compound 12)

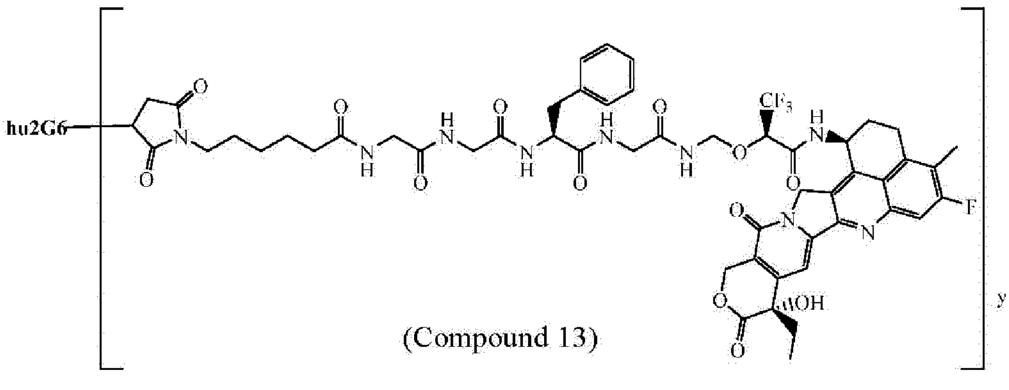

(Compound 13)

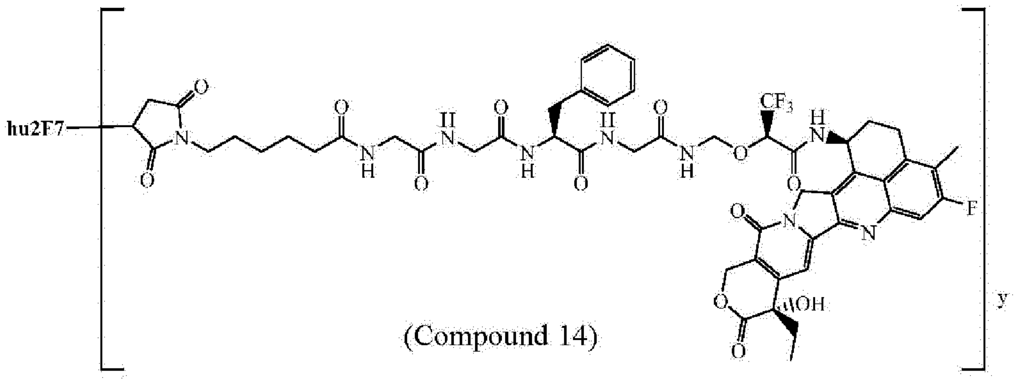

(Compound 14)

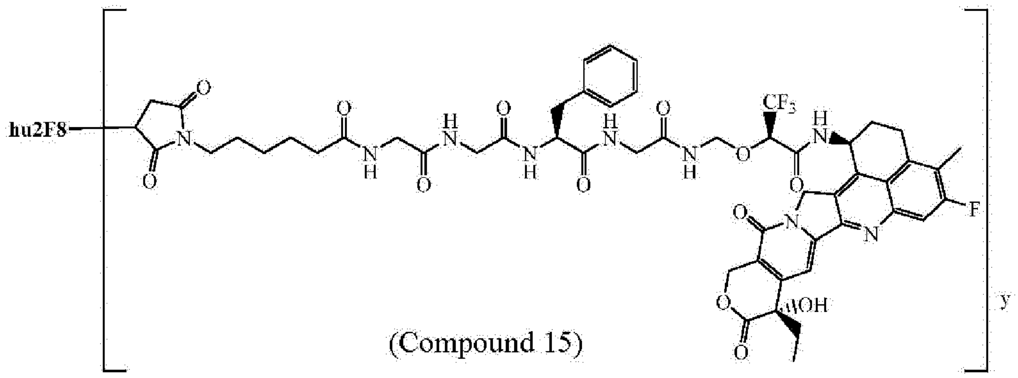

(Compound 15)

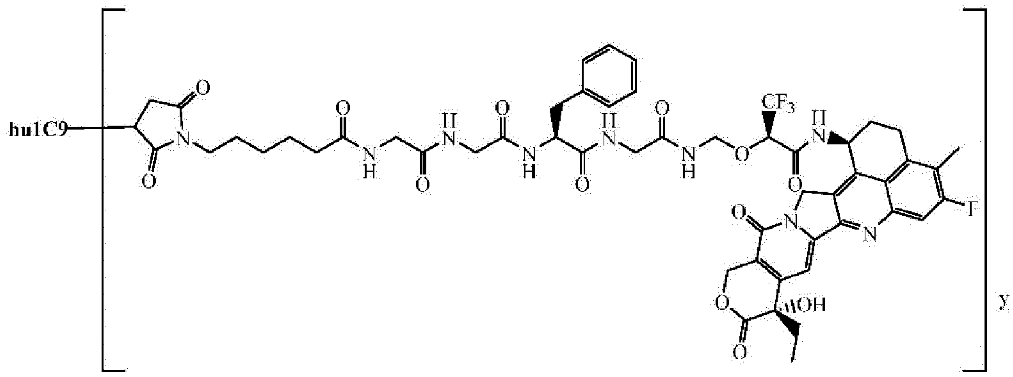

(Compound 16)

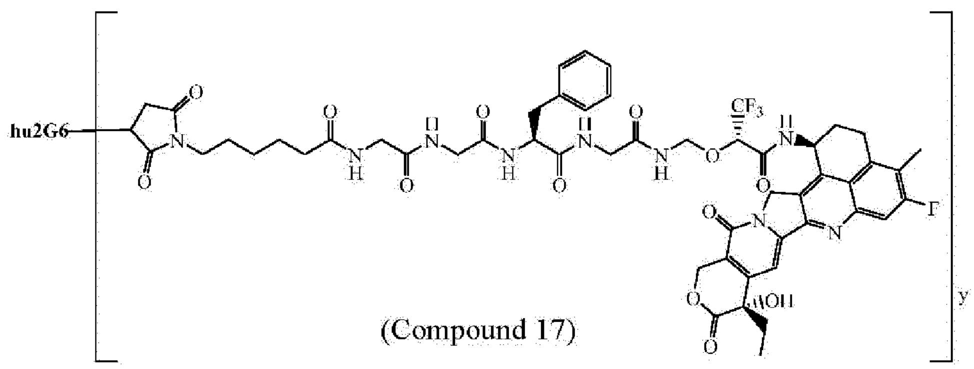

(Compound 17)

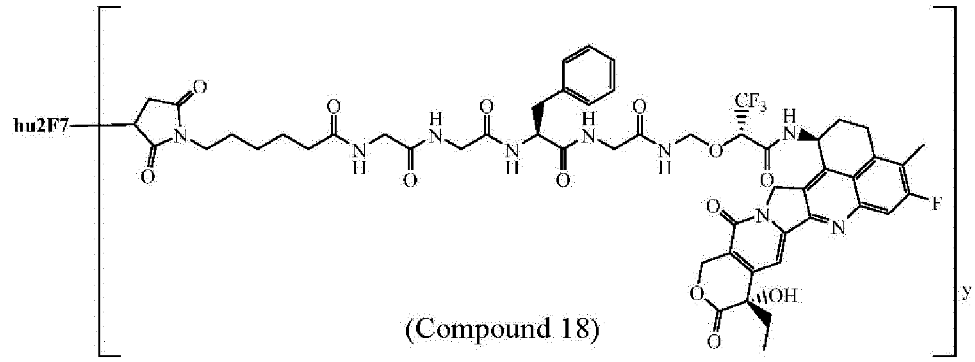

(Compound 18)

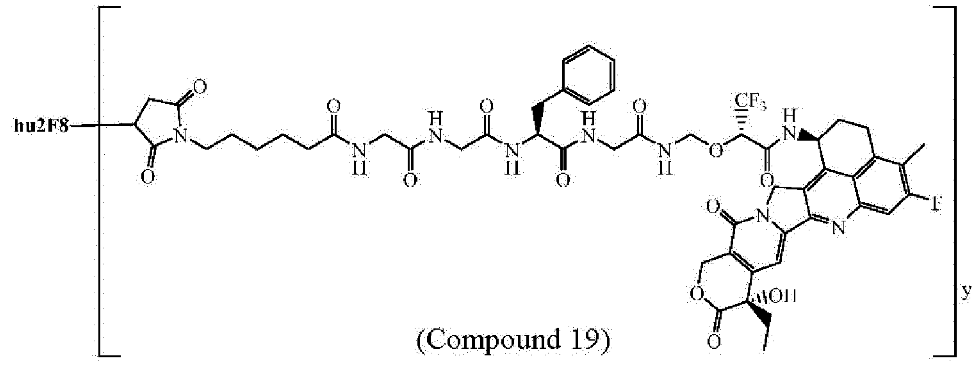

(Compound 19)

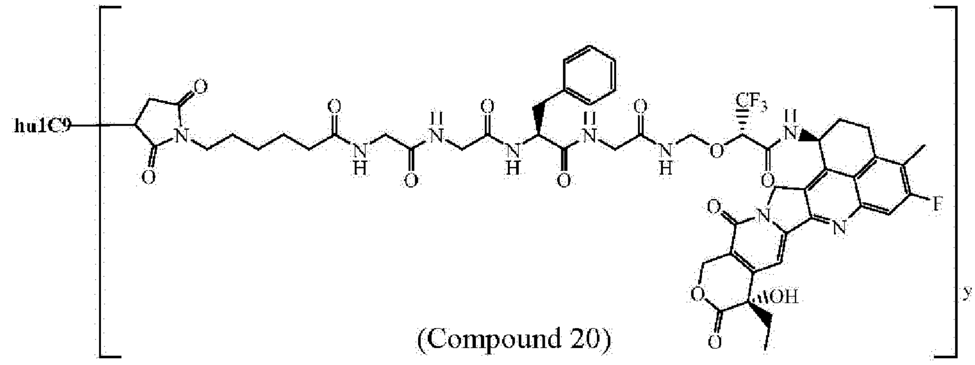

(Compound 20)

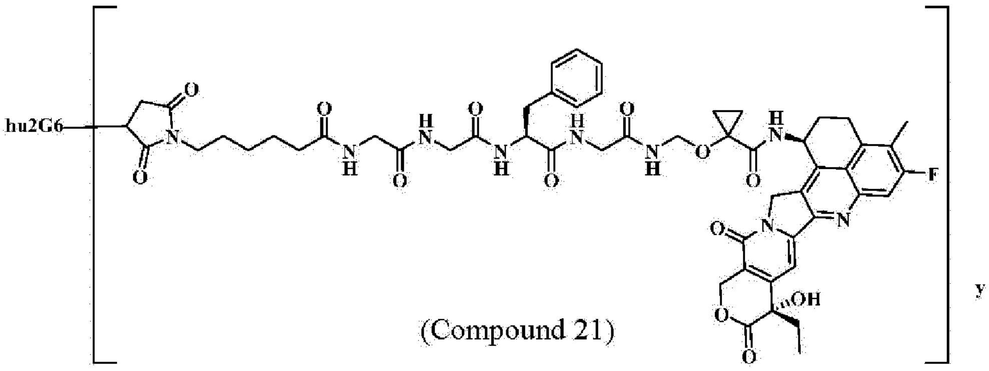

(Compound 21)

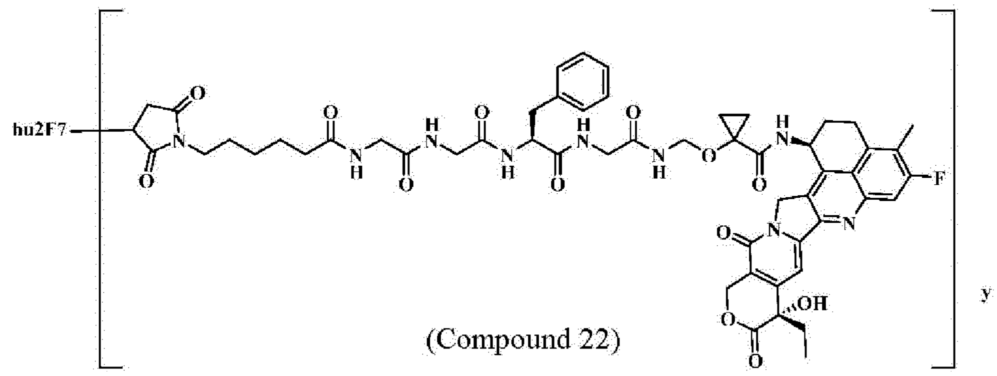

(Compound 22)

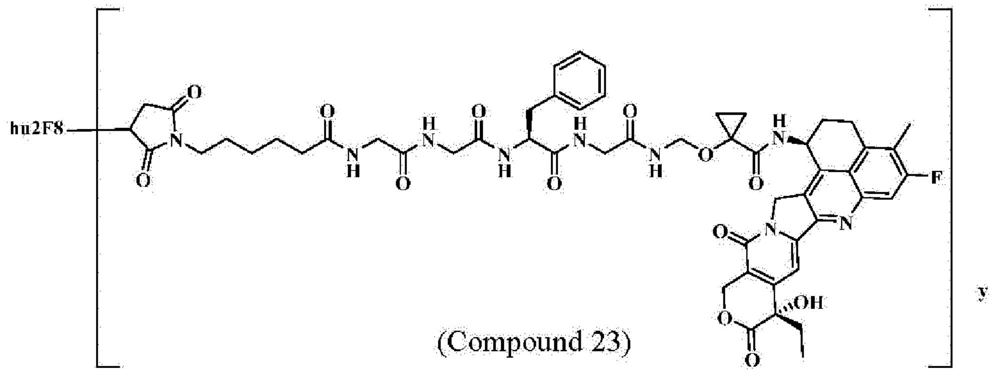

(Compound 23)

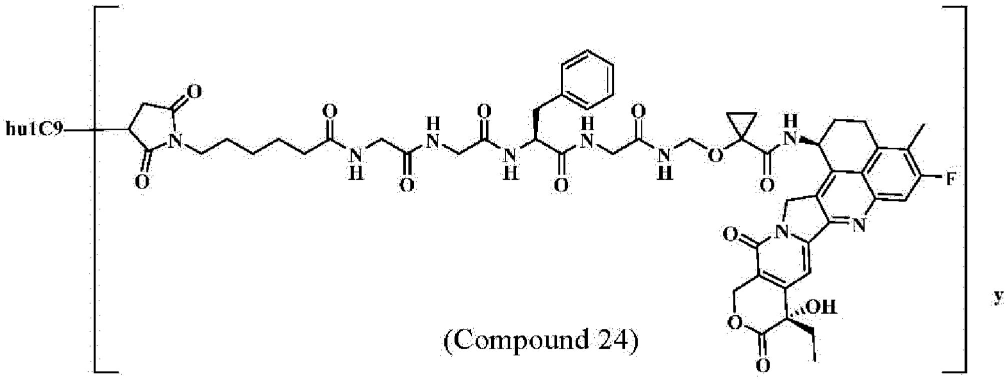

(Compound 24)

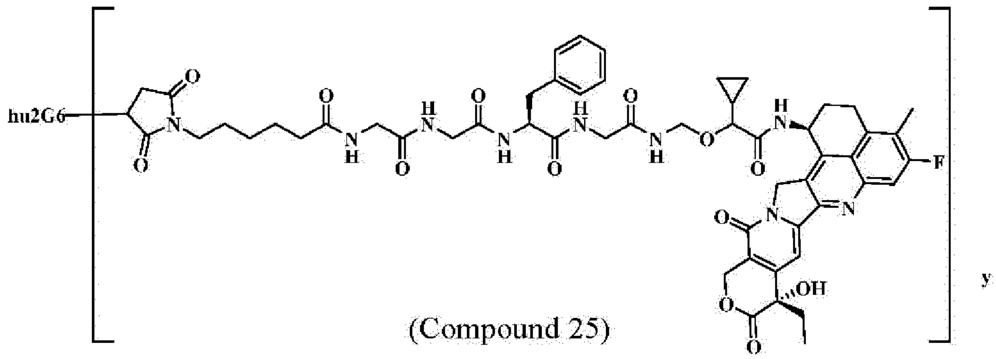

(Compound 25)

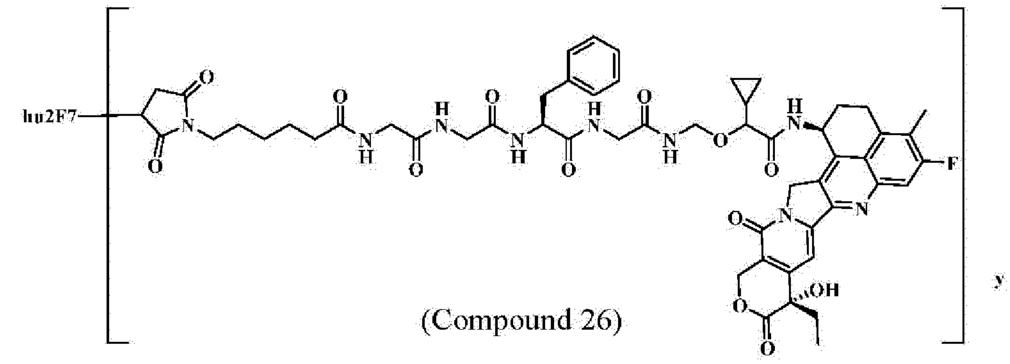

(Compound 26)

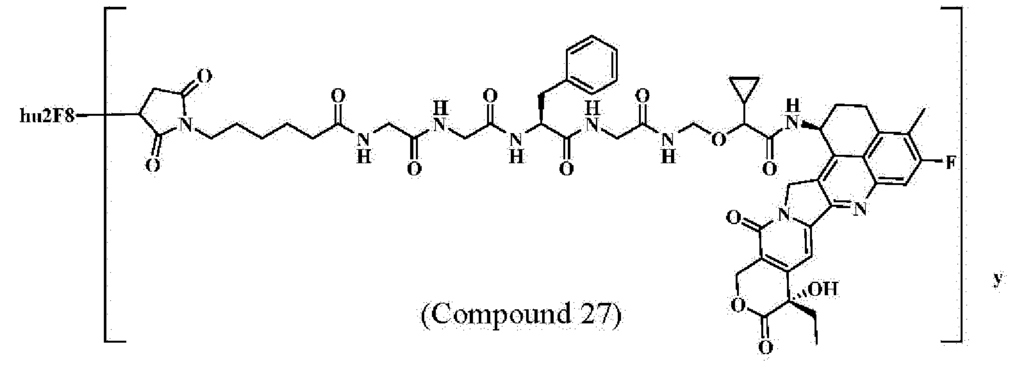

(Compound 27)

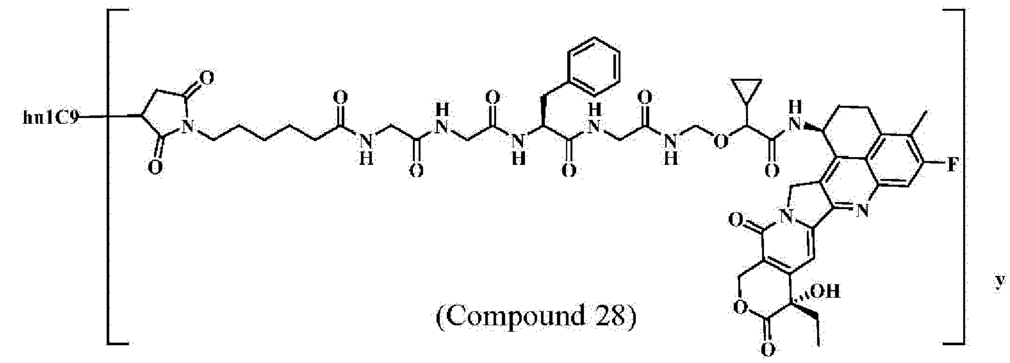

(Compound 28)

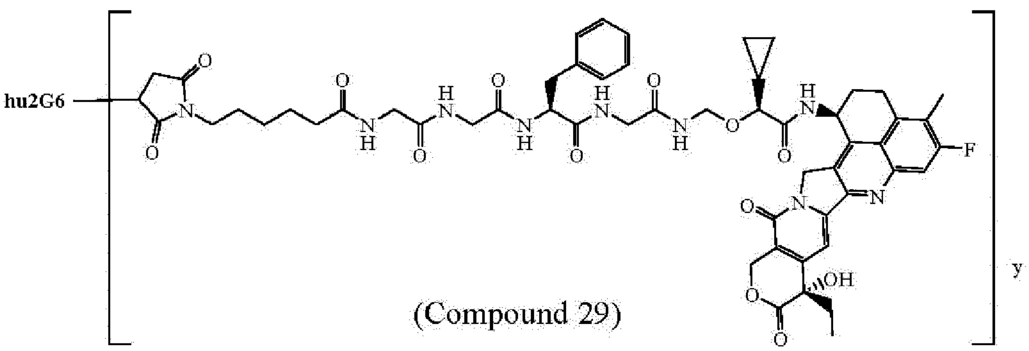

(Compound 29)

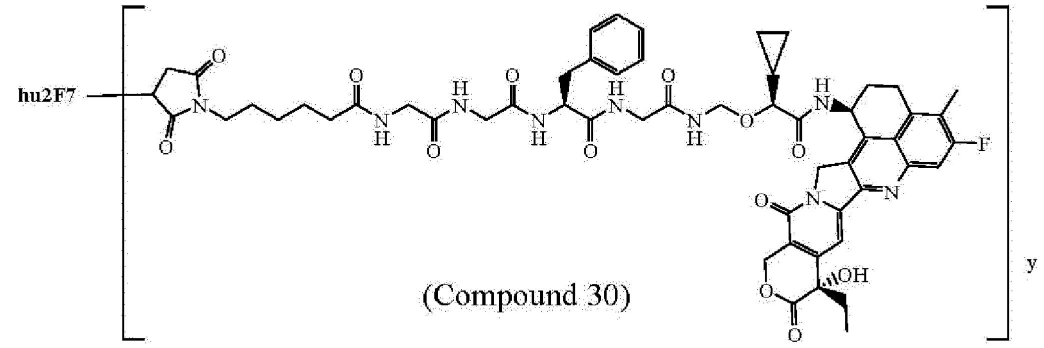

(Compound 30)

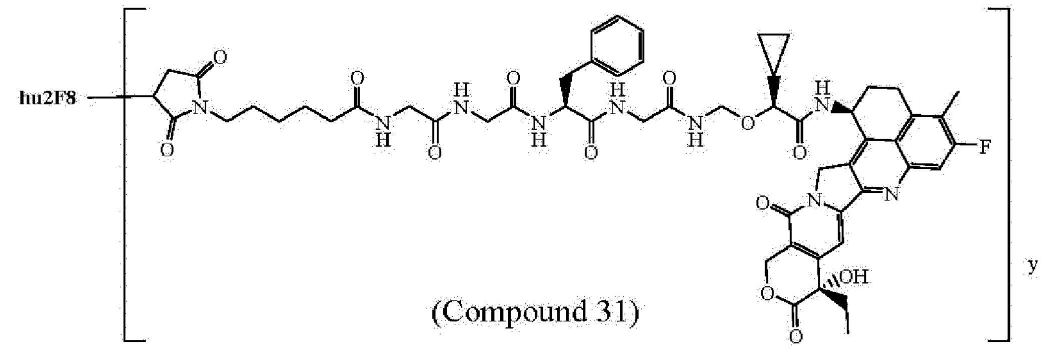

(Compound 31)

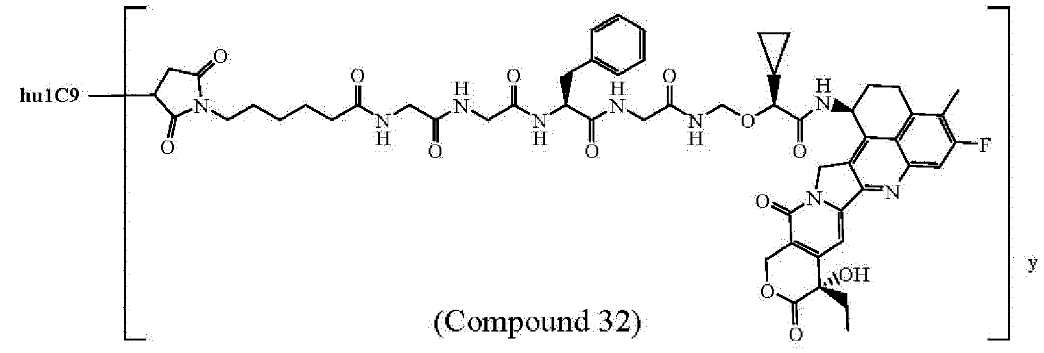

(Compound 32)

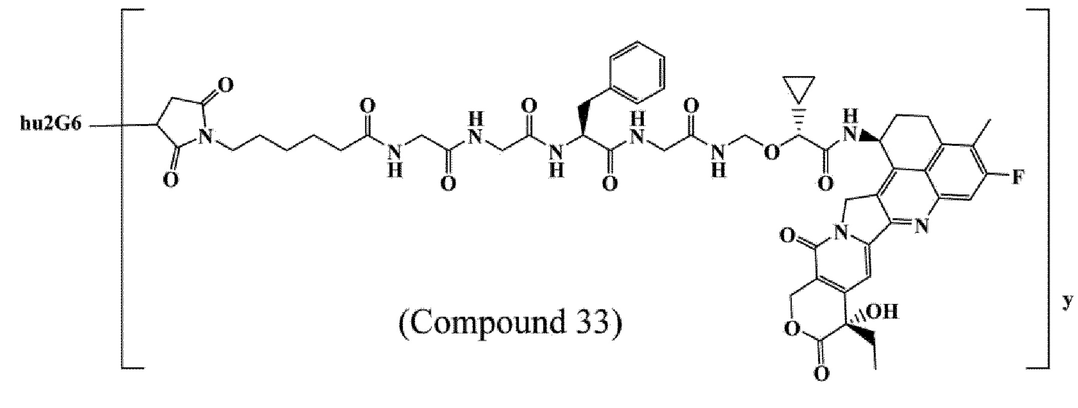

(Compound 33)

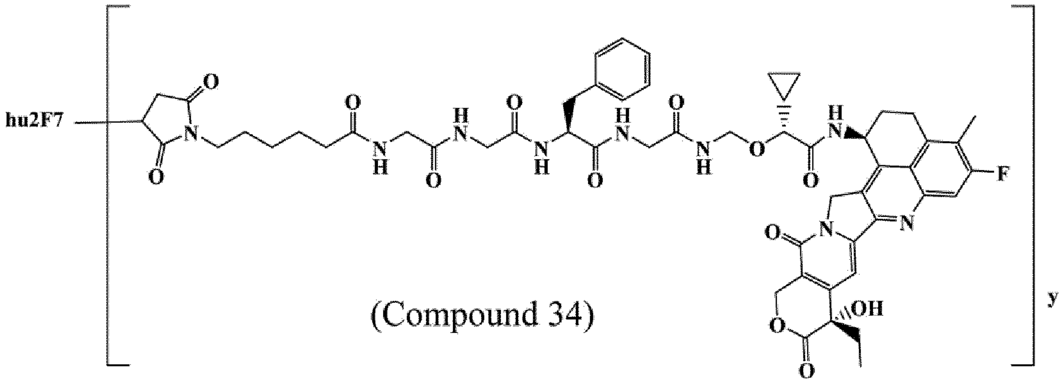

(Compound 34)

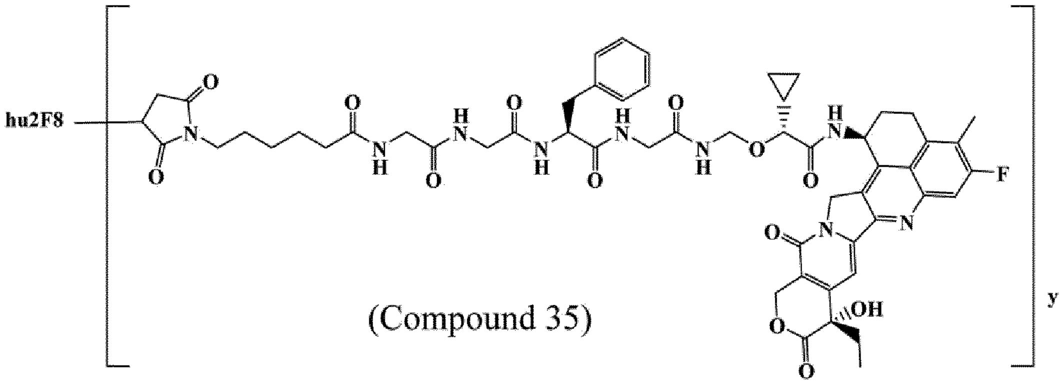

(Compound 35)

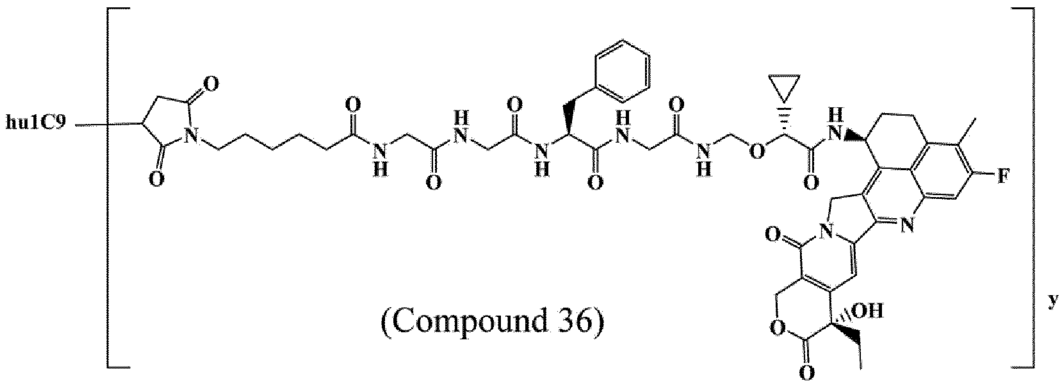

(Compound 36)

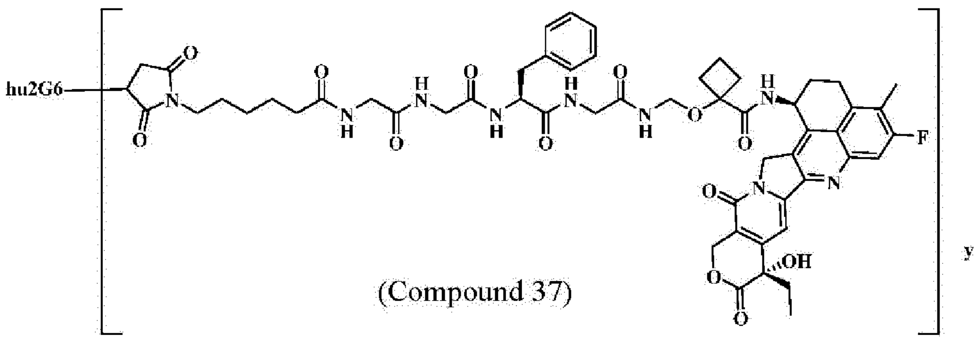

(Compound 37)

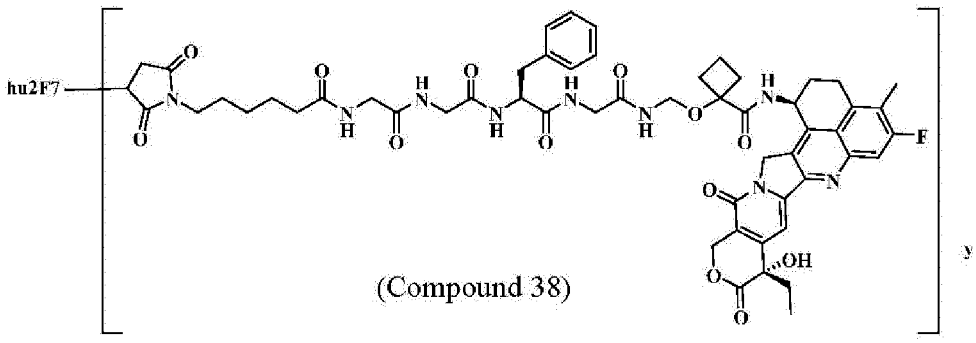

(Compound 38)

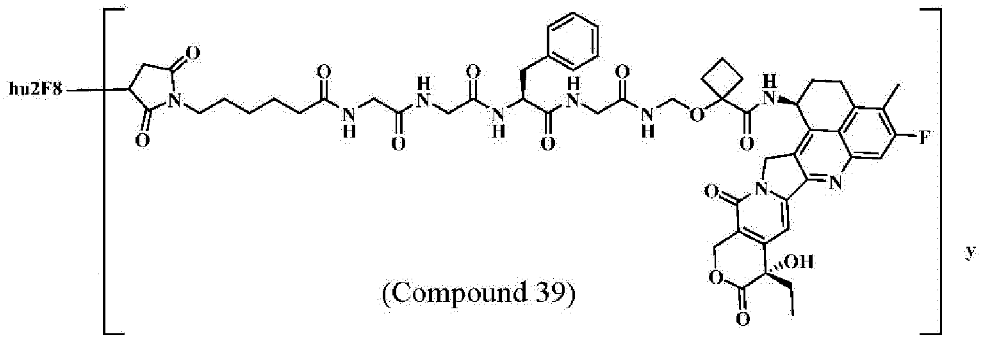

(Compound 39)

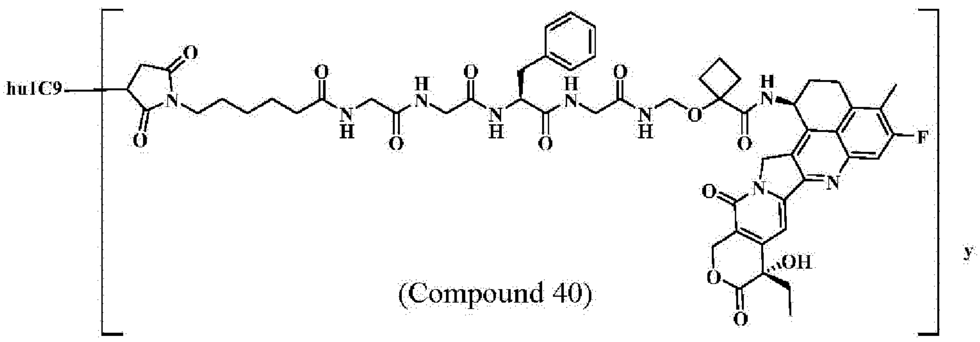

(Compound 40)

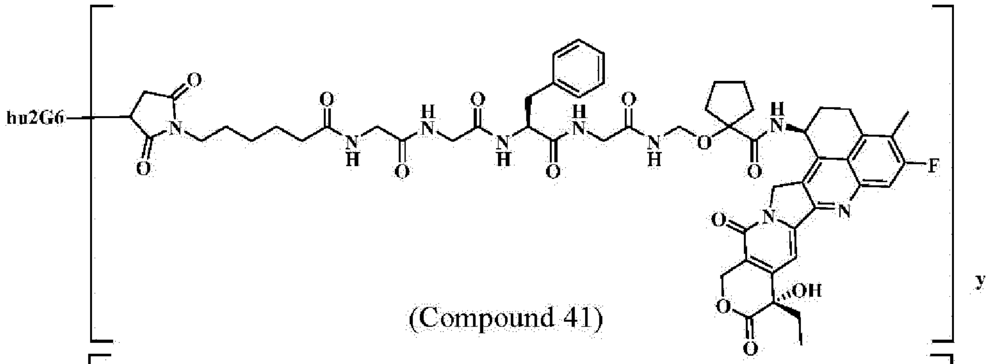

(Compound 41)

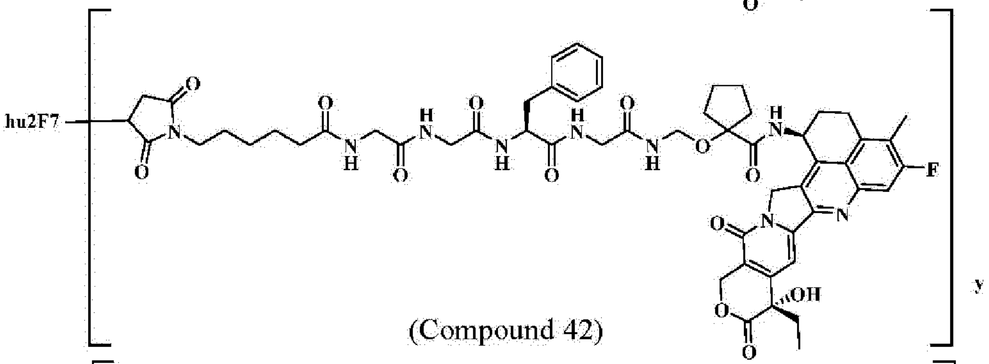

(Compound 42)

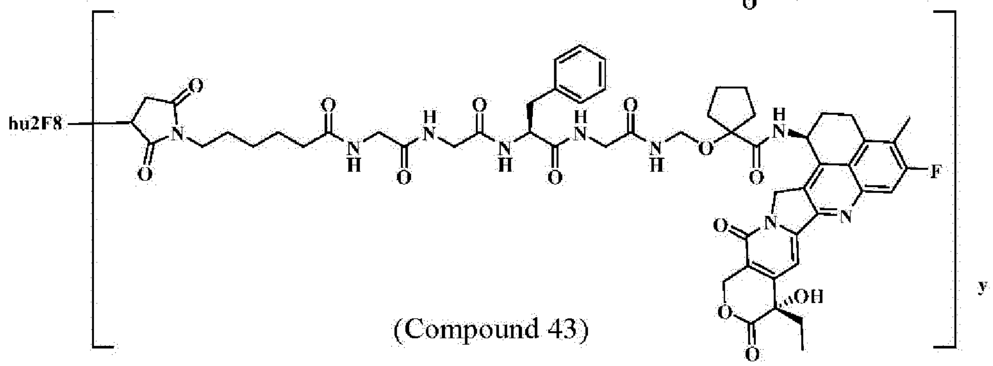

(Compound 43)

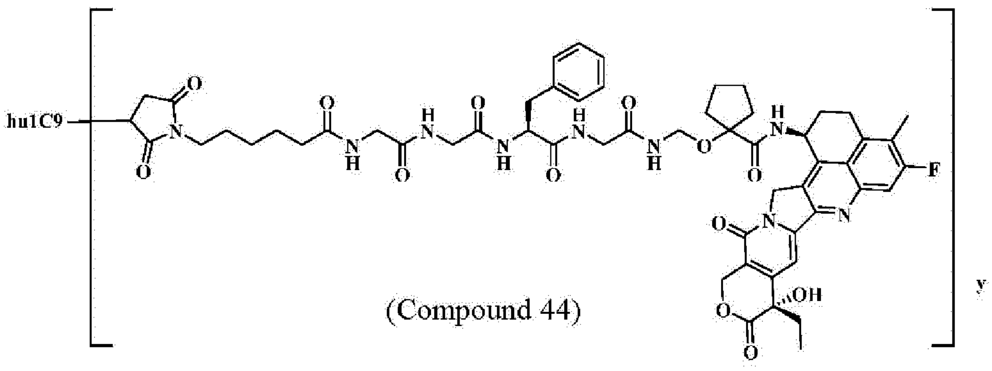

(Compound 44)

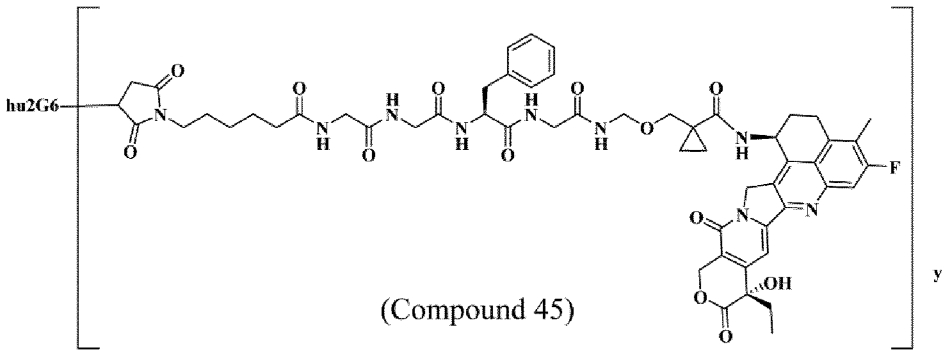

(Compound 45)

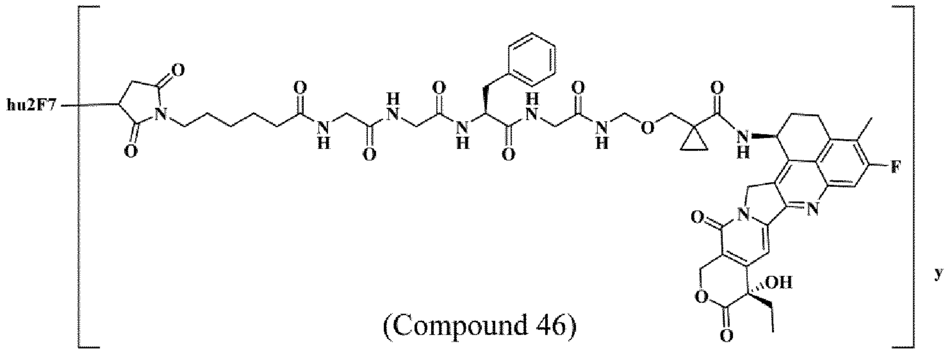

(Compound 46)

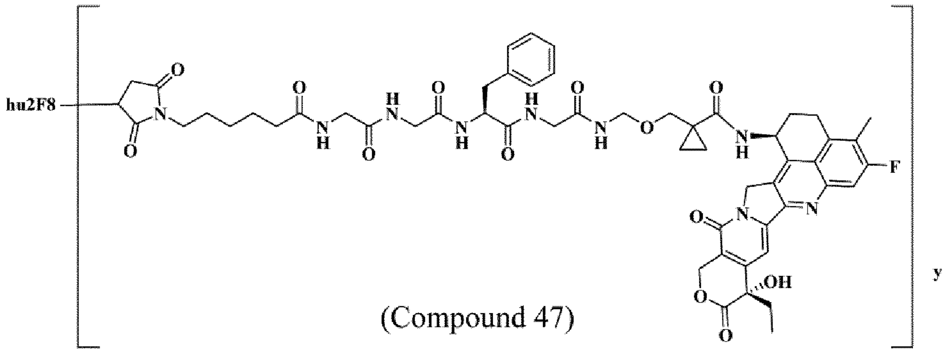

(Compound 47)

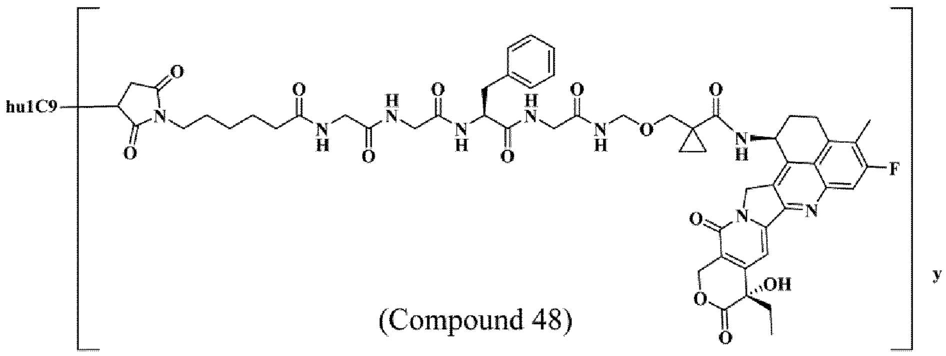

(Compound 48)

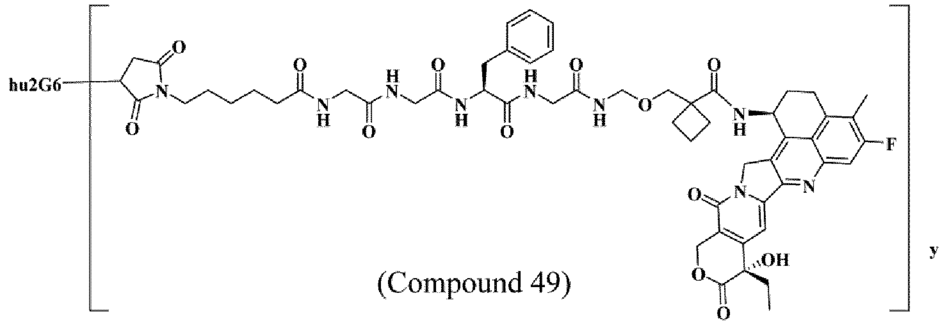

(Compound 49)

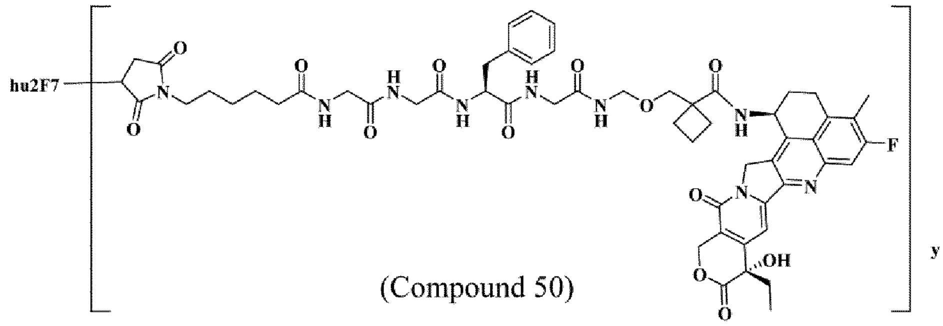

(Compound 50)

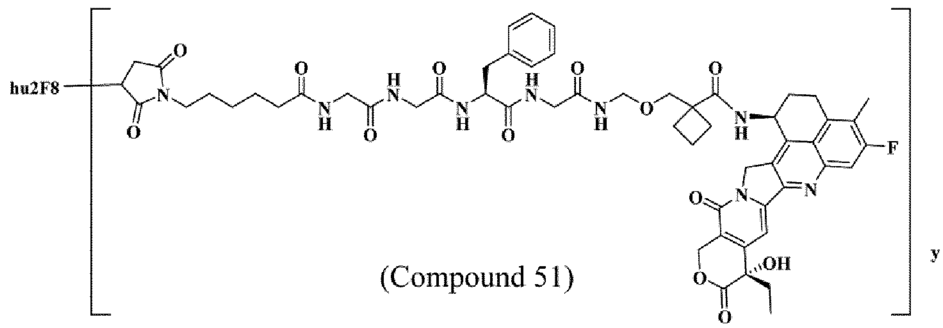

(Compound 51)

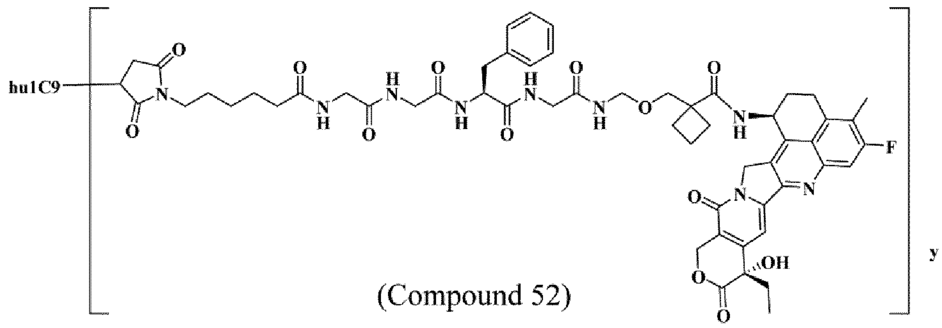

(Compound 52)

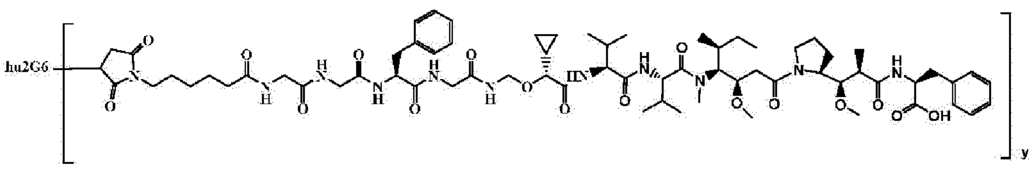

(Compound 53)

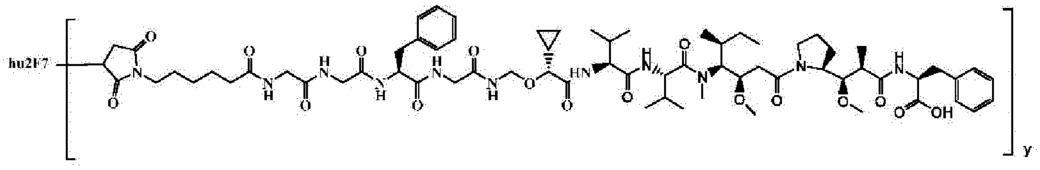

(Compound 54)

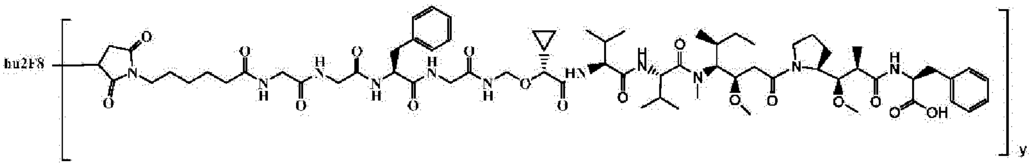

(Compound 55)

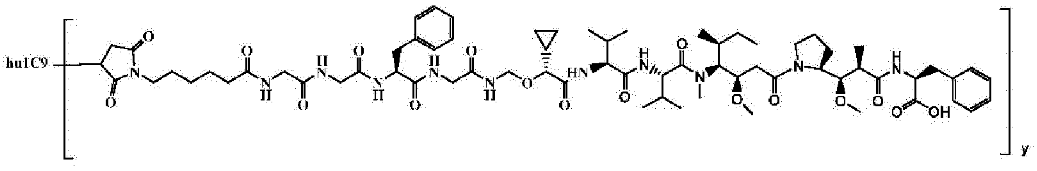

(Compound 56)

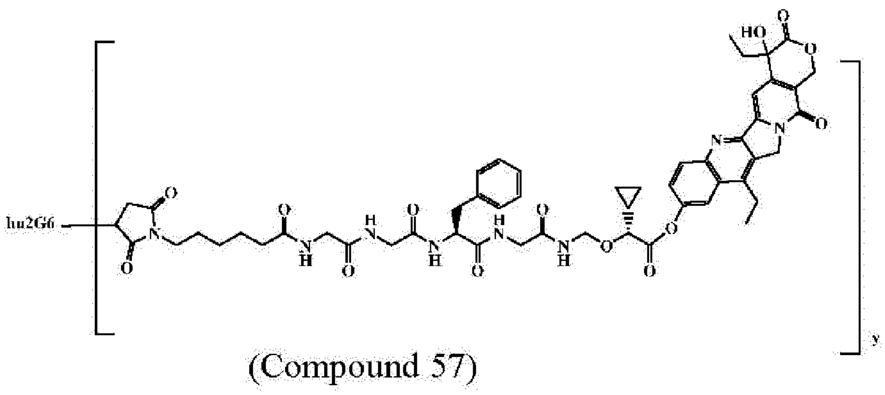

(Compound 57)

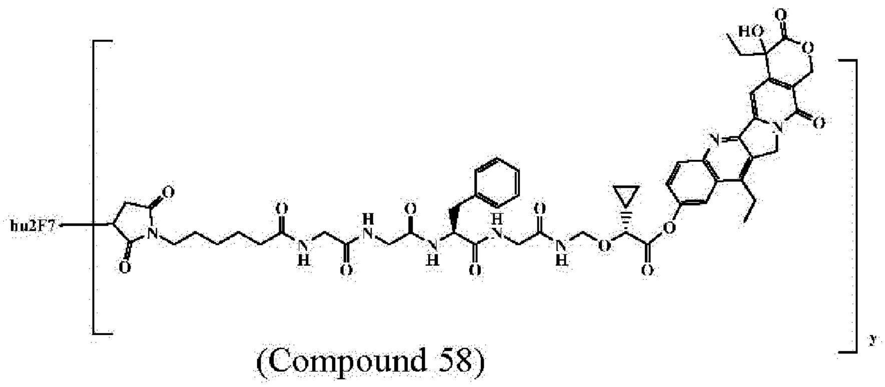

(Compound 58)

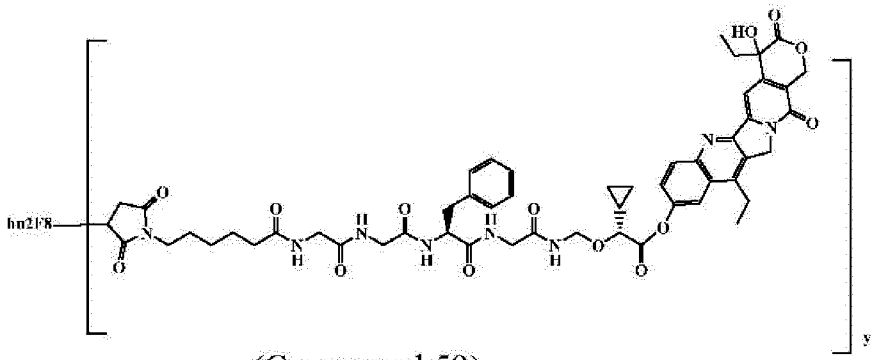

(Compound 59)

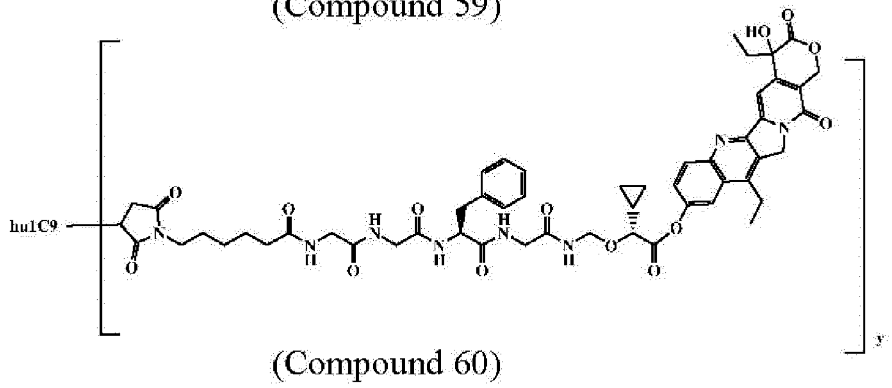

(Compound 60)

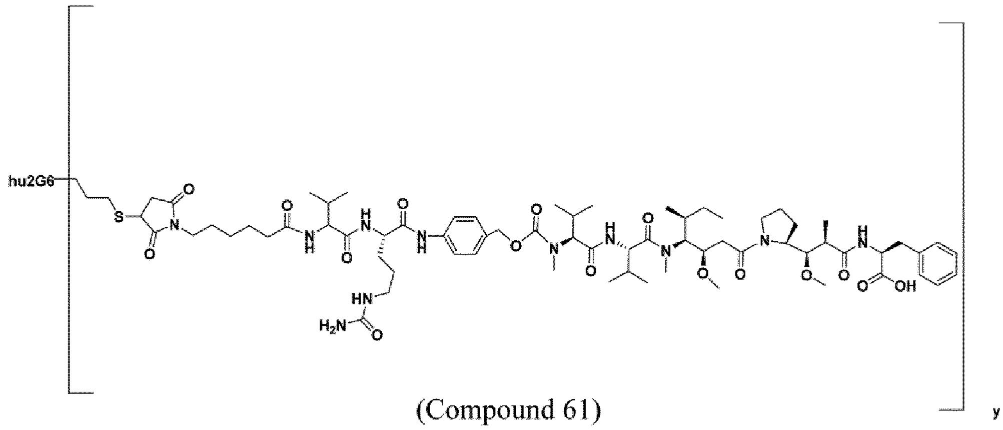

(Compound 61)

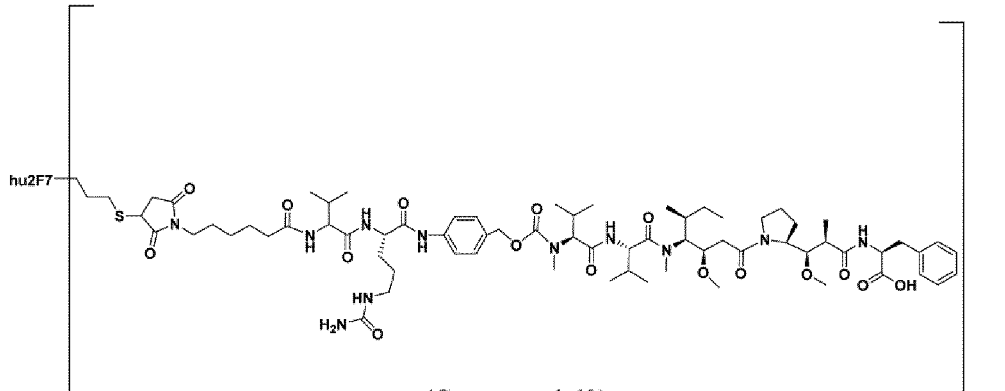

(Compound 62)

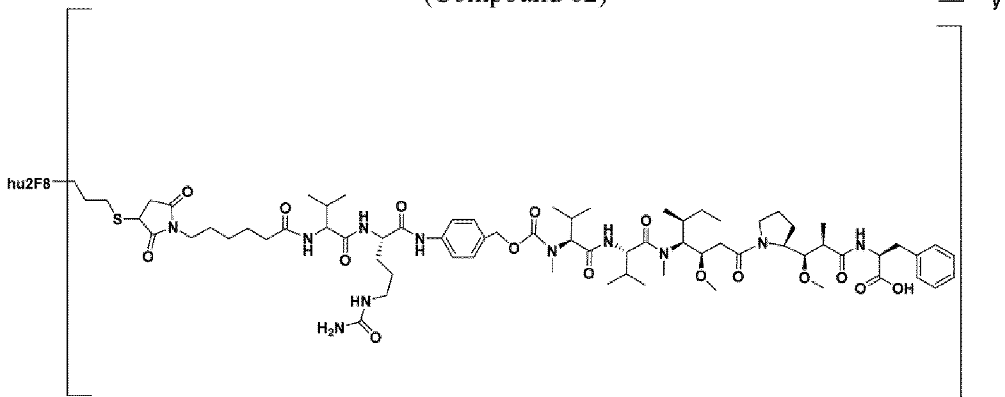

(Compound 63)

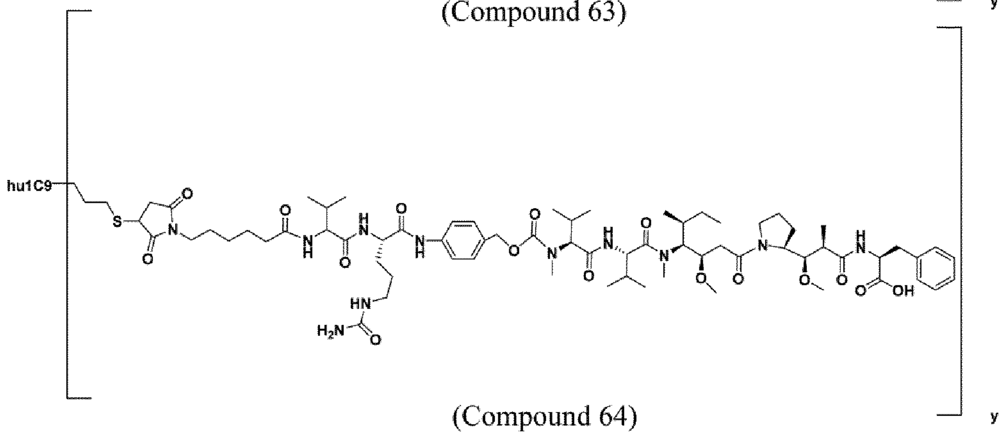

(Compound 64)

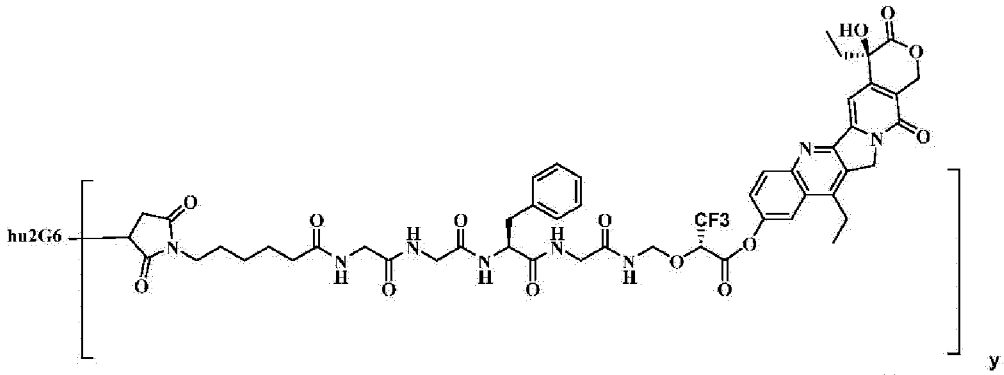

(Compound 65)

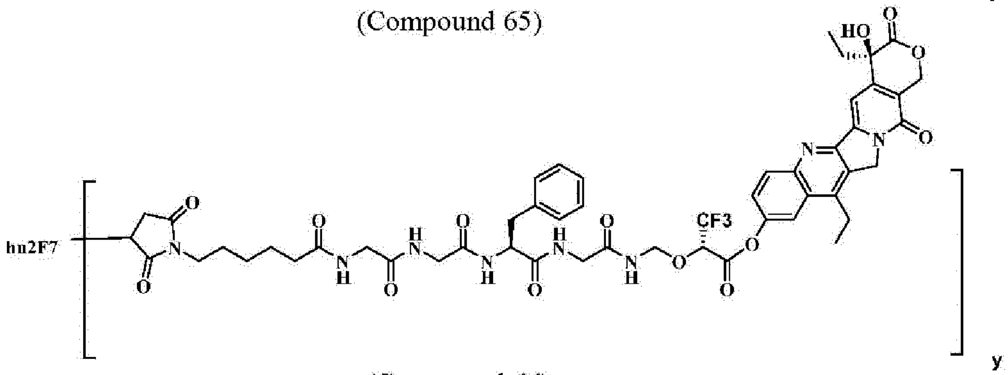

(Compound 66)

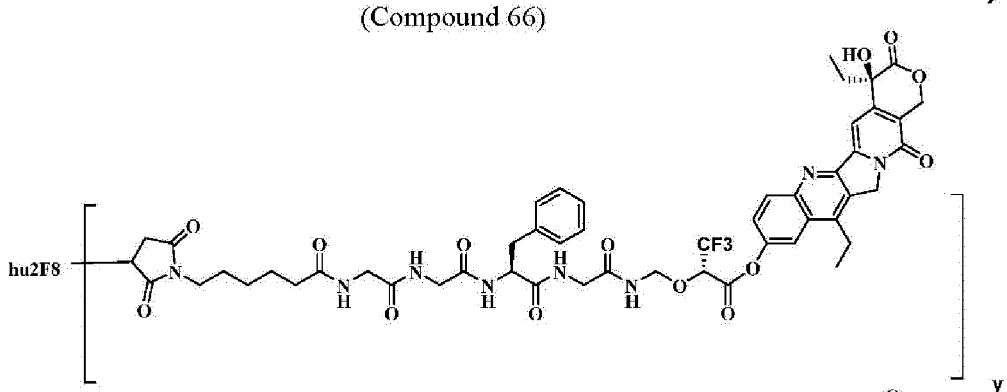

(Compound 67)

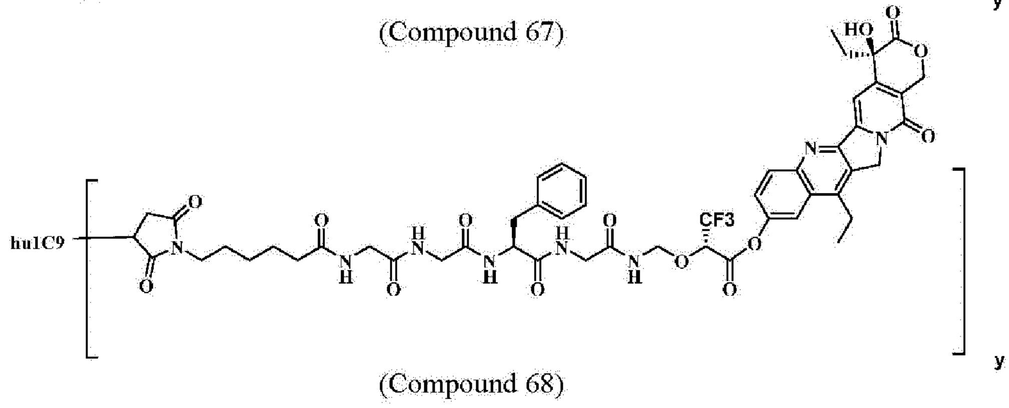

(Compound 68)

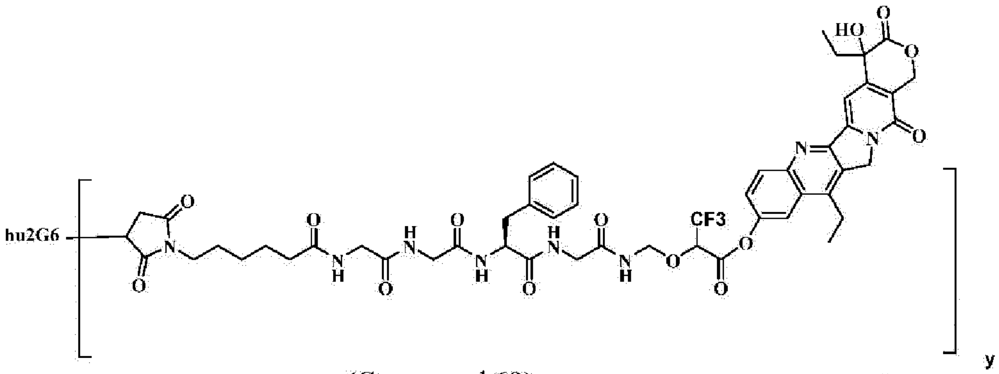

(Compound 69)

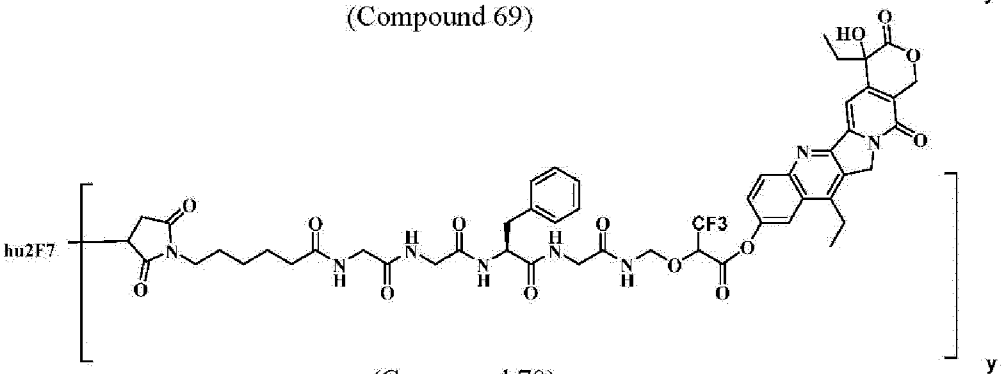

(Compound 70)

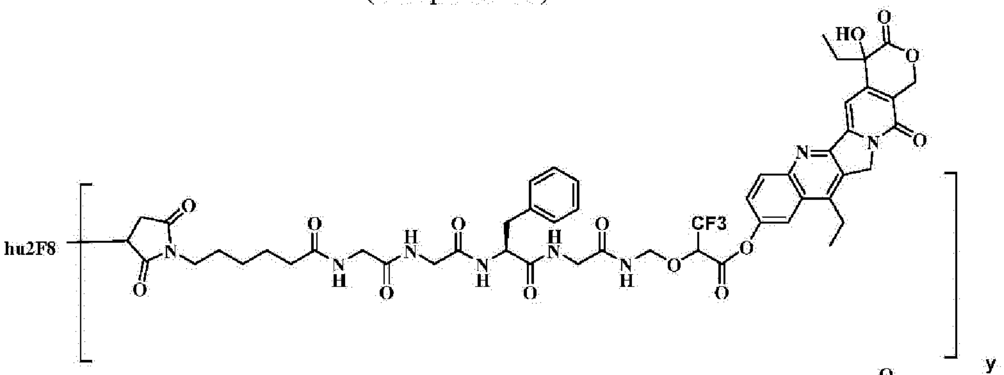

(Compound 71)

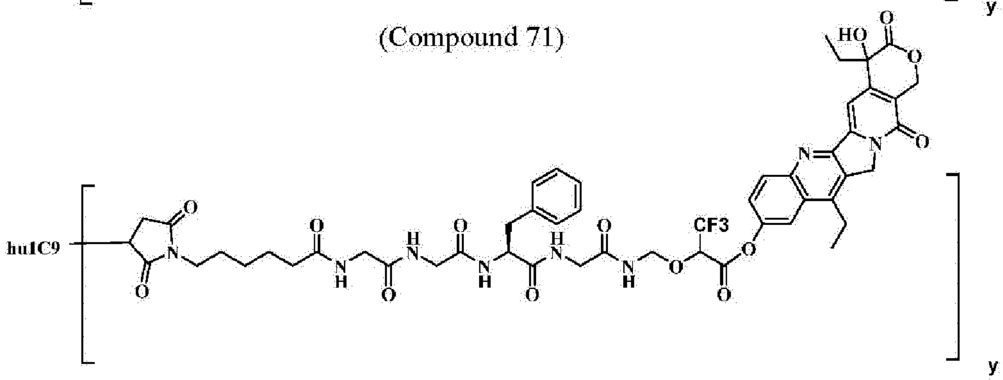

(Compound 72)

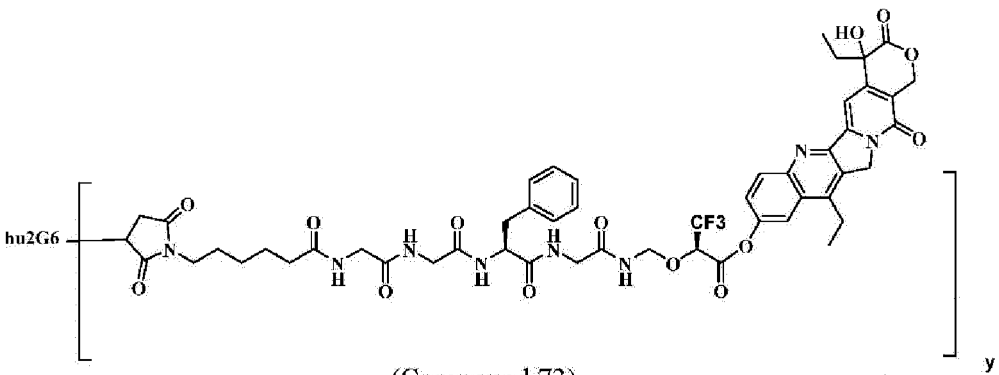

(Compound 73)

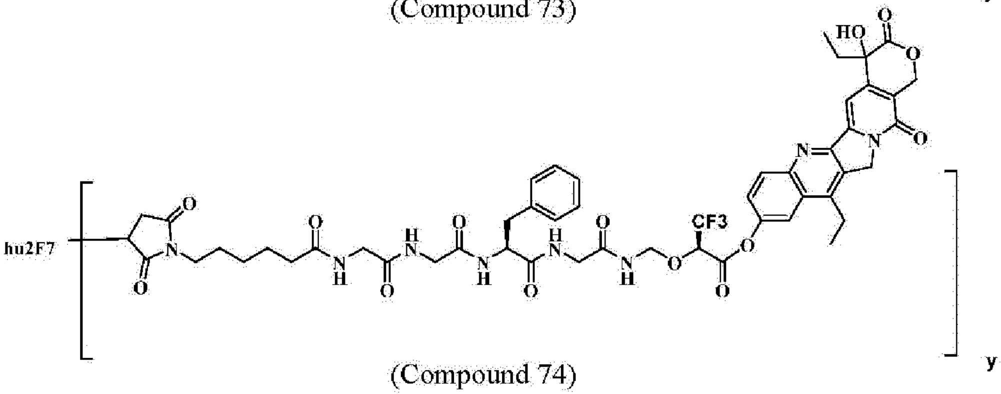

(Compound 74)

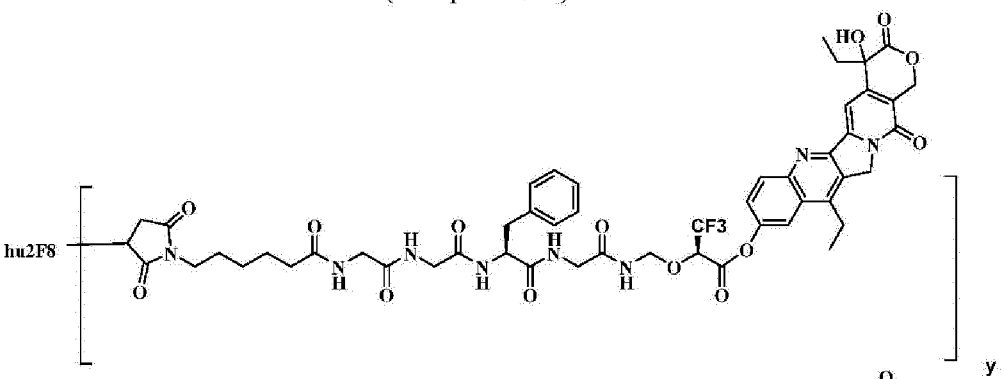

(Compound 75)

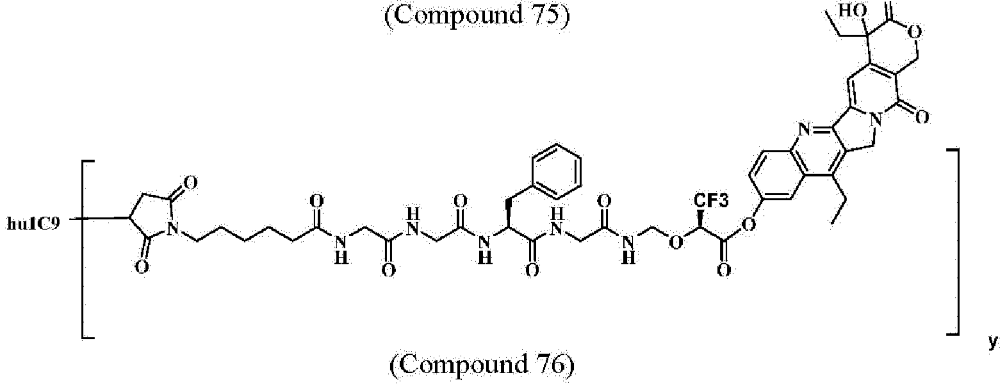

(Compound 76)

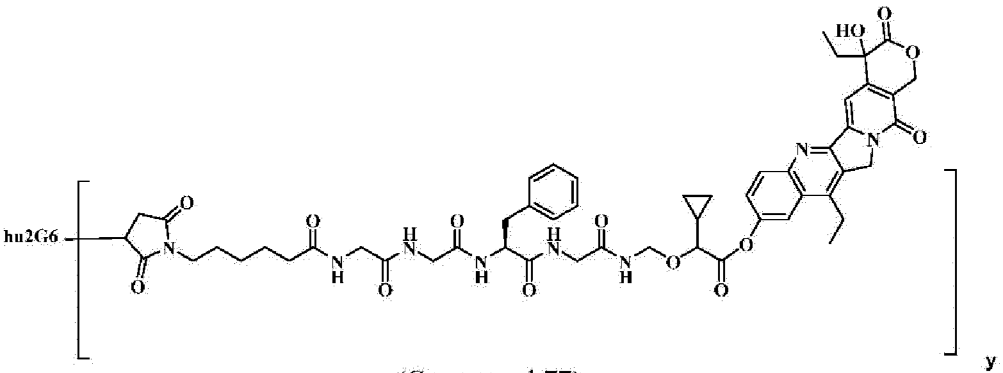

(Compound 77)

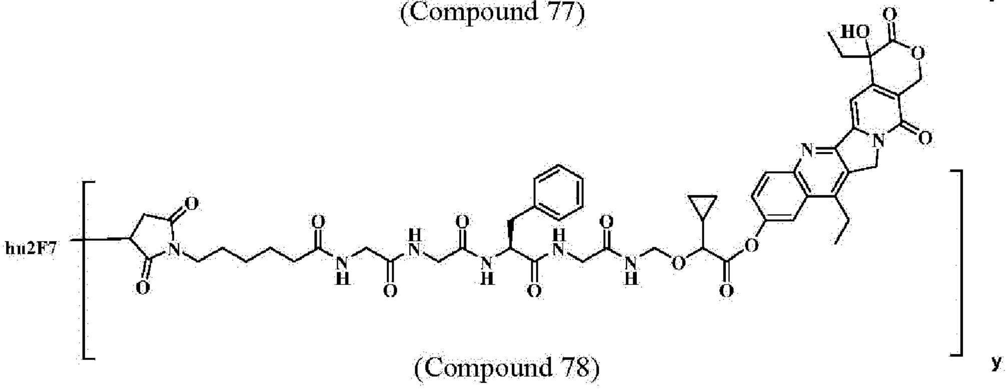

(Compound 78)

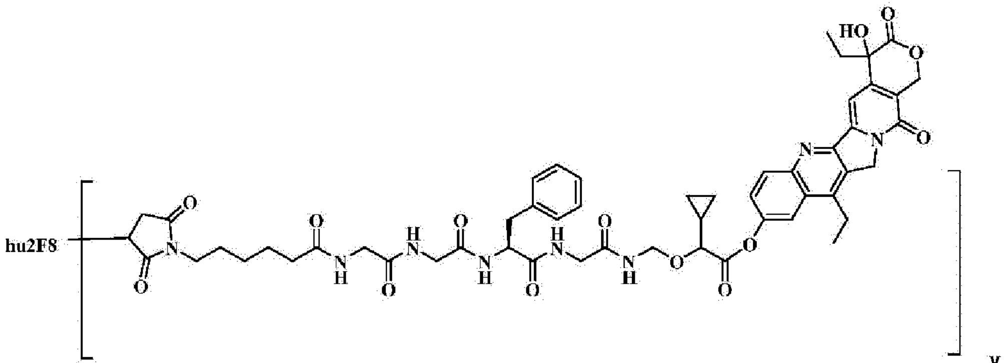

(Compound 79)

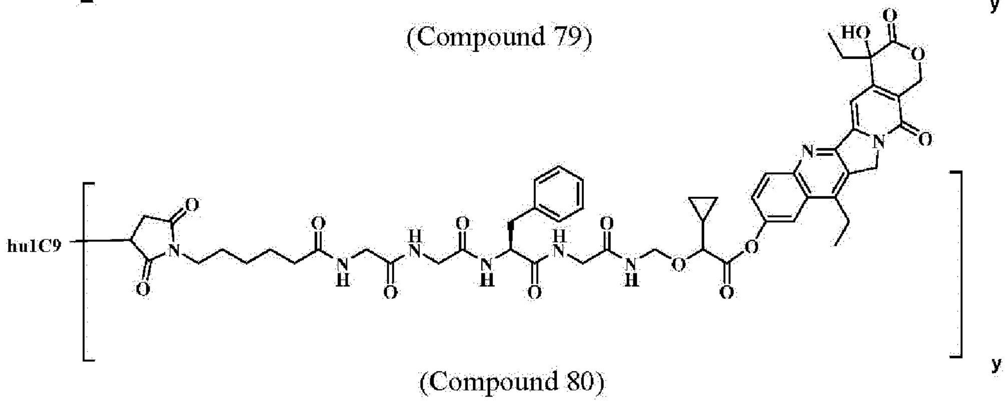

(Compound 80)

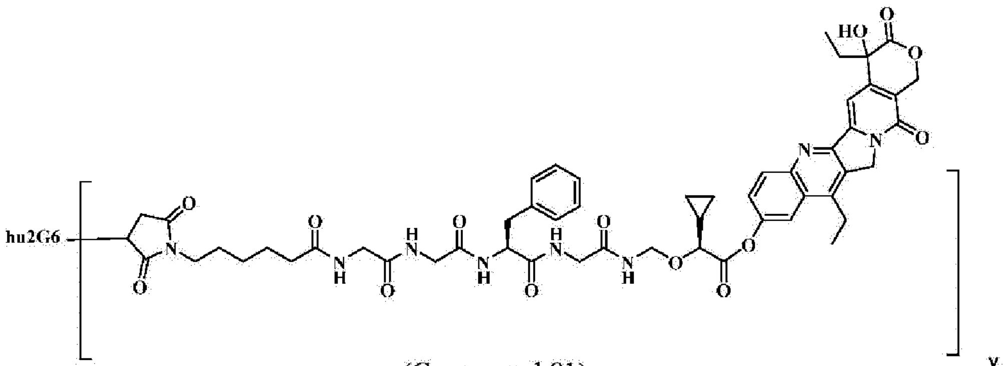

(Compound 81)

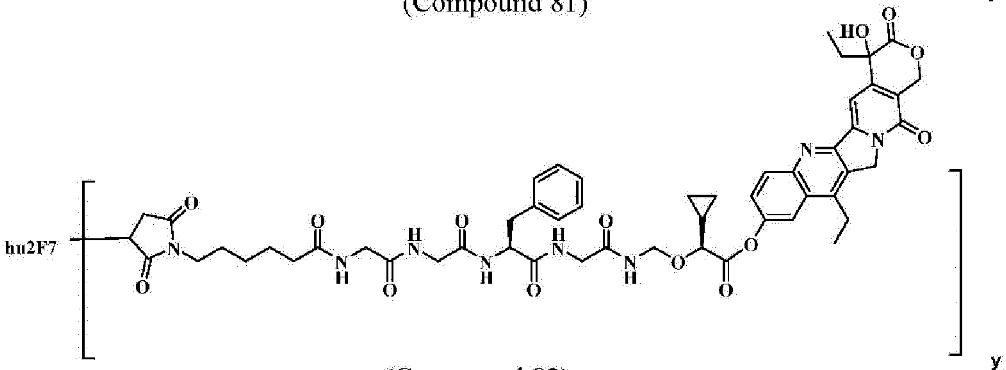

(Compound 82)

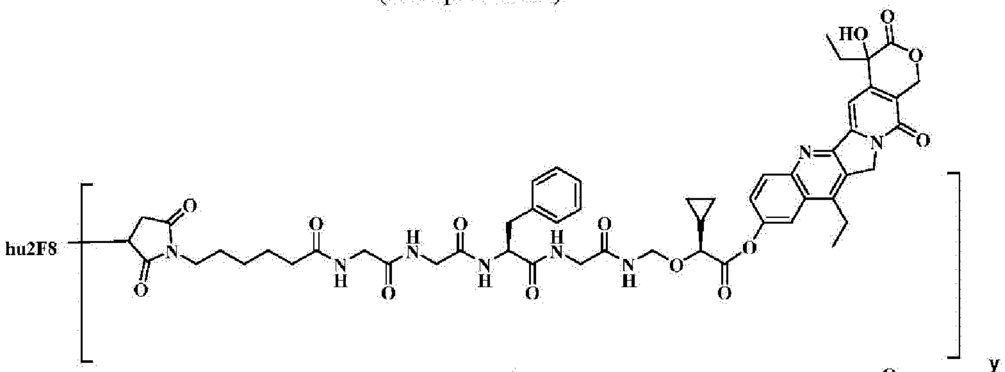

(Compound 83)

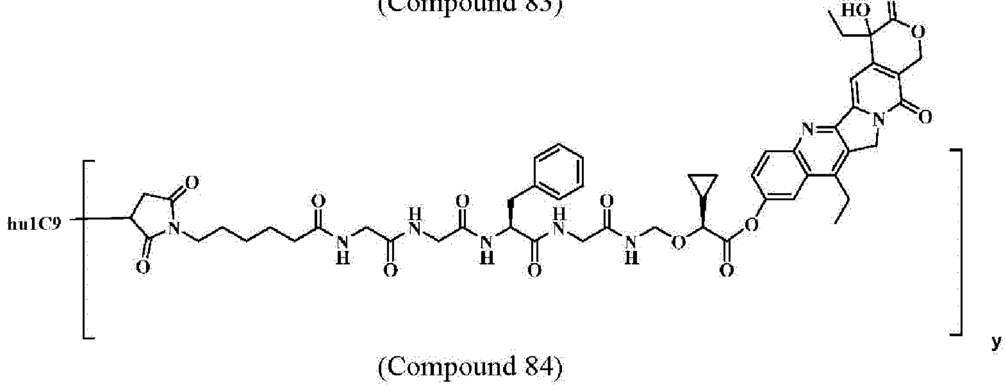

(Compound 84)

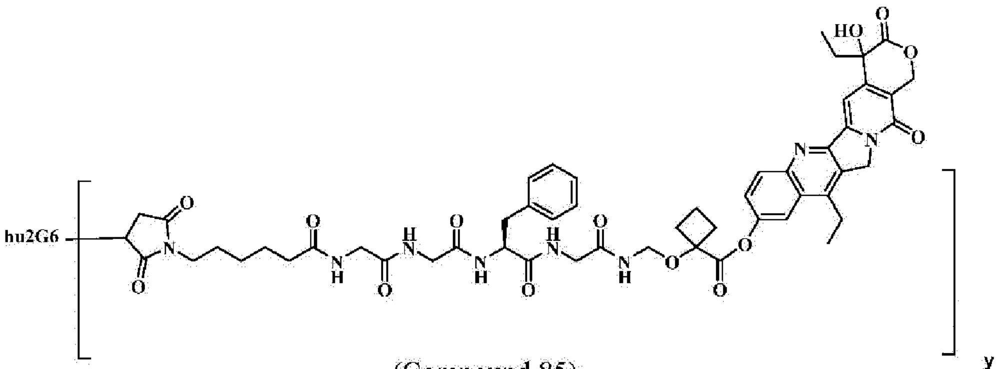

(Compound 85)

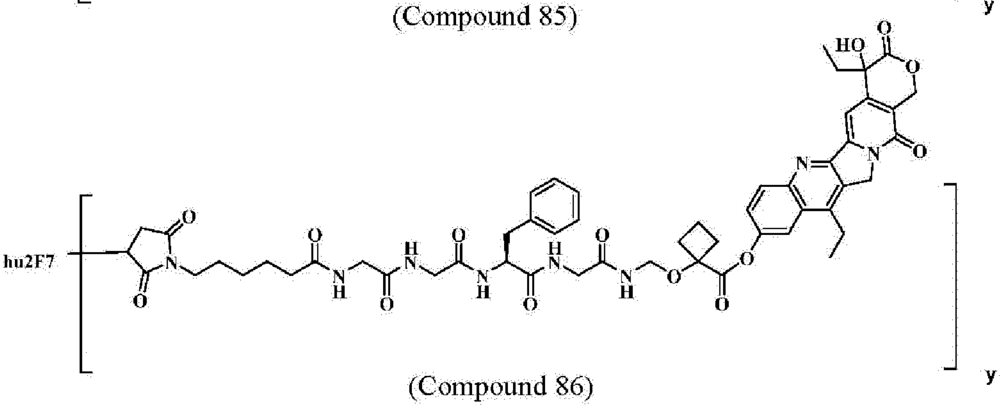

(Compound 86)

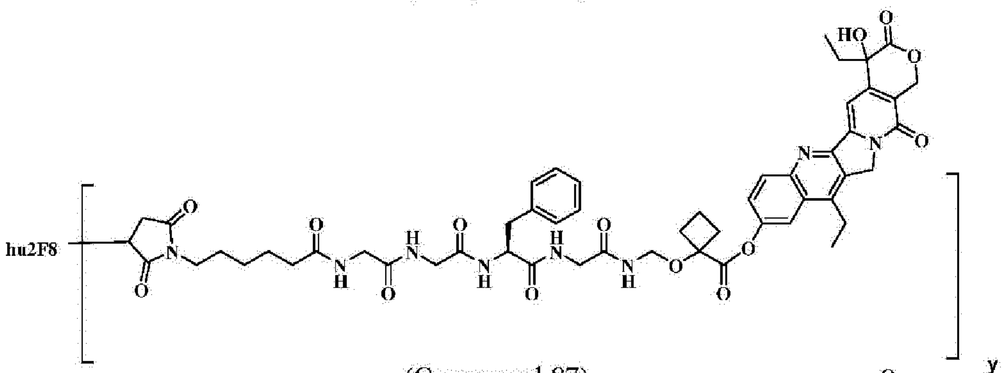

(Compound 87)

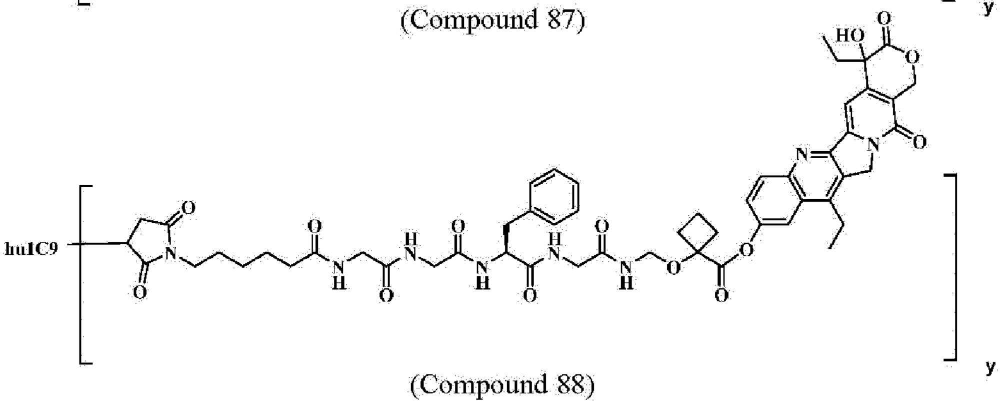

(Compound 88)

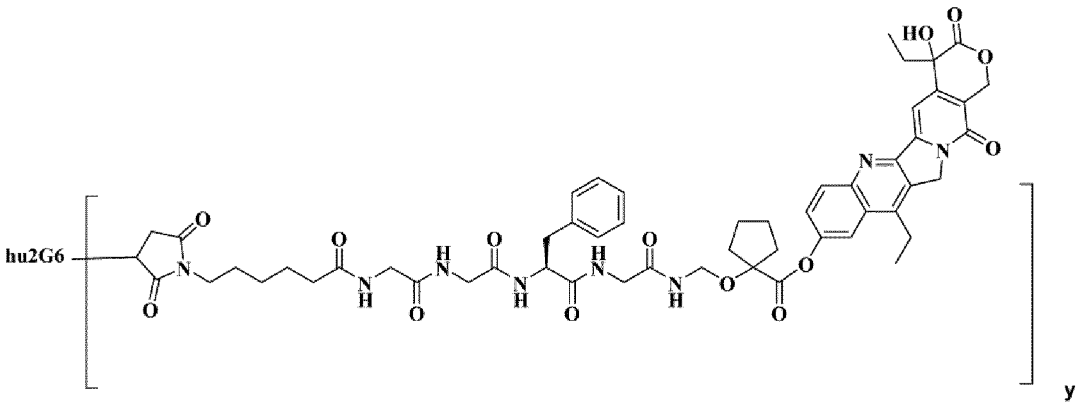

(Compound 89)

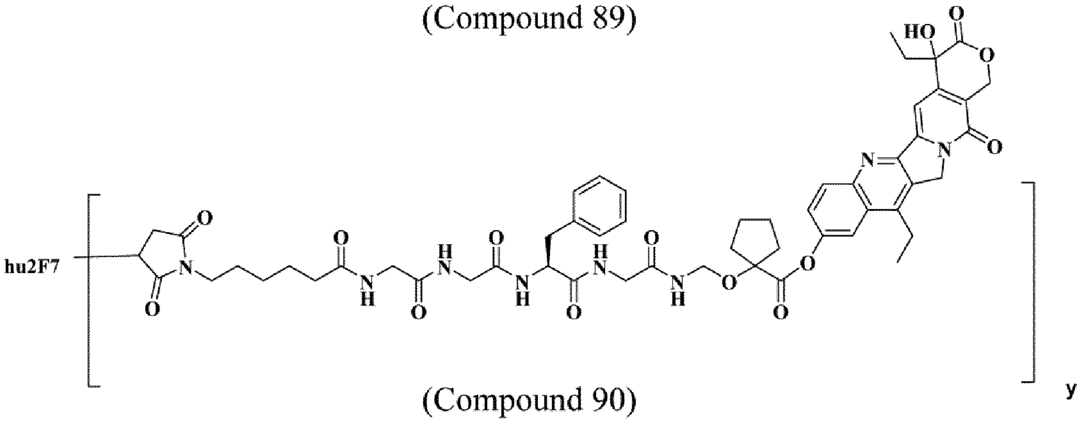

(Compound 90)

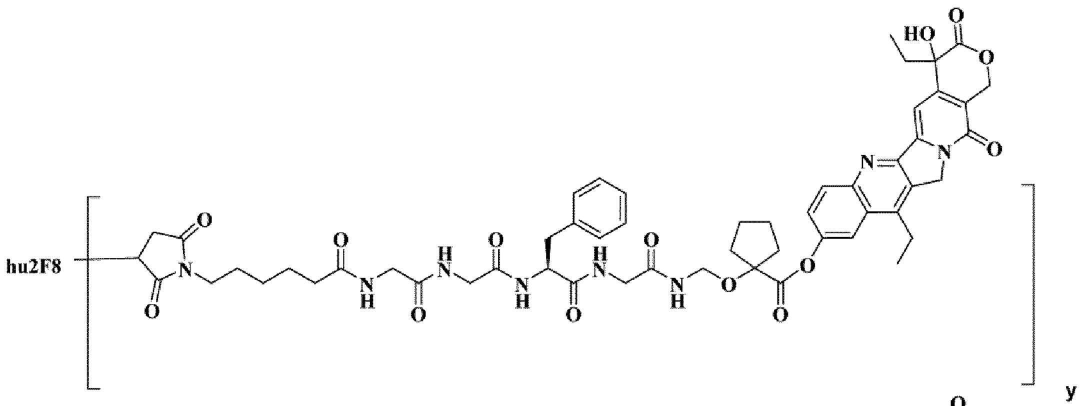

(Compound 91)

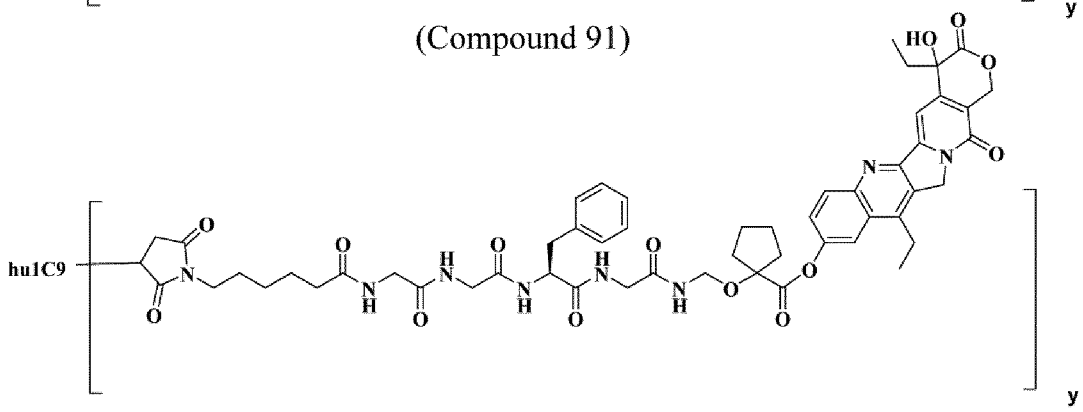

(Compound 92)

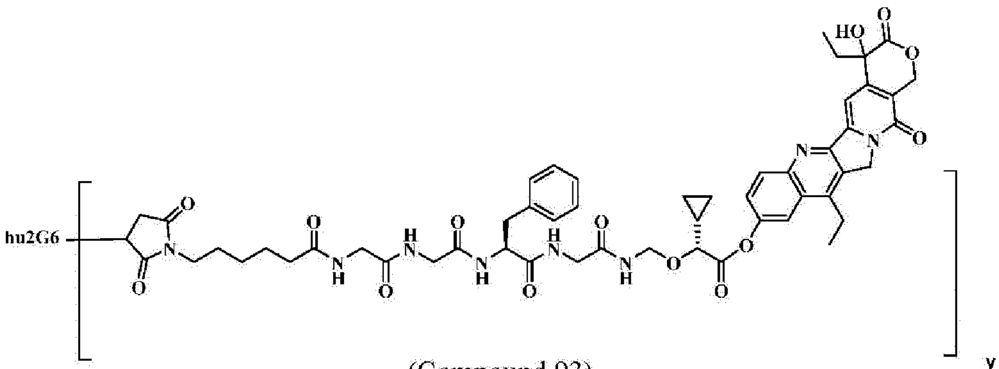

(Compound 93)

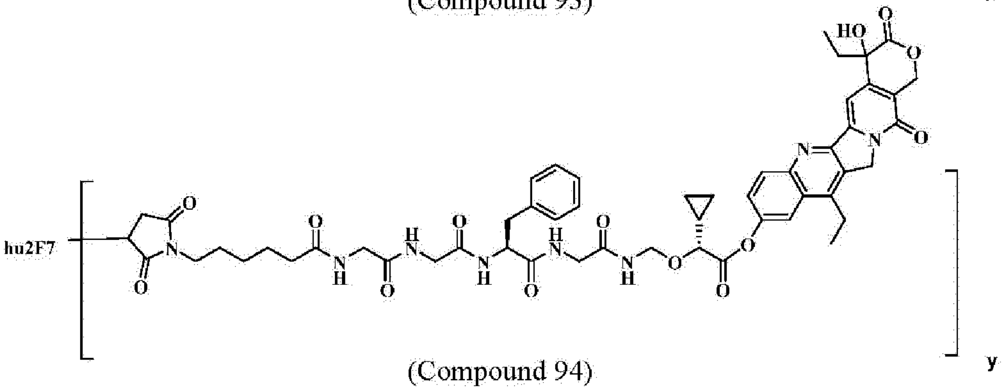

(Compound 94)

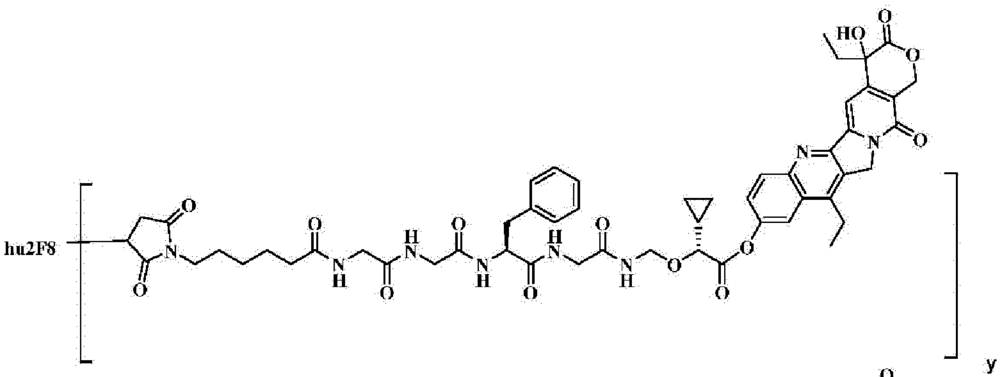

(Compound 95)

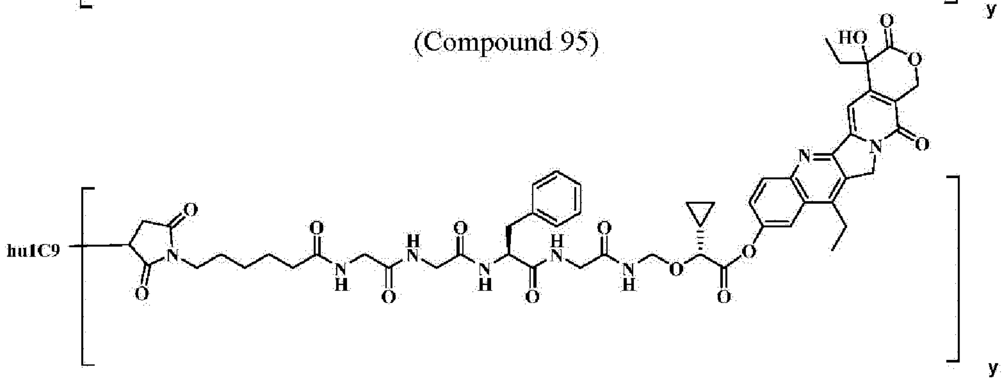

(Compound 96)

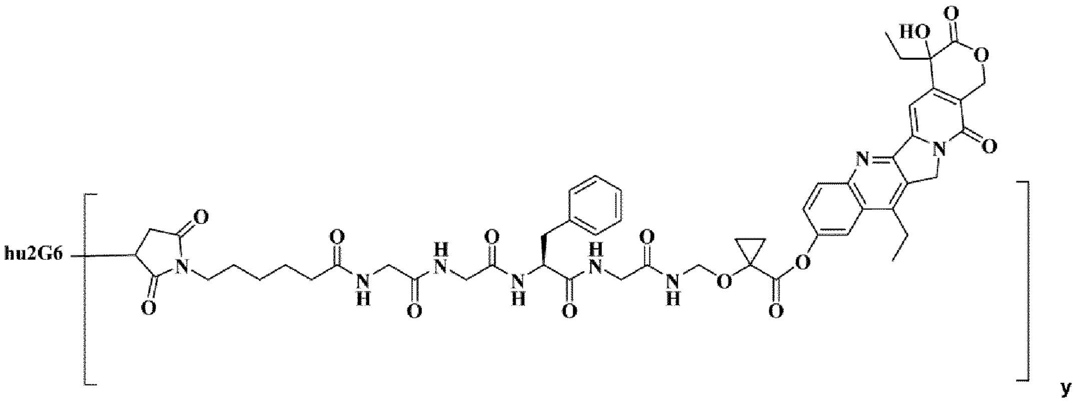

(Compound 97)

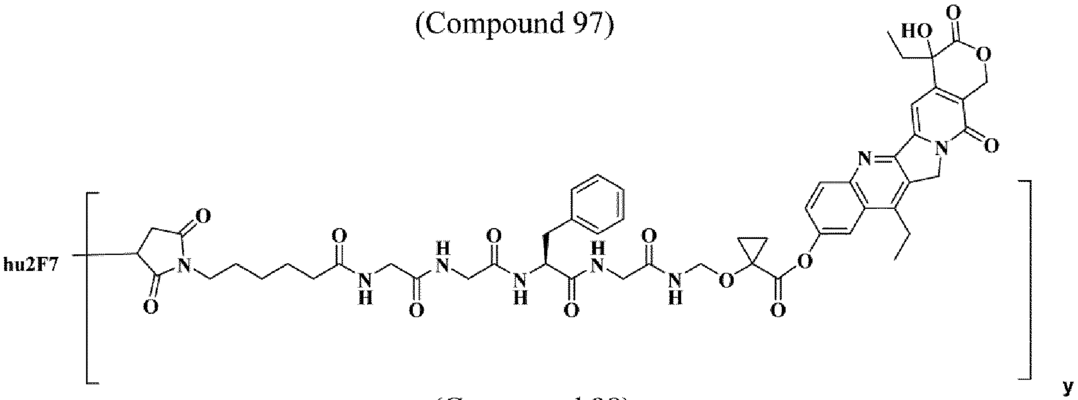

(Compound 98)

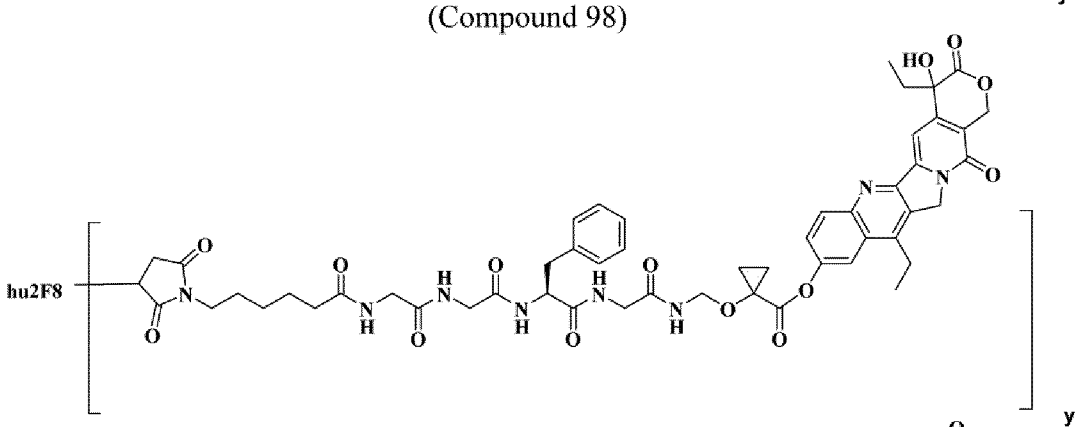

(Compound 99)

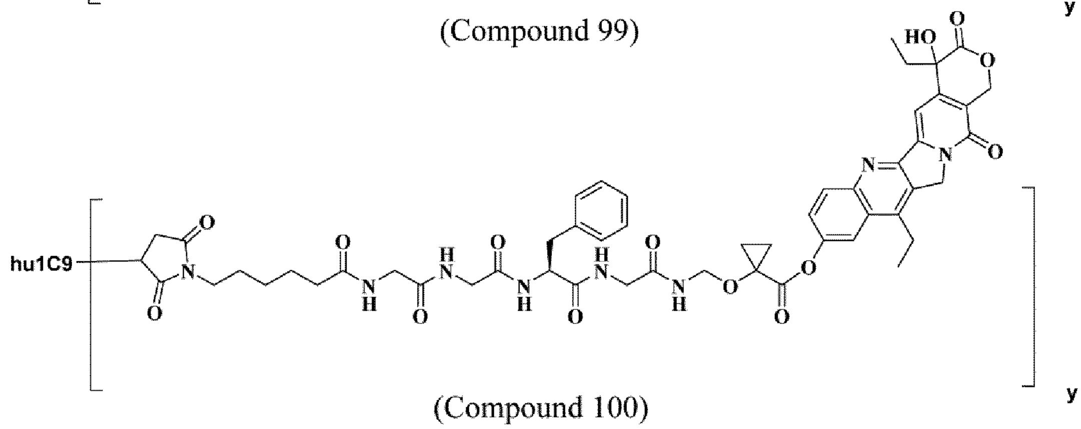

(Compound 100)

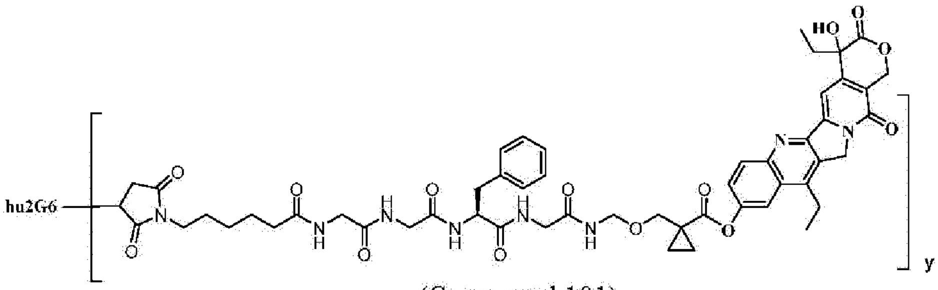

(Compound 101)

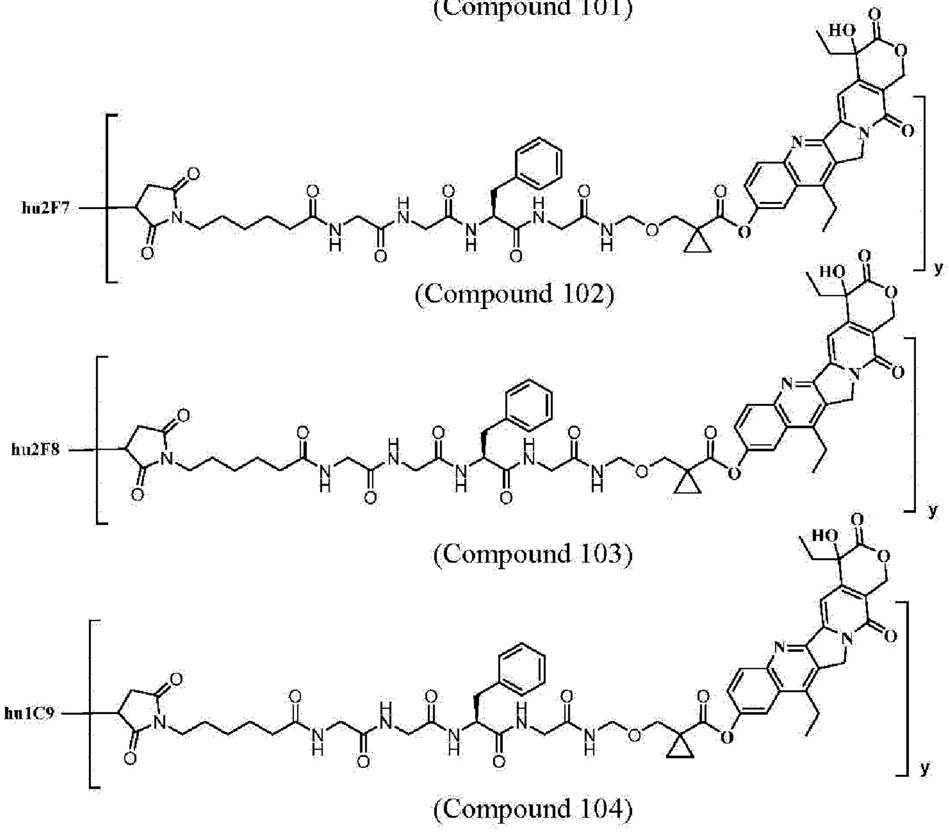

(Compound 102)

(Compound 103)

(Compound 104)

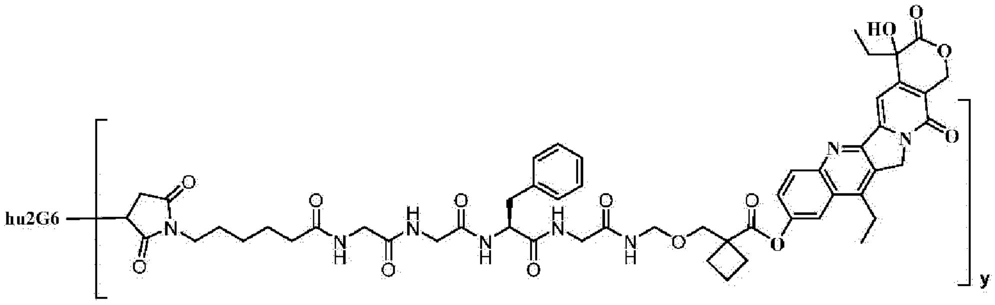

(Compound 105)

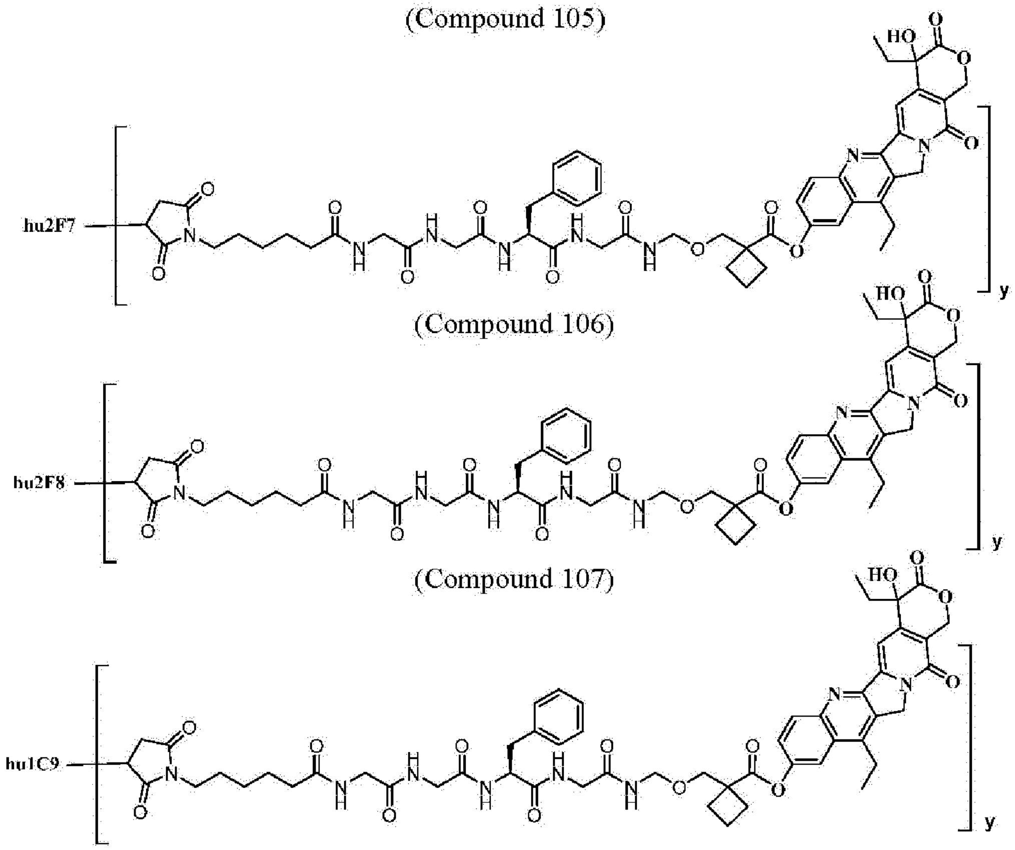

(Compound 106)

(Compound 107)

(Compound 108)

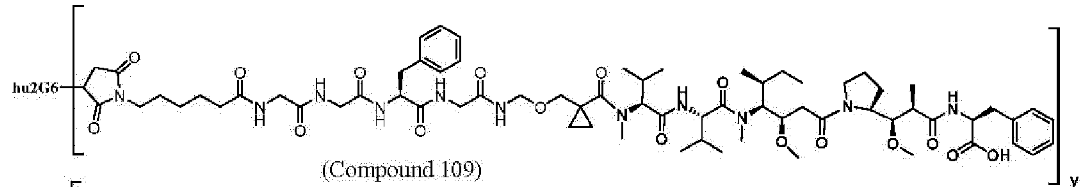

(Compound 109)

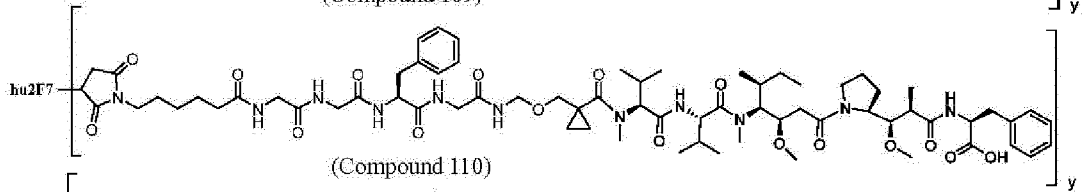

(Compound 110)

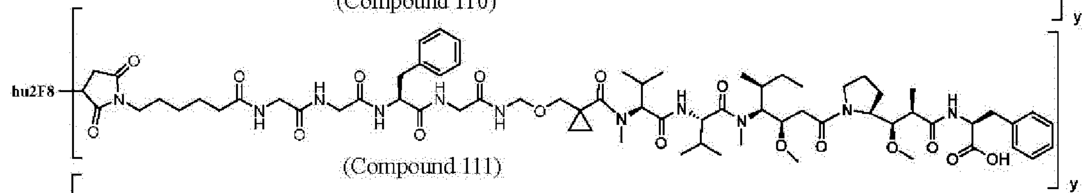

(Compound 111)

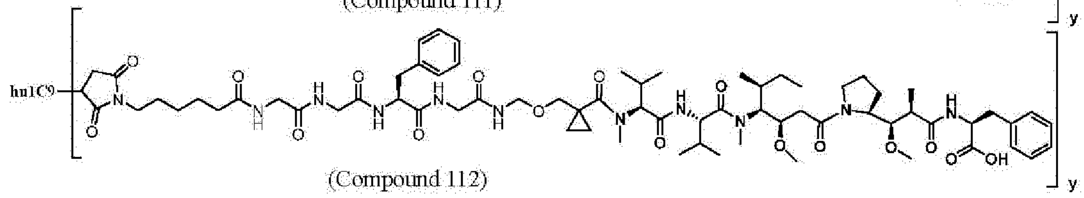

(Compound 112)

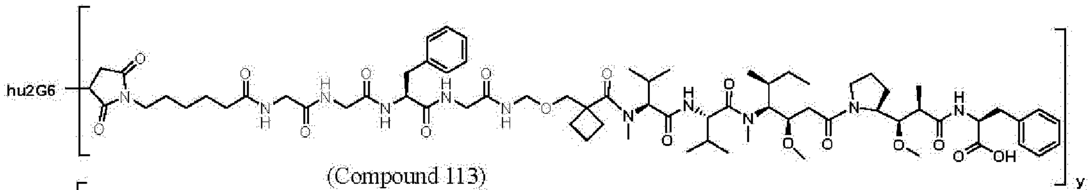

(Compound 113)

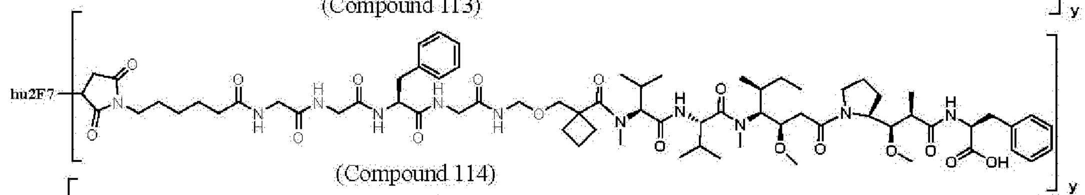

(Compound 114)

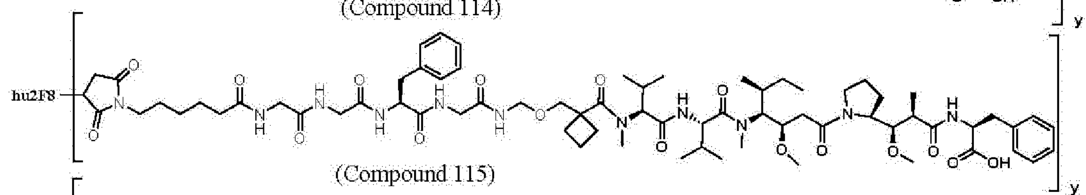

(Compound 115)

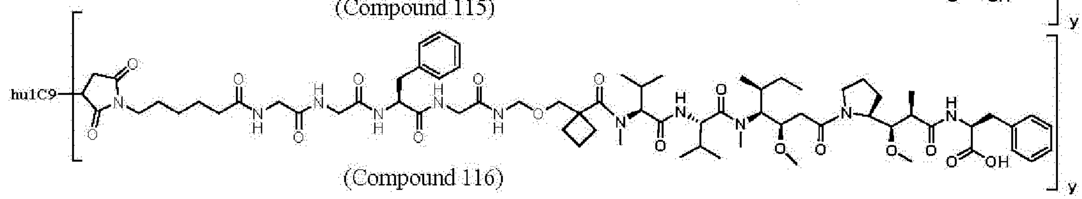

(Compound 116)

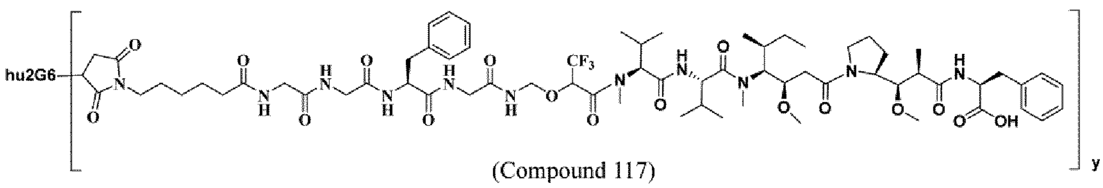

(Compound 117)

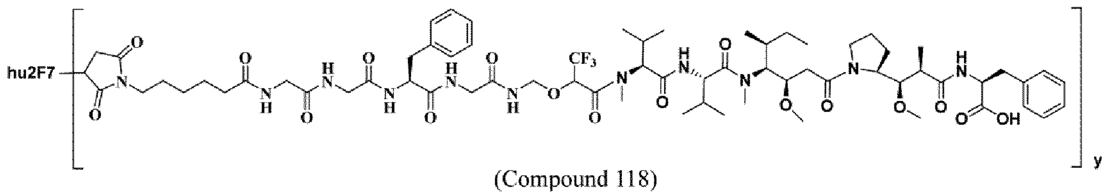

(Compound 118)

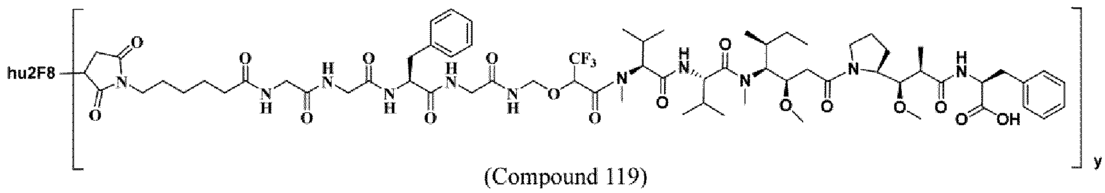

(Compound 119)

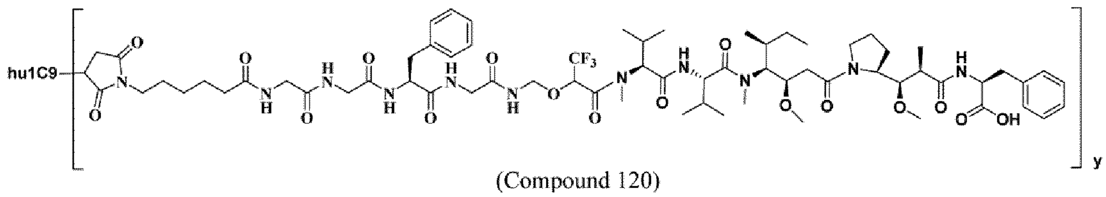

(Compound 120)

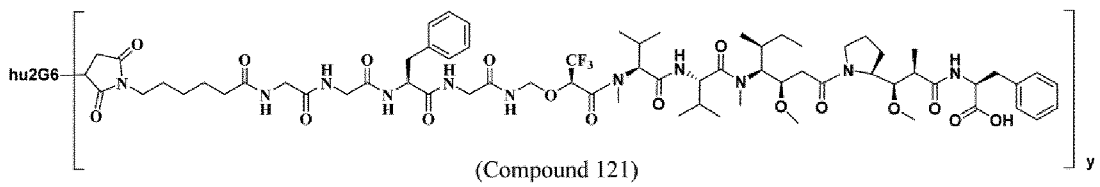

(Compound 121)

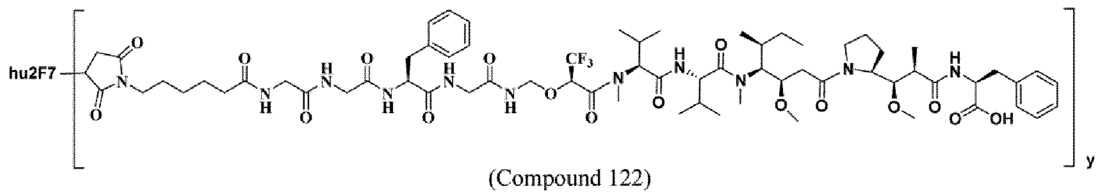

(Compound 122)

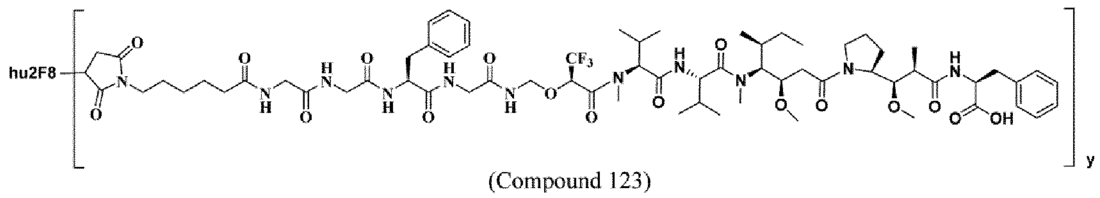

(Compound 123)

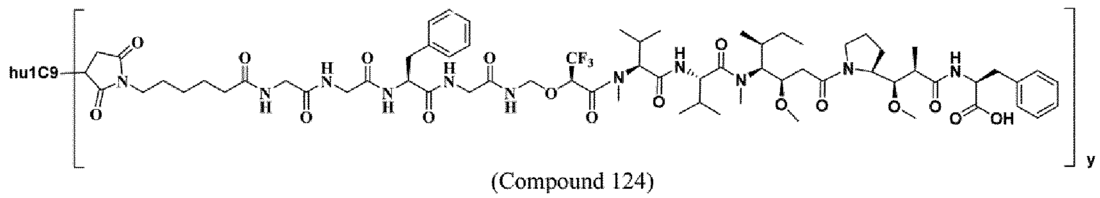

(Compound 124)

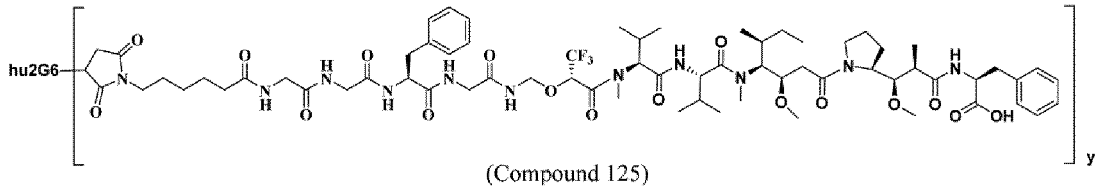

(Compound 125)

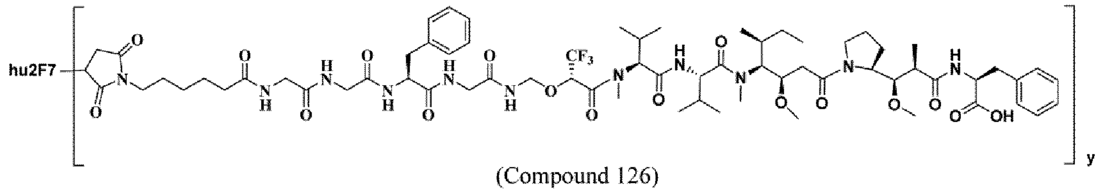

(Compound 126)

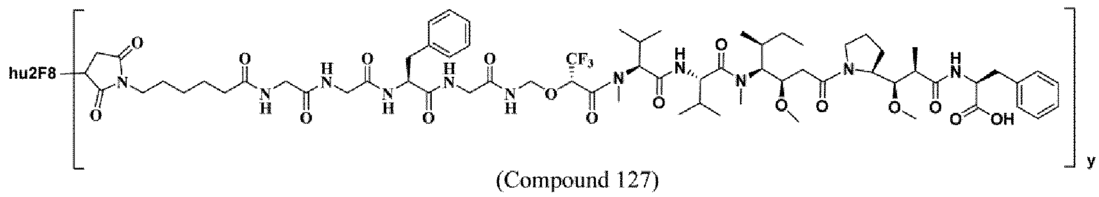

(Compound 127)

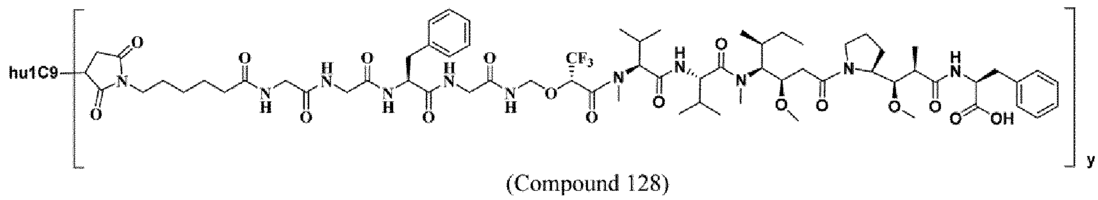

(Compound 128)

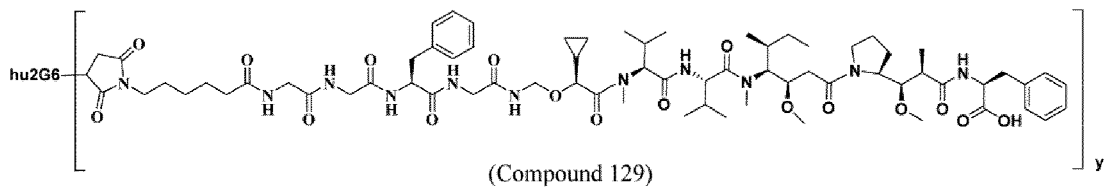

(Compound 129)

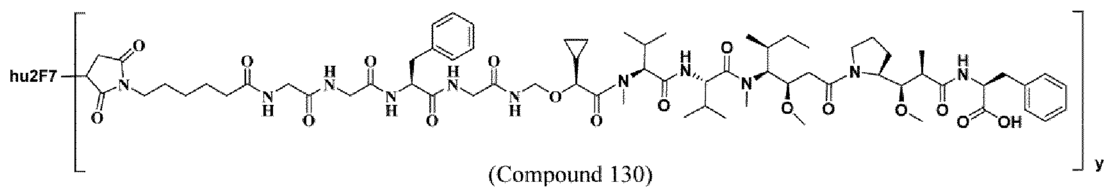

(Compound 130)

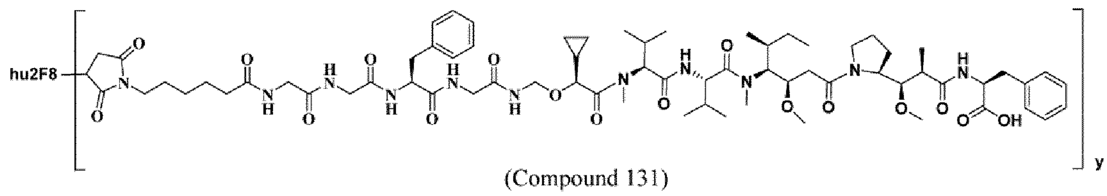

(Compound 131)

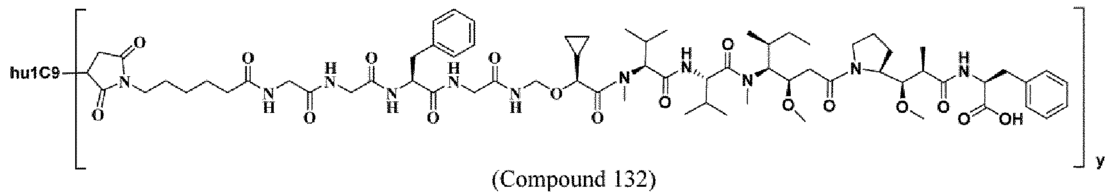

(Compound 132)

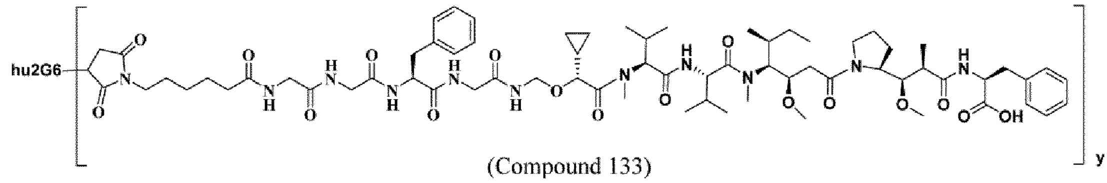

(Compound 133)

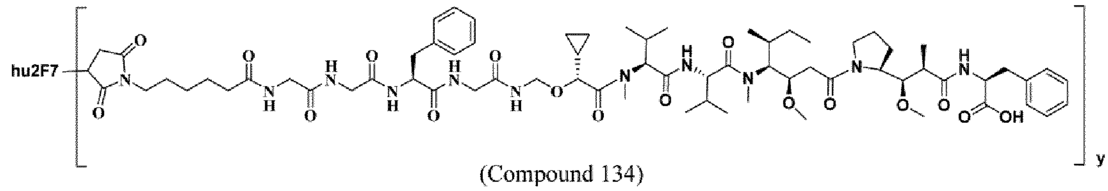

(Compound 134)

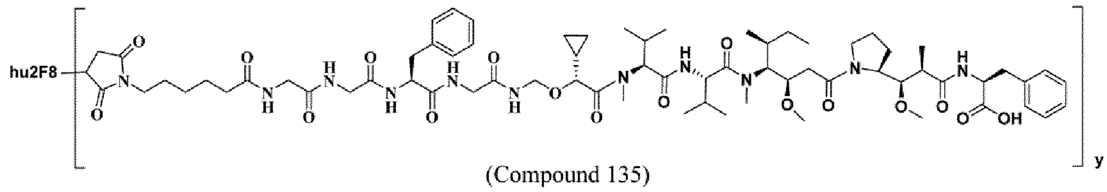

(Compound 135)

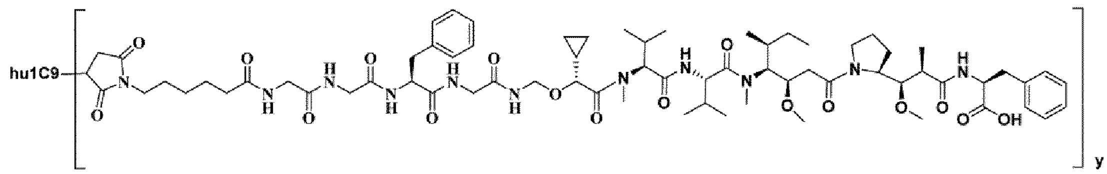

(Compound 136)

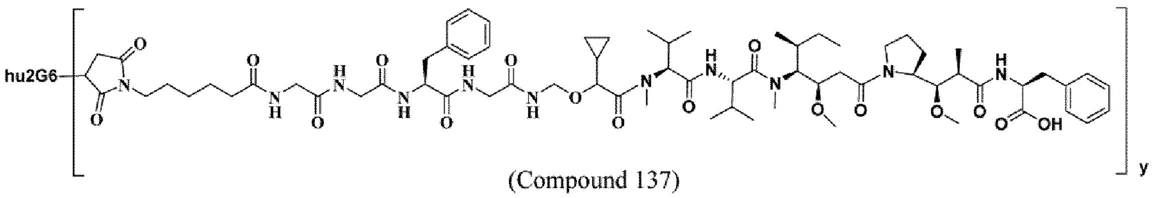

(Compound 137)

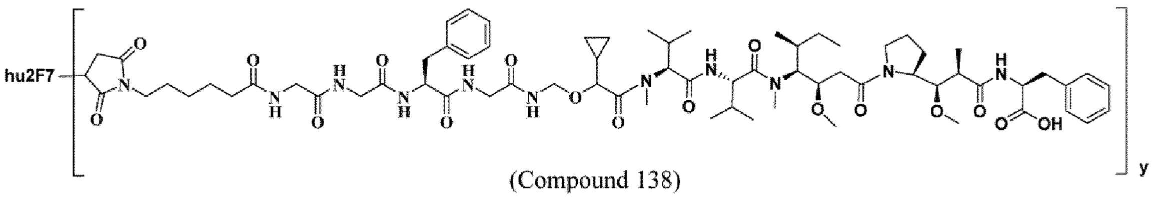

(Compound 138)

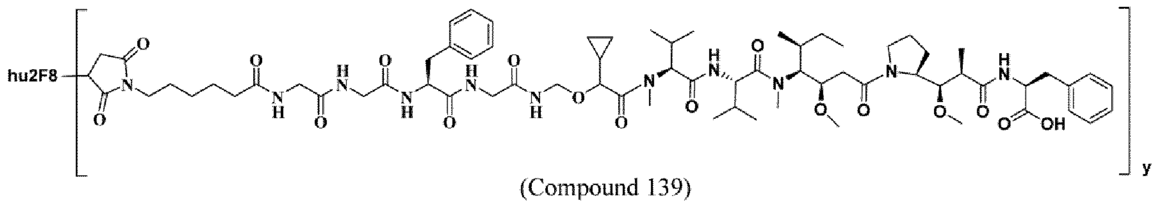

(Compound 139)

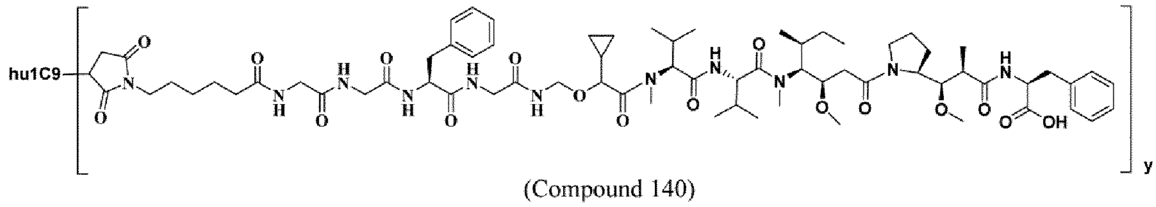

(Compound 140)

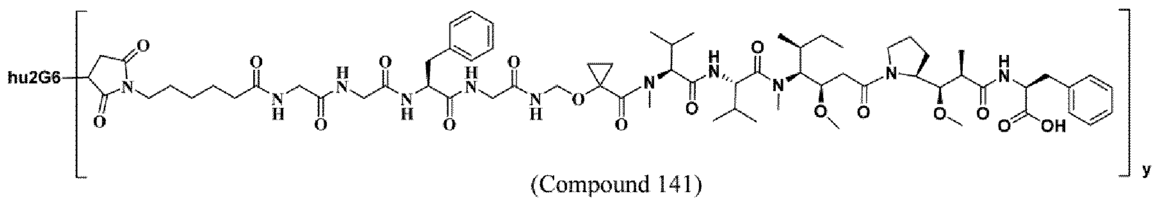

(Compound 141)

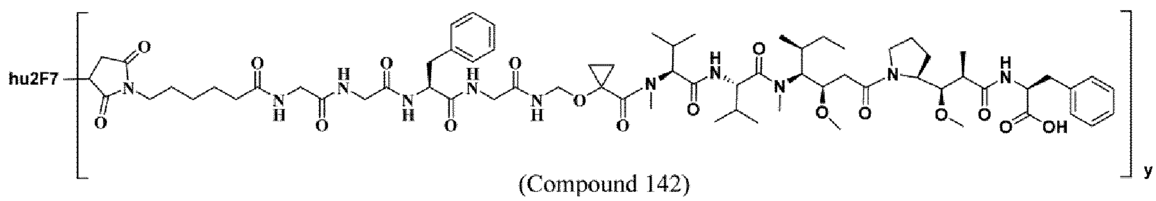

(Compound 142)

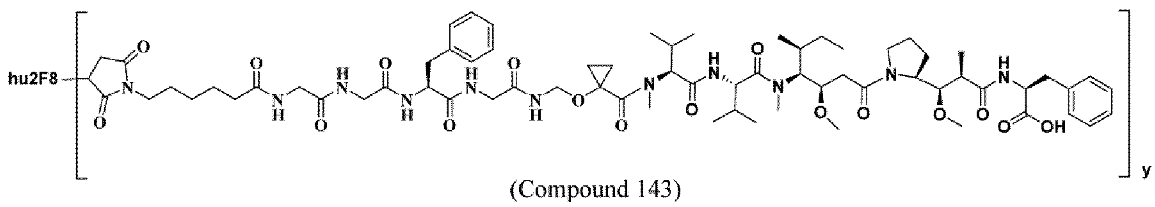

(Compound 143)

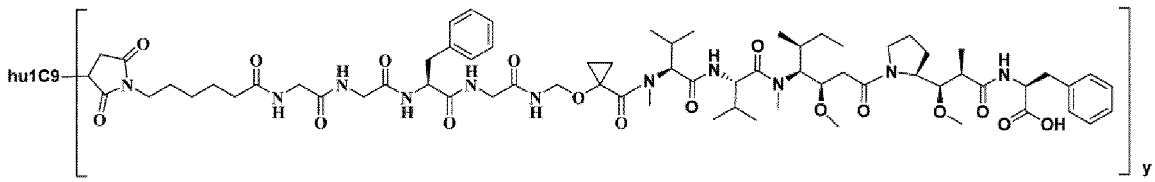

(Compound 144)

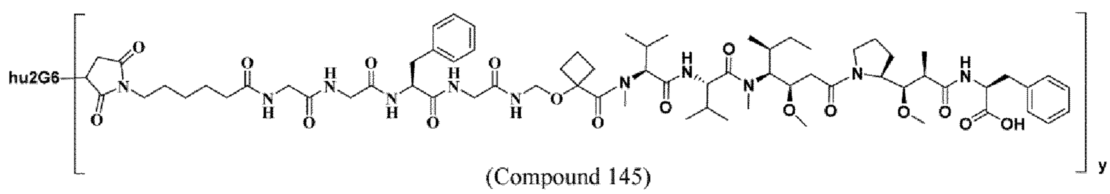

(Compound 145)

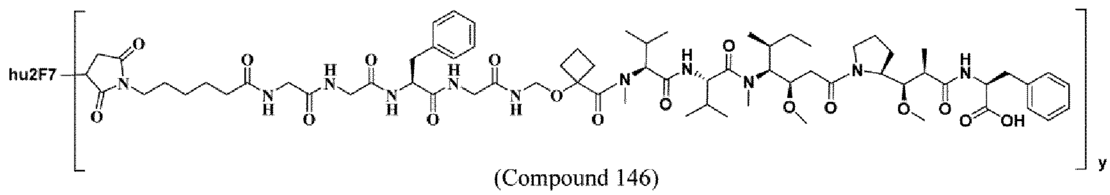

(Compound 146)

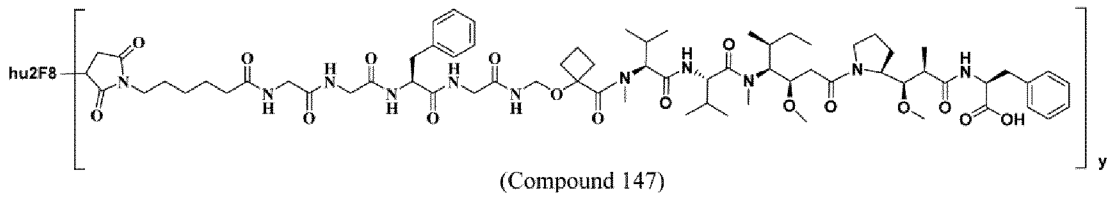

(Compound 147)

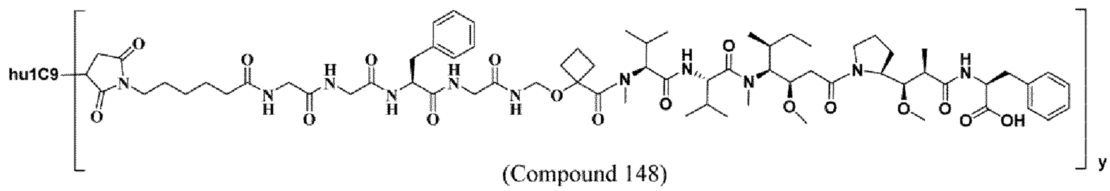

(Compound 148)

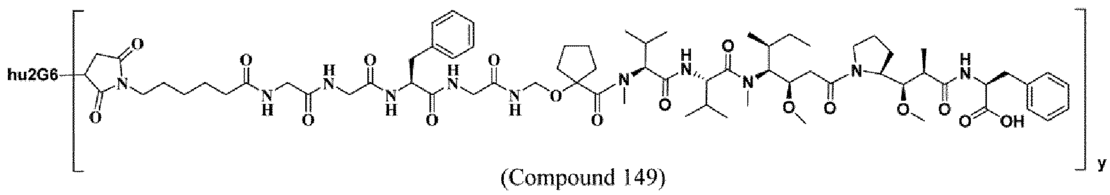

(Compound 149)

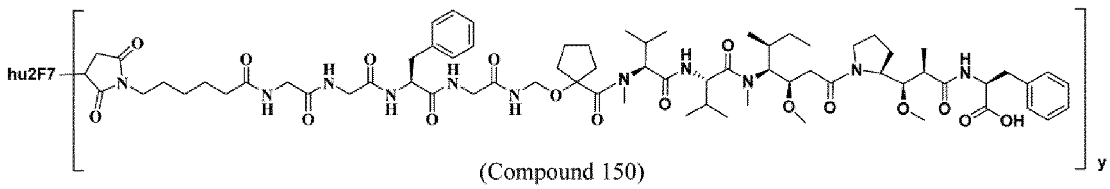

(Compound 150)

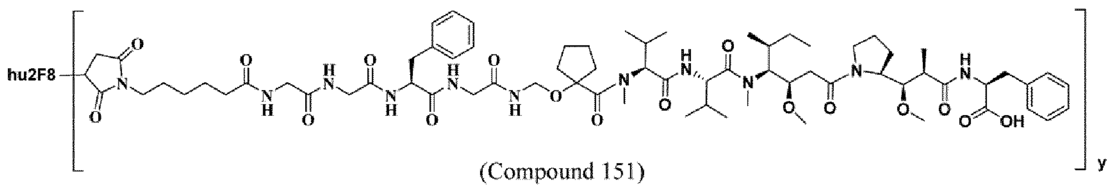

(Compound 151)

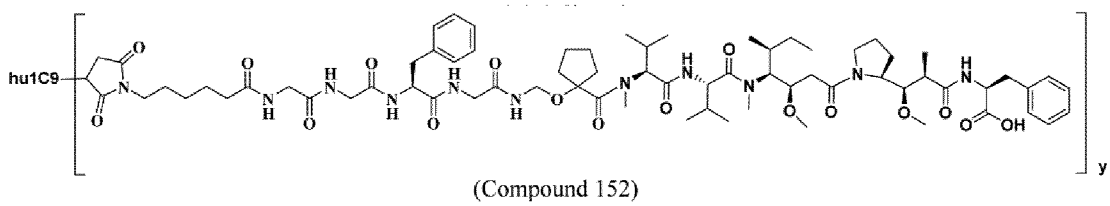

(Compound 152)

wherein, y is selected from 2 to 10, preferably 4 to 8, more preferably 6to 8, further preferably 7to 8, and most preferably 8.

**36.** A method for preparing the antibody-drug conjugate of general formula (IV) or the pharmaceutically acceptable salt or solvate thereof, which comprises the following steps:

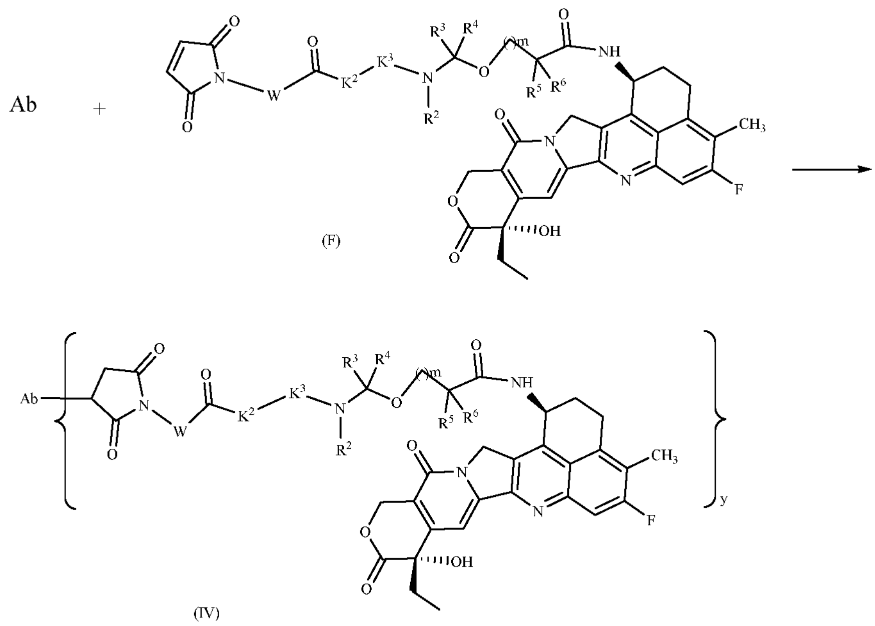
(F)

(IV)

once Ab is reduced, it is subjected to coupling reaction with general formula (F) to obtain the compound of general formula (IV);
wherein:

Ab is an anti-B7-H4 antibody or antigen-binding fragment thereof;
W, $K^2$, $K^3$, $R^2$ to $R^6$, m and y are as defined in claim 29.

**37.** The method according to claim 36, wherein the general formula (F) is a compound of general formula (F-1):

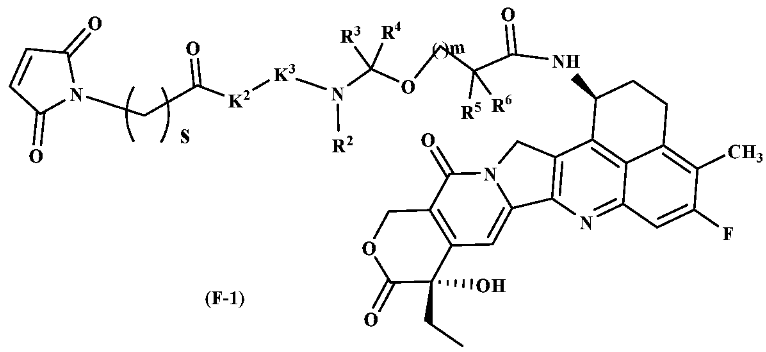
(F-1)

or a tautomer, mesomer, racemate, enantiomer, diastereomer or mixture form thereof, or the pharmaceutically acceptable salt thereof,
wherein
$K^2$, $K^3$, $R^2$ to $R^6$, s and m are as defined in claim 28.

**38.** The compound of general formula (F) or general formula (F-1), which is selected from the group consisting of:

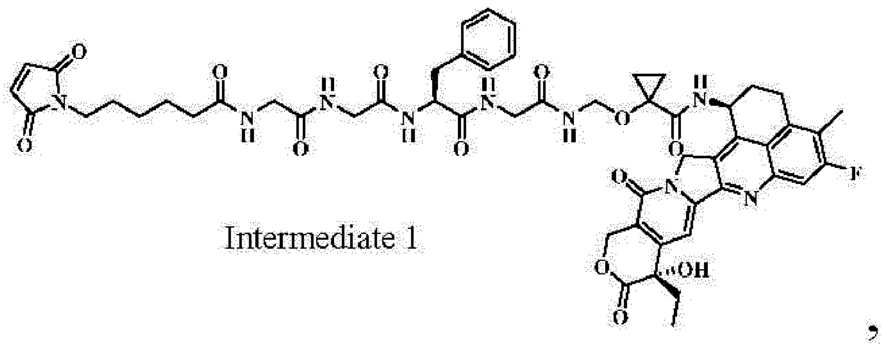

Intermediate 1,

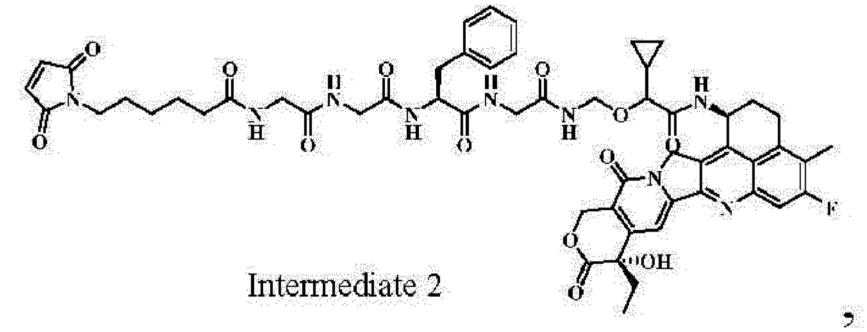

Intermediate 2,

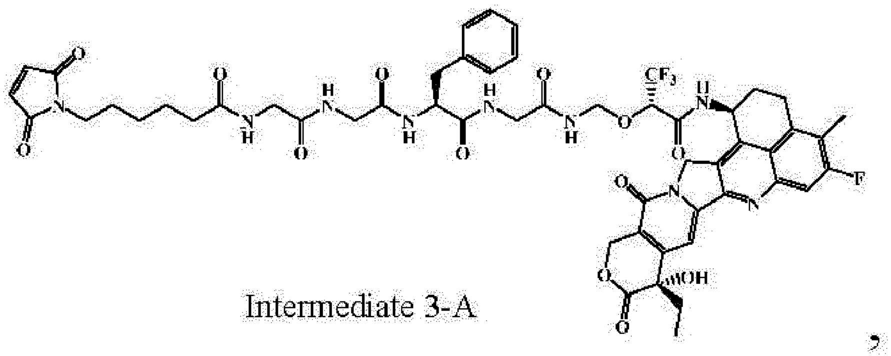

Intermediate 3-A,

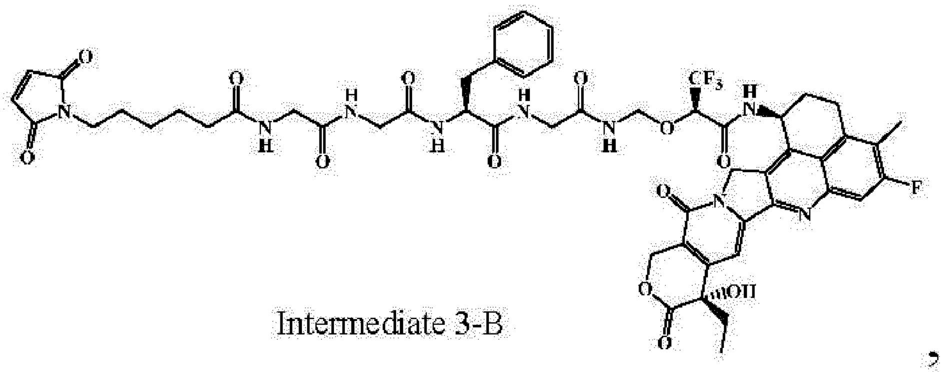

Intermediate 3-B,

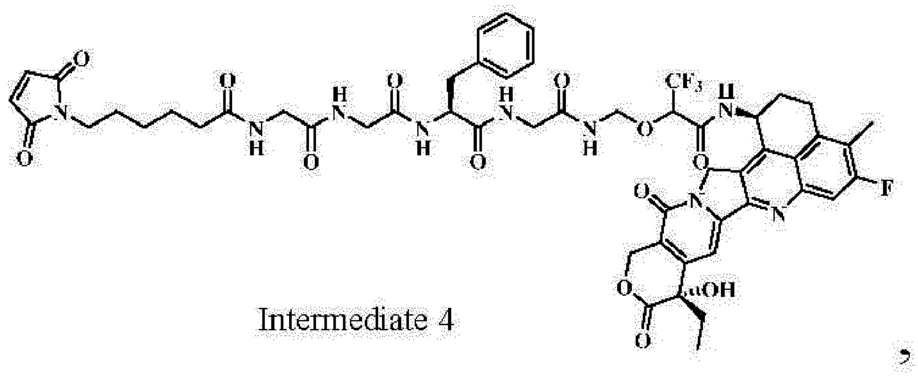

Intermediate 4

,

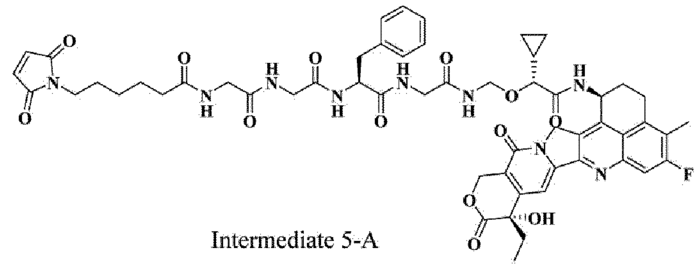

Intermediate 5-A

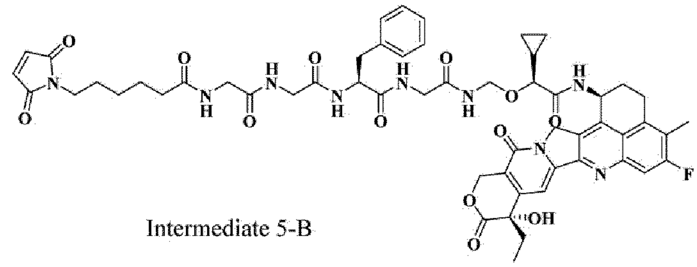

Intermediate 5-B

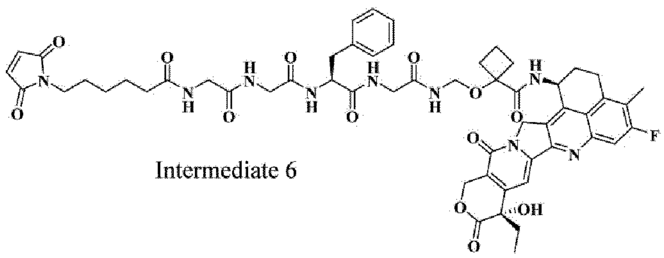

Intermediate 6

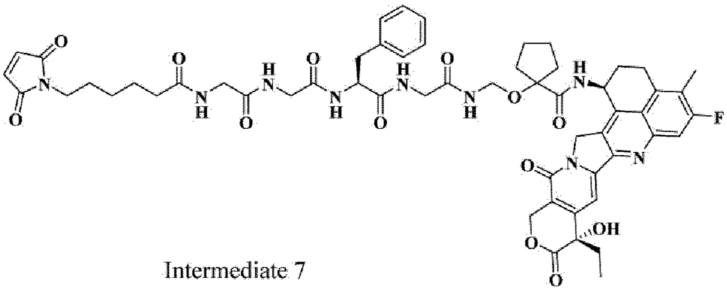

Intermediate 7

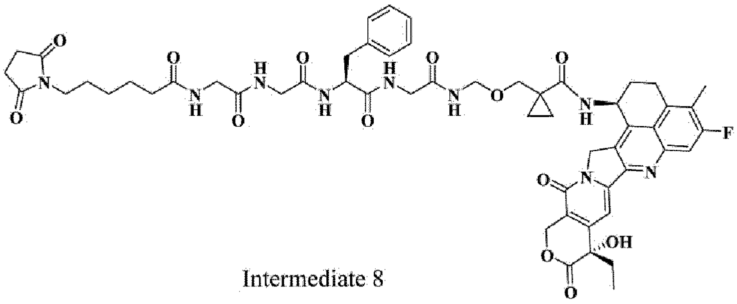

Intermediate 8

and

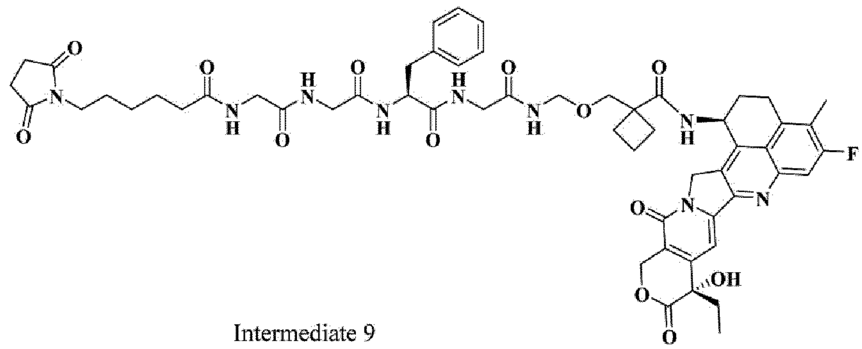

Intermediate 9

.

**39.** A pharmaceutical composition comprising the antibody-drug conjugate according to any one of claims 1 to 35, or the pharmaceutically acceptable salt or solvate thereof, and one or more pharmaceutically acceptable carriers.

**40.** Use of the antibody-drug conjugate or the pharmaceutically acceptable salt or solvate thereof according to any one of claims 1 to 35 and the pharmaceutical composition according to claim 39, in the manufacture of a medicament for treating a disease related to human B7-H4.

**41.** The use according to claim 40, **characterized in that** it is for the manufacture of a medicament for treating a cancer with high B7-H4 expression, wherein the cancer is selected from the group consisting of astroblastoma of human brain, human pharyngeal cancer, adrenal tumor, AIDS-related cancer, alveolar soft-part sarcoma, astrocytoma, bladder cancer, bone cancer, brain and spinal cord cancer, metastatic brain tumor, breast cancer, carotid body tumor, cervical cancer, chondrosarcoma, chordoma, chromophobe cell carcinoma of kidney, clear cell carcinoma, colon cancer, colorectal cancer, connective tissue proliferative small round cell tumor, ependymoma, Ewing's sarcoma, extraosseous mucoid chondrosarcoma, fibrogenesis imperfecta ossium of bone, fibrous dysplasia of bone, gallbladder or cholangiocarcinoma, gastric cancer, gestational trophoblastic disease, germ cell tumor, head and neck cancer, hepatocellular carcinoma, islet cell tumor, Kaposi's sarcoma, kidney cancer, leukemia, liposarcoma/malignant lipomatous tumor, liver cancer, lymphoma, lung cancer, medulloblastoma, melanoma, meningioma, multiple endocrine neoplasia, multiple myeloma, myelodysplastic syndrome, neuroblastoma, neuroendocrine tumor, ovarian cancer, pancreatic cancer, papillary thyroid cancer, parathyroid adenoma, pediatric cancer, peripheral schwannoma, pheocytoma, pituitary tumor, prostate cancer, posterior uveal melanoma, renal metastatic cancer, rhabdoid tumor, rhabdomyosarcoma, sarcoma, skin cancer, soft tissue sarcoma, squamous cell carcinoma, synovial sarcoma, testicular cancer, thymic cancer, thyroid metastatic cancer and uterine cancer.

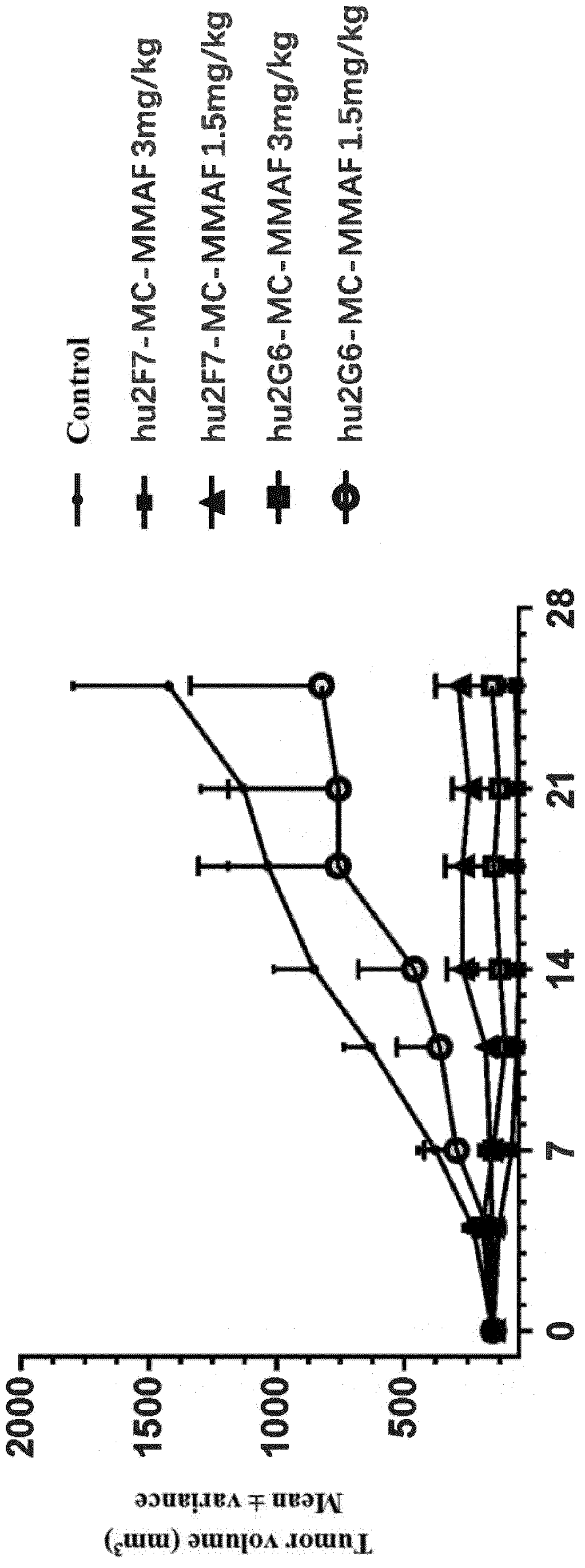
Control
hu2F7-MC-MMAF 3mg/kg
hu2F7-MC-MMAF 1.5mg/kg
hu2G6-MC-MMAF 3mg/kg
hu2G6-MC-MMAF 1.5mg/kg
Tumor volume (mm³)
Mean ± variance
2000
1500
1000
500
0
7
14
21
28

Figure 1

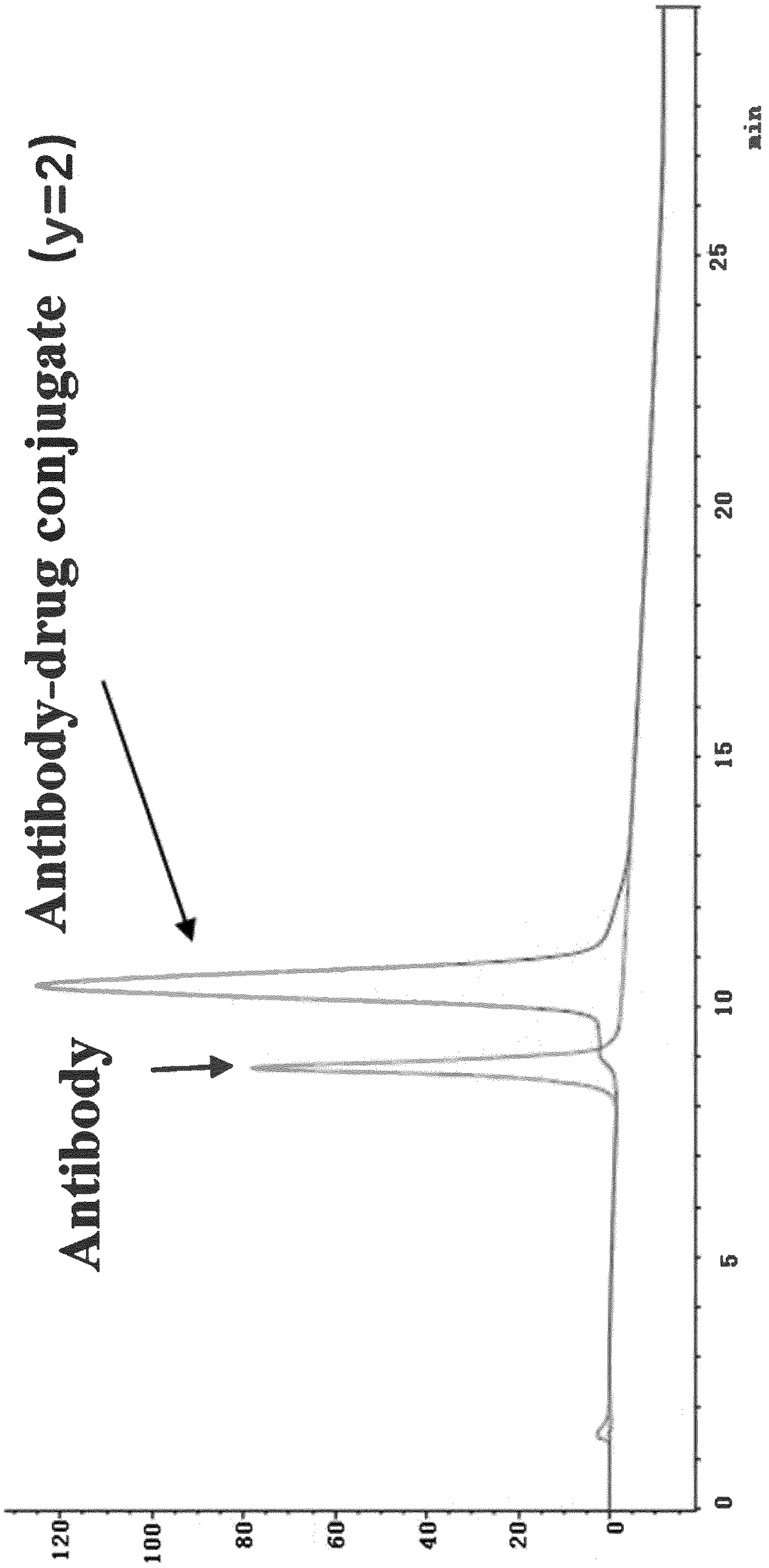
Antibody-drug conjugate (y=2)
Antibody
120
100
80
60
40
20
0
0
5
10
15
20
25
min

**Figure 2**

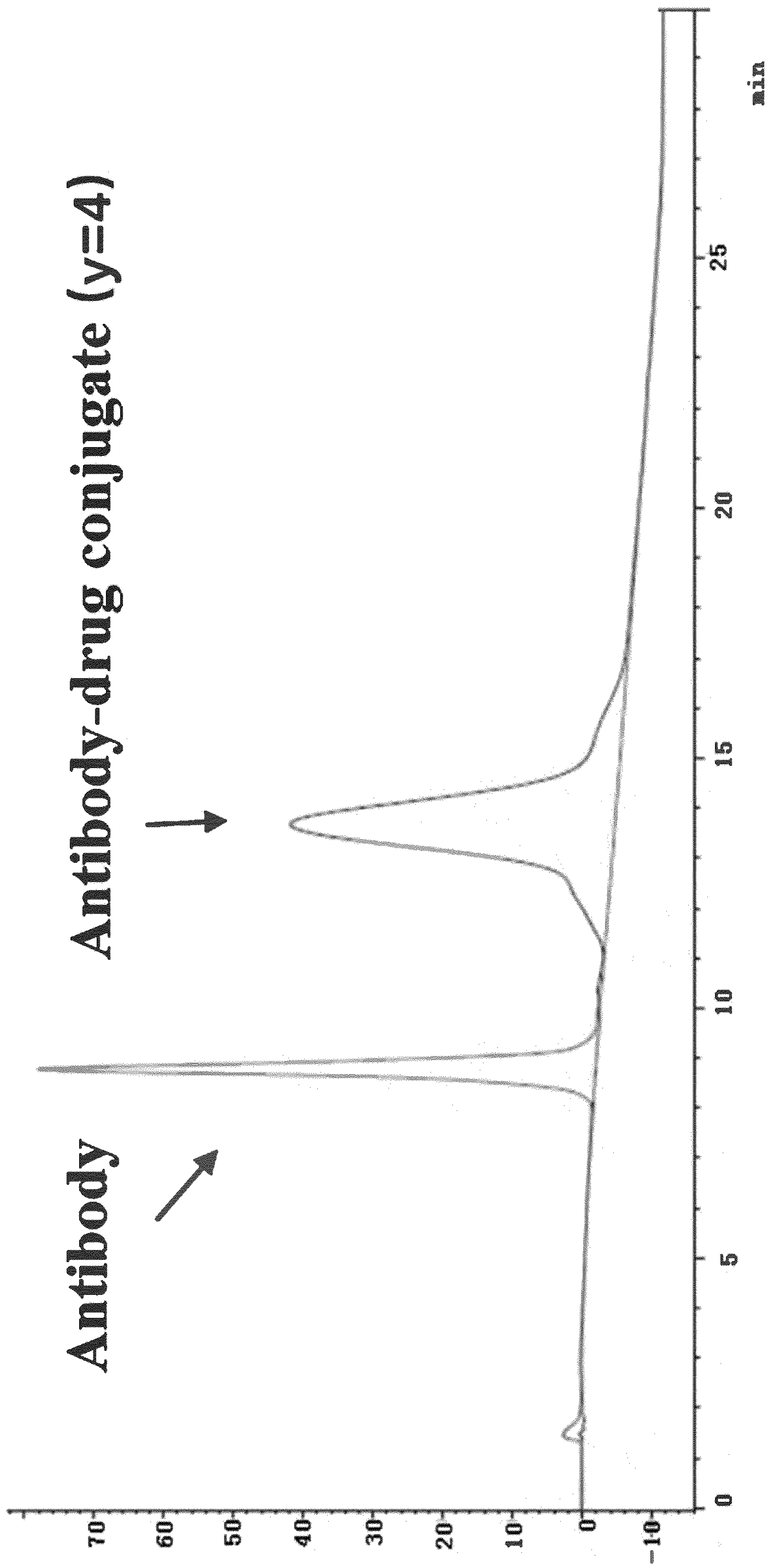


Figure 2 (continue)

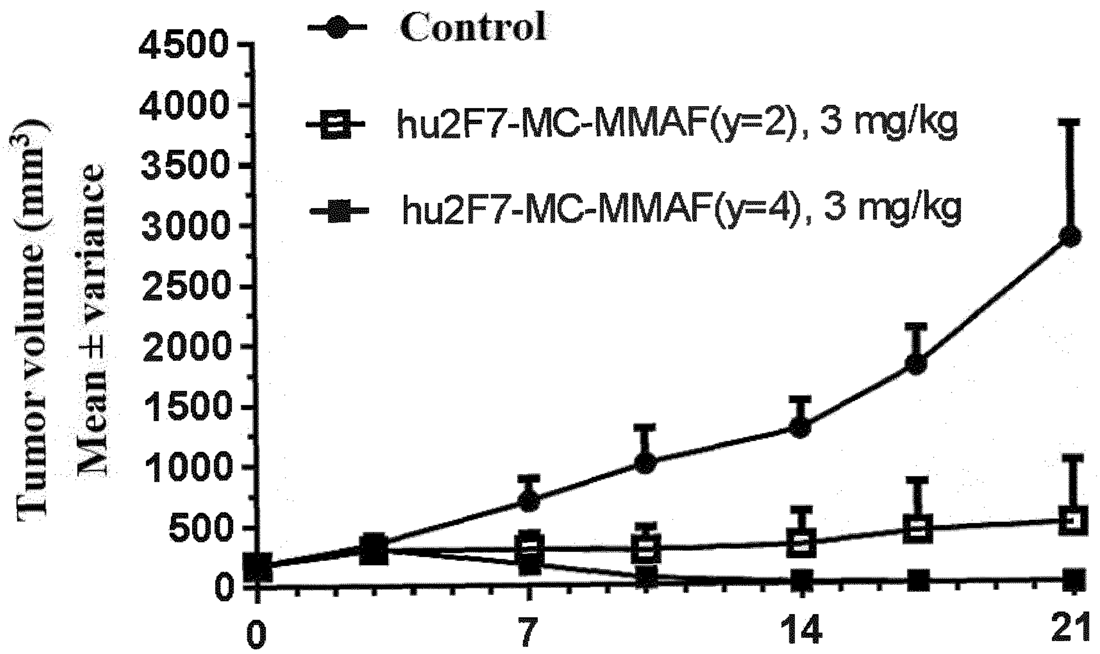
Control
hu2F7-MC-MMAF(y=2), 3 mg/kg
hu2F7-MC-MMAF(y=4), 3 mg/kg
Tumor volume (mm3)
Mean ± variance
4500
4000
3500
3000
2500
2000
1500
1000
500
0
0
7
14
21

Figure 3

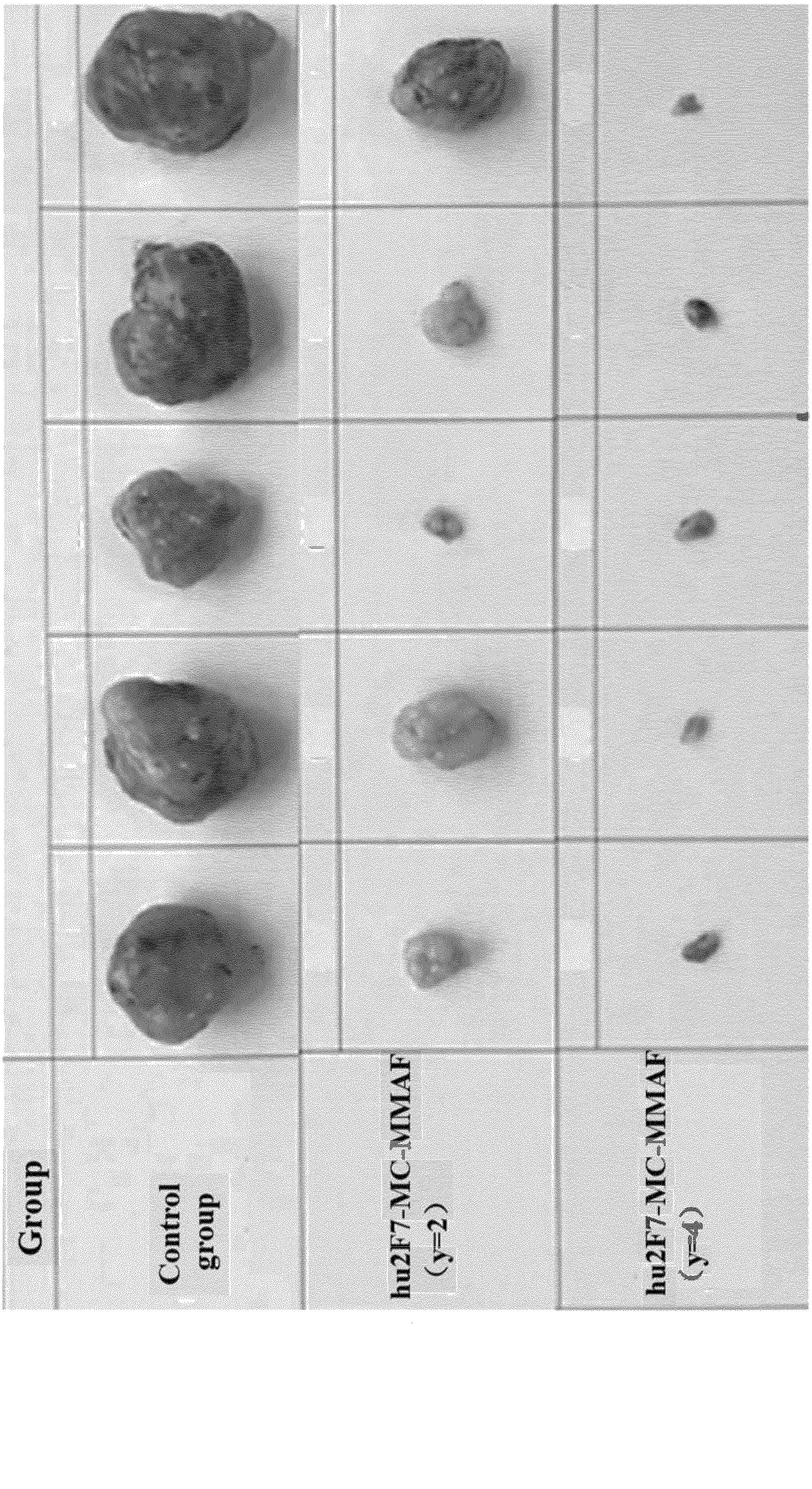


Figure 4

# INTERNATIONAL SEARCH REPORT

International application No.
**PCT/CN2020/094856**

**A. CLASSIFICATION OF SUBJECT MATTER**

A61K 47/68(2017.01)i; A61K 39/00(2006.01)i; C07K 16/18(2006.01)i; A61P 35/00(2006.01)i

According to International Patent Classification (IPC) or to both national classification and IPC

**B. FIELDS SEARCHED**

Minimum documentation searched (classification system followed by classification symbols)

A61K, C07K, A61P

Documentation searched other than minimum documentation to the extent that such documents are included in the fields searched

Electronic data base consulted during the international search (name of data base and, where practicable, search terms used)

CNABS, SIPOABS, DWPI, CNTXT, EPTXT, USTXT, WOTXT, CNKI, 万方, EBI, NCBI, 百度, ISI Web of Knowledge, PUBMED: 翰森, 豪森, 江苏豪森药业集团有限公司, 上海翰森生物医药科技有限公司, 花海清, 刘素霞, 包如迪, B7-H4, B7H4, 抗体-药物偶联物, Antibody-drug conjugate, antibody-drug conjugate, ADC, 药物缀合物, 抗体-药物缀合物

**C. DOCUMENTS CONSIDERED TO BE RELEVANT**

| Category* | Citation of document, with indication, where appropriate, of the relevant passages | Relevant to claim No. |
|---|---|---|
| PY | WO 2019154315 A1 (JIANGSU HANSOH PHARM GROUP CO., LTD. et al.) 15 August 2019 (2019-08-15)<br>Embodiment 2 | 4, 11, 12, 35 |
| X | LEONG, S.R. et al. "An Anti-B7-H4 Antibody−Drug Conjugate for the Treatment of Breast Cancer"<br>*Mol. Pharmaceutics,* Vol. 12, 08 April 2015 (2015-04-08),<br>p. 1717 | 1-3, 5-10, 13-34, 36-41 |
| Y | LEONG, S.R. et al. "An Anti-B7-H4 Antibody−Drug Conjugate for the Treatment of Breast Cancer"<br>*Mol. Pharmaceutics,* Vol. 12, 08 April 2015 (2015-04-08),<br>p. 1717 | 4, 11, 12, 35 |
| X | CN 101951959 A (BRISTOL-MYERS SQUIBB COMPANY) 19 January 2011 (2011-01-19)<br>claims 1-39, description pages 44-64 | 1-3, 5-10, 13-34, 36-41 |
| Y | CN 101951959 A (BRISTOL-MYERS SQUIBB COMPANY) 19 January 2011 (2011-01-19)<br>claims 1-39, description pages 44-64 | 4, 11, 12, 35 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **26 August 2020** | **09 September 2020** |
| Name and mailing address of the ISA/CN<br>**China National Intellectual Property Administration (ISA/CN)**<br>**No. 6, Xitucheng Road, Jimenqiao Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/094856**

**C. DOCUMENTS CONSIDERED TO BE RELEVANT**

| Category* | Citation of document, with indication, where appropriate, of the relevant passages | Relevant to claim No. |
|---|---|---|
| X | CN 105189552 A (GENENTECH INC.) 23 December 2015 (2015-12-23) claims 1-57 | 1-3, 5-10, 13-34, 36-41 |
| Y | CN 105189552 A (GENENTECH INC.) 23 December 2015 (2015-12-23) claims 1-57 | 4, 11, 12, 35 |
| A | US 2018021270 A1 (BERG LLC.) 25 January 2018 (2018-01-25) entire document | 1-41 |
| A | WO 2018065501 A1 (F.HOFFMANN-LA ROCHE AG. et al.) 12 April 2018 (2018-04-12) entire document | 1-41 |
| A | CN 103561772 A (BRISTOL-MYERS SQUIBB COMPANY) 05 February 2014 (2014-02-05) entire document | 1-41 |

Form PCT/ISA/210 (second sheet) (January 2015)

**INTERNATIONAL SEARCH REPORT**
**Information on patent family members**

International application No.
**PCT/CN2020/094856**

| Patent document cited in search report | | | Publication date (day/month/year) | Patent family member(s) | | | Publication date (day/month/year) |
|---|---|---|---|---|---|---|---|
| WO | 2019154315 | A1 | 15 August 2019 | TW | 201934581 | A | 01 September 2019 |
| | | | | CN | 110366560 | A | 22 October 2019 |
| CN | 101951959 | A | 19 January 2011 | TW | 200938224 | A | 16 September 2009 |
| | | | | US | 2011085970 | A1 | 14 April 2011 |
| | | | | MX | 2010005830 | A | 14 September 2010 |
| | | | | IL | 205993 | D0 | 30 November 2010 |
| | | | | JP | 2011505372 | A | 24 February 2011 |
| | | | | AU | 2008334063 | A2 | 20 January 2011 |
| | | | | CA | 2706926 | A1 | 11 June 2009 |
| | | | | AR | 069747 | A1 | 17 February 2010 |
| | | | | AU | 2008334063 | A1 | 11 June 2009 |
| | | | | CL | 2008003526 | A1 | 11 January 2010 |
| | | | | WO | 2009073533 | A3 | 26 November 2009 |
| | | | | BR | PI0818963 | A2 | 05 May 2015 |
| | | | | WO | 2009073533 | A2 | 11 June 2009 |
| | | | | EA | 201000910 | A1 | 29 April 2011 |
| | | | | EP | 2224958 | A2 | 08 September 2010 |
| | | | | KR | 20100093578 | A | 25 August 2010 |
| CN | 105189552 | A | 23 December 2015 | SG | 11201507037X | A | 29 October 2015 |
| | | | | US | 2019241663 | A1 | 08 August 2019 |
| | | | | US | 2016017040 | A1 | 21 January 2016 |
| | | | | WO | 2014159835 | A1 | 02 October 2014 |
| | | | | HK | 1218423 | A1 | 17 February 2017 |
| | | | | PE | 20151672 | A1 | 27 November 2015 |
| | | | | JP | 6436965 | B2 | 12 December 2018 |
| | | | | MX | 2015010777 | A | 25 April 2016 |
| | | | | EP | 3299391 | B1 | 04 December 2019 |
| | | | | AU | 2014244424 | A1 | 27 August 2015 |
| | | | | JP | 6677784 | B2 | 08 April 2020 |
| | | | | EP | 2970474 | B1 | 20 December 2017 |
| | | | | CL | 2015002562 | A1 | 04 March 2016 |
| | | | | JP | 2019058174 | A | 18 April 2019 |
| | | | | CR | 20150482 | A | 26 October 2015 |
| | | | | PH | 12015501954 | A1 | 18 January 2016 |
| | | | | IL | 240517 | A | 29 October 2015 |
| | | | | IL | 240517 | D0 | 29 October 2015 |
| | | | | CA | 2902865 | A1 | 02 October 2014 |
| | | | | EA | 201591750 | A1 | 31 May 2016 |
| | | | | JP | 2016523810 | A | 12 August 2016 |
| | | | | EP | 3299391 | A1 | 28 March 2018 |
| | | | | CN | 105189552 | B | 02 August 2019 |
| | | | | US | 10150813 | B2 | 11 December 2018 |
| | | | | EP | 2970474 | A1 | 20 January 2016 |
| | | | | KR | 20150127199 | A | 16 November 2015 |
| US | 2018021270 | A1 | 25 January 2018 | WO | 2018018018 | A1 | 25 January 2018 |
| WO | 2018065501 | A1 | 12 April 2018 | EP | 3522933 | A1 | 14 August 2019 |
| | | | | CN | 110139674 | A | 16 August 2019 |
| | | | | US | 2019201544 | A1 | 04 July 2019 |
| | | | | JP | 2019537558 | A | 26 December 2019 |

Form PCT/ISA/210 (patent family annex) (January 2015)

**INTERNATIONAL SEARCH REPORT**
**Information on patent family members**

International application No.
**PCT/CN2020/094856**

| Patent document cited in search report | Publication date (day/month/year) | Patent family member(s) | Publication date (day/month/year) |
|---|---|---|---|
| CN 103561772 A | 05 February 2014 | KR 102070464 B1 | 29 January 2020 |
| | | MA 35153 B1 | 02 June 2014 |
| | | US 2014364585 A1 | 11 December 2014 |
| | | IL 229477 A | 29 June 2017 |
| | | CN 103561772 B | 17 August 2016 |
| | | BR 112013029764 A2 | 28 March 2017 |
| | | US 9186416 B2 | 17 November 2015 |
| | | CA 2837327 C | 15 August 2017 |
| | | EA 201391754 A1 | 30 April 2014 |
| | | CL 2013003393 A1 | 13 June 2014 |
| | | TW 201300401 A | 01 January 2013 |
| | | ZA 201406168 B | 25 March 2015 |
| | | SG 195011 A1 | 30 December 2013 |
| | | CO 6831975 A2 | 10 January 2014 |
| | | AU 2012258750 B2 | 03 March 2016 |
| | | JP 6105566 B2 | 29 March 2017 |
| | | PE 20141480 A1 | 23 October 2014 |
| | | UY 34100 A | 30 November 2012 |
| | | JP 6359133 B2 | 18 July 2018 |
| | | JP 2017128571 A | 27 July 2017 |
| | | JP 2014517842 A | 24 July 2014 |
| | | MX 336806 B | 02 February 2016 |
| | | ZA 201309669 B | 28 October 2015 |
| | | KR 20140036252 A | 25 March 2014 |
| | | EA 024844 B1 | 31 October 2016 |
| | | US 2012301490 A1 | 29 November 2012 |
| | | CA 2837327 A1 | 29 November 2012 |
| | | WO 2012162482 A1 | 29 November 2012 |
| | | AU 2012258750 A1 | 16 January 2014 |
| | | EP 2714092 A1 | 09 April 2014 |
| | | AR 086568 A1 | 08 January 2014 |
| | | MX 2013013402 A | 31 January 2014 |
| | | US 8852599 B2 | 07 October 2014 |
| | | IL 229477 D0 | 30 January 2014 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013025779 A
- US 20140322129 A
- WO 2005081711 A


### Non-patent literature cited in the description


- **CHOI IH et al.** *J Immunol,* 01 November 2003, vol. 171 (9), 4650-4
- **SICA GL et al.** *Immunity,* June 2003, vol. 18 (6), 849-61
- **ZHU G et al.** *Blood,* 19 February 2009, vol. 113 (8), 1759-67
- **SUH WK et al.** Blood. *Mol Cell Biol,* September 2006, vol. 26 (17), 6403-11
- **KRYCZEK, I. et al.** *J. Exp. Med.,* 2006, vol. 203 (4), 871-881
- *J. Biol. Chem,* 1968, vol. 243, 3558
- **HOLLIGER ; HUDSON.** *Nat. Biotechnol.,* 2005, vol. 23, 1126-1136

SEQUENCE LISTING

<110> SHANGHAI HANSOH BIOMEDICAL CO., LTD.
JIANGSU HANSOH PHARMACEUTICAL GROUP CO., LTD.

<120> ANTI-B7-H4 ANTIBODY-DRUG CONJUGATE AND MEDICINAL USE THEREOF

<130> 702041CPCT

<140> PCT/CN2020/094856
<141> 2020-06-08

<150> 201910498993.2
<151> 2019-06-06

<150> 202010215322.3
<151> 2020-03-24

<160> 55

<170> SIPOSequenceListing 1.0

<210> 1
<211> 276
<212> PRT
<213> Artificial Sequence

<220>
<223> huB7-H4-His

<400> 1
Met Ala Ser Leu Gly Gln Ile Leu Phe Trp Ser Ile Ile Ser Ile Ile
1 5 10 15
Ile Ile Leu Ala Gly Ala Ile Ala Leu Ile Ile Gly Phe Gly Ile Ser
20 25 30
Gly Arg His Ser Ile Thr Val Thr Thr Val Ala Ser Ala Gly Asn Ile
35 40 45
Gly Glu Asp Gly Ile Leu Ser Cys Thr Phe Glu Pro Asp Ile Lys Leu
50 55 60
Ser Asp Ile Val Ile Gln Trp Leu Lys Glu Gly Val Leu Gly Leu Val
65 70 75 80
His Glu Phe Lys Glu Gly Lys Asp Glu Leu Ser Glu Gln Asp Glu Met
85 90 95
Phe Arg Gly Arg Thr Ala Val Phe Ala Asp Gln Val Ile Val Gly Asn
100 105 110
Ala Ser Leu Arg Leu Lys Asn Val Gln Leu Thr Asp Ala Gly Thr Tyr
115 120 125
Lys Cys Tyr Ile Ile Thr Ser Lys Gly Lys Gly Asn Ala Asn Leu Glu
130 135 140
Tyr Lys Thr Gly Ala Phe Ser Met Pro Glu Val Asn Val Asp Tyr Asn
145 150 155 160
Ala Ser Ser Glu Thr Leu Arg Cys Glu Ala Pro Arg Trp Phe Pro Gln
165 170 175
Pro Thr Val Val Trp Ala Ser Gln Val Asp Gln Gly Ala Asn Phe Ser
180 185 190
Glu Val Ser Asn Thr Ser Phe Glu Leu Asn Ser Glu Asn Val Thr Met
195 200 205
Lys Val Val Ser Val Leu Tyr Asn Val Thr Ile Asn Asn Thr Tyr Ser
210 215 220
Cys Met Ile Glu Asn Asp Ile Ala Lys Ala Thr Gly Asp Ile Lys Val
225 230 235 240
Thr Glu Ser Glu Ile Lys Arg Arg Ser His Leu Gln Leu Leu Asn Ser

```
                245                 250                 255
Lys Ala Asp Tyr Lys Asp Asp Asp Asp Lys Gly Ser His His His His
            260                 265                 270
His His His His
        275

<210>  2
<211>  463
<212>  PRT
<213>  Artificial Sequence

<220>
<223>  huB7-H4-Fc


<400>  2
Phe Gly Ile Ser Gly Arg His Ser Ile Thr Val Thr Thr Val Ala Ser
1               5                   10                  15
Ala Gly Asn Ile Gly Glu Asp Gly Ile Leu Ser Cys Thr Phe Glu Pro
            20                  25                  30
Asp Ile Lys Leu Ser Asp Ile Val Ile Gln Trp Leu Lys Glu Gly Val
        35                  40                  45
Leu Gly Leu Val His Glu Phe Lys Glu Gly Lys Asp Glu Leu Ser Glu
    50                  55                  60
Gln Asp Glu Met Phe Arg Gly Arg Thr Ala Val Phe Ala Asp Gln Val
65                  70                  75                  80
Ile Val Gly Asn Ala Ser Leu Arg Leu Lys Asn Val Gln Leu Thr Asp
                85                  90                  95
Ala Gly Thr Tyr Lys Cys Tyr Ile Ile Thr Ser Lys Gly Lys Gly Asn
            100                 105                 110
Ala Asn Leu Glu Tyr Lys Thr Gly Ala Phe Ser Met Pro Glu Val Asn
        115                 120                 125
Val Asp Tyr Asn Ala Ser Ser Glu Thr Leu Arg Cys Glu Ala Pro Arg
    130                 135                 140
Trp Phe Pro Gln Pro Thr Val Val Trp Ala Ser Gln Val Asp Gln Gly
145                 150                 155                 160
Ala Asn Phe Ser Glu Val Ser Asn Thr Ser Phe Glu Leu Asn Ser Glu
                165                 170                 175
Asn Val Thr Met Lys Val Val Ser Val Leu Tyr Asn Val Thr Ile Asn
            180                 185                 190
Asn Thr Tyr Ser Cys Met Ile Glu Asn Asp Ile Ala Lys Ala Thr Gly
        195                 200                 205
Asp Ile Lys Val Thr Glu Ser Glu Ile Lys Arg Arg Ser His Leu Gln
    210                 215                 220
Leu Leu Asn Ser Lys Ala Gly Ser Gly Gly Gly Gly Asp Lys Thr His
225                 230                 235                 240
Thr Cys Pro Pro Cys Pro Ala Pro Glu Leu Leu Gly Gly Pro Ser Val
                245                 250                 255
Phe Leu Phe Pro Pro Lys Pro Lys Asp Thr Leu Met Ile Ser Arg Thr
            260                 265                 270
Pro Glu Val Thr Cys Val Val Val Asp Val Ser His Glu Asp Pro Glu
        275                 280                 285
Val Lys Phe Asn Trp Tyr Val Asp Gly Val Glu Val His Asn Ala Lys
    290                 295                 300
Thr Lys Pro Arg Glu Glu Gln Tyr Asn Ser Thr Tyr Arg Val Val Ser
305                 310                 315                 320
Val Leu Thr Val Leu His Gln Asp Trp Leu Asn Gly Lys Glu Tyr Lys
                325                 330                 335
Cys Lys Val Ser Asn Lys Ala Leu Pro Ala Pro Ile Glu Lys Thr Ile
            340                 345                 350
Ser Lys Ala Lys Gly Gln Pro Arg Glu Pro Gln Val Tyr Thr Leu Pro
        355                 360                 365
Pro Ser Arg Asp Glu Leu Thr Lys Asn Gln Val Ser Leu Thr Cys Leu
```

```
    370                 375                 380
Val Lys Gly Phe Tyr Pro Ser Asp Ile Ala Val Glu Trp Glu Ser Asn
385                 390                 395                 400
Gly Gln Pro Glu Asn Asn Tyr Lys Thr Thr Pro Pro Val Leu Asp Ser
                405                 410                 415
Asp Gly Ser Phe Phe Leu Tyr Ser Lys Leu Thr Val Asp Lys Ser Arg
            420                 425                 430
Trp Gln Gln Gly Asn Val Phe Ser Cys Ser Val Met His Glu Ala Leu
        435                 440                 445
His Asn His Tyr Thr Gln Lys Ser Leu Ser Leu Ser Pro Gly Lys
    450                 455                 460
```

<210> 3
<211> 10
<212> PRT
<213> Artificial Sequence

<220>
<223> HCDR1

<400> 3

```
Gly Phe Thr Phe Ser Arg Tyr Gly Met Ser
1               5                   10
```

<210> 4
<211> 17
<212> PRT
<213> Artificial Sequence

<220>
<223> HCDR2

<400> 4

```
Gly Ile Asn Gly Gly Gly Ser Tyr Thr Tyr Tyr Leu Asp Thr Val Lys
1               5                   10                  15
Gly
```

<210> 5
<211> 9
<212> PRT
<213> Artificial Sequence

<220>
<223> HCDR3

<400> 5

```
Gln Gly Ser Asn Tyr Tyr Phe Asp Tyr
1               5
```

<210> 6
<211> 11
<212> PRT
<213> Artificial Sequence

<220>
<223> LCDR1

<400> 6

His Ala Ser Gln Gly Ile Ser Ser Asn Ile Gly
1               5                   10

<210>  7
<211>  7
<212>  PRT
<213>  Artificial Sequence

<220>
<223>  LCDR2

<400>  7
His Gly Thr Asn Leu Glu Asp
1               5

<210>  8
<211>  9
<212>  PRT
<213>  Artificial Sequence

<220>
<223>  LCDR3

<400>  8
Val Gln Tyr Ala Gln Phe Pro Tyr Thr
1               5

<210>  9
<211>  10
<212>  PRT
<213>  Artificial Sequence

<220>
<223>  HCDR1

<400>  9
Gly Phe Thr Phe Ser Asn Tyr Tyr Met Ser
1               5                   10

<210>  10
<211>  17
<212>  PRT
<213>  Artificial Sequence

<220>
<223>  HCDR2

<400>  10
Tyr Val Ser Ser Gly Gly Gly Ser Thr Tyr Tyr Ser Asp Ser Val Lys
1               5                   10                  15
Gly

<210>  11
<211>  11
<212>  PRT
<213>  Artificial Sequence

<220>

<223>  HCDR3

<400>  11
```
Glu Ser Tyr Ser Gln Gly Asn Tyr Phe Asp Tyr
1               5                   10
```

<210>  12
<211>  11
<212>  PRT
<213>  Artificial Sequence

<220>
<223>  LCDR1

<400>  12
```
Arg Ala Ser Gln Ser Ile Ser Asp Tyr Leu His
1               5                   10
```

<210>  13
<211>  7
<212>  PRT
<213>  Artificial Sequence

<220>
<223>  LCDR2

<400>  13
```
Phe Ala Ser Gln Ser Ile Ser
1               5
```

<210>  14
<211>  9
<212>  PRT
<213>  Artificial Sequence

<220>
<223>  LCDR3

<400>  14
```
Gln Asn Gly His Ser Phe Ser Leu Thr
1               5
```

<210>  15
<211>  118
<212>  PRT
<213>  Artificial Sequence

<220>
<223>  hu2G6 HCVR

<400>  15
```
Glu Val Gln Leu Leu Glu Ser Gly Gly Gly Leu Val Gln Pro Gly Gly
1               5                   10                  15
Ser Leu Arg Leu Ser Cys Ala Ala Ser Gly Phe Thr Phe Ser Arg Tyr
            20                  25                  30
Gly Met Ser Trp Val Arg Gln Ala Pro Gly Lys Gly Leu Glu Trp Val
        35                  40                  45
Ser Gly Ile Asn Gly Gly Gly Ser Tyr Thr Tyr Tyr Leu Asp Thr Val
```

```
    50                  55                  60
Lys Gly Arg Phe Thr Ile Ser Arg Asp Asn Ala Arg Asn Thr Leu Tyr
65                  70                  75                  80
Leu Gln Met Ser Ser Leu Arg Ala Glu Asp Thr Ala Val Tyr Tyr Cys
                85                  90                  95
Val Ser Gln Gly Ser Asn Tyr Tyr Phe Asp Tyr Trp Gly Gln Gly Thr
            100                 105                 110
Leu Val Thr Val Ser Ser
        115

<210>  16
<211>  107
<212>  PRT
<213>  Artificial Sequence

<220>
<223>  hu2G6 LCVR


<400>  16
Asp Ile Arg Met Thr Gln Ser Pro Ser Ser Leu Ser Ala Ser Val Gly
1               5                   10                  15
Asp Arg Val Thr Ile Thr Cys His Ala Ser Gln Gly Ile Ser Ser Asn
            20                  25                  30
Ile Gly Trp Leu Gln Gln Lys Pro Gly Lys Ala Pro Lys Ala Leu Ile
        35                  40                  45
Tyr His Gly Thr Asn Leu Glu Asp Gly Val Pro Ser Arg Phe Ser Gly
    50                  55                  60
Ser Gly Ser Gly Ala Asp Tyr Thr Leu Thr Ile Ser Ser Leu Gln Pro
65                  70                  75                  80
Glu Asp Phe Ala Thr Tyr Tyr Cys Val Gln Tyr Ala Gln Phe Pro Tyr
                85                  90                  95
Thr Phe Gly Gly Gly Thr Lys Val Glu Ile Lys
            100                 105

<210>  17
<211>  120
<212>  PRT
<213>  Artificial Sequence

<220>
<223>  hu2F7 HCVR


<400>  17
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Asn Tyr
            20                  25                  30
Tyr Met Ser Trp Val Arg Gln Ala Pro Gly Lys Gly Leu Glu Trp Val
        35                  40                  45
Ala Tyr Val Ser Ser Gly Gly Gly Ser Thr Tyr Tyr Ser Asp Ser Val
    50                  55                  60
Lys Gly Arg Phe Thr Ile Ser Arg Asp Asn Ala Lys Asn Thr Leu Tyr
65                  70                  75                  80
Leu Gln Met Ser Ser Leu Arg Ala Glu Asp Thr Ala Val Tyr Tyr Cys
                85                  90                  95
Thr Arg Glu Ser Tyr Ser Gln Gly Asn Tyr Phe Asp Tyr Trp Gly Gln
            100                 105                 110
Gly Thr Thr Val Thr Val Ser Ser
        115                 120

<210>  18
```

<211> 107
<212> PRT
<213> Artificial Sequence

<220>
<223> hu2F7 LCVR

<400> 18
Glu Ile Val Met Thr Gln Ser Pro Ala Thr Leu Ser Leu Ser Pro Gly
1 5 10 15
Glu Arg Ala Thr Leu Ser Cys Arg Ala Ser Gln Ser Ile Ser Asp Tyr
20 25 30
Leu His Trp Tyr Gln Gln Lys Pro Gly Gln Ser Pro Arg Leu Leu Ile
35 40 45
Lys Phe Ala Ser Gln Ser Ile Ser Gly Ile Pro Ala Arg Phe Ser Gly
50 55 60
Ser Gly Ser Gly Thr Asp Phe Thr Leu Thr Ile Ser Ser Leu Glu Pro
65 70 75 80
Glu Asp Phe Ala Val Tyr Tyr Cys Gln Asn Gly His Ser Phe Ser Leu
85 90 95
Thr Phe Gly Gln Gly Thr Lys Leu Glu Ile Lys
100 105

<210> 19
<211> 448
<212> PRT
<213> Artificial Sequence

<220>
<223> hu2G6 HC

<400> 19
Glu Val Gln Leu Leu Glu Ser Gly Gly Gly Leu Val Gln Pro Gly Gly
1 5 10 15
Ser Leu Arg Leu Ser Cys Ala Ala Ser Gly Phe Thr Phe Ser Arg Tyr
20 25 30
Gly Met Ser Trp Val Arg Gln Ala Pro Gly Lys Gly Leu Glu Trp Val
35 40 45
Ser Gly Ile Asn Gly Gly Gly Ser Tyr Thr Tyr Tyr Leu Asp Thr Val
50 55 60
Lys Gly Arg Phe Thr Ile Ser Arg Asp Asn Ala Arg Asn Thr Leu Tyr
65 70 75 80
Leu Gln Met Ser Ser Leu Arg Ala Glu Asp Thr Ala Val Tyr Tyr Cys
85 90 95
Val Ser Gln Gly Ser Asn Tyr Tyr Phe Asp Tyr Trp Gly Gln Gly Thr
100 105 110
Leu Val Thr Val Ser Ser Ala Ser Thr Lys Gly Pro Ser Val Phe Pro
115 120 125
Leu Ala Pro Ser Ser Lys Ser Thr Ser Gly Gly Thr Ala Ala Leu Gly
130 135 140
Cys Leu Val Lys Asp Tyr Phe Pro Glu Pro Val Thr Val Ser Trp Asn
145 150 155 160
Ser Gly Ala Leu Thr Ser Gly Val His Thr Phe Pro Ala Val Leu Gln
165 170 175
Ser Ser Gly Leu Tyr Ser Leu Ser Ser Val Val Thr Val Pro Ser Ser
180 185 190
Ser Leu Gly Thr Gln Thr Tyr Ile Cys Asn Val Asn His Lys Pro Ser
195 200 205
Asn Thr Lys Val Asp Lys Lys Val Glu Pro Lys Ser Cys Asp Lys Thr
210 215 220
His Thr Cys Pro Pro Cys Pro Ala Pro Glu Leu Leu Gly Gly Pro Ser

```
225                 230                 235                 240
Val Phe Leu Phe Pro Pro Lys Pro Lys Asp Thr Leu Met Ile Ser Arg
                245                 250                 255
Thr Pro Glu Val Thr Cys Val Val Val Asp Val Ser His Glu Asp Pro
            260                 265                 270
Glu Val Lys Phe Asn Trp Tyr Val Asp Gly Val Glu Val His Asn Ala
        275                 280                 285
Lys Thr Lys Pro Arg Glu Glu Gln Tyr Asn Ser Thr Tyr Arg Val Val
    290                 295                 300
Ser Val Leu Thr Val Leu His Gln Asp Trp Leu Asn Gly Lys Glu Tyr
305                 310                 315                 320
Lys Cys Lys Val Ser Asn Lys Ala Leu Pro Ala Pro Ile Glu Lys Thr
                325                 330                 335
Ile Ser Lys Ala Lys Gly Gln Pro Arg Glu Pro Gln Val Tyr Thr Leu
            340                 345                 350
Pro Pro Ser Arg Glu Glu Met Thr Lys Asn Gln Val Ser Leu Thr Cys
        355                 360                 365
Leu Val Lys Gly Phe Tyr Pro Ser Asp Ile Ala Val Glu Trp Glu Ser
    370                 375                 380
Asn Gly Gln Pro Glu Asn Asn Tyr Lys Thr Thr Pro Pro Val Leu Asp
385                 390                 395                 400
Ser Asp Gly Ser Phe Phe Leu Tyr Ser Lys Leu Thr Val Asp Lys Ser
                405                 410                 415
Arg Trp Gln Gln Gly Asn Val Phe Ser Cys Ser Val Met His Glu Ala
            420                 425                 430
Leu His Asn His Tyr Thr Gln Lys Ser Leu Ser Leu Ser Pro Gly Lys
        435                 440                 445
```

```
<210>  20
<211>  214
<212>  PRT
<213>  Artificial Sequence

<220>
<223>  hu2G6 LC


<400>  20
Asp Ile Arg Met Thr Gln Ser Pro Ser Ser Leu Ser Ala Ser Val Gly
1               5                   10                  15
Asp Arg Val Thr Ile Thr Cys His Ala Ser Gln Gly Ile Ser Ser Asn
            20                  25                  30
Ile Gly Trp Leu Gln Gln Lys Pro Gly Lys Ala Pro Lys Ala Leu Ile
        35                  40                  45
Tyr His Gly Thr Asn Leu Glu Asp Gly Val Pro Ser Arg Phe Ser Gly
    50                  55                  60
Ser Gly Ser Gly Ala Asp Tyr Thr Leu Thr Ile Ser Ser Leu Gln Pro
65                  70                  75                  80
Glu Asp Phe Ala Thr Tyr Tyr Cys Val Gln Tyr Ala Gln Phe Pro Tyr
                85                  90                  95
Thr Phe Gly Gly Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
```

```
        195                 200                 205
Phe Asn Arg Gly Glu Cys
    210

<210>  21
<211>  450
<212>  PRT
<213>  Artificial Sequence

<220>
<223>  hu2F7 HC


<400>  21
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Asn Tyr
            20                  25                  30
Tyr Met Ser Trp Val Arg Gln Ala Pro Gly Lys Gly Leu Glu Trp Val
        35                  40                  45
Ala Tyr Val Ser Ser Gly Gly Gly Ser Thr Tyr Tyr Ser Asp Ser Val
    50                  55                  60
Lys Gly Arg Phe Thr Ile Ser Arg Asp Asn Ala Lys Asn Thr Leu Tyr
65                  70                  75                  80
Leu Gln Met Ser Ser Leu Arg Ala Glu Asp Thr Ala Val Tyr Tyr Cys
                85                  90                  95
Thr Arg Glu Ser Tyr Ser Gln Gly Asn Tyr Phe Asp Tyr Trp Gly Gln
            100                 105                 110
Gly Thr Thr Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Glu Glu Met Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
```

```
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys
    450
```

<210>  22
<211>  214
<212>  PRT
<213>  Artificial Sequence

<220>
<223>  hu2F7 LC

<400>  22

```
Glu Ile Val Met Thr Gln Ser Pro Ala Thr Leu Ser Leu Ser Pro Gly
1               5                   10                  15
Glu Arg Ala Thr Leu Ser Cys Arg Ala Ser Gln Ser Ile Ser Asp Tyr
            20                  25                  30
Leu His Trp Tyr Gln Gln Lys Pro Gly Gln Ser Pro Arg Leu Leu Ile
        35                  40                  45
Lys Phe Ala Ser Gln Ser Ile Ser Gly Ile Pro Ala Arg Phe Ser Gly
    50                  55                  60
Ser Gly Ser Gly Thr Asp Phe Thr Leu Thr Ile Ser Ser Leu Glu Pro
65                  70                  75                  80
Glu Asp Phe Ala Val Tyr Tyr Cys Gln Asn Gly His Ser Phe Ser Leu
                85                  90                  95
Thr Phe Gly Gln Gly Thr Lys Leu Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210
```

<210>  23
<211>  10
<212>  PRT
<213>  Artificial Sequence

<220>
<223>  HCDR1

<400>  23

```
Gly Tyr Thr Phe Thr Asn Ser Trp Met Asn
1               5                   10
```

<210>  24

<211> 17
<212> PRT
<213> Artificial Sequence

<220>
<223> HCDR2

<400> 24
Gly Ile Tyr Pro Asn Ser Gly Asn Ile Glu Tyr Asn Glu Lys Phe Lys
1 5 10 15
Gly

<210> 25
<211> 6
<212> PRT
<213> Artificial Sequence

<220>
<223> HCDR3

<400> 25
Asp Ser Arg Phe Ser Tyr
1 5

<210> 26
<211> 11
<212> PRT
<213> Artificial Sequence

<220>
<223> LCDR1

<400> 26
Lys Ala Ser Gln Asp Val Arg Thr Ala Val Ala
1 5 10

<210> 27
<211> 7
<212> PRT
<213> Artificial Sequence

<220>
<223> LCDR2

<400> 27
Ser Thr Ser Tyr Arg Tyr Thr
1 5

<210> 28
<211> 9
<212> PRT
<213> Artificial Sequence

<220>
<223> LCDR3

<400> 28

Gln Gln His Tyr Ser Thr Pro Leu Thr
1               5

<210>  29
<211>  7
<212>  PRT
<213>  Artificial Sequence

<220>
<223>  HCDR1

<400>  29
Gly Asp Thr Phe Thr Thr Tyr
1               5

<210>  30
<211>  6
<212>  PRT
<213>  Artificial Sequence

<220>
<223>  HCDR2

<400>  30
Tyr Leu Asn Ser Gly Ser
1               5

<210>  31
<211>  6
<212>  PRT
<213>  Artificial Sequence

<220>
<223>  HCDR3

<400>  31
Asp Ser Arg Phe Ser Tyr
1               5

<210>  32
<211>  11
<212>  PRT
<213>  Artificial Sequence

<220>
<223>  LCDR1

<400>  32
Lys Ala Ser Gln Asp Val Ser Thr Ala Val Ala
1               5                   10

<210>  33
<211>  7
<212>  PRT
<213>  Artificial Sequence

<220>
<223>  LCDR2

<400> 33
Ser Ala Ser Tyr Arg Tyr Thr
1 5

<210> 34
<211> 9
<212> PRT
<213> Artificial Sequence

<220>
<223> LCDR3

<400> 34
Gln Gln His Tyr Asn Thr Pro Leu Thr
1 5

<210> 35
<211> 115
<212> PRT
<213> Artificial Sequence

<220>
<223> hu2F8 HCVR

<400> 35
Glu Val Gln Leu Val Gln Ser Gly Ala Glu Val Lys Lys Pro Gly Ala
1 5 10 15
Ser Val Lys Val Ser Cys Lys Ala Ser Gly Tyr Thr Phe Thr Ser Ser
20 25 30
Trp Met Asn Trp Val Arg Gln Ala Pro Gly Gln Arg Leu Glu Trp Met
35 40 45
Gly Gly Ile Tyr Pro Asn Arg Gly Asn Ile Glu Tyr Asn Glu Lys Phe
50 55 60
Lys Gly Arg Val Thr Leu Thr Val Asp Thr Ser Ala Ser Thr Ala Tyr
65 70 75 80
Met Glu Leu Ser Ser Leu Arg Ser Glu Asp Thr Ala Val Tyr Tyr Cys
85 90 95
Ala Arg Asp Ser Arg Phe Ser Tyr Trp Gly Gln Gly Thr Leu Val Thr
100 105 110
Val Ser Ser
115

<210> 36
<211> 107
<212> PRT
<213> Artificial Sequence

<220>
<223> hu2F8 LCVR

<400> 36
Asp Ile Gln Met Thr Gln Ser Pro Ser Ser Leu Ser Ala Ser Val Gly
1 5 10 15
Asp Arg Val Thr Ile Thr Cys Lys Ala Ser Gln Asp Val Arg Thr Ala
20 25 30
Val Ala Trp Tyr Gln Gln Lys Pro Gly Lys Ala Pro Lys Leu Leu Ile
35 40 45
Ser Ser Thr Ser Tyr Arg Tyr Thr Gly Val Pro Ser Arg Phe Ser Gly
50 55 60

Ser Gly Ser Gly Thr Asp Phe Thr Phe Thr Ile Ser Ser Leu Gln Pro
65 70 75 80
Glu Asp Ile Ala Thr Tyr Tyr Cys Gln Gln His Tyr Ser Thr Pro Leu
85 90 95
Thr Phe Gly Gly Gly Thr Lys Val Glu Ile Lys
100 105

<210> 37
<211> 115
<212> PRT
<213> Artificial Sequence

<220>
<223> hu1C9 HCVR

<400> 37
Glu Val Gln Leu Val Gln Ser Gly Ala Glu Val Lys Lys Pro Gly Ala
1 5 10 15
Ser Val Lys Val Ser Cys Lys Ala Ser Gly Asp Thr Phe Thr Thr Tyr
20 25 30
Trp Met Asn Trp Val Arg Gln Ala Pro Gly Gln Arg Leu Glu Trp Met
35 40 45
Gly Gly Ile Tyr Leu Asn Arg Gly Ser Ser Glu Tyr Asn Glu Lys Phe
50 55 60
Lys Gly Arg Val Thr Leu Thr Val Asp Thr Ser Ala Ser Thr Ala Tyr
65 70 75 80
Met Glu Leu Ser Ser Leu Arg Ser Glu Asp Thr Ala Val Tyr Tyr Cys
85 90 95
Ala Arg Asp Ser Arg Phe Ser Tyr Trp Gly Gln Gly Thr Leu Val Thr
100 105 110
Val Ser Ser
115

<210> 38
<211> 107
<212> PRT
<213> Artificial Sequence

<220>
<223> hu1C9 LCVR

<400> 38
Asp Ile Gln Met Thr Gln Ser Pro Ser Ser Leu Ser Ala Ser Val Gly
1 5 10 15
Asp Arg Val Thr Ile Thr Cys Lys Ala Ser Gln Asp Val Ser Thr Ala
20 25 30
Val Ala Trp Tyr Gln Gln Lys Pro Gly Lys Ala Pro Lys Leu Leu Ile
35 40 45
Ser Ser Ala Ser Tyr Arg Tyr Thr Gly Val Pro Ser Arg Phe Ser Gly
50 55 60
Ser Gly Ser Gly Thr Asp Phe Thr Phe Thr Ile Ser Ser Leu Gln Pro
65 70 75 80
Glu Asp Ile Ala Thr Tyr Tyr Cys Gln Gln His Tyr Asn Thr Pro Leu
85 90 95
Thr Phe Gly Gly Gly Thr Lys Val Glu Ile Lys
100 105

<210> 39
<211> 445
<212> PRT
<213> Artificial Sequence

<220>
<223> hu2F8 HC

<400> 39
Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ser Ser
            20                  25                  30
Trp Met Asn Trp Val Arg Gln Ala Pro Gly Gln Arg Leu Glu Trp Met
        35                  40                  45
Gly Gly Ile Tyr Pro Asn Arg Gly Asn Ile Glu Tyr Asn Glu Lys Phe
    50                  55                  60
Lys Gly Arg Val Thr Leu Thr Val Asp Thr Ser Ala Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Asp Ser Arg Phe Ser Tyr Trp Gly Gln Gly Thr Leu Val Thr
            100                 105                 110
Val Ser Ser Ala Ser Thr Lys Gly Pro Ser Val Phe Pro Leu Ala Pro
        115                 120                 125
Ser Ser Lys Ser Thr Ser Gly Gly Thr Ala Ala Leu Gly Cys Leu Val
    130                 135                 140
Lys Asp Tyr Phe Pro Glu Pro Val Thr Val Ser Trp Asn Ser Gly Ala
145                 150                 155                 160
Leu Thr Ser Gly Val His Thr Phe Pro Ala Val Leu Gln Ser Ser Gly
                165                 170                 175
Leu Tyr Ser Leu Ser Ser Val Val Thr Val Pro Ser Ser Ser Leu Gly
            180                 185                 190
Thr Gln Thr Tyr Ile Cys Asn Val Asn His Lys Pro Ser Asn Thr Lys
        195                 200                 205
Val Asp Lys Lys Val Glu Pro Lys Ser Cys Asp Lys Thr His Thr Cys
    210                 215                 220
Pro Pro Cys Pro Ala Pro Glu Leu Leu Gly Gly Pro Ser Val Phe Leu
225                 230                 235                 240
Phe Pro Pro Lys Pro Lys Asp Thr Leu Met Ile Ser Arg Thr Pro Glu
                245                 250                 255
Val Thr Cys Val Val Val Asp Val Ser His Glu Asp Pro Glu Val Lys
            260                 265                 270
Phe Asn Trp Tyr Val Asp Gly Val Glu Val His Asn Ala Lys Thr Lys
        275                 280                 285
Pro Arg Glu Glu Gln Tyr Asn Ser Thr Tyr Arg Val Val Ser Val Leu
    290                 295                 300
Thr Val Leu His Gln Asp Trp Leu Asn Gly Lys Glu Tyr Lys Cys Lys
305                 310                 315                 320
Val Ser Asn Lys Ala Leu Pro Ala Pro Ile Glu Lys Thr Ile Ser Lys
                325                 330                 335
Ala Lys Gly Gln Pro Arg Glu Pro Gln Val Tyr Thr Leu Pro Pro Ser
            340                 345                 350
Arg Glu Glu Met Thr Lys Asn Gln Val Ser Leu Thr Cys Leu Val Lys
        355                 360                 365
Gly Phe Tyr Pro Ser Asp Ile Ala Val Glu Trp Glu Ser Asn Gly Gln
    370                 375                 380
Pro Glu Asn Asn Tyr Lys Thr Thr Pro Pro Val Leu Asp Ser Asp Gly
385                 390                 395                 400
Ser Phe Phe Leu Tyr Ser Lys Leu Thr Val Asp Lys Ser Arg Trp Gln
                405                 410                 415
Gln Gly Asn Val Phe Ser Cys Ser Val Met His Glu Ala Leu His Asn
            420                 425                 430
His Tyr Thr Gln Lys Ser Leu Ser Leu Ser Pro Gly Lys
        435                 440                 445

<210> 40
<211> 214
<212> PRT
<213> Artificial Sequence

<220>
<223> hu2F8 LC

<400> 40

```
Asp Ile Gln Met Thr Gln Ser Pro Ser Ser Leu Ser Ala Ser Val Gly
1               5                   10                  15
Asp Arg Val Thr Ile Thr Cys Lys Ala Ser Gln Asp Val Arg Thr Ala
            20                  25                  30
Val Ala Trp Tyr Gln Gln Lys Pro Gly Lys Ala Pro Lys Leu Leu Ile
        35                  40                  45
Ser Ser Thr Ser Tyr Arg Tyr Thr Gly Val Pro Ser Arg Phe Ser Gly
    50                  55                  60
Ser Gly Ser Gly Thr Asp Phe Thr Phe Thr Ile Ser Ser Leu Gln Pro
65                  70                  75                  80
Glu Asp Ile Ala Thr Tyr Tyr Cys Gln Gln His Tyr Ser Thr Pro Leu
                85                  90                  95
Thr Phe Gly Gly Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210
```

<210> 41
<211> 445
<212> PRT
<213> Artificial Sequence

<220>
<223> hu1C9HC

<400> 41

```
Glu Val Gln Leu Val Gln Ser Gly Ala Glu Val Lys Lys Pro Gly Ala
1               5                   10                  15
Ser Val Lys Val Ser Cys Lys Ala Ser Gly Asp Thr Phe Thr Thr Tyr
            20                  25                  30
Trp Met Asn Trp Val Arg Gln Ala Pro Gly Gln Arg Leu Glu Trp Met
        35                  40                  45
Gly Gly Ile Tyr Leu Asn Arg Gly Ser Ser Glu Tyr Asn Glu Lys Phe
    50                  55                  60
Lys Gly Arg Val Thr Leu Thr Val Asp Thr Ser Ala Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Asp Ser Arg Phe Ser Tyr Trp Gly Gln Gly Thr Leu Val Thr
            100                 105                 110
```

Val Ser Ser Ala Ser Thr Lys Gly Pro Ser Val Phe Pro Leu Ala Pro
115 120 125
Ser Ser Lys Ser Thr Ser Gly Gly Thr Ala Ala Leu Gly Cys Leu Val
130 135 140
Lys Asp Tyr Phe Pro Glu Pro Val Thr Val Ser Trp Asn Ser Gly Ala
145 150 155 160
Leu Thr Ser Gly Val His Thr Phe Pro Ala Val Leu Gln Ser Ser Gly
165 170 175
Leu Tyr Ser Leu Ser Ser Val Val Thr Val Pro Ser Ser Ser Leu Gly
180 185 190
Thr Gln Thr Tyr Ile Cys Asn Val Asn His Lys Pro Ser Asn Thr Lys
195 200 205
Val Asp Lys Lys Val Glu Pro Lys Ser Cys Asp Lys Thr His Thr Cys
210 215 220
Pro Pro Cys Pro Ala Pro Glu Leu Leu Gly Gly Pro Ser Val Phe Leu
225 230 235 240
Phe Pro Pro Lys Pro Lys Asp Thr Leu Met Ile Ser Arg Thr Pro Glu
245 250 255
Val Thr Cys Val Val Val Asp Val Ser His Glu Asp Pro Glu Val Lys
260 265 270
Phe Asn Trp Tyr Val Asp Gly Val Glu Val His Asn Ala Lys Thr Lys
275 280 285
Pro Arg Glu Glu Gln Tyr Asn Ser Thr Tyr Arg Val Val Ser Val Leu
290 295 300
Thr Val Leu His Gln Asp Trp Leu Asn Gly Lys Glu Tyr Lys Cys Lys
305 310 315 320
Val Ser Asn Lys Ala Leu Pro Ala Pro Ile Glu Lys Thr Ile Ser Lys
325 330 335
Ala Lys Gly Gln Pro Arg Glu Pro Gln Val Tyr Thr Leu Pro Pro Ser
340 345 350
Arg Glu Glu Met Thr Lys Asn Gln Val Ser Leu Thr Cys Leu Val Lys
355 360 365
Gly Phe Tyr Pro Ser Asp Ile Ala Val Glu Trp Glu Ser Asn Gly Gln
370 375 380
Pro Glu Asn Asn Tyr Lys Thr Thr Pro Pro Val Leu Asp Ser Asp Gly
385 390 395 400
Ser Phe Phe Leu Tyr Ser Lys Leu Thr Val Asp Lys Ser Arg Trp Gln
405 410 415
Gln Gly Asn Val Phe Ser Cys Ser Val Met His Glu Ala Leu His Asn
420 425 430
His Tyr Thr Gln Lys Ser Leu Ser Leu Ser Pro Gly Lys
435 440 445

<210> 42
<211> 214
<212> PRT
<213> Artificial Sequence

<220>
<223> hu1C9 LC

<400> 42
Asp Ile Gln Met Thr Gln Ser Pro Ser Ser Leu Ser Ala Ser Val Gly
1 5 10 15
Asp Arg Val Thr Ile Thr Cys Lys Ala Ser Gln Asp Val Ser Thr Ala
20 25 30
Val Ala Trp Tyr Gln Gln Lys Pro Gly Lys Ala Pro Lys Leu Leu Ile
35 40 45
Ser Ser Ala Ser Tyr Arg Tyr Thr Gly Val Pro Ser Arg Phe Ser Gly
50 55 60
Ser Gly Ser Gly Thr Asp Phe Thr Phe Thr Ile Ser Ser Leu Gln Pro
65 70 75 80

```
Glu Asp Ile Ala Thr Tyr Tyr Cys Gln Gln His Tyr Asn Thr Pro Leu
                85                  90                  95
Thr Phe Gly Gly Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210

<210>  43
<211>  10
<212>  PRT
<213>  Artificial Sequence

<220>
<223>  Optimized HCDR1


<400>  43
Gly Tyr Thr Phe Thr Ser Ser Trp Met Asn
1               5                   10

<210>  44
<211>  17
<212>  PRT
<213>  Artificial Sequence

<220>
<223>  Optimized HCDR2


<400>  44
Gly Ile Tyr Pro Asn Arg Gly Asn Ile Glu Tyr Asn Glu Lys Phe Lys
1               5                   10                  15
Gly


<210>  45
<211>  6
<212>  PRT
<213>  Artificial Sequence

<220>
<223>  Optimized HCDR2


<400>  45
Tyr Leu Asn Arg Gly Ser
1               5

<210>  46
<211>  118
<212>  PRT
```

<213> Artificial Sequence

<220>
<223> 2G6 HCVR

<400> 46
```
Glu Val Gln Leu Val Glu Ser Gly Gly Gly Leu Val Lys Pro Gly Gly
1               5                   10                  15
Ser Leu Lys Leu Ser Cys Ala Ala Ser Gly Phe Thr Phe Ser Arg Tyr
            20                  25                  30
Gly Met Ser Trp Val Arg Gln Thr Pro Glu Lys Arg Leu Glu Trp Val
        35                  40                  45
Ala Gly Ile Asn Gly Gly Gly Ser Tyr Thr Tyr Tyr Leu Asp Thr Val
    50                  55                  60
Lys Gly Arg Phe Thr Ile Ser Arg Asp Asn Ser Arg Asn Thr Leu Tyr
65                  70                  75                  80
Leu Gln Met Ser Ser Leu Arg Ser Glu Asp Thr Ala Met Tyr Tyr Cys
                85                  90                  95
Val Ser Gln Gly Ser Asn Tyr Tyr Phe Asp Tyr Trp Gly Gln Gly Thr
            100                 105                 110
Thr Leu Thr Val Ser Ser
        115
```

<210> 47
<211> 107
<212> PRT
<213> Artificial Sequence

<220>
<223> 2G6 LCVR

<400> 47
```
Asp Ile Arg Met Thr Gln Ser Pro Ser Ser Met Ser Val Ser Leu Gly
1               5                   10                  15
Asp Thr Val Ser Ile Thr Cys His Ala Ser Gln Gly Ile Ser Ser Asn
            20                  25                  30
Ile Gly Trp Leu Gln Gln Lys Pro Gly Lys Ser Phe Lys Ala Leu Ile
        35                  40                  45
Tyr His Gly Thr Asn Leu Glu Asp Gly Val Pro Ser Arg Phe Ser Gly
    50                  55                  60
Ser Gly Ser Gly Ala Asp Tyr Ser Leu Thr Ile Ser Ser Leu Glu Ser
65                  70                  75                  80
Glu Asp Phe Ala Asp Tyr Tyr Cys Val Gln Tyr Ala Gln Phe Pro Tyr
                85                  90                  95
Thr Phe Gly Gly Gly Thr Lys Leu Glu Ile Lys
            100                 105
```

<210> 48
<211> 120
<212> PRT
<213> Artificial Sequence

<220>
<223> 2F7 HCVR

<400> 48
```
Glu Val Gln Leu Val Glu Ser Gly Gly Gly Leu Val Gln Pro Gly Gly
1               5                   10                  15
Ser Leu Lys Leu Ser Cys Ala Ala Ser Gly Phe Thr Phe Ser Asn Tyr
            20                  25                  30
```

Tyr Met Ser Trp Val Arg Gln Thr Pro Glu Lys Arg Leu Glu Trp Val
35 40 45
Ala Tyr Val Ser Ser Gly Gly Gly Ser Thr Tyr Tyr Ser Asp Ser Val
50 55 60
Lys Gly Arg Phe Thr Ile Ser Arg Asp Asn Ala Lys Asn Thr Leu Tyr
65 70 75 80
Leu Gln Met Ser Ser Leu Lys Pro Glu Asp Thr Ala Met Tyr Tyr Cys
85 90 95
Thr Arg Glu Ser Tyr Ser Gln Gly Asn Tyr Phe Asp Tyr Trp Gly Gln
100 105 110
Gly Thr Thr Leu Thr Val Ser Ser
115 120

<210> 49
<211> 107
<212> PRT
<213> Artificial Sequence

<220>
<223> 2F7 LCVR

<400> 49
Asp Ile Val Met Thr Gln Ser Pro Ala Thr Leu Ser Val Thr Pro Gly
1 5 10 15
Asp Arg Val Ser Leu Ser Cys Arg Ala Ser Gln Ser Ile Ser Asp Tyr
20 25 30
Leu His Trp Tyr Gln Gln Lys Ser His Glu Ser Pro Arg Leu Leu Ile
35 40 45
Lys Phe Ala Ser Gln Ser Ile Ser Gly Ile Pro Ser Arg Phe Ser Gly
50 55 60
Ser Gly Ser Gly Ser Asp Phe Thr Leu Ser Ile Asn Ser Val Glu Pro
65 70 75 80
Glu Asp Val Gly Val Tyr Tyr Cys Gln Asn Gly His Ser Phe Ser Leu
85 90 95
Thr Phe Gly Ala Gly Thr Lys Leu Glu Leu Lys
100 105

<210> 50
<211> 115
<212> PRT
<213> Artificial Sequence

<220>
<223> 2F8 HCVR

<400> 50
Gln Val Gln Leu Gln Gln Pro Gly Ser Val Leu Val Arg Pro Gly Ala
1 5 10 15
Ser Val Lys Leu Ser Cys Lys Ala Ser Gly Tyr Thr Phe Thr Asn Ser
20 25 30
Trp Met Asn Trp Ala Lys Leu Arg Pro Gly Gln Gly Leu Glu Trp Ile
35 40 45
Gly Gly Ile Tyr Pro Asn Ser Gly Asn Ile Glu Tyr Asn Glu Lys Phe
50 55 60
Lys Gly Lys Ala Thr Leu Thr Val Asp Thr Ser Ser Ser Thr Ala Tyr
65 70 75 80
Met Asp Leu Thr Ser Leu Thr Ser Glu Asp Ser Ala Val Tyr Tyr Cys
85 90 95
Ala Arg Asp Ser Arg Phe Ser Tyr Trp Gly Gln Gly Thr Leu Val Thr
100 105 110
Val Ser Ala

115

<210> 51
<211> 107
<212> PRT
<213> Artificial Sequence

<220>
<223> 2F8 LCVR

<400> 51
Asp Ile Val Met Thr Gln Ser His Lys Phe Met Ser Thr Ser Val Gly
1 5 10 15
Asp Arg Val Ser Ile Thr Cys Lys Ala Ser Gln Asp Val Arg Thr Ala
20 25 30
Val Ala Trp Tyr Gln Gln Lys Pro Gly Gln Ser Pro Lys Leu Leu Ile
35 40 45
Ser Ser Thr Ser Tyr Arg Tyr Thr Gly Val Pro Asp Arg Phe Thr Gly
50 55 60
Ser Gly Ser Gly Thr Asp Phe Thr Phe Ile Ile Ser Ser Val Gln Ala
65 70 75 80
Glu Asp Leu Ala Val Tyr Tyr Cys Gln Gln His Tyr Ser Thr Pro Leu
85 90 95
Thr Phe Gly Ala Gly Thr Lys Leu Glu Leu Lys
100 105

<210> 52
<211> 115
<212> PRT
<213> Artificial Sequence

<220>
<223> 1C9 HCVR

<400> 52
Gln Val Gln Leu Gln Gln Pro Gly Ser Val Leu Val Arg Pro Gly Ala
1 5 10 15
Ser Val Lys Leu Ser Cys Lys Ala Ser Gly Asp Thr Phe Thr Thr Tyr
20 25 30
Trp Met Asn Trp Val Lys Gln Arg Pro Gly Gln Gly Leu Glu Trp Ile
35 40 45
Gly Gly Ile Tyr Leu Asn Ser Gly Ser Ser Glu Tyr Asn Glu Lys Phe
50 55 60
Lys Gly Lys Ala Thr Leu Ser Val Asp Thr Ser Ser Ser Thr Ala Tyr
65 70 75 80
Met Asp Leu Ser Ser Leu Thr Ser Glu Asp Ser Ala Val Tyr Tyr Cys
85 90 95
Ala Arg Asp Ser Arg Phe Ser Tyr Trp Gly Gln Gly Thr Leu Val Thr
100 105 110
Val Ser Ala
115

<210> 53
<211> 107
<212> PRT
<213> Artificial Sequence

<220>
<223> 1C9 LCVR

<400> 53
Asp Ile Val Met Thr Gln Ser His Lys Phe Leu Ser Thr Ser Val Gly
1 5 10 15
Asp Arg Val Ser Ile Thr Cys Lys Ala Ser Gln Asp Val Ser Thr Ala
20 25 30
Val Ala Trp Tyr Gln Gln Lys Pro Gly Gln Ser Pro Glu Leu Leu Ile
35 40 45
Ser Ser Ala Ser Tyr Arg Tyr Thr Gly Val Pro Asp Arg Phe Thr Gly
50 55 60
Ser Gly Ser Gly Thr Asp Phe Thr Phe Thr Ile Ser Ser Val Gln Ala
65 70 75 80
Glu Asp Leu Ala Val Tyr Tyr Cys Gln Gln His Tyr Asn Thr Pro Leu
85 90 95
Thr Phe Gly Ala Gly Thr Gln Leu Glu Leu Lys
100 105

<210> 54
<211> 330
<212> PRT
<213> Artificial Sequence

<220>
<223> IgG1 C

<400> 54
Ala Ser Thr Lys Gly Pro Ser Val Phe Pro Leu Ala Pro Ser Ser Lys
1 5 10 15
Ser Thr Ser Gly Gly Thr Ala Ala Leu Gly Cys Leu Val Lys Asp Tyr
20 25 30
Phe Pro Glu Pro Val Thr Val Ser Trp Asn Ser Gly Ala Leu Thr Ser
35 40 45
Gly Val His Thr Phe Pro Ala Val Leu Gln Ser Ser Gly Leu Tyr Ser
50 55 60
Leu Ser Ser Val Val Thr Val Pro Ser Ser Ser Leu Gly Thr Gln Thr
65 70 75 80
Tyr Ile Cys Asn Val Asn His Lys Pro Ser Asn Thr Lys Val Asp Lys
85 90 95
Lys Val Glu Pro Lys Ser Cys Asp Lys Thr His Thr Cys Pro Pro Cys
100 105 110
Pro Ala Pro Glu Leu Leu Gly Gly Pro Ser Val Phe Leu Phe Pro Pro
115 120 125
Lys Pro Lys Asp Thr Leu Met Ile Ser Arg Thr Pro Glu Val Thr Cys
130 135 140
Val Val Val Asp Val Ser His Glu Asp Pro Glu Val Lys Phe Asn Trp
145 150 155 160
Tyr Val Asp Gly Val Glu Val His Asn Ala Lys Thr Lys Pro Arg Glu
165 170 175
Glu Gln Tyr Asn Ser Thr Tyr Arg Val Val Ser Val Leu Thr Val Leu
180 185 190
His Gln Asp Trp Leu Asn Gly Lys Glu Tyr Lys Cys Lys Val Ser Asn
195 200 205
Lys Ala Leu Pro Ala Pro Ile Glu Lys Thr Ile Ser Lys Ala Lys Gly
210 215 220
Gln Pro Arg Glu Pro Gln Val Tyr Thr Leu Pro Pro Ser Arg Glu Glu
225 230 235 240
Met Thr Lys Asn Gln Val Ser Leu Thr Cys Leu Val Lys Gly Phe Tyr
245 250 255
Pro Ser Asp Ile Ala Val Glu Trp Glu Ser Asn Gly Gln Pro Glu Asn
260 265 270
Asn Tyr Lys Thr Thr Pro Pro Val Leu Asp Ser Asp Gly Ser Phe Phe
275 280 285
Leu Tyr Ser Lys Leu Thr Val Asp Lys Ser Arg Trp Gln Gln Gly Asn

```
    290                 295                 300
Val Phe Ser Cys Ser Val Met His Glu Ala Leu His Asn His Tyr Thr
305                 310                 315                 320
Gln Lys Ser Leu Ser Leu Ser Pro Gly Lys
                325                 330

<210>  55
<211>  107
<212>  PRT
<213>  Artificial Sequence

<220>
<223>  Ig kappa C


<400>  55
Arg Thr Val Ala Ala Pro Ser Val Phe Ile Phe Pro Pro Ser Asp Glu
1               5                   10                  15
Gln Leu Lys Ser Gly Thr Ala Ser Val Val Cys Leu Leu Asn Asn Phe
            20                  25                  30
Tyr Pro Arg Glu Ala Lys Val Gln Trp Lys Val Asp Asn Ala Leu Gln
        35                  40                  45
Ser Gly Asn Ser Gln Glu Ser Val Thr Glu Gln Asp Ser Lys Asp Ser
    50                  55                  60
Thr Tyr Ser Leu Ser Ser Thr Leu Thr Leu Ser Lys Ala Asp Tyr Glu
65                  70                  75                  80
Lys His Lys Val Tyr Ala Cys Glu Val Thr His Gln Gly Leu Ser Ser
                85                  90                  95
Pro Val Thr Lys Ser Phe Asn Arg Gly Glu Cys
            100                 105
```